(19) Europäisches Patentamt
European Patent Office
Office européen des brevets

(11) Veröffentlichungsnummer: **0 023 569**
**B1**

(12) # EUROPÄISCHE PATENTSCHRIFT

(45) Veröffentlichungstag der Patentschrift: **22.06.83**

(21) Anmeldenummer: **80103670.8**

(22) Anmeldetag: **28.06.80**

(51) Int. Cl.3: **C 07 D295/14**, C 07 C103/84, C 07 D211/14, C 07 D217/04, C 07 D223/16, C 07 D265/28, C 07 D279/12, C 07 D491/113, A 61 K 31/165, A 61 K 31/395

(54) **Carbonsäure-Derivate, deren Herstellung und diese Verbindungen enthaltende Arzneimittel.**

(30) Priorität: **13.07.79 DE 2928352**
**07.12.79 DE 2949259**
**30.04.80 DE 3016651**
**30.04.80 DE 3016650**

(43) Veröffentlichungstag der Anmeldung: **11.02.81 Patentblatt 81/06**

(45) Bekanntmachung des Hinweises auf die Patenterteilung: **22.06.83 Patentblatt 83/25**

(84) Benannte Vertragsstaaten: **AT BE CH DE FR GB IT LI LU NL SE**

(56) Entgegenhaltungen:
**AT-B-347 922**
**DE-A-2 604 560**
**DE-A-2 655 144**
**FR-A-2 381 028**
**US-A-3-936 467**

(73) Patentinhaber: **Dr. Karl Thomae GmbH**
**Postfach 1755**
**D-7950 Biberach (Riss) (DE)**

(72) Erfinder: **Griss, Gerhart, Dr. Dipl.-Chem.**
**Schopperweg 1**
**D-7950 Biberach 1 (DE)**
Erfinder: **Sauter, Robert, Dr. Dipl.-Chem.**
**Albert-Schweizer Weg 9**
**D-7958 Laupheim (DE)**
Erfinder: **Grell, Wolfgang, Dr. Dipl.-Chem.**
**Amriswilstrasse 7**
**D-7950 Biberach 1 (DE)**
Erfinder: **Hurnaus, Rudolf, Dr. Dipl.-Chem.**
**Silcherstrasse 19**
**D-7950 Biberach 1 (DE)**
Erfinder: **Rupprecht, Eckhard, Dr. Dipl.-Biologe**
**Riedbachstrasse 15**
**D-7960 Aulendorf-Tannhausen (DE)**
Erfinder: **Kaubisch, Nikolaus, Dr. Dipl.-Chem.**
**Dinglingerstrasse 45**
**D-7950 Biberach 1 (DE)**
Erfinder: **Kähling, Joachim, Dr.**
**Haydnweg 8**
**D-7950 Biberach 1 (DE)**
Erfinder: **Eisele, Bernhard, Dr.**
**Beethovenstrasse 12**
**D-7950 Biberach 1 (DE)**
Erfinder: **Piper, Helmut, Dr. Dipl.-Chem.**
**Kapellenweg 5**
**D-7950 Biberach 1 (DE)**
Erfinder: **Noll, Klaus, Dr. Dipl.-Chem.**
**Im Schönblick 3**
**D-7951 Warthausen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 023 569 B1

# Carbonsäure-Derivate, deren Herstellung und diese Verbindungen enthaltende Arzneimittel

In der AT-B-347 922 werden u. a. bereits 4-[2-(2-Alkylamino-benzamido)-äthyl]-benzoesäuren und in der DE-A1-2 604 560 u. a. β-[4-[2-(2-N,N-Dialkylamino-benzamido)-äthyl]-phenyl]-propionsäuren beschrieben. So wird in Beispiel 5 der AT-B-347 922 die Verbindung 4-[2-(2-Äthylamino-benzamido)-äthyl]-benzoesäure (≙ Beispiel 5 der BE-B-837 311) und in Beispiel 12a der DE-A1-2 604 560 die Verbindung β-[4-[2-(2-N,N-Dimethylamino-benzamido)-äthyl]-phenyl]-propionsäure beschrieben.

Es wurde nun gefunden, daß die neuen Carbonsäurederivate der allgemeinen Formel I

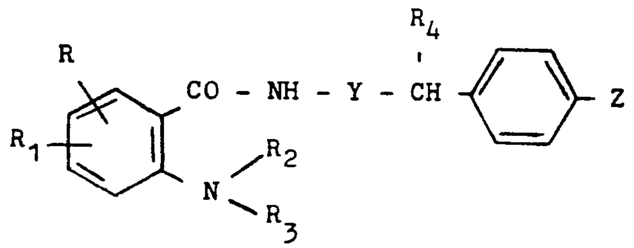

(I)

und deren physiologisch verträgliche Salze mit anorganischen oder organischen Säuren oder auch Basen, wenn Z eine Carboxygruppe darstellt oder enthält, überlegene blutzuckersenkende Eigenschaften aufweisen.

Gegenstand der Erfindung sind somit neue Carbonsäurederivate der allgemeinen Formel I, deren physiologisch verträgliche Salze mit anorganischen oder organischen Säuren oder auch Basen, wenn Z eine Carboxygruppe darstellt oder enthält, und Verfahren zu ihrer Herstellung sowie die neuen Verbindungen enthaltende Arzneimittel.

In der allgemeinen Formel I bedeutet

R ein Wasserstoff-, Chlor- oder Bromatom oder eine cyclische Alkyleniminogruppe mit 4 bis 7 Kohlenstoffatomen im Iminoring,

$R_1$ ein Wasserstoff-, Fluor-, Chlor- oder Bromatom, eine Alkyl- oder Alkoxygruppe mit jeweils 1 bis 6 Kohlenstoffatomen, eine durch eine Phenylgruppe substituierte Alkoxygruppe mit 1 bis 3 Kohlenstoffatomen, eine Hydroxy-, Nitro-, Amino-, Cyano- oder Carboxygruppe, eine Alkanoylamino-, Alkoxycarbonyl- oder Dialkylamidosulfonylgruppe, wobei der Alkylteil jeweils 1 bis 3 Kohlenstoffatome enthalten kann,

$R_2$ und $R_3$, die gleich oder verschieden sein können, Alkylgruppen mit 1 bis 7 Kohlenstoffatomen, Alkenylgruppen mit 3 bis 7 Kohlenstoffatomen, Cycloalkylgruppen mit 3 bis 7 Kohlenstoffatomen, durch eine Phenylgruppe substituierte Alkylgruppen mit 1 bis 3 Kohlenstoffatomen, Phenyl- oder Adamantylgruppen oder

$R_2$ und $R_3$ zusammen mit dem dazwischenliegenden Stickstoffatom eine unverzweigte Alkyleniminogruppe mit 3 bis 12 Kohlenstoffatomen im Iminoring, eine unverzweigte Alkyleniminogruppe mit 4 bis 6 Kohlenstoffatomen im Iminoring, der durch eine oder zwei Alkylgruppen mit jeweils 1 bis 4 Kohlenstoffatomen, eine Alkoxygruppe mit 1 bis 3 Kohlenstoffatomen, eine Hydroxy-, Phenyl-, Carboxy- oder Alkoxycarbonylgruppe mit insgesamt 2 bis 4 Kohlenstoffatomen substituiert ist und/oder in dem eine Methylengruppe durch eine Iminogruppe, welche durch eine Alkylgruppe mit 1 bis 3 Kohlenstoffatomen, eine Alkoxycarbonylgruppe mit insgesamt 2 bis 4 Kohlenstoffatomen, eine Phenyl-, Halogenphenyl-, Benzyl-, Pyridyl- oder Furoylgruppe substituiert sein kann, durch ein Sauerstoff- oder Schwefelatom, durch eine Carbonyl-, Sulfoxid- oder Sulfonylgruppe ersetzt ist, eine gesättigte oder teilweise ungesättigte Azabicycloalkylgruppe mit 7 bis 10 Kohlenstoffatomen, eine in 3- und 5-Stellung durch insgesamt 3 oder 4 Alkylgruppen mit jeweils 1 bis 3 Kohlenstoffatomen substituierte Piperidinogruppe, eine 1,4-Dioxa-8-aza-spiro-alkylgruppe mit 6 bis 9 Kohlenstoffatomen, eine Pyrrolyl- oder Tetrahydro-pyridinogruppe,

$R_4$ ein Wasserstoffatom oder eine Alkylgruppe mit 1 bis 3 Kohlenstoffatomen,

Y ein Sauerstoffatom, eine Iminogruppe oder eine gegebenenfalls durch eine oder zwei Alkylgruppen mit jeweils 1 bis 3 Kohlenstoffatomen substituierte Methylengruppe und

Z ein Wasserstoff- oder Halogenatom, eine Carboxy-, Cyano-, Formyl-, Hydroxymethyl-, Hydroxycarbonyläthylen-, Nitro-, Amino-, Allyloxycarbonyl-, Phenoxycarbonyl-, Benzyloxycarbonyl- oder Phenyläthoxycarbonylgruppe, eine Alkoxycarbonylgruppe mit insgesamt 2 bis 8 Kohlenstoffatomen, eine Cycloalkoxycarbonylgruppe mit insgesamt 4 bis 8 Kohlenstoffatomen, eine gegebenenfalls durch 2 oder 3 Alkoxygruppen, durch eine Carboxy-, Alkoxycarbonyl- oder Äthylendioxygruppe substituierte Methylgruppe, wobei die Alkoxygruppe jeweils 1 bis 3 Kohlenstoffatome enthalten kann, eine gegebenenfalls durch eine Carboxygruppe oder Alkoxycarbonylgruppe mit insgesamt 2 bis 4 Kohlenstoffatomen substituierte Acetylgruppe, eine durch eine oder zwei Alkoxycarbonylgruppen mit insgesamt je 2 bis 4 Kohlenstoffatomen oder durch zwei Carboxygruppen substituierte Äthyl- oder Äthylengruppe, eine gegebenenfalls durch eine Alkylgruppe mit 1 bis 7 Kohlenstoffatomen, eine Cycloalkylgruppe mit 3 bis 7 Kohlenstoffatomen und/oder Alkenylgruppe mit 3 bis 7 Kohlenstoffatomen mono- oder disubstituierte Aminocarbonylgruppe, eine Piperidinocarbonyl-, Morpholinocarbonyl-, Thiomorpholinocarbonyl- oder N-Alkyl-piperazinocarbonylgruppe, wobei die Alkylgruppe 1 bis 3 Kohlenstoffatome enthalten kann, oder auch eine durch eine Carboxygruppe substituierte Äthylgruppe, wenn die Reste $R_2$ und $R_3$ zusammen mit

dem dazwischenliegenden Stickstoffatom eine der eingangs erwähnten cyclischen Iminoreste bedeuten.

Für die bei der Definition der Reste R, $R_1$ bis $R_4$, Y und Z eingangs erwähnten Bedeutungen kommen also

für R die Bedeutung des Wasserstoff-, Chlor- oder Bromatoms, der Pyrrolidino-, Piperidino- oder Hexamethyleniminogruppe,

für $R_1$ die des Wasserstoff-, Fluor-, Chlor- oder Bromatoms, die der Methyl-, Äthyl-, Propyl-, Isopropyl-, Butyl-, Isobutyl-, Pentyl-, Isopentyl-, Hexyl-, Hydroxy-, Methoxy-, Athoxy-, Propoxy-, Isopropoxy-, Butoxy-, Isobutoxy-, tert. Butoxy-, Pentyloxy-, Isopentyloxy-, Neopentyloxy-, tert. Pentyloxy-, Hexyloxy-, Benzyloxy-, 1-Phenyläthoxy-, 2-Phenyläthoxy-, 1-Phenylpropoxy-, 2-Phenylpropoxy-, 3-Phenylpropoxy-, Nitro-, Cyano-, Amino-, Formylamino-, Acetamido-, Propionylamino-, Carboxy-, Methoxycarbonyl-, Äthoxycarbonyl-, Propoxycarbonyl-, Isopropoxycarbonyl-, Dimethylamidosulfonyl-, Diäthylamidosulfonyl-, Dipropylamidosulfonyl-, Äthyl-methylamidosulfonyl-, Methyl-propylamidosulfonyl- oder Äthyl-propylamidosulfonylgruppe,

für $R_2$ und $R_3$ zusammen mit dem dazwischenliegenden Stickstoffatom die der Dimethylamino-, Diäthylamino-, Dipropylamino-, Diisopropylamino-, Dibutylamino-, Diisobutylamino-, Dipentylamino-, Dihexylamino-, Diheptylamino-, N-Methyl-äthylamino-, N-Methyl-propylamino-, N-Isopropyl-propylamino-, N-Isobutyl-propylamino-, N-Methyl-isopropylamino-, N-Methyl-butylamino-, N-Äthyl-butylamino-, N-Äthylisopropylamino-, N-Äthyl-pentylamino-, N-Propyl-butylamino-, N-Propyl-heptylamino-, Dicyclohexylamino-, N-Methyl-cyclohexylamino-, N-Äthyl-cyclohexylamino-, N-Propyl-cyclohexylamino-, N-Isobutyl-cyclohexylamino-, Dibenzylamino-, N-Methyl-benzylamino-, N-Äthyl-benzylamino-, N-Propyl-benzylamino-, N-Isopropyl-benzylamino-, N-Butyl-benzylamino-, N-Heptyl-benzylamino-, N-Methyl-phenyläthylamino-, N-Methyl-phenylpropylamino-, N-Äthyl-phenyläthylamino-, N-Propyl-phenyläthylamino-, N-Butyl-phenylpropylamino-, Diallylamino-, Dibutenylamino-, Dipentenylamino-, Diheptenylamino-, N-Methyl-adamantylamino-, N-Äthyl-adamantylamino-, N-Propyl-adamantylamino-, Trimethylenimino-, Pyrrolidino-, Piperidino-, Hexamethylenimino-, Heptamethylenimino-, Octamethylenimino-, Nonamethylenimino-, Decamethylen,-imino-, Undecamethylenimino-, Dodecamethylenimino-, Methyl-pyrrolidino-, Dimethylpyrrolidino-, 1,2,3, 6-Tetrahydro-pyridino-, Methyl-piperidino-, Dimethyl-piperidino-, Trimethyl-piperidino-, Tetramethyl-piperidino-, Äthyl-piperidino-, Diäthyl-piperidino-, Methyläthyl-piperidino-, Propyl-piperidino-, Dipropyl-piperidino-, Methyl-propyl-piperidino-, Isopropyl-piperidino-, Äthyl-propyl-piperidino-, Butyl-piperidino-, Isobutyl-piperidino-, tert. Butyl-piperidino-, cis-3,5-Dimethyl-piperidino-, trans-3,5-Dimethyl-piperidino-, cis-3,5-Diäthyl-piperidino-, trans-3,5-Dipropyl-piperidino-, Hydroxy-pyrrolidino-, Hydroxy-piperidino-, Methoxypyrrolidino-, Methoxy-piperidino-, Äthoxy-piperidino-, Propoxy-piperidino-, Isopropoxy-piperidino-, Phenyl-piperidino-, Hydroxycarbonyl-pyrrolidino-, Hydroxycarbonyl-piperidino-, Methoxycarbonyl-piperidino-, Äthoxycarbonyl-piperidino-, Propoxycarbonyl-piperidino-, Isopropoxycarbonyl-piperidino-, 3,3,5,5-Tetramethyl-piperidino-, 3,3,5,5-Tetraäthyl-piperidino-, 3,3,5,5-Tetrapropyl-piperidino-, Pyrrolidon-1-yl-, Piperidon-1-yl-, Hexahydroazepinon-1-yl-, Morpholino-, Methyl-morpholino-, Dimethyl-morpholino-, Propyl-morpholino-, Thiomorpholino-, Methyl-thiomorpholino-, Dimethyl-thiomorpholino-, 1-Oxidothiomorpholino-, Dimethyl-1-oxidothiomorpholino-, 1,1-Dioxido-thiomorpholino-, Dimethyl-1,1-dioxidothiomorpholino-, Piperazino-, N-Methyl-piperazino-, N-Äthyl-piperazino-, N-Propyl-piperazino-, N-Isopropyl-piperazino-, N-Phenyl-piperazino-, N-Chlor-phenyl-piperazino-, N-Bromphenyl-piperazino-, N-Pyridyl-piperazino-, N-Methoxycarbonyl-piperazino-, N-Äthoxycarbonyl-piperazino-, N-Propoxycarbonyl-piperazino-, N-Furoyl-piperazino-, Tetrahydro-isochinolin-2-yl-, Octahydro-isochinolin-2-yl-, Decahydro-isochinolin-2-yl-, Tetrahydro-3-benzazepin-3-yl-, Decahydro-3-benzazepin-3-yl-, Octahydro-isoindol-2-yl-, 3-Aza-bicyclononan-3-yl-, 1,4-Dioxa-8-aza-spiro [4,5] decan-8-yl- oder 1,4-Dioxa-8-aza-spiro [4,6] undecan-8-yl-gruppe,

für $R_4$ die des Wasserstoffatoms, der Methyl-, Äthyl-, Propyl- oder Isopropylgruppe,

für Y die des Sauerstoffatoms, die der Imino-, Methylen-, Methyl-methylen-, Äthyl-methylen-, Propyl-methylen-, Dimethyl-methylen-, Diäthyl-methylen- oder Methyl-propyl-methylen-gruppe und

für Z die Bedeutung des Wasserstoff-, Chlor- oder Bromatoms, die der Nitro-, Amino-, Cyano-, Formyl-, Hydroxymethyl-, Carboxy-, Methoxycarbonyl-, Äthoxycarbonyl-, Propoxycarbonyl-, Isopropoxycarbonyl-, Butoxycarbonyl-, Isobutoxycarbonyl-, tert. Butoxycarbonyl-, Pentoxycarbonyl-, Hexoxycarbonyl-, Heptoxycarbonyl-, Allyloxycarbonyl-, Phenoxycarbonyl-, Benzyloxycarbonyl-, Phenyläthoxycarbonyl-, Cyclopropoxycarbonyl-, Cyclopentoxycarbonyl-, Cyclohexyloxycarbonyl-, Cycloheptoxycarbonyl-, Methyl-, Dimethoxymethyl-, Diäthoxymethyl-, Triäthoxymethyl-, Dipropoxymethyl-, Tripropoxymethyl-, Hydroxycarbonylmethyl-, Methoxycarbonylmethyl-, Äthoxycarbonylmethyl-, Propoxycarbonylmethyl-, 1,3-Dioxolan-2-yl-, Acetyl-, Hydroxycarbonylacetyl-, Methoxycarbonylacetyl-, Äthoxycarbonylacetyl-, Isopropoxycarbonylacetyl-, 2-Hydroxycarbonyläthylen-, 2-Methoxycarbonyläthylen-, 2-Äthoxycarbonyläthylen-, 2-Hydroxycarbonyläthyl-, 2-Methoxycarbonyl-äthyl-, 2-Äthoxycarbonyl-äthyl-, 2-Isopropoxycarbonyl-äthyl-, 2,2-Bis-hydroxycarbonyl-äthyl-, 2,2-Bis-äthoxycarbonyl-äthyl-, Aminocarbonyl-, Methylaminocarbonyl-, Äthylaminocarbonyl-, Isopropylaminocarbonyl-, Butylaminocarbonyl-, Pentylaminocarbonyl-, Hexylaminocarbonyl-, Heptylaminocarbonyl-, Allylaminocarbonyl-, Diallylaminocarbonyl-, Dimethylaminocarbonyl-, Diäthylaminocarbonyl-, Dipropylaminocarbonyl-, Dihexylaminocarbonyl-, N-Methyläthylaminocarbonyl-, Cyclopropylaminocarbonyl-, Cyclopentylaminocarbonyl-, Cyclohexylaminocarbonyl-, Cycloheptylaminocarbonyl-, Dicyclohexylaminocarbonyl-, N-Methylcyclohexylaminocarbonyl-, N-Äthylcyclohexylaminocarbonyl-, N-Propyl-cyclohexylaminocarbonyl-, N-Pentyl-cyclohexylaminocarbonyl-, Piperidinocarbonyl-, Morpholinocarbonyl-, Thiomorpholinocarbonyl-, N-Methyl-piperazinocarbonyl-, N-Äthyl-piperazinocarbonyl- oder N-Propyl-piperazinocarbonylgruppe in Betracht.

Bevorzugte Verbindungen der allgemeinen Formel I sind jedoch diejenigen, in denen

R ein Wasserstoffatom,

$R_1$ ein Wasserstoff-, Fluor-, Chlor- oder Bromatom, eine Alkoxygruppe mit 1 bis 6 Kohlenstoffatomen, eine Alkylgruppe mit 1 bis 4 Kohlenstoffatomen, eine Alkoxycarbonylgruppe mit insgesamt 2 bis 4 Kohlenstoffatomen, eine Hydroxy-, Cyano-, Carboxy-, Nitro-, Amino-, Acetamido-, Dimethylaminosulfonyl- oder Benzyloxygruppe,

$R_2$ eine Alkylgruppe mit 1 bis 6 Kohlenstoffatomen, eine Cyclohexyl-, Phenyl-, Benzyl-, Adamantyl- oder Allylgruppe,

$R_3$ eine Alkylgruppe mit 1 bis 6 Kohlenstoffatomen oder eine Allylgruppe oder

$R_2$ und $R_3$ zusammen mit dem dazwischenliegenden Stickstoffatom eine unverzweigte Alkyleniminogruppe mit 4 bis 12 Kohlenstoffatomen im Iminoring, eine durch eine Alkylgruppe mit 1 bis 4 Kohlenstoffatomen, eine Phenyl-, Hydroxy-, Methoxy-, Carboxy- oder Alkoxycarbonylgruppe mit insgesamt 2 bis 4 Kohlenstoffatomen substituierte Piperidinogruppe, eine in 3- und 5-Stellung je durch eine Alkylgruppe mit 1 bis 3 Kohlenstoffatomen disubstituierte Piperidinogruppe, eine in 3- und 5-Stellung je durch zwei Alkylgruppen mit jeweils 1 bis 3 Kohlenstoffatomen tetrasubstituierte Piperidinogruppe, eine gegebenenfalls durch eine oder zwei Methylgruppen substituierte Morpholino-, Thiomorpholino-, 1-Oxido-thiomorpholino- oder 1,1-Dioxido-thiomorpholino-gruppe, eine gegebenenfalls in 4-Stellung durch eine Methyl-, Benzyl-, Phenyl-, Chlor-phenyl-, Pyridyl-, Furoyl- oder Alkoxycarbonylgruppe mit insgesamt 2 bis 4 Kohlenstoffatomen substituierte Piperazinogruppe, eine Pyrrolyl-, Piperidon-(2)-yl-(1)-, 1,2,3,6-Tetrahydropyridino-, 1,4-Dioxa-8-aza-spiro [4,5] decan-8-yl-, 1,4-Dioxa-8-aza-spiro [4,6] undecan-8-yl-, Octahydro-isoindol-2-yl-, 1,2,3,4-Tetrahydro-isochinolin-2-yl-, 1,2,3,4,5,6,7,8-Octahydro-isochinolin-2-yl-, Decahydro-isochinolin-2-yl-, 1,2,4,5-Tetrahydro-3-benzazepin-3-yl-, Decahydro-3-benzazepin-3-yl- oder 3-Aza-bicyclo-nonan-3-yl-gruppe,

$R_4$ ein Wasserstoffatom oder eine Methylgruppe,

Y eine Methylen-, Methyl-methylen- oder Dimethylmethylengruppe, eine NH-Gruppe oder ein Sauerstoffatom und

Z eine Carboxy-, Cyano-, Formyl- oder Hydroxymethylgruppe, eine Alkoxycarbonylgruppe mit insgesamt 2 bis 7 Kohlenstoffatomen, eine Cyclohexyloxycarbonyl-, Benzyloxycarbonyl-, Diäthoxymethyl-, Hydroxycarbonylmethyl-, Bis-2,2-äthoxycarbonyl-äthyl-, 2-Hydroxy-carbonyl-äthylen- oder 2-Äthoxycarbonyl-äthylgruppe, eine gegebenenfalls durch eine Hydroxycarbonyl- oder Äthoxycarbonylgruppe substituierte Acetylgruppe oder auch eine 2-Hydroxycarbonyl-äthylgruppe, wenn die Reste $R_2$ und $R_3$ zusammen mit dem dazwischenliegenden Stickstoffatom eine der eingangs erwähnten cyclischen Iminoreste darstellen, bedeuten, und deren Salze, insbesondere jedoch diejenigen Verbindungen, in denen der Rest $R_1$ sich in Position 5 des Benzolringes befindet.

Besonders bevorzugte Verbindungen der allgemeinen Formel I sind jedoch diejenigen, in denen

R ein Wasserstoffatom,

$R_1$ in 5-Position ein Wasserstoff-, Fluor-, Chlor- oder Bromatom, eine Alkyl- oder Alkoxygruppe mit jeweils 1 bis 3 Kohlenstoffatomen, eine Carboxy-, Cyano- oder Nitrogruppe,

$R_2$ und $R_3$ zusammen mit dem dazwischenliegenden Stickstoffatom eine N,N-Dialkylamino- oder N-Alkyl-cyclohexylaminogruppe mit jeweils 1 bis 3 Kohlenstoffatomen im Alkylteil, eine Pyrrolidino-, Piperidino-, Hexamethylenimino-, Heptamethylenimino-, Octamethylenimino- oder Nonamethyleniminogruppe, eine durch eine Alkylgruppe mit 1 bis 4 Kohlenstoffatomen, durch eine Methoxy- oder Phenylgruppe substituierte Piperidinogruppe, eine in 3- und 5-Stellung jeweils durch eine oder zwei Methyl- oder Äthylgruppen substituierte Piperidinogruppe, eine gegebenenfalls in 2- und 6-Stellung je durch eine Methylgruppe substituierte Morpholino- oder Thiomorpholinogruppe, eine 1,4-Dioxa-8-aza-spiro [4,5] decan-8-yl-, 1,4-Dioxa-8-aza-spiro [4,6] undecan-yl-, Octahydro-isoindol-2-yl-, 1,2,3,4-Tetrahydro-isochinolin-2-yl-, 1,2,3,4,5,6,7,8-Octahydro-isochinolin-2-yl-, Decahydro-isochinolin-2-yl-, 1,2,4,5-Tetrahydro-3H-3-benzazepin-3-yl-, Decahydro-3H-3-benzazepin-3-yl-, 3-Aza-bicyclo [3,2,2] nonan-3-yl- oder N-Methyl-adamantyl-(1)-amino-Gruppe,

$R_4$ ein Wasserstoffatom oder eine Methylgruppe,

Y eine Methylen-, Methyl-methylen-, Dimethyl-methylen- oder NH-Gruppe oder ein Sauerstoffatom und,

Z eine Alkoxycarbonylgruppe mit insgesamt 2 bis 4 Kohlenstoffatomen, eine Carboxy-, Formyl- oder Hydroxymethylgruppe bedeuten, und deren physiologisch verträgliche Säureadditionssalze mit anorganischen oder organischen Säuren und auch Basen, wenn Z eine Carboxygruppe darstellt.

Erfindungsgemäß erhält man die neuen Verbindungen nach folgenden Verfahren :

a) Umsetzung einer Aminobenzoesäure der allgemeinen Formel II

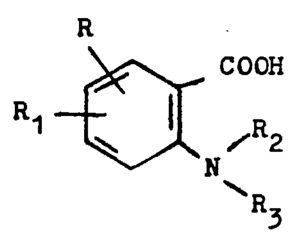

(II)

in der R, $R_1$, $R_2$ und $R_3$ wie eingangs definiert sind, oder deren gegebenenfalls im Reaktionsgemisch hergestelltes reaktionsfähiges Derivat mit einem Amin der allgemeinen Formel III

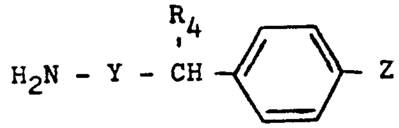

(III)

in der $R_4$, Y und Z wie eingangs definiert sind, oder mit einem gegebenenfalls im Reaktionsgemisch gebildeten N-aktivierten Amin der allgemeinen Formel III, wenn eine Aminobenzoesäure der allgemeinen Formel II eingesetzt wird und wenn Z in einem N-aktivierten Amin der allgemeinen Formel III keine Carboxy- oder Aminogruppe enthält.

Das Verfahren betrifft somit die Acylierung eines Amins der allgemeinen Formel III mit einer Aminobenzoesäure der allgemeinen Formel II in Gegenwart eines die Säure aktivierenden oder eines wasserentziehenden Mittels oder mit deren funktionellen Derivaten oder die Umsetzung einer Aminobenzoesäure der allgemeinen Formel II mit einem Amin der allgemeinen Formel III, in der Z keine Carboxy- oder Aminogruppe darstellt, in Gegenwart eines die Aminogruppe aktivierenden Mittels oder mit dessen reaktionsfähigen Derivaten.

Als gegebenenfalls im Reaktionsgemisch hergestellte funktionelle Derivate einer Aminobenzoesäure der allgemeinen Formel II kommen beispielsweise deren Alkyl-, Aryl- oder Aralkylester oder -thioester wie der Methyl-, Äthyl-, Phenyl- oder Benzylester, dessen Kupfer-Komplexe, deren Imidazolide, deren Säurehalogenide wie das Säurechlorid oder -bromid, deren Anhydride, deren gemischte Anhydride mit aliphatischen oder aromatischen Carbon-, Sulfen-, Sulfin- oder Sulfonsäuren oder Kohlensäureestern, z. B. der Essigsäure, Propionsäure, p-Toluolsulfonsäure oder der O-Äthylkohlensäure, deren O-Triphenylphosphonium-Komplexe, deren N-Acyloxyimide, deren Azide oder Nitrile oder die entsprechenden Amino-thiobenzoesäure-Derivate, und als gegebenenfalls im Reaktionsgemisch hergestellte reaktionsfähige Derivate eines Amins der allgemeinen Formel III, wenn Z keine Carboxy- oder Aminogruppe enthält, deren Phosphazoderivate in Betracht.

Als säureaktivierende und/oder wasserentziehende Mittel kommen beispielsweise ein Chlorameisensäureester wie Chlorameisensäureäthylester, Thionylchlorid, Phosphortrichlorid, Phosphorpentoxid, N,N'-Dicyclohexylcarbodiimid, N,N'-Carbonyldiimidazol, N,N'-Thionyldiimidazol, Bortrifluoridätherat oder Triphenylphosphin/Tetrachlorkohlenstoff in Betracht.

Die Umsetzung wird zweckmäßigerweise in einem Lösungsmittel wie Methylenchlorid, Chloroform, Tetrachlorkohlenstoff, Äther, Tetrahydrofuran, Dioxan, Benzol, Toluol oder Dimethylformamid, gegebenenfalls in Gegenwart einer anorganischen Base wie Natriumcarbonat oder einer tertiären organischen Base wie Triäthylamin oder Pyridin, welche gleichzeitig als Lösungsmittel dienen kann, und gegebenenfalls in Gegenwart eines säureaktivierenden Mittels bei Temperaturen zwischen − 25 und 250 °C, vorzugsweise jedoch bei Temperaturen zwischen − 10 °C und der Siedetemperatur des verwendeten Lösungsmittels, durchgeführt. Hierbei braucht ein gegebenenfalls im Reaktionsgemisch entstandenes funktionelles Derivat einer Verbindung der allgemeinen Formel II oder III nicht isoliert zu werden, ferner kann die Umsetzung auch ohne Lösungsmittel durchgeführt werden. Desweiteren kann während der Umsetzung entstehendes Wasser durch azeotrope Destillation, z. B. durch Erhitzen mit Toluol am Wasserabscheider, oder durch Zugabe eines Trockenmittels wie Magnesiumsulfat oder eines Molekularsiebes abgetrennt werden.

b) Zur Herstellung von Verbindungen der allgemeinen Formel I, in der $R_1$ mit Ausnahme der Hydroxy- und Aminogruppe wie eingangs definiert ist und Y eine gegebenenfalls durch eine oder zwei Alkylgruppen mit jeweils 1 bis 3 Kohlenstoffatomen substituierte Methylengruppe darstellt:

Umsetzung einer Verbindung der allgemeinen Formel IV

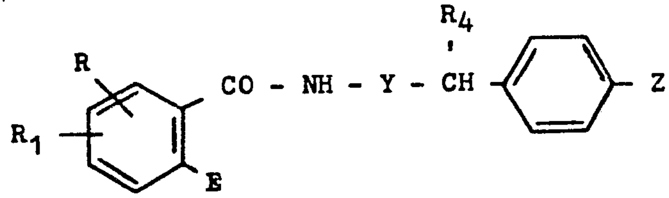

(IV)

in der

R, $R_4$ und Z wie eingangs und

$R_1$ und Y wie oben definiert sind und

E ein Halogenatom darstellt, mit einem Amin der allgemeinen Formel V

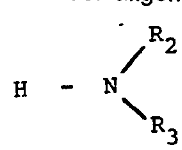

(V)

in der $R_2$ und $R_3$ wie eingangs definiert sind.

Unter den bei der Definition des austauschbaren Restes E verwendeten Begriff « ein Halogenatom » ist insbesondere ein Chlor- oder Bromatom oder auch ein Fluoratom, wenn $R_1$ die Nitrogruppe darstellt, zu verstehen.

Die Umsetzung wird zweckmäßigerweise in einem Lösungsmittel wie Wasser, Wasser/Methanol, Wasser/Äthanol, Wasser/Isopropanol, Dimethylformamid oder in einem Überschuß des eingesetzten Amins der allgemeinen Formel V gegebenenfalls in Gegenwart einer anorganischen oder tertiären organischen Base, gegebenenfalls in Gegenwart eines Reaktionsbeschleunigers wie Kupfer und gegebenenfalls in einem Druckgefäß bei Temperaturen zwischen 20 und 150 °C, vorzugsweise jedoch bei der Siedetemperatur des Reaktionsgemisches, z. B. bei 100 °C, durchgeführt. Die Umsetzung kann jedoch auch ohne Lösungsmittel durchgeführt werden.

c) Zur Herstellung von Verbindungen der allgemeinen Formel I, in der Z eine Carboxygruppe und Y keine NH-Gruppe darstellt :

Oxidation einer Verbindung der allgemeinen Formel VI

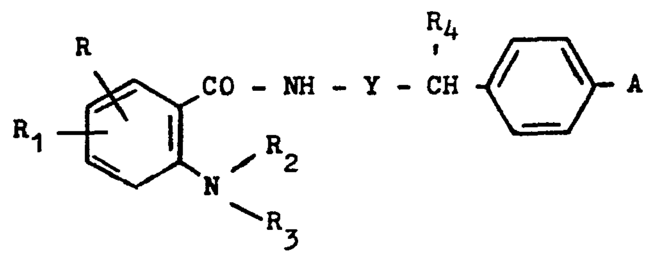

(VI)

in der

R und $R_1$ bis $R_4$ wie eingangs definiert sind,

Y mit Ausnahme der NH-Gruppe wie eingangs definiert ist und

A eine durch Oxidation in eine Carboxygruppe überführbare Gruppe bedeutet.

Als eine derartige oxidierbare Gruppe kommt beispielsweise die Formylgruppe und deren Acetale, die Hydroxymethylgruppe und deren Äther, eine unsubstituierte oder substituierte Acylgruppe wie die Acetyl-, Chloracetyl-, Propionyl-, Malonsäure-(1)-yl- oder Malonester-(1)-yl-gruppe in Betracht.

Die Umsetzung wird mit einem Oxidationsmittel in einem geeigneten Lösungsmittel wie Wasser, Eisessig, Pyridin oder Tetrachlorkohlenstoff bei Temperaturen zwischen 0 und 100 °C, zweckmäßigerweise jedoch bei Temperaturen zwischen 20 und 50 °C, durchgeführt. Die Umsetzung wird jedoch vorzugsweise mit Silberoxid/Natronlauge, Mangandioxid/Aceton oder Methylenchlorid, Wasserstoffperoxid/Natronlauge, Brom oder Chlor/Natron- oder Kalilauge oder Chromtrioxid/Pyridin durchgeführt.

d) Zur Herstellung von Verbindungen der allgemeinen Formel I, in der Z eine Carboxygruppe darstellt :

Hydrolyse einer Verbindung der allgemeinen Formel VII

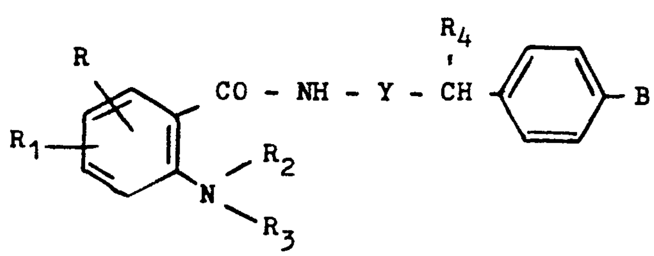

(VII)

in der

R, $R_1$ bis $R_4$ und Y wie eingangs definiert sind und

B eine durch Hydrolyse in eine Carboxygruppe überführbare Gruppe darstellt.

Als derartige hydrolysierbare Gruppen kommen beispielsweise die Nitrilgruppe, funktionelle Derivate der Carboxygruppe wie deren unsubstituierte oder substituierte Amide, Ester, Thioester, Orthoester, Iminoäther, Amidine oder Anhydride, eine Malonester-(1)-yl-gruppe, die Tetrazolylgruppe, eine gegebenenfalls substituierte 1,3-Oxazol-(2)-yl- oder Dihydro-1,3-oxazol-(2)-yl-gruppe in Betracht.

Die Hydrolyse wird zweckmäßigerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Äthanol, Wasser/Äthanol, Wasser/Isopropanol oder Wasser/Dioxan bei Temperaturen zwischen − 10 und panol oder Wasser/Dioxan bei Temperaturen zwischen − 10 und 120 °C, z. B. bei Temperaturen zwischen Raumtemperatur und der Siedetemperatur des Reaktionsgemisches, durchgeführt.

Bedeutet in einer Verbindung der allgemeinen Formel VII B die Cyangruppe, so wird die Umsetzung zweckmäßigerweise in Gegenwart von Äthanol/Chlorwasserstoff durchgeführt, hierbei bildet sich im Reaktionsgemisch der entsprechende Imino- und Orthoester bzw. nach Wasserzugabe der entsprechende Ester, welcher nach Zugabe von Wasser hydrolysiert wird.

e) Zur Herstellung von Verbindungen der allgemeinen Formel I, in der $R_2$ eine Alkylgruppe mit 1 bis 7 Kohlenstoffatomen, eine Cycloalkylgruppe mit 3 bis 7 Kohlenstoffatomen, eine durch eine Phenylgruppe substituierte Alkylgruppe mit 1 bis 3 Kohlenstoffatomen oder eine Alkenylgruppe mit 3 bis 7 Kohlenstoffatomen, $R_3$ eine Alkylgruppe mit 1 bis 7 Kohlenstoffatomen, eine Cycloalkylgruppe mit 3 bis 7 Kohlenstoffatomen, eine Alkenylgruppe mit 3 bis 7 Kohlenstoffatomen, eine durch eine Phenylgruppe substituierte Alkylgruppe mit 1 bis 3 Kohlenstoffatomen oder eine Adamantylgruppe oder $R_2$ und $R_3$ zusammen mit dem dazwischenliegenden Stickstoffatom einen 5- bis 7-gliedrigen Alkyleniminoring, eine durch eine Alkylgruppe mit 1 bis 4 Kohlenstoffatomen substituierte Piperidinogruppe oder eine in 3- und 5-Stellung jeweils durch eine Alkylgruppe mit 1 bis 3 Kohlenstoffatomen substituierte Piperidinogruppe und Y eine gegebenenfalls durch eine oder zwei Alkylgruppen mit jeweils 1 bis 3 Kohlenstoffatomen substituierte Methylengruppe bedeuten :

Umsetzung einer gegebenenfalls im Reaktionsgemisch gebildeten Verbindung der allgemeinen Formel VIII

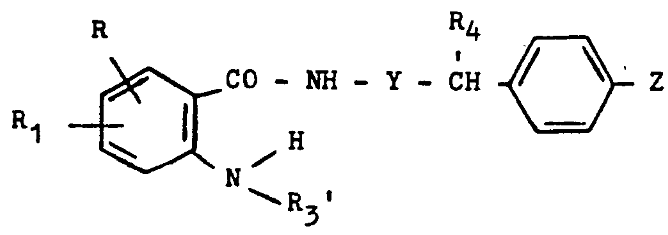

(VIII)

in der

R, $R_1$, $R_4$ und Z wie eingangs definiert sind,

$R_3'$ ein Wasserstoffatom darstellt oder die für $R_3$ vorstehend erwähnten Bedeutungen besitzt, und

Y die oben erwähnten Bedeutungen besitzt, mit einer Verbindung der allgemeinen Formel IX

$$R_2'—G \qquad (IX)$$

in der

$R_2'$ die für $R_2$ eingangs erwähnten Bedeutungen besitzt oder zusammen mit dem Rest $R_3'$ der Formel IX eine geradkettige Alkylengruppe mit 4 bis 6 Kohlenstoffatomen, eine durch eine Alkylgruppe mit 1 bis 4 Kohlenstoffatomen substituierte n-Pentylengruppe oder eine in 2- und 4-Stellung jeweils durch eine Alkylgruppe mit 1 bis 3 Kohlenstoffatomen substituierte n-Pentylengruppe darstellt und

G eine nukleophile Austrittsgruppe wie ein Halogenatom oder eine Sulfonyloxygruppe, z. B. ein Chlor-, Brom- oder Jodatom, eine Methansulfonyloxy- oder p-Toluolsulfonyloxygruppe, bedeutet.

Als ein Alkylierungsmittel der Formel IX kommen somit beispielsweise die entsprechenden Halogenide oder Sulfate wie Methyljodid, Äthyljodid, Propylbromid, Benzylchlorid, Benzylbromid, Dimethylsulfat oder Diäthylsulfat in Betracht.

Die Umsetzung wird zweckmäßigerweise in einem Lösungsmittel wie Aceton, Tetrahydrofuran, Dimethylformamid, Dimethylsulfoxid oder Hexamethylphosphorsäuretriamid, gegebenenfalls in Gegenwart einer anorganischen Base wie Natriumcarbonat, Kaliumcarbonat oder Kalium-tert. butylat oder einer tertiären organischen Base wie Pyridin, bei Temperaturen zwischen 0 und 150 °C, vorzugsweise jedoch bei Temperaturen zwischen 20 und 75 °C, durchgeführt. Setzt man eine Carbonsäure der allgemeinen Formel VIII ein, so kann diese je nach den verwendeten Reaktionsbedingungen, z. B. bei Temperaturen oberhalb Raumtemperatur und in Gegenwart eines Alkoholats als Base, gleichzeitig in den entsprechenden Ester übergeführt werden.

Die Methylierung kann auch in der Weise durchgeführt werden, daß eine Verbindung der allgemeinen Formel VIII mit Formalin in Gegenwart eines Reduktionsmittels, z. B. Ameisensäure oder Wasserstoff in Gegenwart eines Hydrierungskatalysators, z. B. Palladium oder Platin, gegebenenfalls in einem Lösungsmittel wie Ameisensäure oder Eisessig bei Temperaturen bis zur Siedetemperatur des Reaktionsgemisches umgesetzt wird.

Eine Verbindung der allgemeinen Formel VIII kann auch im Reaktionsgemisch durch Umsetzung eines entsprechend substituierten Isatosäureanhydrids mit einem entsprechenden Amin der allgemeinen Formel III hergestellt werden.

f) Zur Herstellung von Verbindungen der allgemeinen Formel I, in der Y die NH-Gruppe oder ein Sauerstoffatom darstellt :

Umsetzung einer Verbindung der allgemeinen Formel X

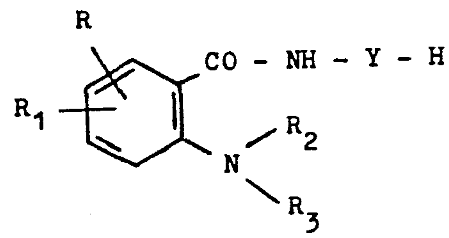

(X)

in der

R und $R_1$ bis $R_3$ wie eingangs definiert sind und

Y die oben erwähnten Bedeutungen besitzt, oder deren Alkalisalz mit einem Phenylderivat der allgemeinen Formel XI

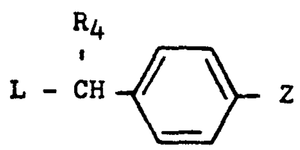

(XI)

in der

$R_4$ und Z wie eingangs definiert sind und

L eine nukleophile Austrittsgruppe wie ein Halogenatom oder eine Sulfonyloxygruppe, z. B. ein Chlor-, Brom- oder Jodatom, eine Methylsulfonyloxy-, p-Toluolsulfonyloxy- oder Methoxysulfonyloxygruppe, bedeutet.

Die Umsetzung wird zweckmäßigerweise in einem Lösungsmittel wie Wasser/Äthanol, Wasser/Isopropanol, Tetrahydrofuran, Dioxan, Aceton, Dimethylformamid, Dimethylsulfoxid oder Hexamethylphosphorsäuretriamid vorzugsweise in Gegenwart einer Base wie Natriumcarbonat, Natriumhydroxid, Kaliumhydroxid oder Kalium-tert. butylat bei Temperaturen zwischen 0 und 150 °C, vorzugsweise jedoch bei der Siedetemperatur des Reaktionsgemisches, z. B. bei Temperaturen zwischen 50 und 100 °C, durchgeführt. Setzt man hierbei einen Ester ein, so kann dieser je nach den verwendeten Reaktionsbedingungen, z. B. bei erhöhten Temperaturen und in Gegenwart eines Überschusses der eingesetzten Base, gleichzeitig in die entsprechende Carbonsäure übergeführt werden.

g) Zur Herstellung von Verbindungen der allgemeinen Formel I, in der

$R_1$ mit Ausnahme der Hydroxy-, Carboxy-, Amino- und Alkanoylaminogruppe wie eingangs definiert ist und

Y eine durch eine oder zwei Alkylgruppen mit jeweils 1 bis 3 Kohlenstoffatomen substituierte Methylengruppe darstellt :

Umsetzung eines Amids der allgemeinen Formel XII

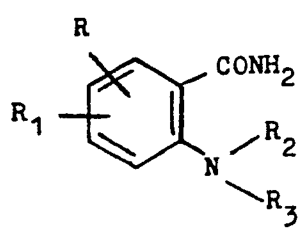

(XII)

in der

R, $R_2$ und $R_3$ wie eingangs definiert sind und

$R_1$ die oben erwähnten Bedeutungen besitzt, oder dessen Alkalisalz mit einer Verbindung der allgemeinen Formel XIII

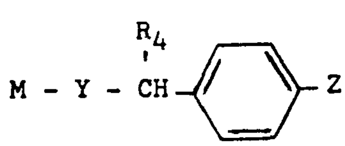

(XIII)

in der

$R_4$ und Z wie eingangs definiert sind,

Y die oben erwähnten Bedeutungen besitzt und

M eine nukleophile Austrittsgruppe wie ein Halogenatom oder eine Sulfonyloxygruppe darstellt.

Die Umsetzung wird zweckmäßigerweise in einem Lösungsmittel wie Tetrahydrofuran, Dioxan, Toluol, Dimethylformamid, Dimethylsulfoxid oder Hexamethylenphosphorsäuretriamid gegebenenfalls in Gegenwart einer Base wie Natriumhydrid oder Kalium-tert. butylat bei Temperaturen zwischen 20 und 180 °C, vorzugsweise jedoch bei Temperaturen zwischen 50 und 150 °C, durchgeführt.

h) Zur Herstellung von Verbindungen der allgemeinen Formel I, in der

$R_1$ ein Wasserstoff-, Fluor-, Chlor-, oder Bromatom, eine Alkyl- oder Alkoxygruppe mit jeweils 1 bis 6 Kohlenstoffatomen, eine durch eine Phenylgruppe substituierte Alkoxygruppe mit 1 bis 3 Kohlenstoffatomen, eine Hydroxy-, Nitro-, Carboxy- oder Alkanoylaminogruppe,

Y eine gegebenenfalls durch eine oder zwei Alkylgruppen mit jeweils 1 bis 3 Kohlenstoffatomen substituierte Methylengruppe und

Z eine Carboxygruppe darstellt :

Acylierung einer Verbindung der allgemeinen Formel XIV

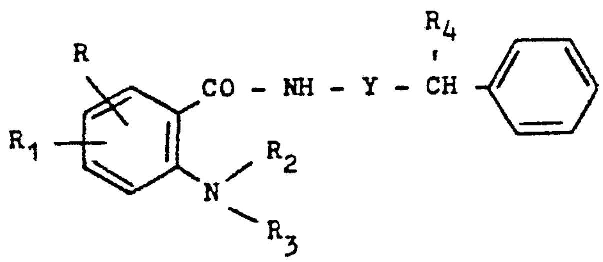

(XIV)

in der

R und $R_2$ bis $R_4$ wie eingangs definiert sind,

$R_1$ und Y die oben erwähnten Bedeutungen besitzen, mit einem Oxalylhalogenid oder Phosgen in Gegenwart einer Lewis-Säure.

Die Friedel-Crafts-Acylierung wird zweckmäßigerweise in einem Lösungsmittel wie Nitrobenzol oder Schwefelkohlenstoff in Gegenwart einer Lewis-Säure wie Aluminiumchlorid bei Temperaturen zwischen 0 und 80 °C, vorzugsweise jedoch bei Temperaturen zwischen 20 und 60 °C, durchgeführt.

i) Zur Herstellung von Verbindungen der allgemeinen Formel I, in der $R_1$ ein Wasserstoff-, Fluor-, Chlor- oder Bromatom, eine Alkyl- oder Alkoxygruppe mit jeweils 1 bis 6 Kohlenstoffatomen, eine durch eine Phenylgruppe substituierte Alkoxygruppe mit 1 bis 3 Kohlenstoffatomen, eine Nitro-, Carboxy-, Alkanoylamino- oder Alkoxycarbonylgruppe mit jeweils 1 bis 3 Kohlenstoffatomen im Alkylteil,

Y eine gegebenenfalls durch eine oder zwei Alkylgruppen mit jeweils 1 bis 3 Kohlenstoffatomen substituierte Methylengruppe und

Z die Carboxygruppe bedeutet :

Umsetzung einer Verbindung der allgemeinen Formel XV

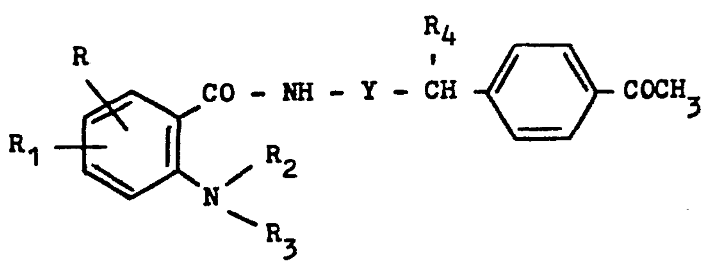

(XV)

in der

R und $R_2$ bis $R_4$ wie eingangs definiert sind,

$R_1$ und Y die oben erwähnten Bedeutungen besitzen, mit einem gegebenenfalls im Reaktionsgemisch hergestellten Hypohalogenit.

Die Umsetzung wird zweckmäßigerweise in einem Lösungsmittel wie Wasser/Tetrahydrofuran oder Wasser/Dioxan bei Temperaturen zwischen 0 und 80 °C, vorzugsweise jedoch bei Temperaturen zwischen 25 und 50 °C, durchgeführt.

Erhält man erfindungsgemäß ein Carbonsäureamid der allgemeinen Formel I, in der Z eine Carboxygruppe darstellt, so kann dieses gewünschtenfalls mittels Veresterung bzw. mittels Amidierung in eine entsprechende Verbindung der allgemeinen Formel I, in der Z eine veresterte Carboxygruppe oder eine gegebenenfalls durch eine Alkylgruppe mit 1 bis 7 Kohlenstoffatomen, eine Cycloalkylgruppe mit 3 bis 7 Kohlenstoffatomen und/oder Alkenylgruppe mit 3 bis 7 Kohlenstoffatomen mono- oder disubstituierte Aminocarbonylgruppe, eine Piperidinocarbonyl-, Morpholinocarbonyl-, Thiomorpholinocarbonyl- oder N-Alkyl-piperazinocarbonylgruppe darstellt, übergeführt werden, und/oder

ein Carbonsäureamid der allgemeinen Formel I, in der $R_1$ und/oder Z eine Nitrogruppe darstellen, so kann dieses mittels Reduktion in eine entsprechende Verbindung der allgemeinen Formel I, in der $R_1$ und/oder Z eine Aminogruppe darstellen, übergeführt werden, und/oder

ein Carbonsäureamid der allgemeinen Formel I, in der $R_1$ und/oder Z eine Aminogruppe darstellen, so kann dieses über ein entsprechendes Diazoniumsalz in eine entsprechende Verbindung der allgemeinen Formel I, in der $R_1$ die Hydroxy- oder Cyangruppe, ein Fluor-, Chlor- oder Bromatom und/oder Z ein Chlor- oder Bromatom oder die Nitrilgruppe darstellen, übergeführt werden, wobei eine so gegebenenfalls erhaltene Verbindung der allgemeinen Formel I, in der $R_1$ die Hydroxygruppe darstellt, anschliessend mittels Alkylierung in eine entsprechende Verbindung der allgemeinen Formel I übergeführt werden kann, in der $R_1$ eine Alkoxygruppe mit 1 bis 6 Kohlenstoffatomen oder eine durch eine Phenylgruppe substituierte Alkoxygruppe mit 1 bis 3 Kohlenstoffatomen darstellt, und/oder

ein Carbonsäureamid der allgemeinen Formel I, in der $R_1$ die Aminogruppe darstellt, so kann diese mittels Acylierung in eine entsprechende Verbindung der allgemeinen Formel I, in der $R_1$ eine Alkanoylaminogruppe mit 1 bis 3 Kohlenstoffatomen im Alkanoylteil darstellt, übergeführt werden, und/oder

ein Carbonsäureamid der allgemeinen Formel I, in der $R_1$ eine Alkoxycarbonylgruppe und/oder $R_2$ und $R_3$ zusammen mit dem dazwischenliegenden Stickstoffatom eine durch eine Alkoxycarbonylgruppe substituierte Iminogruppe mit 4 bis 6 Kohlenstoffatomen darstellt, wobei der Alkoxyteil jeweils 1 bis 3 Kohlenstoffatome enthalten kann, mittels Hydrolyse in eine entsprechende Verbindung der allgemeinen Formel I, in der $R_1$ die Carboxygruppe und/oder $R_2$ und $R_3$ zusammen mit dem dazwischenliegenden Stickstoffatom eine durch eine Carboxygruppe substituierte Iminogruppe mit 4 bis 6 Kohlenstoffatomen im Iminoring darstellt, übergeführt werden, und/oder

ein Carbonsäureamid der allgemeinen Formel I, in der $R_2$ und $R_3$ zusammen mit dem dazwischenliegenden Stickstoffatom eine N-Benzylpiperazinogruppe darstellt, mittels Entbenzylierung in eine entsprechende Verbindung der allgemeinen Formel I, in der $R_2$ und $R_3$ zusammen mit dem dazwischenliegenden Stickstoffatom eine Piperazinogruppe darstellt, übergeführt werden, und/oder

ein Carbonsäureamid der allgemeinen Formel I, in der $R_1$ ein Chlor- oder Bromatom darstellt, mittels Enthalogenierung in eine entsprechende Verbindung der allgemeinen Formel I, in der $R_1$ ein Wasserstoffatom darstellt, übergeführt werden, und/oder

ein Carbonsäureamid der allgemeinen Formel I, in der Z eine gegebenenfalls veresterte Carboxygruppe darstellt, so kann diese mittels Reduktion mit einem Metallhydrid in eine entsprechende Verbindung der allgemeinen Formel I, in der Z die Hydroxymethylgruppe darstellt, übergeführt werden, und/oder

ein Carbonsäureamid der allgemeinen Formel I, in der Z die Hydroxymethylgruppe darstellt, so kann diese mittels Oxidation in eine entsprechende Verbindung der allgemeinen Formel I, in der Z die Formylgruppe darstellt, übergeführt werden, und/oder

ein Carbonsäureamid der allgemeinen Formel I, in der Z die Hydroxymethylgruppe darstellt, so kann diese mittels Halogenierung und anschließende Umsetzung mit einem Malonsäureester in eine entsprechende Verbindung der allgemeinen Formel I, in der Z eine durch zwei Alkoxycarbonylgruppen substituierte Äthylgruppe darstellt, übergeführt werden, und/oder

ein Carbonsäureamid der allgemeinen Formel I, in der Z die Formylgruppe darstellt, so kann dieses mittels Acetalisierung in eine entsprechende Verbindung der allgemeinen Formel I, in der Z eine Dialkoxymethylgruppe darstellt, übergeführt werden, und/oder

ein Carbonsäureamid der allgemeinen Formel I, in der Z die Formylgruppe darstellt, so kann dieses mittels Kondensation und gegebenenfalls anschließende Hydrolyse in eine entsprechende Verbindung der allgemeinen Formel I, in der Z eine durch eine Hydroxycarbonyl- oder Alkoxycarbonylgruppe substituierte Äthylengruppe darstellt, übergeführt werden, und/oder

ein Carbonsäureamid der allgemeinen Formel I, in der Z ein Wasserstoffatom darstellt, so kann dieses mittels Friedel-Crafts-Acylierung in eine entsprechende Verbindung der allgemeinen Formel I, in der Z eine gegebenenfalls durch eine Alkoxycarbonylgruppe substituierte Acetylgruppe darstellt, übergeführt werden, und/oder

ein Carbonsäureamid der allgemeinen Formel I, in der Z eine Nitrilgruppe darstellt, so kann dieses mittels Alkoholyse über einen entsprechenden Iminoester in eine entsprechende Verbindung der allgemeinen Formel I, in der Z eine Trialkoxymethylgruppe darstellt, übergeführt werden, und/oder

ein Carbonsäureamid der allgemeinen Formel I, in der Z eine Trialkoxymethylgruppe darstellt, mittels Hydrolyse in eine entsprechende Verbindung der allgemeinen Formel I, in der Z eine Alkoxycarbonylgruppe darstellt, übergeführt werden, und/oder

ein Carbonsäureamid der allgemeinen Formel I, in der Z die Acetylgruppe darstellt, so kann dieses durch Erhitzen mit einem Amin und Schwefel und anschließend mit einer anorganischen Base in eine entsprechende Verbindung der allgemeinen Formel I, in der Z die 2-Hydroxycarbonylmethylgruppe darstellt, übergeführt werden, und/oder

ein Carbonsäureamid der allgemeinen Formel I, in der Z die Carboxygruppe darstellt, so kann dieses mittels Überführung in ein Sulfonsäurehydrazid und anschließende Disproportionierung in eine entsprechende Verbindung der allgemeinen Formel I, in der Z die Formylgruppe darstellt, übergeführt werden, und/oder

ein Carbonsäureamid der allgemeinen Formel I, in der $R_1$ ein Wasserstoff-, Fluor-, Chlor-, oder Bromatom, eine Alkyl- oder Alkoxygruppe mit jeweils 1 bis 6 Kohlenstoffatomen oder eine durch eine Phenylgruppe substituierte Alkoxygruppe mit 1 bis 3 Kohlenstoffatomen, Y eine gegebenenfalls durch eine oder zwei Alkylgruppen mit jeweils 1 bis 3 Kohlenstoffatomen substituierte Methylengruppe und Z ein Chlor- oder Bromatom bedeuten, so kann dieses nach Überführung in eine entsprechende metallorganische Verbindung mit Kohlendioxid in eine entsprechende Verbindung der allgemeinen Formel I, in der Z die Carboxygruppe darstellt, übergeführt werden.

Die nachträgliche Veresterung bzw. Amidierung wird zweckmäßigerweise in einem geeigneten Lösungsmittel, z. B. in einem entsprechenden Alkohol oder Amin, Pyridin, Toluol oder Dioxan, in Gegenwart eines säureaktivierenden und/oder wasserentziehenden Mittels wie Thionylchlorid, Chlorameisensäureäthylester, N,N'-Dicyclohexylcarbodiimid oder Carbonyldiimidazol oder durch Umesterung, z. B. mit einem entsprechenden Kohlensäurediester, bei Temperaturen zwischen 0 und 50 °C, vorzugsweise jedoch bei Raumtemperatur, durchgeführt.

Die nachträgliche Reduktion der Nitroverbindung wird vorzugsweise in einem Lösungsmittel wie Wasser, Wasser/Äthanol, Methanol, Eisessig, Essigsäureäthylester oder Dimethylformamid zweckmäßigerweise mit Wasserstoff in Gegenwart eines Hydrierungskatalysators wie Raney-Nickel, Platin oder Palladium/Kohle, mit Metallen wie Eisen, Zinn oder Zink in Gegenwart einer Säure, mit Salzen wie Eisen(II)sulfat, Zinn(II)-chlorid oder Natriumdithionit, oder mit Hydrazin in Gegenwart von Raney-Nickel bei Temperaturen zwischen 0 und 50 °C, vorzugsweise jedoch bei Raumtemperatur, durchgeführt.

Die nachträgliche Umsetzung eines Diazoniumsalzes, z. B. des Fluoroborats, des Hydrosulfats in Schwefelsäure oder des Hydrochlorids in Gegenwart von Kupfer oder eines entsprechenden Kupfer(I)-Salzes wie Kupfer(I)-chlorid/Salzsäure, Kupfer(I)-bromid/Bromwasserstoffsäure oder Trinatrium-kupfer(I)-tetracyanid bei pH 7, wird bei leicht erhöhten Temperaturen, z. B. bei Temperaturen zwischen 15 und 100 °C, durchgeführt. Das hierzu erforderliche Diazoniumsalz wird zweckmäßigerweise in einem geeigneten Lösungsmittel, z. B. in Wasser/Salzsäure, Methanol/Salzsäure oder Dioxan/Salzsäure, durch Diazotierung einer entsprechenden Aminoverbindung mit einem Nitrit, z. B. Natriumnitrit oder einem Ester der salpetrigen Säure, bei niederen Temperaturen, z. B. bei Temperaturen zwischen − 10 und 5 °C, hergestellt.

Die nachträgliche Acylierung wird zweckmäßigerweise in einem Lösungsmittel wie Methylenchlorid, Äther, Tetrahydrofuran oder in einem Überschuß der verwendeten Acylierungsmittels, z. B. Ameisensäure, Essigsäure- oder Propionsäure bzw. deren Anhydriden, Säurechloriden oder Estern, gegebenenfalls in Gegenwart einer anorganischen oder einer tertiären organischen Base, welche gleichzeitig auch als Lösungsmittel dienen kann, und gegebenenfalls in Gegenwart eines die Säure aktivierenden oder wasserentziehenden Mittels bei Temperaturen zwischen − 25 und 150 °C, vorzugsweise jedoch bei Temperaturen zwischen − 10 °C und der Siedetemperatur des Reaktionsgemisches, durchgeführt.

Die nachträgliche Hydrolyse wird zweckmäßigerweise entweder in Gegenwart einer Säure wie Salzsäure, Schwefelsäure- Phosphorsäure oder Trifluoressigsäure oder in Gegenwart einer Base wie Natriumhydroxid oder Kaliumhydroxid in einem geeigneten Lösungsmittel wie Äthanol, Wasser/Äthanol, Wasser/Isopropanol oder Wasser/Dioxan bei erhöhten Temperaturen, z. B. bei der Siedetemperatur des Reaktionsgemisches, durchgeführt.

Die nachträgliche Entbenzylierung und/oder Enthalogenierung wird Zweckmäßigerweise in einem Lösungsmittel wie Methanol, Äthanol, Essigester oder Eisessig mittels katalytisch angeregtem Wasserstoff, z. B. mit Wasserstoff in Gegenwart von Platin oder Palladium/Kohle, bei Temperaturen zwischen 0 und 75 °C, vorzugsweise jedoch bei Raumtemperatur, und bei einem Wasserstoffdruck von 1-5 bar durchgeführt.

Die nachträgliche O-Alkylierung wird zweckmäßigerweise mit einem entsprechenden Halogenid oder Sulfonsäureester, z. B. mit Methyljodid, Dimethylsulfat, Äthylbromid, p-Toluolsulfonsäure-benzylester oder Methansulfonsäure-isopropylester, gegebenenfalls in Gegenwart einer Base wie Natriumhydrid, Kaliumhydroxid oder Kalium-tert. butylat und vorzugsweise in einem Lösungsmittel wie Diäthyläther, Tetrahydrofuran, Dioxan, Äthanol, Pyridin oder Dimethylformamid bei Temperaturen zwischen 0 und 75 °C, vorzugsweise jedoch bei Raumtemperatur, durchgeführt.

Die nachträgliche Reduktion mit einem Metallhydrid wird zweckmäßigerweise mit einem komplexen Metallhydrid wie Lithiumaluminiumhydrid in einem Lösungsmittel wie Diäthyläther, Tetrahydrofuran oder Dioxan bei Temperaturen zwischen 0 und 100 °C, vorzugsweise jedoch bei der Siedetemperatur des Reaktionsgemisches, durchgeführt.

Die nachträgliche Oxidation einer Hydroxymethylgruppe wird zweckmäßigerweise mit einem Metalloxid wie Mangandioxid in einem Lösungsmittel wie Aceton oder Dichlormethan bei Temperaturen zwischen 0 und 50 °C, vorzugsweise jedoch bei Raumtemperatur, durchgeführt.

Die nachträgliche Überführung einer Hydroxymethylgruppe in eine Halogenmethylgruppe wird mit einem Halogenierungsmittel wie Thionylchlorid, Phosphortrichlorid, Phosphortribromid oder Phosphorpentachlorid in einem Lösungsmittel wie Methylenchlorid, Tetrachlorkohlenstoff, Benzol oder Nitrobenzol und deren anschließende Umsetzung mit einem Malonsäureester, z. B. mit einem Alkalisalz des Malonsäurediäthylester, bei Temperaturen zwischen 0 und 100 °C, vorzugsweise jedoch bei Temperaturen zwischen 20 und 50 °C, durchgeführt.

Die nachträgliche Acetalbildung wird zweckmäßigerweise in dem entsprechenden Alkohol als

Lösungsmittel, z. B. in Methanol oder Äthanol, in Gegenwart einer Säure wie Salzsäure oder Schwefelsäure, oder durch Umacetalisierung mit einem entsprechenden Orthoester, z. B. Orthoameisensäuretriäthylester, bei Temperaturen zwischen 0 und 100 °C, vorzugsweise jedoch bei Temperaturen zwischen 30 und 60 °C, durchgeführt.

Die nachträgliche Kondensation einer Formyl-Verbindung wird zweckmäßigerweise in einem Lösungsmittel wie Pyridin oder Tetrahydrofuran mit Malonsäure, mit einem Malonsäureester oder einem Trialkylphosphon-essigsäureester gegebenenfalls in Gegenwart einer Base als Kondensationsmittel, z. B. in Gegenwart von Piperidin, Kalium-tert. butylat oder Natriumhydrid, bei Temperaturen zwischen 0 und 100 °C durchgeführt ; durch anschließendes Ansäuern, z. B. mit Salzsäure oder Schwefelsäure, erhält man die gewünschte Säure.

Die nachträgliche Friedel-Crafts-Acylierung wird mit einem entsprechenden Säurehalogenid oder Säureanhydrid in einem geeigneten Lösungsmittel wie Schwefelkohlenstoff, Methylenchlorid, Dichloräthan oder Nitrobenzol und in Gegenwart eines Friedel-Crafts-Katalysators wie Aluminiumchlorid bei Temperaturen zwischen 0 und 50 °C, vorzugsweise jedoch bei Raumtemperatur, durchgeführt.

Die nachträgliche Alkoholyse wird vorzugsweise in einem entsprechenden wasserfreien Alkohol als Lösungsmittel, z. B. in wasserfreiem Methanol, Äthanol oder Propanol, in Gegenwart einer Säure wie Chlorwasserstoff oder Schwefelsäure bei erhöhten Temperaturen, vorzugsweise jedoch bei der Siedetemperatur des Reaktionsgemisches, durchgeführt.

Die nachträgliche Willgerodt-Reaktion wird zweckmäßigerweise in eine alkoholischen Lösungsmittel wie Methanol, Äthanol oder Isopropanol durch Erhitzen der Acetylverbindung mit einem Amin, z. B. mit Morpholin, in Gegenwart von Schwefel durchgeführt ; das so erhaltene entsprechende Thioamid wird anschließend durch Erhitzen in Gegenwart einer anorganischen Base wie Natriumhydroxid in das entsprechende Essigsäure-Derivat übergeführt, besonders vorteilhaft wird die Umsetzung bei der Siedetemperatur des Reaktionsgemisches durchgeführt.

Die nachträgliche Hydrolyse eines Orthoesters wird zweckmäßigerweise in einem Lösungsmittel wie Wasser/Methanol, Wasser/Dioxan, Wasser/Äthanol oder Wasser/Propanol in Gegenwart einer Säure wie Salzsäure oder Schwefelsäure bei niederen Temperaturen, z. B. bei Raumtemperatur, durchgeführt.

Die nachträgliche Disproportionierung eines Sulfonsäurehydrazids, welches man durch Umsetzung eines entsprechenden Hydrazins mit einem entsprechenden reaktionsfähigen Carbonsäurederivat erhält, wird in gegenwart einer Base wie Natriumcarbonat in einem Lösungsmittel wie Äthylenglykol bei Temperaturen zwischen 100 und 200 °C, vorzugsweise jedoch bei 160-170 °C, durchgeführt.

Die Überführung einer entsprechenden Verbindung der allgemeinen Formel I in eine entsprechende metallorganische Verbindung erfolgt zweckmäßigerweise in einem inerten Lösungsmittel wie Diäthyläther, Tetrahydrofuran, Dioxan oder Tetrahydrofuran/n-Hexan gegebenenfalls unter Schutzgas, z. B. unter Stickstoff, vorzugsweise mit einer entsprechenden Lithiumverbindung, z. B. Butyl-Lithium in n-Hexan, bei Temperaturen zwischen − 60 und 50 °C. Anschließend wird eine so erhaltene Lösung einer entsprechenden metallorganischen Verbindung gegebenenfalls unter Schutzgas vorzugsweise in festes Kohlendioxid eingetragen.

Die erhaltenen Verbindungen der allgemeinen Formel I lassen sich ferner in ihre physiologisch verträglichen Salze mit anorganischen oder organischen Säuren oder auch Basen, wenn Z eine Carboxygruppe darstellt oder Z eine Carboxygruppe enthält, überführen. Als Säuren kommen hierbei beispielsweise Salzsäure, Bromwasserstoffsäure, Schwefelsäure, Phosphorsäure, Milchsäure, Zitronensäure, Weinsäure, Bernsteinsäure, Maleinsäure oder Fumarsäure und als Basen Natriumhydroxid, Kaliumhydroxid oder Cyclohexylamin in Betracht.

Die als Ausgangsstoffe verwendeten Verbindungen der allgemeinen Formeln II bis XV sind literaturbekannt, bzw. man erhält sie nach an sich bekannten Verfahren. So erhält man beispielsweise eine Verbindung der allgemeinen Formel II durch Umsetzung einer 2-Chlor- oder 2-Brom-nitro-carbonsäure oder deren Derivate mit einem entsprechenden Amin, anschließende Reduktion der Nitrogruppe in einer so erhaltenen 2-Aminoverbindung mittels katalytisch angeregtem Wasserstoff, mittels nascierendem Wasserstoff, mittels Metallen oder Metallsalzen und Überführung der so erhaltenen Aminogruppe über ein entsprechendes Diazoniumsalz in eine entsprechende Verbindung der allgemeinen Formel II. Zur Herstellung einer Ausgangsverbindung der allgemeinen Formel II, in der $R_1$ eine Alkoxy- oder Phenylalkyloxygruppe darstellt, wird eine so hergestellte Hydroxy-carbonsäure anschließend alkyliert und erforderlichenfalls anschließend hydrolysiert.

Eine Verbindung deßallgemeinen Formel III, in der Y keine NH-Gruppe und keine Sauerstoffatom darstellt, erhält man beispielsweise durch Umsetzung eines entsprechenden 4-(α-Bromalkyl)-benzol-Derivates mit Natriumcyanid und anschließende katalytische Hydrierung der so erhaltenen Cyanoverbindung.

Eine Verbindung der allgemeinen Formel III, in der Y die NH-Gruppe oder ein Sauerstoffatom darstellt, erhält man beispielsweise durch Umsetzung eines entsprechenden 4-(α-Bromalkyl)- benzol-Derivates mit einer Hydroxamsäure oder deren Ester oder mit einem Acyl-hydrazin und erforderlichenfalls anschließende Hydrolyse.

Eine Verbindung der allgemeinen Formel III, in der Y die NH-Gruppe darstellt, erhält man auch durch Umsetzung eines 4-Formyl- oder 4-Acyl-benzol-Derivates mit einem N-Acyl-hydrazin, anschließende Reduktion des erhaltenen Hydrazons, z. B. mittels katalytischer Hydrierung, und anschließende hydrolytische Abspaltung des Acylrestes. Ein so erhaltener Ester der allgemeinen Formel III kann gewünschtenfalls mittels Hydrolyse in die entsprechende Carbonsäure übergeführt werden.

Eine als Ausgangsstoff verwendete Verbindung der allgemeinen Formeln IV, VI bis VIII, XII, XIV und XV erhält man durch Umsetzung einer entsprechenden Carbonsäure mit Ammoniak oder einem entsprechenden Amin in Gegenwart eines die Säure aktivierenden oder wasserentziehenden Mittels.

Eine als Ausgangsstoff verwendete Verbindung der allgemeinen Formel X erhält man durch Umsetzung eines entsprechenden Carbonsäureesters mit Hydrazin oder Hydroxylamin.

Eine als Ausgangsstoff verwendete Verbindung der der allgemeinen Formeln XI oder XIII erhält man durch Halogenierung eines entsprechenden Alkohols oder Umsetzung eines Sulfonsäurehalogenids mit einem entsprechenden Alkohol in Gegenwart einer Base.

Wie bereits eingangs erwähnt, weisen die neuen Verbindungen der allgemeinen Formel I wertvolle pharmakologische Eigenschaften auf, nämlich eine Wirkung auf den Stoffwechsel. So besitzen die Verbindungen der allgemeinen Formel I insbesondere eine blutzuckersenkende und/oder lipidsenkende Wirkung.

Beispielsweise wurden die Verbindungen

A = 4-[2-(5-Chlor-2-dimethylamino-benzylamino)-äthyl]-benzoesäure,
B = 4-[2-(5-Brom-2-dimethylamino-benzoylamino)-äthyl]-benzoesäure,
C = 4-[2-(5-Chlor-2-piperidino-benzoylamino)-äthyl]-benzoesäure-methylester,
D = 4-[2-(5-Chlor-2-piperidino-benzoylamino)-äthyl]-benzoesäure,
E = 4-[2-(5-Chlor-2-(2-methyl-piperidino)-benzoylamino)-äthyl]-benzoesäure,
F = 4-[2-(5-Chlor-2-(3-methyl-piperidino)-benzolamino)-äthyl]-benzoesäure,
G = 4-[2-(5-Chlor-2-(4-methyl-piperidino)-benzoylamino)-äthyl]-benzoesäure,
H = 4-[2-(5-Chlor-2-(3,5-dimethyl-piperidino)-benzoylamino)-äthyl]-benzoesäure-methylester,
I = 4-[2-(5-Chlor-2-(3,5-dimethyl-piperidino)-benzoylamino)-äthyl]-benzoesäure,
K = 4-[2-(5-Chlor-2-(4-methoxy-piperidino)-benzoylamino)-äthyl]-benzoesäure,
L = 4-[2-(5-Methoxy-2-piperidino-benzoylamino)-äthyl]-benzoesäure-hydrochlorid,
M = 4-[2-(5-Chlor-2-heptamethylenimino-benzoylamino)-äthyl]-benzoesäure,
N = 4-[2-5-Chlor-2-(1,4-dioxa-8-aza-spiro[4,5]-decan-8-yl)-benzoylamino)-äthyl]-benzoesäure,
O = 4-[2-(5-Chlor-2-hexamethylenimino-benzoylamino)-äthyl]-benzoesäure,
P = 4-[2-(5-Chloro-2-thiomorpholino-benzoylamino)-äthyl]-benzoesäure,
Q = 4-[2-(5-Brom-2-piperidino-benzoylamino)-äthyl]-benzoesäure,
R = 4-[2-(5-Chlor-2-piperidino-benzoylamino)-1-methyl-äthyl]-benzoesäure,
S = 4-[2-(5-Brom-2-(2-methyl-piperidino)-benzoylamino)-äthyl]-benzoesäure,
T = 4-[2-(5-Chlor-2-(2,6-dimethyl-morpholino)-benzoylamino)-äthyl]-benzoesäure,
U = 4-[2-(5-Chlor-2-(2,6-dimethyl-thiomorpholino)-benzoylamino)-äthyl]-benzoesäure,
V = 4-[2-(5-Brom-2-heptamethylenimino-benzoylamino)-äthyl]-benzoesäure-äthylester,
W = 4-[2-(5-Brom-2-heptamethylenimino-benzoylamino)-äthyl]-benzoesäure,
X = 4-[2-(5-Chlor-2-octamethylenimino-benzoylamino)-äthyl]-benzoesäure,
Y = 4-[2-(5-Chlor-2-(decahydro-isochinolin-2-yl)-benzoylamino)-äthyl]-benzoesäure-hydrochlorid,
Z = 4-[2-(5-Brom-2-octamethylenimino-benzoylamino)-äthyl]-benzoesäure-hydrochlorid,
AA = 4-[2-(5-Äthyl-2-piperidino-benzoylamino)-äthyl]-benzoesäure,
BB = 4-[2-(5-Methyl-2-piperidino-benzoylamino)-äthyl]-benzoesäure,
CC = 4-[2-(2-(N-Adamantyl-(1)-N-methyl-amino)-5-chlor-benzoylamino)-äthyl]-benzoesäure,
DD = 4-[2-(5-Chlor-2-octamethylenimino-benzoylamino)-äthyl]-benzoesäure-äthylester,
EE = 4-[2-Chlor-2-octamethylenimino-benzoylamino)-äthyl]-benzylalkohol,
FF = $N^1$-(1-(4-Carboxyphenyl)-äthyl)-$N^2$-(5-chlor-2-piperidino-benzoyl)-hydrazin
und
GG = 4-[1-(5-Chlor-2-octamethylenimino-benzoylaminoxy)-äthyl]-benzoesäure
im Vergleich zu
HH = 4-[2-(2-Äthylamino-5-chlor-benzoylamino)-äthyl]-benzoesäure
(siehe Beispiel 5 der BE-PS 837 311)

auf ihre blutzuckersenkenden Eigenschaften wie folgt untersucht :

1. Blutzuckersenkende Wirkung :

Die blutzuckersenkende Wirkung der zu untersuchenden Substanzen wurde an weiblichen Ratten eigener Zucht mit dem Gewicht von 180-220 g geprüft, welche 24 Stunden vor Versuchsbeginn nüchtern gesetzt wurden. Die zu untersuchenden Substanzen wurden unmittelbar vor Versuchsbeginn in 1,5 %-iger Methylcellulose suspendiert und per Schlundsonde appliziert.

Die Blutentnahme erfolgte unmittelbar vor Substanzapplikation, sowie 1, 2, 3 und 4 Stunden danach jeweils aus dem retroorbitalen Venenplexus. Hiervon wurden jeweils 50 µl mit 0,5 ml 0,33 N Perchlorsäure enteiweißt und zentrifugiert. Im Überstand wurde Glukose nach der Hexokinase-Methode mit Hilfe eines Analysenphotometers bestimmt. Die statistische Auswertung erfolgte nach dem t-Test nach Student

mit p =0,05 als Signifikanzgrenze.

Die nachfolgende Tabelle enthält die gefundenen Werte in Prozent gegenüber Kontrolle :

Tabelle 1

| Substanz | 25 mg/kg | | | | 10 mg/kg | | | | 5 mg/kg | | | |
|---|---|---|---|---|---|---|---|---|---|---|---|---|
| | 1 | 2 | 3 | 4 | 1 | 2 | 3 | 4 | 1 | 2 | 3 | 4 |
| | Stunden | | | | Stunden | | | | Stunden | | | |
| A | -31 | -21 | -10 | -10 | -32 | -18 | n.s. | n.s. | -12 | n.s. | n.s. | n.s. |
| B | -33 | -23 | n.s. | n.s. | -14 | - 9 | n.s. | n.s. | n.s. | n.s. | n.s. | n.s. |
| C | -44 | -37 | -23 | -24 | -41 | -26 | -23 | -14 | -31 | -21 | -18 | -15 |
| D | -44 | -43 | -41 | -38 | -33 | -37 | -36 | -26 | -37 | -34 | -28 | -30 |
| E | -42 | -43 | -38 | -31 | -34 | -24 | -14 | n.s. | -31 | -18 | n.s. | n.s. |
| F | -51 | -48 | -41 | -40 | -41 | -40 | -44 | -39 | -44 | -42 | -38 | -35 |
| G | -30 | -24 | -27 | -25 | -24 | -26 | n.s. | n.s. | -24 | -20 | -21 | -13 |
| H | -35 | -41 | -44 | -38 | -39 | -46 | -33 | -20 | -42 | -47 | -42 | -43 |
| I | -41 | -37 | -38 | -40 | -39 | -38 | -30 | -40 | -47 | -46 | -49 | -50 |
| K | -43 | -44 | -39 | -34 | -40 | -34 | -16 | n.s. | -40 | -32 | -20 | n.s. |
| L | -47 | -39 | -29 | -27 | -34 | n.s. | -15 | -16 | -37 | -29 | n.s. | n.s. |
| M | -38 | -40 | -38 | -36 | -38 | -37 | -38 | -36 | -42 | -41 | -40 | -34 |
| N | -43 | -41 | -36 | -25 | -35 | -34 | -24 | -19 | -36 | -17 | -10 | n.s. |
| O | -41 | -37 | -33 | -24 | -42 | -39 | -25 | -22 | -33 | -26 | -28 | -15 |
| P | -37 | -41 | -32 | -30 | -37 | -30 | -26 | -21 | -29 | -19 | n.s. | n.s. |
| Q | -38 | -39 | -34 | -37 | -40 | -42 | -43 | -44 | -32 | -40 | -32 | -23 |
| R | -52 | -37 | -37 | -30 | -25 | -28 | -21 | -21 | -27 | -25 | n.s. | n.s. |
| S | -42 | -44 | -38 | -32 | -39 | -34 | -24 | -12 | | | | |
| T | -48 | -29 | -25 | -33 | | | | | | | | |
| U | | | | | -41 | -43 | -41 | -40 | | | | |
| V | -34 | -43 | -39 | -40 | -19 | -19 | -24 | -26 | | | | |
| W | -44 | -49 | -41 | -46 | -34 | -36 | -38 | -38 | | | | |
| X | -51 | -44 | -39 | -41 | -45 | -43 | -45 | -46 | -37 | -43 | -37 | -49 |
| Y | -40 | -45 | -45 | -49 | | | | | -46 | -79 | -38 | -46 |
| Z | | | | | | | | | -45 | -43 | -42 | -35 |
| AA | | | | | -40 | -44 | -29 | -39 | -41 | -40 | -32 | -32 |
| BB | | | | | | | | | -40 | -31 | -14 | n.s. |
| CC | | | | | | | | | -39 | -42 | -41 | -38 |
| DD | -42 | -41 | -43 | -41 | -42 | -41 | -40 | -43 | -42 | -41 | -40 | -43 |
| EE | | | | | | | | | -35 | -36 | -28 | -23 |
| FF | | | | | n.s. | -31 | -33 | -22 | -15 | -25 | -13 | n.s. |
| GG | | | | | | | | | -41 | -21 | n.s. | n.s. |
| HH | n.s. | n.s. | n.s. | n.s. | | | | | | | | |

n.s. ≙ statistisch nicht signifikant

2. Akute Toxizität :

Bei weiblichen und männlichen und männlichen Mäusen eigener Zucht mit dem Gewicht von 20-26 g wurde die toxische Wirkung der Substanzen nach oraler Gabe (Suspension in 1 %-iger Methylcellulose) einer einmaligen Dosis bei einer Nachbeobachtungszeit von mindestens 7 Tagen geprüft. Die nachfolgende Tabelle enthält die gefundenen Werte :

| Substanz | orientierende Toxizität |
|---|---|
| A | > 2 000 mg/kg p.o. (0 von 6 Tieren gestorben) |
| B | > 1 000 mg/kg p.o. (0 von 5 Tieren gestorben) |
| D | > 2 000 mg/kg p.o. (0 von 6 Tieren gestorben) |
| H | > 1 000 mg/kg p.o. (0 von 6 Tieren gestorben) |
| O | > 1 000 mg/kg p.o. (0 von 10 Tieren gestorben) |
| T | > 1 000 mg/kg p.o. (0 von 10 Tieren gestorben) |
| V | > 1 000 mg/kg p.o. (0 von 6 Tieren gestorben) |
| W | > 1 000 mg/kg p.o. (0 von 6 Tieren gestorben) |
| X | > 1 000 mg/kg p.o. (0 von 6 Tieren gestorben) |

Aufgrund ihrer pharmakologischen Eigenschaften eignen sich die erfindungsgemäß hergestellten Verbindungen der allgemeinen Formel I und deren physiologisch verträgliche Salze zur Behandlung des Diabetes mellitus. Hierzu lassen sie sich, gegebenenfalls in Kombination mit anderen Wirksubstanzen, in die üblichen galenischen Zubereitungsformen wie Tabletten, Dragées, Kapseln, Pulver oder Suspensionen einarbeiten. Die Einzeldosis am Erwachsenen beträgt hierbei 1-50 mg, vorzugsweise jedoch 2,5-20 mg, 1 oder 2 mal täglich.

Die nachfolgenden Beispiele sollen die Erfindung näher erläutern :

Herstellung der Ausgangsprodukte :

Beispiel A

2-(Decahydro-isochinolin-2-yl)-5-nitro-benzoesäure

In 500 ml Äthanol werden 19 g (0,136 Mol) Decahydro-isochinolin, 27,3 g (0,136 Mol) 2-Chlor-5-nitro-benzoesäure und 38,6 g Kaliumcarbonat unter Rühren 18 Stunden unter Rückfluß erhitzt. Nach Abdestillieren des Äthanols wird der Rückstand in 800 ml Wasser gelöst und durch Zugabe von 2N Salzsäure auf pH 4 eingestellt, wobei das Produkt auskristallisiert.

Ausbeute : 38 g (92 % der Theorie),
Schmelzpunkt : 132-134 °C (Isopropanol)

| | | | |
|---|---|---|---|
| Ber. : | C 63,14 | H 6,62 | N 9,20 |
| Gef. : | 63,02 | 6,48 | 9,38 |

Beispiel B

2-(1,4-Dioxa-8-aza-spiro[4,5]decan-8-yl)-5-nitro-benzoesäure

20,1 g (0,1 Mol) 2-Chlor-5-nitro-benzoesäure werden in 200 ml Äthanol mit 42,9 g (0,3 Mol) 1,4-Dioxa-8-aza-spiro[4,5]decan 8 Stunden auf Rückflußtemperatur erhitzt. Nach dem Abdestillieren des Lösungsmittels wird der Abdampfrückstand in Wasser aufgenommen und mit 2N Salzsäure auf pH 5,2 eingestellt, dabei fällt das Produkt aus. Nach Extraktion mit Chloroform und Trocknung über Natriumsulfat, kristallisiert nach dem Abdestillieren des Lösungsmittels die Verbindung aus.

Ausbeute : 12 g (39 % der Theorie),
Schmelzpunkt : 155 °C (Äthanol).

| | | | |
|---|---|---|---|
| Ber. : | C 54,54 | H 5,23 | N 9,09 |
| Gef. : | 54,20 | 5,13 | 8,97 |

Analog den Beispielen A und B wurden folgende Verbindungen hergestellt :

2-(2-Methyl-piperidino)-5-nitro-benzoesäure
Ausbeute : 99 % der Theorie, Schmelzpunkt : 164 °C.
2-(3-Methyl-piperidino)-5-nitro-benzoesäure
Ausbeute : 85 % der Theorie, Schmelzpunkt : 161 °C.
2-(4-methyl-piperidino)-5-nitro-benzoesäure
Ausbeute : 85 % der Theorie, Schmelzpunkt : 155 °C.
2-(3-Äthyl-6-methyl-piperidino)-5-nitro-benzoesäure

Ausbeute : 76 % der Theorie, Schmelzpunkt : < 20 °C.
2-(3,5-Dimethyl-piperidino)-5-nitro-benzoesäure
Ausbeute : 65 % der Theorie, Schmelpunkt : 172 °C.
2-(4-Methoxy-piperidino)-5-nitro-benzoesäure
Ausbeute : 68 % der Theorie, Schmelzpunkt : 140 °C.
5-Nitro-2-(4-phenyl-piperidino)-benzoesäure
Ausbeute : 88 % der Theorie, Schmelzpunkt : 196 °C.
2-(4-Äthoxycarbonyl-piperidino)-5-nitro-benzoesäure
Ausbeute : 82 % der Theorie, Schmelzpunkt : 160 °C.
5-Nitro-2-thiomorpholino-benzoesäure
Ausbeute : 80 % der Theorie, Schmelzpunkt : 235 °C.
5-Nitro-2-(1,2,4,5-tetrahydro-3-benzazepino)-benzoesäure
Ausbeute : 68 % der Theorie, Schmelzpunkt : 222 °C.
5-Nitro-2-(1,2,3,4-tetrahydro-isochinolino)-benzoesäure
Ausbeute : 70 % der Theorie, Schmelzpunkt : 195 °C.
5-Nitro-2-(4-phenyl-piperazino)-benzoesäure
Ausbeute 88 % der Theorie, Schmelzpunkt : 196 °C.
5-Nitro-2-(4-pyridyl-(2)-piperazino)-benzoesäure
Ausbeute : 66 % der Theorie, Schmelzpunkt : 192 °C.
2-(trans-3,5-Dimethylpiperidino)-5-nitro-benzoesäure
Ausbeute : 63 % der Theorie, Schmelzpunkt : 132 °C.
2-(3,3,5,5-Tetramethyl-piperidino)-5-nitro-benzoesäure
Ausbeute : 98 % der Theorie, Schmelzpunkt : 138 °C.
2-(3,5-Dimethyl-morpholino)-5-nitro-benzoesäure
Ausbeute : 75 % der Theorie, Schmelzpunkt : 164 °C.
2-(3,5-Dimethyl-thiomorpholino)-5-nitro-benzoesäure
Ausbeute : 70 % der Theorie, Schmelzpunkt : 118 °C.
2-(3-Aza-bicyclo [3,2,2] nonan-3-yl)-5-nitro-benzoesäure
Ausbeute : 72 % der Theorie, Schmelzpunkt : 221 °C.
5-Nitro-2-nonamethylenimino-benzoesäure
Ausbeute : 80 % Theorie, Schmelzpunkt : 127 °C.
5-Nitro-2-decamethylenimino-benzoesäure
Ausbeute : 92 % der Theorie, Schmelzpunkt : 128 °C.
5-Nitro-2-undecamethylenimino-benzoesäure
Ausbeute : 91 % der Theorie, Schmelzpunkt : 120 °C.
5-Nitro-2-dodecamethylenimino-benzoesäure
Ausbeute : 95 % der Theorie, Schmelzpunkt : 115 °C.
2-(N-Methyl-N-phenylamino)-5-nitro-benzoesäure
Ausbeute : 10 % der Theorie, Schmelzpunkt : 115 °C.
2-(N-Äthyl-N-cyclohexylamino)-5-nitro-benzoesäure
Ausbeute : 78 % der Theorie, Schmelzpunkt : 74 °C.
2-(N-Butyl-N-cyclohexylamino)-5-nitro-benzoesäure
Ausbeute : 84 % der Theorie, Schmelzpunkt : 56 °C.
2-(N-Cyclohexyl-N-isobutylamino)-5-nitro-benzoesäure
Ausbeute : 63 % der Theorie, Schmelzpunkt : < 20 °C.
2-(Decahydro-3-benzazepin-3-yl)-5-nitro-benzoesäure
Ausbeute : 98 % der Theorie, Schmelzpunkt : < 20 °C.
2-(Octahydro-isoindol-2-yl)-5-nitro-benzoesäure
Ausbeute : 80 % der Theorie, Schmelzpunkt : 128 °C.
2-(4-Isopropyl-piperidino)-5-nitro-bensoesäure
Ausbeute : 79 % der Theorie, Schmelzpunkt : 142 °C.
2-(4-tert.Butyl-piperidino)-5-nitro-benzoesäure
Ausbeute : 57 % der Theorie, Schmelzpunkt : 136 °C.
2-(1,4-Dioxa-8-aza-spiro [4,6] undecan-8-yl)-5-nitro-benzoesäure
Ausbeute : 75 % der Theorie, Schmelzpunkt : 135 °C.
2,4-Dipiperidino-5-nitro-benzoesäure
Ausbeute : 31 % der Theorie, Schmelzpunkt : 152 °C.
4-Chlor-2-piperidino-5-nitro-benzoesäure
Ausbeute : 18 % der Theorie, Schmelzpunkt : 133 °C.
5-Nitro-2-(1,2,3,6-tetrahydro-pyridino)-benzoesäure
Ausbeute : 58 % der Theorie, Schmelzpunkt : 215 °C.
2-(N-Methyl-N-benzylamino)-5-nitro-benzoesäure
Ausbeute : 93% der Theorie, Schmelzpunkt : 123-126 °C.
2-[4-(4-Chlorphenyl)-piperazino]-5-nitro-benzoesäure-hydrochlorid
Ausbeute : 71,5 % der Theorie, Schmelzpunkt : 225-227 °C (Zers.).

2-(4-Carbäthoxy-piperazino)-5-nitro-benzosäure
Ausbeute : 23,1 % der Theorie, Schmelzpunkt : 155-156 °C.
2-[4-(2-Furoyl)-piperazino]-5-nitro-benzoesäure
Ausbeute : 64,8 % der Theorie, Schmelzpunkt : 200-205 °C.
2-(4-Benzyl-piperazino)-5-nitro-benzoesäure-hydrochlorid
Ausbeute : 86,6 % der Theorie, Schmelzpunkt : 142-145 °C.

Beispiel C

2-Hexamethylenimino-5-nitro-benzoesäurenitril

18,4 g (0,11 Mol) 2-Chlor-5-nitro-benzoesäurenitril werden in 250 ml Äthanol mit 22,4 g (0,21 Mol) Hexamethylenimin 4 Stunden auf Rückflußtemperatur erhitzt. Nach dem Abkühlen wird durch Zugabe von 500 ml Wasser das Produkt ölig ausgefällt. Die Fällung wird in Chloroform aufgenommen. Nach Trocknung mit Natriumsulfat und Abdestillation des Chloroforms wird der Abdampfrückstand aus Äthanol umkristallisiert.
Ausbeute : 19,7 g (73 % der Theorie),
Schmelzpunkt : 70 °C.

| | | | |
|---|---|---|---|
| Ber. : | C 63,65 | H 6,16 | H 17,13 |
| Gef. : | 63,80 | 6,07 | 17,05 |

Beispiel D

5-Amino-2-(decahydro-isochinolin-2-yl)-benzoesäure

In 250 ml Dimethylformamid werden 36 g (0,118 Mol) 2-(Decahydroisochinolin-2-yl)-5-nitro-benzoesäure gelöst und bei einem Wasserstoffdruck von 5 bar mit 10 %iger Palladiumkohle als Katalysator bei Raumtemperatur hydriert. Nach beendeter Wasserstoffaufnahme wird der Katalysator abfiltriert, das Lösungsmittel im Vakuum abdestilliert und der Rückstand aus Äthanol umkristallisiert.
Ausbeute : 31,2 g (96 % der Theorie),
Schmelzpunkt : 252 °C.

| | | | |
|---|---|---|---|
| Ber. : | C 70,04 | H 8,08 | N 10,20 |
| Gef. : | 70,09 | 7,85 | 10,12 |

Beispiel E

5-Amino-2-(1,4-dioxa-8-aza-spiro [4,5] decan-8-yl)-benzoesäure

12 g (0,039 Mol) 2-(1,4-Dioxa-8-aza-spiro [4,5] decan-8-yl)-5-nitro-benzoesäure werden in 100 ml Dimethylformamid bei einem Wasserstoffdruck von 5 bar bei Raumtemperatur mit 10 %iger Palladiumkohle als Katalysator hydriert. Nach beendeter Wasserstoffaufnahme wird der Katalysator abfiltriert, das Lösungsmittel abdestilliert und aus Äthanol umkristallisiert.
Ausbeute : 9 g (83 % der Theorie),
Schmelzpunkt : 209 °C.

| | | | |
|---|---|---|---|
| Ber. : | C 60,42 | H 6,52 | N 10,07 |
| Gef. : | 60,18 | 6,58 | 10,12 |

Analog den Beispielen D und E wurden folgende Verbindungen hergestellt :

5-Amino-2-pyrrolidino-benzoesäure
Ausbeute : 79 % der Theorie, Schmelzpunkt : 208 °C.
5-Amino-2-(2-methyl-piperidino)-benzoesäure
Ausbeute : 84 % der Theorie, Schmelzpunkt : 240 °C.
5-Amino-2-(3-methyl-piperidino)-benzoesäure
Ausbeute : 75 % der Theorie, Schmelzpunkt : 192 °C.
5-Amino-2-(4-methyl-piperidino)-benzoesäure
Ausbeute : 88 % der Theorie, Schmelzpunkt : 215 °C.
5-Amino-2-(3-äthyl-6-methyl-piperidino)-benzoesäure
Ausbeute : 59 % der Theorie, Schmelzpunkt : 219 °C.
5-Amino-2-(cis-3,5-dimethyl-piperidino)-benzoesäure
Ausbeute : 87 % der Theorie, Schmelzpunkt : 234 °C.
5-Amino-2-(4-methoxy-piperidino)-benzoesäure
Ausbeute : 80 % der Theorie, Schmelzpunkt : 228 °C.
5-Amino-2-heptamethylenimino-benzoesäure
Ausbeute : 64 % der Theorie, Schmelzpunkt : 214 °C.
5-Amino-2-(4-phenyl-piperidino)-benzoesäure

Ausbeute : 76 % der Theorie, Schmelzpunkt : 275 °C.
5-Amino-2-(4-äthoxycarbonyl-piperidino)-benzoesäure
Ausbeute : 85 % der Theorie, Schmelzpunkt : 203 °C.
5-Amino-2-thiomorpholino-benzoesäure
Ausbeute : 76 % der Theorie, Schmelzpunkt : 193 °C.
5-Amino-2-(1,2,4,5-tetrahydro-3-benzazepino)-benzoesäure
Ausbeute : 86 % der Theorie, Schmelzpunkt : 258 °C.
5-Amino-2-(1,2,3,4-tetrahydro-isochinolino)-benzoesäure
Ausbeute : 66 % der Theorie, Schmelzpunkt : 220 °C.
5-Amino-2-(4-phenyl-piperazino)-benzoesäure
Ausbeute : 83 % der Theorie, Schmelzpunkt : 255 °C.
5-Amino-2-(4-pyridyl-(2)-piperazino)-benzoesäure
Ausbeute : 80 % der Theorie, Schmelzpunkt : 248 °C.
5-Amino-2-(trans-3,5-dimethyl-piperidino)-benzoesäure
Ausbeute : 89 % der Theorie, Schmelzpunkt : 156 °C.
5-amino-2-(3,3,5,5-tetramethyl-piperidino)-benzoesäure
Ausbeute : 98 % der Theorie, Schmelzpunkt : < 20 °C.
5-Amino-2-(3,5-dimethyl-morpholino)-benzoesäure
Ausbeute : 83 % der Theorie, Schmelzpunkt : 255 °C.
5-Amino-2-(3,5-dimethyl-thiomorpholino)-benzoesäure
Ausbeute : 50 % der Theorie, Schmelzpunkt : 233 °C.
5-Amino-2-(3-aza-bicyclo [3,2,2] nonan-3-yl)-benzoesäure
Ausbeute : 86 % der Theorie, Schmelzpunkt : 288 °C.
5-Amino-2-octamethylenimino-benzoesäure
Ausbeute : 88 % der Theorie, Schmelzpunkt : 191 °C.
5-Amino-2-nonamethylenimino-benzoesäure
Ausbeute : 80 % der Theorie, Schmelzpunkt : 212 °C.
5-Amino-2-decamethylenimino-benzoesäure
Ausbeute : 52 % der Theorie, Schmelzpunkt : 202 °C.
5-Amino-2-undecamethylenimino-benzoesäure
Ausbeute : 93 % der Theorie, Schmelzpunkt : 242 °C.
5-Amino-2-dodecamethylenimino-benzoesäure
Ausbeute : 59 % der Theorie, Schmelzpunkt : 224 °C.
5-Amino-2-(N-methyl-N-phenylamino)-benzoesäure
Ausbeute : 47 % der Theorie, Schmelzpunkt : 184 °C.
5-Amino-2-(N-äthyl-N-cyclohexylamino)-benzoesäure
Ausbeute : 66 % der Theorie, Schmelzpunkt : 160 °C.
5-Amino-2-(N-butyl-N-cyclohexylamino)-benzoesäure
Ausbeute : 48 % der Theorie, Schmelzpunkt : 140 °C.
5-Amino-2-(N-cyclohexyl-N-isobutylamino)-benzoesäure
Ausbeute : 62 % der Theorie, Schmelzpunkt : < 20 °C.
5-Amino-2-(decahydro-3-benzazepin-3-yl)-benzoesäure
Ausbeute : 54 % der Theorie, Schmelzpunkt : 204 °C.
5-Amino-2-(octahydro-isoindol-2-yl)-benzoesäure
Ausbeute : 43 % der Theorie, Schmelzpunkt : 228 °C.
5-Amino-2-(4-isopropyl-piperidino)-bensoesäure
Ausbeute : 50 % der Theorie, Schmelzpunkt : 231 °C.
5-Amino-2-(4-tert.butyl-piperidino)-benzoesäure
Ausbeute : 81 % der Theorie, Schmelzpunkt : 276 °C.
5-Amino-2-(1,4-dioxa-8-aza-spiro [4,6] undecan-8-yl)-bensoesäure
Ausbeute : 49 % der Theorie, Schmelzpunkt : 235 °C.
5-Amino-2-(1,2,3,6-tetrahydro-pyridino)-benzoesäure
Ausbeute : 51 % der Theorie, Schmelzpunkt : 232 °C.
5-Amino-2-(4-methyl-piperazino)-benzoesäure-hydrochlorid
Ausbeute : 90 % der Theorie, Schmelzpunkt : < 20 °C.
5-Amino-2-(N-methyl-N-benzylamino)-benzoesäure
Ausbeute : 95 % der Theorie, Schmelzpunkt : < 20 °C.
5-Amino-2-[4-(4-chlor-phenyl)-piperazino]-benzoesäure-hydrochlorid
Ausbeute : 80,5 % der Theorie, Schmelzpunkt : 305 °C (Zersetzung).
5-Amino-2-(4-carbäthoxy-piperazino)-benzoesäure
Ausbeute : 87,5 % der Theorie, Schmelzpunkt : 195-197 °C.
5-Amino-2-[4-(2-furoyl)-piperazino]-benzoesäure
Ausbeute : 97 % der Theorie, Schmelzpunkt : < 20 °C.
5-Amino-2-(4-benzyl-piperazino)-benzoesäure-hydrochlorid
Ausbeute : 80 % der Theorie, Schmelzpunkt : 200-210 °C.

Beispiel F

5-Chlor-2-(decahydro-isochinolin-2-yl)-benzoesäure

In 55 ml halbkonzentrierter Salzsäure werden 10 g (0,036 5 Mol) 5-Amino-2-(decahydro-isochinolin-2-yl)-benzoesäure gelöst und bei 0 °C mit einer Lösung von 2,7 g (0,039 Mol) Natriumnitrit in 10 ml Wasser bei tropfenweiser Zugabe diazotiert. Nach abgeschlossener Zugabe wird 15 Minuten nachgerührt und anschließend die Diazoniumsalzlösung in eine Suspension von 4 g Kupferpulver in 40 ml konz. Salzsäure eingetropft. Nach Rühren über Nacht entsteht eine tiefgrüne homogene Lösung, die nach Verdünnen mit 100 ml Wasser mit Chloroform erschöpfend extrahiert wird. Nach Trocknung über Natriumsulfat wird der Chloroformabdampfrückstand über eine Kieselsäule mit einem Gemisch von Essigsäureäthylester/Methanol = 9,5 : 0,5 chromatographisch gereinigt.

Ausbeute : 4,8 g (45 % der Theorie),
Schmelzpunkt : 138 °C.

| | | | | |
|---|---|---|---|---|
| Ber. : | C 65,41 | H 6,85 | N 4,76 | Cl 12,06 |
| Gef. : | 65,51 | 7,07 | 4,89 | 12,32 |

Beispiel G

5-Chlor-2-(1,4-dioxa-8-aza-spiro [4,5] decan-8-yl)-benzoesäure

8,5 g (0,031 Mol) 5-Amino-2-(1,4-dioxa-8-aza-spiro [4,5] decan-8-yl)-benzoesäure werden in 28 ml halbkonzentrierter Salzsäure gelöst und bei 0 °C mit einer Lösung von 2,4 g (0,034 Mol) Natriumnitrit in 10 ml Wasser diazotiert. Die Diazoniumsalzlösung wird unter Rühren zu einer Suspension von 3 g Kupferpulver in 3 ml konzentrierter Salzsäure zugetropft. Nach beendeter Stickstoffentwicklung wird zwei Stunden nachgerührt, mit Wasser verdünnt und mit Chloroform ausgeschüttelt. Nach Trocknung über Natriumsulfat wird das Lösungsmittel abdestilliert. Beim Digerieren des Abdampfrückstandes mit Petroläther werden 6,1 g (66 % der Ausbeute) erhalten.

Schmelzpunkt : 180 °C.

| | | | |
|---|---|---|---|
| Ber. : | C 56,47 | H 5,42 | N 4,71 |
| Gef. : | 56,11 | 5,37 | 4,83 |

Analog den Beispielen F und G wurden folgende Verbindungen hergestellt :

5-Chlor-2-pyrrolidino-benzoesäure
Ausbeute : 30 % der Theorie, Schmelzpunkt : 164 °C.
5-Chlor-2-(2-methyl-piperidino)-benzoesäure
Ausbeute : 74 % der Theorie, Schmelzpunkt : 124 °C.
5-Chlor-2-(3-methyl-piperidino)-benzoesäure
Ausbeute : 47 % der Theorie, Schmelzpunkt : 165 °C.
5-Chlor-2-(4-methyl-piperidino)-benzoesäure
Ausbeute : 52 % der Theorie, Schmelzpunkt : 107 °C.
2-(3-Äthyl-6-methyl-piperidino)-5-chlor-benzoesäure
Ausbeute : 73 % der Theorie, Schmelzpunkt : < 20 °C.
5-Chlor-2-(cis-3,5-dimethyl-piperidino)-benzoesäure
Ausbeute : 46 % der Theorie, Schmelzpunkt : 167 °C.
5-Chlor-2-(trans-3,5-dimethyl-piperidino)-benzoesäure
Ausbeute : 63 % der Theorie, Schmelzpunkt : 132 °C.
5-Chlor-2-(4-methoxy-piperidino)-benzoesäure
Ausbeute : 63 % der Theorie, Schmelzpunkt : 136 °C.
5-Chlor-2-heptamethylenimino-benzoesäure
Ausbeute : 58 % der Theorie, Schmelzpunkt : < 20 °C.
5-Chlor-2-(4-phenyl-piperidino)-benzoesäure
Ausbeute : 51 % der Theorie, Schmelzpunkt : 217 °C.
5-Chlor-2-(4-äthoxycarbonyl-piperidino)-benzoesäure
Ausbeute : 97 % der Theorie, Schmelzpunkt : < 20 °C.
5-Chlor-2-hexamethylenimino-benzoesäure
Ausbeute : 34 % der Theorie, Schmelzpunkt : 113 °C.
5-Chlor-2-thiomorpholino-benzoesäure
Ausbeute : 16 % der Theorie, Schmelzpunkt : 160 °C.
5-Chlor-2-(1,2,4,5-tetrahydro-3-benzazepino)-benzoesäure
Ausbeute : 59 % der Theorie, Schmelzpunkt : 174 °C.
5-Chlor-2-(1,2,3,4-tetrahydro-isochinolino)-benzoesäure

Ausbeute : 50 % der Theorie, Schmelzpunkt : 182 °C.
5-Chlor-2-(4-phenyl-piperazino)-benzoesäure
Ausbeute : 42 % der Theorie, Schmelzpunkt : 154 °C.
5-Chlor-2-(4-pyridyl-(2)-piperazino)-benzoesäure
Ausbeute : 45 % der Theorie, Schmelzpunkt : 168 °C.
5-Brom-2-(2-methyl-piperidino)-benzoesäure
Ausbeute : 31 % der Theorie, Schmelzpunkt : 168 °C.
5-Chlor-2-(3,3,5,5-tetramethyl-piperidino)-benzoesäure
Ausbeute : 62 % der Theorie, Schmelzpunkt : < 20 °C.
5-Brom-2-(4-methoxy-piperidino)-benzoesäure
Ausbeute : 48 % der Theorie, Schmelzpunkt : 138 °C.
5-Chlor-2-(3,5-dimethylmorpholino)-benzoesäure
Ausbeute : 50 % der Theorie, Schmelzpunkt : 174 °C.
5-Chlor-2-(3,5-dimethyl-thiomorpholino)-benzoesäure
Ausbeute : 18 % der Theorie, Schmelzpunkt : 134 °C.
5-Brom-2-heptamethylenimino-benzoesäure
Ausbeute : 15 % der Theorie, Schmelzpunkt : 104 °C.
5-Chlor-2-(3-aza-bicyclo [3,2,2] nonan-3-yl)-benzoesäure
Ausbeute : 16 % der Theorie, Schmelzpunkt : 199 °C.
5-Chlor-2-octamethylenimino-benzoesäure
Ausbeute : 70 % der Theorie, Schmelzpunkt : 84 °C.
5-Chlor-2-nonamethylenimino-benzoesäure
Ausbeute : 30 % der Theorie, Schmelzpunkt : 78 °C.
5-Chlor-2-decamethylenimino-benzoesäure
Ausbeute : 65 % der Theorie, Schmelzpunkt : 70 °C.
5-Chlor-2-undecamethylenimino-benzoesäure
Ausbeute : 41 % der Theorie, Schmelzpunkt : 41 °C.
5-Chlor-2-dodecamethylenimino-benzoesäure
Ausbeute : 36 % der Theorie, Schmelzpunkt : 40 °C.
5-Chlor-2-(N-phenyl-N-methylamino)-benzoesäure
Ausbeute : 27 % der Theorie, Schmelzpunkt : 164 °C.
2-(N-Äthyl-N-cyclohexylamino)-5-chlor-benzoesäure
Ausbeute : 24 % der Theorie, Schmelzpunkt : 152 °C.
2-(N-Butyl-N-cyclohexylamino)-5-chlor-benzoesäure
Ausbeute : 16 % der Theorie, Schmelzpunkt : 145 °C.
5-Chlor-2-(N-cyclohexyl-N-isobutylamino)-benzoesäure
Ausbeute : 22 % der Theorie, Schmelzpunkt : 131 °C.
5-Chlor-2-(decahydro-3-benzazepin-3-yl)-benzoesäure
Ausbeute : 70 % der Theorie, Schmelzpunkt : 153 °C.
5-Brom-2-(decahydro-3-benzazepin-3-yl)-benzoesäure
Ausbeute : 54 % der Theorie, Schmekzpunkt : 154 °C.
5-Chlor-2-(octahydro-isoindol-2-yl)-benzoesäure
Ausbeute : 33 % der Theorie, Schmelzpunkt : 164 °C.
5-Brom-2-octamethylenimino-benzoesäure
Ausbeute : 48 % der Theorie, Schmelzpunkt : 94 °C.
5-Chlor-2-(4-isopropyl-piperidino)-benzoesäure
Ausbeute : 43 % der Theorie, Schmelzpunkt : 172 °C.
5-Chlor-2-(4-tert. butyl-piperidino)-benzoesäure
Ausbeute : 35 % der Theorie, Schmelzpunkt : 161 °C.
5-Chlor-2-(1,4-dioxa-8-aza-spiro [4,6] undecan-8-yl)-benzoesäure
Ausbeute : 42 % der Theorie, Schmelzpunkt : 163 °C.
5-Chlor-2-(1,2,3,6-tetrahydro-pyridino)-benzoesäure
Ausbeute : 73 % der Theorie, Schmelzpunkt : 173 °C.
5-Chlor-2-(4-methyl-piperazino)-benzoesäure-hydrochlorid
Ausbeute : 75 % der Theorie, Schmelzpunkt : 132 °C (Zers.).
5-Chlor-2-(N-methyl-N-benzylamino)-benzoesäure
Ausbeute : 18,2 % der Theorie, Schmelzpunkt : 156-157 °C.
5-Chlor-2-[4-(4-chlor-phenyl)-piperazino] benzoesäure
Ausbeute : 30,5 % der Theorie, Schmelzpunkt : 228-230 °C.
2-(4-Carbäthoxy-piperazino)-5-chlor-benzoesäure
Ausbeute : 52 % der Theorie, Schmelzpunkt : 129-130 °C.
5-Chlor-2-[4-(2-furoyl)-piperazino] benzoesäure
Ausbeute : 33,1 % der Theorie, Schmelzpunkt : 200-202 °C.
2-(4-Benzyl-piperazino)-5-chlor-benzoesäure-hydrochlorid
Ausbeute : 42,8 % der Theorie, Schmelzpunkt : 230-232 °C (Zers.).

Beispiel H

5-Amino-2-hexamethylenimino-benzoesäurenitril

21,4 g (0,087 Mol) 2-Hexamethylenimino-5-nitro-benzoesäurenitril werden in 200 ml Dioxan und 500 ml Methanol gelöst und bei Raumtemperatur und einem Wasserstoffdruck von 5 bar in Gegenwart von 10 %iger Palladiumkohle als Katalysator hydriert. Nach Abfiltrieren des Katalysators und Abdestillieren des Lösungsmittels werden 20 g (100 % der Theorie) erhalten.

Schmelzpunkt : < 20 °C.

Beispiel I

5-Chlor-2-hexamethylenimino-benzoesäurenitril

20 g (0,092 Mol) 5-Amino-2-hexamethylenimino-benzoesäurenitril werden in 90 ml halbkonzentrierter Salzsäure gelöst und bei 0 °C mit einer Lösung von 6,5 g (0,094 Mol) Natriumnitrit in 30 ml Wasser bei tropfenweiser Zugabe diazotiert. Nach beendeter Zugabe wird 15 Minuten nachgerührt. Die Diazoniumsalzlösung wird unter Rühren zu einer Lösung von Kupfer(I)-chlorid in konzentrierter Salzsäure, die auf 70 °C erwärmt wird, zugetropft. Nach abgeschlossener Stickstoffentwicklung wird mit Chloroform extrahiert. Nach Trocknung über Natriumsulfat und Abdestillieren des Chloroforms wird der Abdampfrückstand über eine Kieselgelsäule chromatographisch gereinigt. Als Fließmittel wird Toluol verwendet.

Ausbeute : 5 g (23 % der Theorie),
Schmelzpunkt : < 20 °C.

Beispiel J

5-Chlor-2-hexamethylenimino-benzoesäure

5 g (0,021 Mol) 5-Chlor-2-hexamethylenimino-benzoesäurenitril werden in 32 g Kalilauge und 20 ml Wasser 8 Stunden auf 170 °C erhitzt. Die erkaltete Schmelze wird in Wasser gelöst. Durch Ansäuern auf pH 5 wird das Amid quantitativ ausgefällt, welches anschließend mit halbkonzentrierter Salzsäure hydrolysiert wird.

Ausbeute : 3,6 g (67,4 % der Theorie),
Schmelzpunkt : 113 °C.

Analog den Beispielen H bis J wurden folgende Verbindungen hergestellt :

2-Morpholino-5-nitro-benzoesäurenitril
Ausbeute : 78 % der Theorie, Schmelzpunkt : 138 °C.
5-Amino-2-morpholino-benzoesäurenitril
Ausbeute : 68 % der Theorie, Schmelzpunkt : 142 °C.
5-Chlor-2-morpholino-benzoesäurenitril
Ausbeute : 20 % der Theorie, Schmelzpunkt : 57 °C.
5-Chlor-2-morpholino-benzamid
Ausbeute : 98 % der Theorie, Schmelzpunkt : 280 °C.
5-Chlor-2-morpholino-benzoesäure
Ausbeute : 60 % der Theorie, Schmelzpunkt : 157 °C.

Beispiel K

5-Cyano-2-octamethylenimino-benzoesäure

26,2 g (0,1 Mol) 5-Amino-2-octamethylenimino-benzoesäure werden in 38 ml konzentrierter Salzsäure gelöst, mit 280 ml Wasser verdünnt und bei 0 °C mit einer Lösung von 7,6 g (0,11 Mol) Natriumnitrit in 30 ml Wasser bei tropfenweiser Zugabe diazotiert. Nach halbstündigem Nachrühren wird die Lösung mit Natriumkarbonat auf pH 7 gestellt. Zu der Diazoniumsalzlösung wird anschließend eine Lösung von Trinatrium-tetracyano-kupfer(I)-Komplex bei 0 °C zugetropft.

Diese Kupfer(I)-Komplex-Lösung wird wie folgt erhalten : 32 g (0,128 Mol) Kupfersulfat × 5 $H_2O$ und 8,7 g Natriumchlorid in 100 ml Wasser werden mit einer Natriumhydrogensulfitlösung, bestehend aus 6,6 g (0,063 5 Mol) Natriumhydrogensulfit, 4,4 g Natriumhydroxid in 50 ml Wasser zum Kupfer(I)-Chlorid reduziert. Das ausgefallene Kupfer(I)-Chlorid wird abgesaugt, in 50 ml Wasser suspendiert und in einer Lösung von 17 g (0,346 Mol) Natriumcyanid in 30 ml Wasser gelöst.

Nach abgeschlossener Stickstoffentwicklung wird das Reaktionsgemisch eine Stunde auf 70 °C erwärmt. Nach dem Erkalten wird mit 2N Salzsäure pH 5,5 eingestellt und mit Chloroform extrahiert. Die Chloroformphasen werden über Natriumsulfat getrocknet und nach Abdestillieren des Chloroforms wird

das erhaltene Rohprodukt über eine Kieselgelsäule mit Essigsäureäthylester als Fließmittel gereinigt.

Ausbeute : 9 g (30 % der Theorie),
Schmelzpunkt : < 20 °C.
Ber. : C 70,56 H 7,40 N 10,28
Gef. : 70,38 7,20 10,10

Beispiel L

2-Heptamethylenimino-5-hydroxy-benzoesäure

26,7 g (0,107 Mol) 5-Amino-2-heptamethylenimino-benzoesäure werden in 190 ml 3N Schwefelsäure gelöst und bei 0 °C mit einer Lösung von 8,3 g (0,12 Mol) Natriumnitrit in 30 ml Wasser bei tropfenweiser Zugabe diazotiert. Nach halbstündigem Nachrühren werden 2 g feingepulverter Harnstoff zugesetzt. Bei guter Rührung wird die Diazoniumsalzlösung zu 320 ml 50 %iger Schwefelsäure, die auf 90 °C erwärmt ist, zugetropft. Nach beendeter Stickstoffentwicklung wird bei Raumtemperatur mit Ammoniak auf pH 4 eingestellt und mit Chloroform extrahiert. Der nach Trocknung über Natriumsulfat und Abdestillieren des Chloroforms gewonnene Trockenrückstand wird über eine Kieselgelsäule mit Chloroform als Laufmittel gereinigt.

Nach Umkristallisation aus Isopropanol werden 8 g (30 % der Theorie) erhalten.
Schmelzpunkt : 199 °C.
Ber. : C 67,45 H 7,68 N 5,61
Gef. : 66,87 7,71 5,65

Analog Beispiel 9 wurden folgende Verbindungen hergestellt :

2-Hexamethylenimino-5-hydroxy-benzoesäure
Ausbeute : 24 % der Theorie, Schmelzpunkt : 214 °C.
5-Hydroxy-2-octamethylenimino-benzoesäure
Ausbeute : 27 % der Theorie, Schmelzpunkt : 208 °C.
5-Hydroxy-2-(4-tert.butyl-piperidino)-benzoesäure
Ausbeute : 33 % der Theorie, Schmelzpunkt : 240 °C.
5-Hydroxy-2-(cis-3,5-dimethyl-piperidino)-benzoesäure
Ausbeute : 67,4 % der Theorie, Schmelzpunkt : 248-250 °C.

Beispiel M

5-Methoxy-2-octamethylenimino-benzoesäure

3,2 g (12,2 mMol) 5-Hydroxy-2-octamethylenimino-benzoesäure werden in 20 ml absolutem Dimethylformamid mit 0,6 g (25 mMol) Natriumhydroxid versetzt und auf 50 °C erwärmt, dabei fällt zum Teil das Natriumsalz aus. Nach Zugabe von 5,2 g (36,6 mMol) Methyljodid in 3 ml absolutem Dimethylformamid wird 5 Stunden bei Raumtemperatur gerührt. Nach Abdestillieren des Dimethylformamids wird der rohe 5-Methoxy-2-octamethylenimino-benzoesäure-methylester über eine Kieselgelsäule mit Chloroform als Laufmittel gereinigt.

Ausbeute : 90 % der Theorie, Schmelzpunkt : < 20 °C.

Dieser Ester wird mit Natronlauge bei 80 °C hydrolysiert. Nach dem Ansäuern auf pH 5,2 wird mit Chloroform extrahiert, über Natriumsulfat getrocknet und der Abdampfrückstand mit Petroläther digeriert.

Ausbeute : 85 % der Theorie,
Schmelzpunkt : 84 °C.
Ber. : C 69,28 H 8,35 N 5,04
Gef. : 69,12 8,29 4,95

Analog Beispiel M wurden folgende Verbindungen hergestellt :

2-Hexamethylenimino-5-methoxy-benzoesäure
Ausbeute : 88 % der Theorie, Schmelzpunkt : 141 °C.
2-Heptamethylenimino-5-methoxy-benzoesäure
Ausbeute : 30 % der Theorie, Schmelzpunkt : 120 °C.
2-Heptamethylenimino-5-isopropyloxy-benzoesäure
Ausbeute : 78 % der Theorie, Schmelzpunkt : 120 °C.
5-Äthoxy-2-octamethylenimino-benzoesäure
Ausbeute : 87 % der Theorie, Schmelzpunkt : < 20 °C.
5-Isopropyloxy-2-octamethylenimino-benzoesäure

Ausbeute : 60 % der Theorie, Schmelzpunkt : 78 °C.
5-Butyl-(2)-oxy-2-octamethylenimino-benzoesäure
Ausbeute : 48 % der Theorie, Schmelzpunkt : < 20 °C.
5-Methoxy-2-(4-tert.butyl-piperidino)-benzoesäure
Ausbeute : 22 % der Theorie, Schmelzpunkt : 156 °C.
5-Methoxy-2-(3,5-cis-dimethyl-piperidino)-benzoesäure
Ausbeute : 90 % der Theorie, Schmelzpunkt : 124 °C.
5-Hexyloxy-2-piperidino-benzoesäure
Ausbeute : 73 % der Theorie, Schmelzpunkt : 72 °C.
5-Benzyloxy-2-piperidino-benzoesäure
Ausbeute : 41 % der Theorie, Schmelzpunkt : 188 °C.

Herstellung der Endprodukte der allgemeinen Formel I :

## Beispiel 1

4-[2-(5-Chlor-2-dimethylamino-benzoylamino)-äthyl]-benzoesäure-methylester

Zu einer Lösung von 1,06 g (5,3 mMol) 5-Chlor-2-dimethylamino-benzoesäure in 5 ml absolutem Tetrahydrofuran werden bei Raumtemperatur 1,03 g (6,3 mMol) N,N'-Carbonyl-diimidazol zugesetzt. Nach 1-2 Stunden wird zu dem gebildeten Imidazolid eine Lösung von 1,13 g (6,3 mMol) 4-(2-Amino-äthyl)-benzoesäuremethylester in 2 ml Tetrahydrofuran zugesetzt. Nach 16-stündigem Stehen bei Raumtemperatur wird das Tetrahydrofuran am Rotationsverdampfer abdestilliert. Der rohe Ester wird über eine Kieselgelsäule mit Toluol/Essigsäureäthylester (9 : 1) als Laufmittel chromatographisch gereinigt. Der Trockenrückstand der vereinigten Fraktionen, die den gereinigten Ester enthalten, wird mit Petroläther behandelt.

Ausbeute : 0,9 g (47,5 % der Theorie),
Schmelzpunkt : 94 °C

| | | | |
|---|---|---|---|
| Ber. : | C 62,7 | H 5,88 | N 7,57 |
| Gef. : | 63,3 | 5,86 | 7,75 |

## Beispiel 2

4-[2-(5-Chlor-2-dimethylamino-benzoylamino)-äthyl]-benzoesäure

0,55 g (1,56 mMol) 4-[2-(5-Chlor-2-dimethylamino-benzoylamino)-äthyl]-benzoesäure-methylester werden in 40 ml einer Mischung aus Methanol/Dioxan (2 : 1) gelöst. Nach Zusatz von 0,29 g (4,8 mMol) Kaliumhydroxid, gelöst in 3 ml Wasser, werden bei Raumtemperatur 30 ml Wasser so langsam zugetropft, daß es zu keiner Ausfällung des Esters kommt. Nach einigen Stunden wird das organische Lösungsmittel am Rotationsverdampfer abdestilliert, die wässrige Phase mit Chloroform ausgeschüttelt und die wässrige Phase mit 2N Salzsäure auf pH 5,5 gestellt, dabei fällt die Säure aus.

Ausbeute : 0,38 g (70 % der Theorie),
Schmelzpunkt : 165 °C

| | | | |
|---|---|---|---|
| Ber. : | C 62,45 | H 5,52 | N 8,06 |
| Gef. : | 62,30 | 5,62 | 7,87 |

## Beispiel 3

4-[2-(5-Chlor-2-dimethylamino-benzoylamino)-äthyl]-benzoesäure

0,32 g (mMol) 4-[2-(2-Amino-5-chlor-benzoylamino)-äthyl]-benzoesäure (Schmelzpunkt : 196 °C, hergestellt aus 2-Amino-5-chlor-benzoesäure und 4-(2-Amino-äthyl)-benzoesäure-methylester analog Beispiel 1 und anschließende alkalische Verseifung analog Beispiel 2) werden in 10 ml Formalin und 30 ml Eisessig nach Zusatz von 500 mg 10 %iger Palladiumkohle bei Raumtemperatur im Autoklav bei 5 bar mit Wasserstoff behandelt. Nach Filtration und Abdestillation der Lösungsmittel wird die Säure in Natronlauge gelöst und mit 2N-Salzsäure ausgefällt.

Ausbeute : 0,11 g (30 % der Theorie),
Schmelzpunkt : 165 °C

| | | | |
|---|---|---|---|
| Ber. : | C 62,45 | H 5,52 | N 8,06 |
| Gef. : | 62,38 | 5,68 | 7,90 |

## Beispiel 4

4-[2-(5-Chlor-2-dimethylamino-benzoylamino)-äthyl]-benzoesäure

Hergestellt aus 4-[2-(5-Chlor-2-methylamino-benzoylamino)-äthyl]-benzoesäure (Schmelzpunkt : 205 °C) analog Beispiel 3.
Ausbeute : 30 % der Theorie,
Schmelzpunkt : 165 °C

Beispiel 5

4-[2-(5-Chlor-2-dimethylamino-benzoylamino)-äthyl]-benzoesäure-methylester

1,06 g (5,3 mMol) 5-Chlor-2-dimethylamino-benzoesäure werden mit 7 ml Thionylchlorid bei 40-50 °C in das Säurechlorid überführt. Nach dem Abdestillieren des Thionylchlorids wird das rohe Säurechlorid in 10 ml absolutem Pyridin mit 0,95 g (5,3 mMol) 4-(2-Aminoäthyl)-benzoesäure-methylester umgesetzt. Nach 2-stündigem Rühren bei Raumtemperatur wird ca. 20 Minuten auf 50-70 °C erhitzt und anschließend das Pyridin am Rotationsverdampfer abdestilliert. Der Trockenrückstand wird in Eiswasser gelöst, mit Natronlauge alkalisch gestellt und diese Lösung mit Chloroform extrahiert. Der Trockenrückstand der über Natriumsulfat getrockneten Chloroformextrakte wird über eine Kieselgelsäule mit Toluol : Essigester = 9 : 1 als Fließmittel chromatographisch gereinigt.
Ausbeute : 1,4 g (73 % der Theorie),
Schmelzpunkt : 94 °C

| | | | |
|---|---|---|---|
| Ber. : | C 62,7 | H 5,88 | N 7,57 |
| Gef. : | 62,9 | 5,73 | 7,63 |

Beispiel 6

4-[2-(5-Brom-2-dimethylamino-benzoylamino)-äthyl]-benzoesäure-methylester

Hergestellt aus 5-Brom-2-dimethylamino-benzoesäure und 4-(2-Amino-äthyl)-benzoesäuremethylester analog Beispiel 1.
Ausbeute : 93,5 % der Theorie,
Schmelzpunkt : 99 °C

| | | | |
|---|---|---|---|
| Ber. : | C 56,35 | H 5,21 | N 6,90 |
| Gef. : | 56,10 | 5,32 | 7,01 |

Beispiel 7

4-[2-(5-Brom-2-dimethylamino-benzoylamino)-äthyl]-benzoesäure

Hergestellt aus 4-[2-(5-Brom-2-dimethylamino-benzoylamino)-äthyl]-benzoesäure-methylester durch alkalische Hydrolyse analog Beispiel 2.
Ausbeute : 77 % der Theorie,
Schmelzpunkt : 187 °C

| | | | |
|---|---|---|---|
| Ber. : | C 55,4 | H 4,89 | N 7,16 |
| Gef. : | 55,2 | 4,97 | 7,01 |

Beispiel 8

4-[2-(5-Fluor-2-dimethylamino-benzoylamino)-äthyl]-benzoesäure-methylester

Hergestellt aus 5-Fluor-2-dimethylamino-benzoesäure und 4-(2-Amino-äthyl)-benzoesäure-methylester analog Beispiel 1.
Ausbeute : 44 % der Theorie,
Schmelzpunkt : 56 °C

| | | | |
|---|---|---|---|
| Ber. : | C 66,30 | H 6,14 | N 8,13 |
| Gef. : | 66,28 | 6,22 | 8,13 |

Beispiel 9

4-[2-(5-Fluor-2-dimethylamino-benzoylamino)-äthyl]-benzoesäure

Hergestellt aus 4-[2-(5-Fluor-2-dimethylamino-benzoylamino)-äthyl]-benzoesäure-methylester durch alkalische Hydrolyse analog Beispiel 2.
Ausbeute : 73 % der Theorie,
Schmelzpunkt : 108 °C

| | | | |
|---|---|---|---|
| Ber. : | C 65,55 | H 5,80 | N 8,47 |
| Gef. : | 64,98 | 5,68 | 8,38 |

## Beispiel 10

4-[2-(2-Dimethylamino-benzoylamino)-äthyl]-benzoesäure-methylester

Hergestellt aus 2-Dimethylamino-benzoesäure und 4-(2-Amino-äthyl)-benzoesäure-methylester analog Beispiel 1. Die Reaktion wurde bei 60-70 °C durchgeführt.
Ausbeute : 98 % der Theorie,
Schmelzpunkt : 74 °C

| | | | |
|---|---|---|---|
| Ber. : | C 70,0 | H 6,78 | N 8,56 |
| Gef. : | 69,8 | 6,92 | 8,54 |

## Beispiel 11

4-[2-(2-Dimethylamino-benzoylamino)-äthyl]-benzoesäure

Hergestellt aus 4-[2-(2-Dimethylamino-benzoylamino)-äthyl]-benzoesäure-methylester durch alkalische Hydrolyse analog Beispiel 2.
Ausbeute : 84 % der Theorie,
Schmelzpunkt : 107 °C

| | | | |
|---|---|---|---|
| Ber. : | C 69,25 | H 6,45 | N 8,96 |
| Gef. : | 69,50 | 6,62 | 9,00 |

## Beispiel 12

4-[2-(5-Chlor-2-diäthylamino-benzoylamino)-äthyl]-benzoesäure-methylester

Hergestellt aus 5-Chlor-2-diäthylamino-benzoesäure und 4-(2-Amino-äthyl)-benzoesäure-methylester analog Beispiel 1.
Ausbeute : 51 % der Theorie,
Schmelzpunkt : 93 °C

| | | | |
|---|---|---|---|
| Ber. : | C 64,86 | H 6,48 | N 9,12 |
| Gef. : | 65,01 | 6,54 | 9,38 |

## Beispiel 13

4-[2-(5-Chlor-2-diäthylamino-benzoylamino)-äthyl]-benzoesäure

Hergestellt aus 4-[2-(Chlor-2-diäthylamino-benzoylamino)-äthyl]-benzoesäure-methylester durch alkalische Hydrolyse analog Beispiel 2.
Ausbeute : 80 % der Theorie,
Schmelzpunkt : 95 °C

| | | | |
|---|---|---|---|
| Ber. : | C 64,1 | H 6,17 | N 7,46 |
| Gef. : | 64,2 | 6,09 | 7,32 |

## Beispiel 14

4-[2-(5-Chlor-2-diisobutylamino-benzoylamino)-äthyl]-benzoesäure-äthylester

Hergestellt aus 5-Chlor-2-diisobutylamino-benzoesäure und 4-(2-Amino-äthyl)-benzoesäure-äthylester analog Beispiel 1.
Ausbeute : 20 % der Theorie,
Schmelzpunkt : < 20 °C

| | | | | |
|---|---|---|---|---|
| Ber. : | C 68,03 | H 7,69 | N 6,10 | Cl 7,72 |
| Gef. : | 68,59 | 7,68 | 5,93 | 7,51 |

## Beispiel 15

4-[2-(5-Chlor-2-diisobutylamino-benzoylamino)-äthyl]-benzoesäure

Hergestellt aus 4-[2-(5-Chlor-2-diisobutylamino-benzoylamino)-äthyl]-benzoesäureäthylester durch alkalische Hydrolyse analog Beispiel 2.
Ausbeute : 90 % der Theorie,
Schmelzpunkt : 113 °C

| | | | | |
|---|---|---|---|---|
| Ber. : | C 66,88 | H 7,24 | N 6,49 | Cl 8,22 |
| Gef. : | 66,50 | 7,28 | 6,32 | 8,40 |

Beispiel 16

4-[2-(5-Chlor-2-dipentylamino-benzoylamino)-äthyl]-benzoesäure-äthylester

Hergestellt aus 5-Chlor-2-dipentylamino-benzoesäure und 4-(2-Amino-äthyl)-benzoesäure-äthylester analog Beispiel 1.
Ausbeute : 83 % der Theorie,
Schmelzpunkt : 118-120 °C

| | | | | |
|---|---|---|---|---|
| Ber. : | C 69,05 | H 8,07 | N 5,75 | Cl 7,28 |
| Gef. : | 68,84 | 7,99 | 6,05 | 7,54 |

Beispiel 17

4-[2-(5-Chlor-2-dipentylamino-benzoylamino)-äthyl]-benzoesäure

Hergestellt aus 4-[2-(5-Chlor-2-dipentylamino-benzoylamino-äthyl]-benzoesäure-äthylester durch alkalische Hydrolyse analog Beispiel 2.
Ausbeute : 36,5 % der Theorie,
Schmelzpunkt : < 20 °C.

| | | | | |
|---|---|---|---|---|
| Ber. : | C 68,03 | H 7,68 | N 6,10 | Cl 7,72 |
| Gef. : | 67,93 | 7,64 | 6,02 | 7,86 |

Beispiel 18

4-[2-(5-Chlor-2-(1-pyrrolyl)-benzoylamino)-äthyl]-benzoesäure-methylester

Hergestellt aus 5-Chlor-2-(1-pyrrolyl)-benzoesäure und 4-(2-Amino-äthyl)-benzoesäure-methylester analog Beispiel 1.
Ausbeute : 64 % der Theorie,
Schmelzpunkt : 120-121 °C

| | | | | |
|---|---|---|---|---|
| Ber. : | C 65,88 | H 5,00 | N 7,32 | Cl 9,26 |
| Gef. : | 65,86 | 4,85 | 7,47 | 9,38 |

Beispiel 19

4-[2-(5-Chlor-2-(1-pyrrolyl)-benzoylamino)-äthyl]-benzoesäure

Hergestellt aus 4-[2-(5-Chlor-2-(1-pyrrolyl)-benzoylamino)-äthyl]-benzoesäure-methylester durch alkalische Hydrolyse analog Beispiel 2.
Ausbeute : 26 % der Theorie,
Schmelzpunkt : 250-255 °C

| | | | | |
|---|---|---|---|---|
| Ber. : | C 65,13 | H 4,65 | N 7,59 | Cl 9,61 |
| Gef. : | 65,07 | 4,74 | 7,34 | 9,07 |

Beispiel 20

4-[2-(5-Chlor-2-(N-cyclohexyl-methylamino-benzoylamino)-äthyl]-benzoesäure-äthylester

Hergestellt aus 2-(N-Cyclohexyl-methylamino)-5-chlor-benzoesäure und 4-(2-Amino-äthyl)-benzoesäure-äthylester analog Beispiel 1.
Ausbeute : 45 % der Theorie,
Schmelzpunkt : 98 °C

| | | | |
|---|---|---|---|
| Ber. : | C 67,78 | H 7,05 | N 6,33 |
| Gef. : | 67,60 | 6,81 | 6,28 |

Beispiel 21

4-[2-(5-Chlor-2-[N-cyclohexyl-methylamino]benzoylamino)-äthyl]-benzoesäure

Hergestellt aus 4-[2-(5-Chlor-2-[N-cyclohexylmethylamino]-benzoylamino)-äthyl]-benzoesäure-äthylester durch alkalische Hydrolyse analog Beispiel 2.
Ausbeute : 58 % der Theorie,
Schmelzpunkt : 166 °C

| | | | |
|---|---|---|---|
| Ber. : | C 66,58 | H 6,56 | N 6,75 |
| Gef. : | 66,63 | 6,79 | 6,66 |

Beispiel 22

4-[2-(5-Brom-2-piperidino-benzoylamino)-äthyl]-benzoesäure-äthylester

Hergestellt aus 5-Brom-2-piperidino-benzoesäure und 4-(2-Aminoäthyl)-benzoesäure-äthylester analog Beispiel 1.
Ausbeute : 87 % der Theorie,
Schmelzpunkt : 76 °C
Ber. : C 60,13 H 5,92 N 6,09
Gef. : 60,35 5,97 6,19

Beispiel 23

4-[2-(5-Brom-2-piperidino-benzoylamino)-äthyl]-benzoesäure

Hergestellt aus 4-[2-(5-Brom-2-piperidino-benzoylamino)-äthyl]-benzoesäure-äthylester durch alkalische Hydrolyse analog Beispiel 2.
Ausbeute : 99 % der Theorie,
Schmelzpunkt : 201 °C
Ber. : C 58,47 H 5,37 N 6,49
Gef. : 58,56 5,40 6,55

Beispiel 24

4-[2-(5-Chlor-2-pyrrolidino-benzoylamino)-äthyl]-benzoesäure-methylester

Hergestellt aus 5-Chlor-2-pyrrolidino-benzoesäure und 4-(2-Amino-äthyl)-benzoesäure-methylester analog Beispiel 1.
Ausbeute : 43 % der Theorie,
Schmelzpunkt : 164 °C
Ber. : C 65,19 H 5,99 N 7,24
Gef. : 65,35 6,00 7,23

Beispiel 25

4-[2-(5-Chlor-2-pyrrolidino-benzoylamino)-äthyl]-benzoesäure

Hergestellt aus 4-[2-(5-Chlor-2-pyrrolidino-benzoylamino)-äthyl]-benzoesäure-methylester durch alkalische Hydrolyse analog Beispiel 2.
Ausbeute : 68 % der Theorie,
Schmelzpunkt : 184 °C
Ber. : C 64,42 H 5,68 N 7,52 Cl 9,51
Gef. : 64,46 5,96 7,47 9,38

Beispiel 26

4-[2-(5-Chlor-2-piperidino-benzoylamino)-äthyl]-benzoesäure-methylester

Hergestellt aus 5-Chlor-2-piperidino-benzoesäure und 4-(2-Aminoäthyl)-benzoesäure-methylester analog Beispiel 1.
Ausbeute : 72 % der Theorie,
Schmelzpunkt : 98 °C
Ber. : C 66,00 H 6,29 N 7,00
Gef. : 66,00 6,37 6,81

Beispiel 27

4-[2-(5-Chlor-2-piperidino-benzoylamino)-äthyl]-benzoesäure

Hergestellt aus 4-[2-(5-Chlor-2-piperidino-benzoylamino)-äthyl]-benzoesäure-methylester durch alkalische Hydrolyse analog Beispiel 2.
Ausbeute : 82 % der Theorie,
Schmelzpunkt : 200 °C
Ber. : C 65,20 H 5,98 N 7,24
Gef. : 65,10 6,00 7,30

Beispiel 28

4-[2-(5-Chlor-2-(2-methyl-piperidino)-benzoylamino)-äthyl]-benzoesäure-methylester

Hergestellt aus 5-Chlor-2-(2-methyl-piperidino)-benzoesäure und 4-(2-Aminoäthyl)-benzoesäure-methylester analog Beispiel 1.
Ausbeute : 23 % der Theorie,
Schmelzpunkt : 82 °C

| | | | |
|---|---|---|---|
| Ber. : | C 66,57 | H 6,56 | N 6,75 |
| Gef. : | 66,82 | 6,42 | 6,78 |

Beispiel 29

4-[2-(5-Chlor-2-(2-methyl-piperidino)-benzoylamino)-äthyl]-benzoesäure

Hergestellt aus 4-[2-(5-Chlor-2-(2-methyl-piperidino)-benzoyl-amino-äthyl]-benzoesäure-methylester durch alkalische Verseifung analog Beispiel 2.
Ausbeute : 57 % der Theorie,
Schmelzpunkt : 178 °C

| | | | |
|---|---|---|---|
| Ber. : | C 65,91 | H 6,29 | N 6,99 |
| Gef. : | 65,73 | 6,28 | 7,13 |

Beispiel 30

4-[2-(5-Chlor-2-(3-methyl-piperidino)-benzoylamino)-äthyl]-benzoesäure-methylester

Hergestellt aus 5-Chlor-2-(3-methyl-piperidino)-benzoesäure und 4-(2-Amino-äthyl)-benzoesäure-methylester analog Beispiel 1.
Ausbeute : 51 % der Theorie,
Schmelzpunkt : 93 °C

| | | | |
|---|---|---|---|
| Ber. : | C 66,57 | H 6,56 | N 6,75 |
| Gef. : | 66,52 | 6,38 | 6,81 |

Beispiel 31

4-[2-(5-Chlor-2-(3-methyl-piperidino)-benzoylamino)-äthyl]-benzoesäure

Hergestellt aus 4-[2-(5-Chlor-2-(3-methyl-piperidino)-benzoyl-amino)-äthyl]-benzoesäure-methylester durch alkalische Hydrolyse analog Beispiel 2.
Ausbeute : 83 % der Theorie,
Schmelzpunkt : 194 °C

| | | | |
|---|---|---|---|
| Ber. : | C 65,91 | H 6,29 | N 6,99 |
| Gef. : | 66,20 | 6,25 | 6,95 |

Beispiel 32

4-[2-(5-Chlor-2-(4-methyl-piperidino)-benzoylamino)-äthyl]-benzoesäure-methylester

Hergestellt aus 5-Chlor-2-(4-methyl-piperidino)-benzoesäure und 4-(2-Amino-äthyl)-benzoesäure-methylester analog Beispiel 1.
Ausbeute : 48 % der Theorie,
Schmelzpunkt : 55 °C

| | | | |
|---|---|---|---|
| Ber. : | C 66,57 | H 6,56 | N 6,75 |
| Gef. : | 66,38 | 6,35 | 6,82 |

Beispiel 33

4-[2-(5-Chlor-2-(4-methyl-piperidino)-benzoylamino)-äthyl]-benzoesäure

Hergestellt aus 4-[2-(5-Chlor-2-(4-methyl-piperidino)-benzoylamino)-äthyl]-benzoesäure-methylester durch alkalische Hydrolyse analog Beispiel 2.
Ausbeute : 83 % der Theorie,
Schmelzpunkt : 211 °C

| | | | |
|---|---|---|---|
| Ber. : | C 65,91 | H 6,29 | N 6,99 |
| Gef. : | 66,07 | 6,25 | 7,02 |

Beispiel 34

4-[2-(5-Chlor-2-(5-äthyl-2-methyl-piperidino)-benzoylamino)-äthyl]-benzoesäure-methylester

Hergestellt aus 5-Chlor-2-(5-äthyl-2-methyl-piperidino)-benzoesäure und 4-(2-Amino-äthyl)-benzoesäure-methylester analog Beispiel 1.

Ausbeute : 10 % der Theorie,
Schmelzpunkt : < 20 °C

| | | | |
|---|---|---|---|
| Ber. : | C 67,78 | H 7,05 | N 6,33 |
| Gef. : | 68,00 | 7,10 | 6,50 |

Beispiel 35

4-[2-(5-Chlor-2-(5-äthyl-2-methyl-piperidino)-benzoylamino)-äthyl]-benzoesäure

Hergestellt aus 4-[2-(5-Chlor-2-(5-äthyl-2-methyl-piperidino)-benzoylamino)-äthyl]-benzoesäure-methylester durch alkalische Hydrolyse analog Beispiel 2.

Ausbeute : 60 % der Theorie,
Schmelzpunkt : 40 °C

| | | | |
|---|---|---|---|
| Ber. : | C 67,19 | H 6,81 | N 6,53 |
| Gef. : | 67,30 | 6,98 | 6,42 |

Beispiel 36

4-[2-(5-Chlor-2-(3,5-dimethyl-piperidino)-benzoylamino)-äthyl]-benzoesäure-methylester

Hergestellt aus 5-Chlor-2-(3,5-dimethyl-piperidino)-benzoesäure und 4-(2-Amino-äthyl)-benzoesäure-methylester analog Beispiel 1.

Ausbeute : 85 % der Theorie,
Schmelzpunkt : 95 °C

| | | | |
|---|---|---|---|
| Ber. : | C 67,20 | H 6,81 | N 6,53 |
| Gef. : | 67,14 | 6,62 | 6,68 |

Beispiel 37

4-[2-(5-Chlor-2-(3,5-dimethyl-piperidino)-benzoylamino)-äthyl]-benzoesäure-methylester

2,7 g (0,01 Mol) 5-Chlor-2-(3,5-dimethyl-piperidino)-benzoesäure werden mit 3,57 g (0,03 Mol) Thionylchlorid in 20 ml Chloroform 4,5 Stunden unter Rückfluß erhitzt. Danach wird das Reaktionsgemisch im Vakuum eingeengt. Der Rückstand wird in 10 ml Chloroform gelöst und unter Rühren bei Raumtemperatur in eine Lösung von 2,16 g (0,01 Mol) 4-(2-Amino-äthyl)-benzoesäure-methylesterhydrochlorid und 3, 03 g (0,03 Mol) Triäthylamin, gelöst in 15 ml Chloroform, getropft. Nach 15 Minuten ist die Zugabe beendet. Danach wird noch 30 Minuten zum Rückfluß erhitzt. Nach dem Abkühlen wird das Reaktionsgemisch zweimal mit Wasser und einmal mit verdünnter Essigsäure gewaschen. Die Chloroform-Phase wird über Natriumsulfat getrocknet und eingeengt. Zur weiteren Reinigung wird der erhaltene Rückstand über eine Kieselgel-Säule gegeben (Chloroform/Aceton = 9 : 1). Die reinen Fraktionen werden nach Abdampfen des Lösungsmittels nochmals aus Methylenchlorid/Petroläther (20 : 1) umkristallisiert.

Ausbeute : 3,3 g (77 % der Theorie),
Schmelzpunkt : 94-95 °C

| | | | | |
|---|---|---|---|---|
| Ber. : | C 67,20 | H 6,81 | N 6,53 | Cl 8,27 |
| Gef. : | 67,11 | 6,78 | 6,70 | 8,39 |

Beispiel 38

4-[2-(5-Chlor-2-(3,5-dimethyl-piperidino)-benzoylamino)-äthyl]-benzoesäure

2,15 g (0,005 Mol) 4-[2-(5-Chlor-2-(3,5-dimethyl-piperidino)-benzoylamino)-äthyl]-benzoesäure-methylester werden in 50 ml Äthanol und 10 ml 1N Natronlauge 30 Minuten unter Rückfluß erhitzt. Nach dem Abkühlen wird der größte Teil des Alkohols im Vakuum entfernt und mit 60 ml Wasser versetzt. Beim schwachen Ansäuern mit Essigsäure auf etwa pH 6 fällt ein Niederschlag aus, der nach einigem Stehen abgesaugt und aus Isopropanol umkristallisiert wird.

Ausbeute : 1,82 g (87,5 % der Theorie),
Schmelzpunkt : 204-206 °C

| | | | | |
|---|---|---|---|---|
| Ber. : | C 66,58 | H 6,56 | N 6,75 | Cl 8,55 |
| Gef. : | 66,59 | 6,48 | 6,71 | 8,45 |

Beispiel 39

4-[2-(5-Chlor-2-(4-methoxy-piperidino)-benzoylamino-äthyl]-benzoesäure-äthylester

Hergestellt aus 5-Chlor-2-(4-methoxy-piperidino)-benzoesäure und 4-(2-Aminoäthyl)-benzoesäure-äthylester analog Beispiel 1.
Ausbeute : 60 % der Theorie,
Schmelzpunkt : < 20 °C
Ber. : C 64,78 H 6,57 N 6,30
Gef. : 64,95 6,62 6,15

Beispiel 40

4-[2-(5-Chlor-2-(4-methoxy-piperidino)-benzoylamino)-äthyl]-benzoesäure

Hergestellt aus 4-[2-(5-Chlor-2-(4-methoxy-piperidino)-benzoylamino)-äthyl]-benzoesäure-äthylester durch alkalische Hydrolyse analog Beispiel 2.
Ausbeute : 85 % der Theorie,
Schmelzpunkt : 188 °C
Ber. : C 63,38 H 6,04 N 6,72
Gef. : 63,46 5,95 6,72

Beispiel 41

4-[2-(5-Methoxy-2-piperidino-benzoylamino)-äthyl]-benzoesäure-äthylester

Hergestellt aus 5-Methoxy-2-piperidino-benzoesäure und 4-(2-Aminoäthyl)-benzoesäure-äthylester analog Beispiel 1.
Ausbeute : 66 % der Theorie,
Schmelzpunkt : 118 °C
Ber. : C 70,22 H 7,36 N 6,82
Gef. : 70,76 7,42 7,36

Beispiel 42

4-[2-(5-Methoxy-2-piperidino-benzoylamino)-äthyl]-benzoesäure-hydrochlorid

Hergestellt aus 4-[2-(5-Methoxy-2-piperidino-benzoylamino)-äthyl]-benzoesäure-äthylester durch alkalische Hydrolyse analog Beispiel 2. Die erhaltene Verbindung wurde im Aceton mit isopropanolischer Salzsäure in das Hydrochlorid überführt.
Ausbeute : 91 % der Theorie,
Schmelzpunkt : 158 °C
Ber. : C 63,07 H 6,49 N 6,68
Gef. : 63,00 6,31 6,61

Beispiel 43

4-[2-(5-Chlor-2-heptamethylenimino-benzoylamino)-äthyl]-benzoesäure-äthylester

Hergestellt aus 5-Chlor-2-heptamethylenimino-benzoesäure und 4-(2-Aminoäthyl)-benzoesäure-äthylester analog Beispiel 1.
Ausbeute : 24 % der Theorie,
Schmelzpunkt : < 20 °C
Ber. : C 67,78 H 7,05 N 6,33
Gef. : 67,95 7,16 6,33

Beispiel 44

4-[2-(5-Chlor-2-heptamethylenimino-benzoylamino)-äthyl]-benzoesäure

Hergestellt aus 4-[2-(5-Chlor-2-heptamethylenimino-benzoylamino)-äthyl]-benzoesäure-äthylester durch alkalische Hydrolyse analog Beispiel 2.
Ausbeute : 47 % der Theorie,
Schmelzpunkt : 186 °C
Ber. : C 66,57 H 6,56 N 6,75
Gef. : 66,45 6,43 6,70

Beispiel 45

4-[2-(5-Nitro-2-piperidino-benzoylamino)-äthyl]-benzoesäure-äthylester

Hergestellt aus 5-Nitro-2-piperidino-benzoesäure und 4-(2-Aminoäthyl)-benzoesäure-äthylester analog Beispiel 1.
Ausbeute : 70 % der Theorie,
Schmelzpunkt : 104 °C
Ber. : C 64,92 H 6,40 N 9,87
Gef. : 65,17 6,38 9,67

Beispiel 46

4-[2-(5-Nitro-2-piperidino-benzoylamino)-äthyl]-benzoesäure

Hergestellt aus 4-[2-(5-Nitro-2-piperidino-benzoylamino)-äthyl]-benzoesäure-äthylester durch alkalische Hydrolyse analog Beispiel 2.
Ausbeute : 69 % der Theorie,
Schmelzpunkt : 194-195 °C
Ber. : C 63,46 H 5,83 N 10,57
Gef. : 63,20 5,79 10,38

Beispiel 47

4-[2-(5-Chlor-2-(4-phenyl-piperidino)-benzoylamino)-äthyl]-benzoesäure-methylester

Hergestellt aus 5-Chlor-2-(4-phenyl-piperidino)-benzoesäure und 4-(2-Amino-äthyl)-benzoesäuremethylester analog Beispiel 1.
Ausbeute : 62 % der Theorie,
Schmelzpunkt : 124 °C
Ber. : C 70,50 H 6,13 N 5,87
Gef. : 70,60 6,26 5,84

Beispiel 48

4-[2-(5-Chlor-2-(4-phenyl-piperidino)-benzoylamino)-äthyl]-benzoesäure

Hergestellt aus 4-[2-(5-Chlor-2-(4-phenyl-piperidino)-benzoylamino)-äthyl]-benzoesäure-methylester durch alkalische Hydrolyse analog Beispiel 2.
Ausbeute : 92 % der Theorie,
Schmelzpunkt : 188 °C
Ber. : C 70,04 H 5,88 N 6,05
Gef. : 70,12 6,08 6,05

Beispiel 49

4-[2-(5-Chlor-2-(1,4-dioxa-8-aza-spiro [4,5] decan-yl-(8)-benzoylamino)-äthyl]-benzoesäure-methylester

Hergestellt aus 5-Chlor-2-(1,4-dioxa-8-aza-spiro [4,5] decan-yl-(8))-benzoesäure und 4-(2-Aminoäthyl)-benzoesäure-methylester analog Beispiel 1.
Ausbeute : 45 % der Theorie,
Schmelzpunkt : 167 °C
Ber. : C 62,81 H 5,93 N 6,11
Gef. : 62,80 5,89 5,93

Beispiel 50

4-[2-(5-Chlor-2-(1,4-dioxa-8-aza-spiro [4,5] decan-yl-(8))-benzoylamino)-äthyl]-benzoesäure

Hergestellt aus 4-[2-(5-Chlor-2-(1,4-dioxa-8-aza-spiro [4,5] decanyl-(8))-benzoylamino)-äthyl]-benzoesäure-methylester durch alkalische Hydrolyse analog Beispiel 2.
Ausbeute : 82 % der Theorie,
Schmelzpunkt : 190 °C
Ber. : C 61,95 H 5,88 N 6,28
Gef. : 61,74 6,03 6,52

Beispiel 51

4-[2-(5-Chlor-2-(4-äthoxycarbonyl-piperidino)-benzoylamino)-äthyl]-benzoesäure-methylester

Hergestellt aus 5-Chlor-2-(4-äthoxycarbonyl-piperidino)-benzoesäure und 4-(2-Aminoäthyl)-benzoesäure-methylester analog Beispiel 1.
Ausbeute : 17 % der Theorie,
Schmelzpunkt : 70 °C
Ber. : C 63,48 H 6,18 N 5,92
Gef. : 63,30 6,08 5,91

Beispiel 52

4-[2-(5-Chlor-2-(4-hydroxycarbonyl-piperidino)-benzoylamino)-äthyl]-benzoesäure

Hergestellt aus 4-[2-(5-Chlor-2-(4-äthoxycarbonyl-piperidino)-benzoylamino)-äthyl]-benzoesäure-methylester durch alkalische Hydrolyse analog Beispiel 2.
Ausbeute : 71 % der Theorie,
Schmelzpunkt : 238 °C
Ber. : C 61,32 H 5,38 N 6,50
Gef. : 61,32 5,33 6,71

Beispiel 53

4-[2-(5-Chlor-2-hexamethylenimino-benzoylamino)-äthyl]-benzoesäure-methylester

Hergestellt aus 5-Chlor-2-hexamethylenimino-benzoesäure und 4-(2-Amino-äthyl)-benzoesäure-methylester analog Beispiel 1.
Ausbeute : 72 % der Theorie,
Schmelzpunkt : 81 °C
Ber. : C 66,60 H 6,56 N 6,75
Gef. : 66,28 6,30 6,67

Beispiel 54

4-[2-(5-Chlor-2-hexamethylenimino-benzoylamino)-äthyl]-benzoesäure

Hergestellt aus 4-[2-(5-Chlor-2-hexamethylenimino-benzoylamino)-äthyl]-benzoesäure-methylester durch alkalische Hydrolyse analog Beispiel 2.
Ausbeute : 89 % der Theorie,
Schmelzpunkt : 182 °C
Ber. : C 66,00 H 6,29 N 6,99
Gef. : 66,20 6,40 7,15

Beispiel 55

4-[2-(5-Chlor-2-morpholino-benzoylamino)-äthyl]-benzoesäure-methylester

Hergestellt aus 5-Chlor-2-morpholino-benzoesäure und 4-(2-Amino-äthyl)-benzoesäure-methylester analog Beispiel 1.
Ausbeute : 80 % der Theorie,
Schmelzpunkt : 111 °C
Ber. : C 62,55 H 5,75 N 6,95
Gef. : 62,48 5,74 6,94

Beispiel 56

4-[2-(5-Chlor-2-morpholino-benzoylamino)-äthyl]-benzoesäure

Hergestellt aus 4-[2-(5-Chlor-2-morpholino-benzoylamino)-äthyl]-benzoesäure-methylester durch alkalische Hydrolyse analog Beispiel 2.
Ausbeute : 78 % der Theorie,
Schmelzpunkt : 186 °C
Ber. : C 61,75 H 5,44 N 7,20
Gef. : 61,60 5,41 7,10

Beispiel 57

4-[2-(5-Chlor-2-thiomorpholino-benzoylamino)-äthyl]-benzoesäure-methylester

Hergestellt aus 5-Chlor-2-thiomorpholino-benzoesäure und 4-(2-Amino-äthyl)-benzoesäure-methylester analog Beispiel 1.
Ausbeute : 45 % der Theorie,
Schmelzpunkt : 160 °C
Ber. : C 60,20 H 5,53 N 6,69
Gef. : 60,62 5,76 6,96

Beispiel 58

4-[2-(5-Chlor-2-thiomorpholino-benzoylamino)-äthyl]-benzoesäure

Hergestellt aus 4-[2-(5-Chlor-2-thiomorpholino-benzoylamino)-äthyl]-benzoesäure-methylester durch alkalische Hydrolyse analog Beispiel 2.
Ausbeute : 57 % der Theorie,
Schmelzpunkt : 210 °C
Ber. : C 59,32 H 5,23 N 6,92
Gef. : 59,25 5,19 6,80

Beispiel 59

4-[2-(5-Chlor-2-thiomorpholino-benzoylamino)-äthyl]-benzoesäure-methylester-S-oxyd

Hergestellt aus 5-Chlor-2-thiomorpholino-benzoesäure-S-oxyd und 4-(2-Amino-äthyl)-benzoesäure-methylester analog Beispiel 1.
Ausbeute : 63 % der Theorie,
Schmelzpunkt : 152 °C
Ber. : C 57,99 H 5,33 N 6,44
Gef. : 58,30 5,22 6,52

Beispiel 60

4-[2-(5-Chlor-2-thiomorpholino-benzoylamino)-äthyl]-benzoesäure-S-oxyd

Hergestellt aus 4-[2-(5-Chlor-2-thiomorpholino-benzoylamino)-äthyl]-benzoesäure-methylester-S-oxyd durch alkalische Hydrolyse analog Beispiel 2.
Ausbeute : 82 % der Theorie,
Schmelzpunkt : 202 °C
Ber. : C 57,07 H 5,03 N 6,66
Gef. : 57,46 5,07 6,30

Beispiel 61

4-[2-(5-Chlor-2-[1,2,4,5-tetrahydro-3H-3benzazepin-yl-(3)]-benzoylamino)-äthyl]-benzoesäure-methylester

Hergestellt aus 5-Chlor-2-[1,2,4,5-tetrahydro-3H-3benzazepinyl-(3)]-benzoesäure und 4-(2-Amino-äthyl)-benzoesäure-methylester analog Beispiel 1.
Ausbeute : 69 % der Theorie,
Schmelzpunkt : 126 °C
Ber. : C 70,04 H 5,88 N 6,05
Gef. : 70,20 5,81 5,94

Beispiel 62

4-[2-(5-Chlor-2-[1,2,4,5-tetrahydro-3H-3benzazepin-yl-(3)]-benzoylamino)-äthyl]-benzoesäure

Hergestellt aus 4-[2-(5-Chlor-2-[1,2,4,5-tetrahydro-3H-3-benzazepin-yl-(3)-benzoylamino)-äthyl]-benzoesäure-methylester durch alkalische Hydrolyse analog Beispiel 2.
Ausbeute : 89 % der Theorie,
Schmelzpunkt : 148 °C
Ber. : C 69,55 H 5,61 N 6,24
Gef. : 69,87 5,90 6,10

Beispiel 63

4-[2-(5-Chlor-2-[1,2,3,4-tetrahydro-isochinolin-yl-(2)]-benzoylamino)-äthyl]-benzoesäure-methylester

Hergestellt aus 5-Chlor-2-[1,2,3,4-tetrahydro-isochinolyl-(2)]-benzoesäure und 4-(2-Amino-äthyl)-benzoesäure-methylester analog Beispiel 1.
Ausbeute : 43 % der Theorie,
Schmelzpunkt : 94 °C

| | | | |
|---|---|---|---|
| Ber. : | C 69,55 | H 5,61 | N 6,24 |
| Gef. : | 69,65 | 5,65 | 6,14 |

Beispiel 64

4-[2-(5-Chlor-2-[1,2,3,4-tetrahydro-isochinolin-yl-(2)]-benzoylamino)-äthyl]-benzoesäure

Hergestellt aus 4-[2-(5-Chlor-2-[1,2,3,4-tetrahydro-isochinolinyl-(2)]-benzoylamino)-äthyl]-benzoesäure-methylester durch alkalische Hydrolyse analog Beispiel 2.
Ausbeute : 86 % der Theorie,
Schmelzpunkt : 173 °C

| | | | | |
|---|---|---|---|---|
| Ber. : | C 69,04 | H 5,33 | N 6,44 | Cl 8,15 |
| Gef. : | 68,45 | 5,56 | 6,21 | 8,57 |

Beispiel 65

4-[2-(5-Chlor-2-(4-phenyl-piperazino)-benzoylamino)-äthyl]-benzoesäure-methylester

Hergestellt aus 5-Chlor-2-(4-phenyl-piperazino)-benzoesäure und 4-(2-Amino-äthyl)-benzoesäure-methylester analog Beispiel 1.
Ausbeute : 40 % der Theorie,
Schmelzpunkt : 132 °C

| | | | |
|---|---|---|---|
| Ber. : | C 67,84 | H 5,90 | N 8,79 |
| Gef. : | 67,72 | 5,92 | 8,66 |

Beispiel 66

4-[2-(5-Chlor-2-(4-phenyl-piperazino)-benzoylamino)-äthyl]-benzoesäure

Hergestellt aus 4-[2-(5-Chlor-2-(4-phenyl-piperazino)-benzoylamino)-äthyl]-benzoesäure-methylester durch alkalische Hydrolyse analog Beispiel 2.
Ausbeute : 82 % der Theorie,
Schmelzpunkt : 166 °C

| | | | |
|---|---|---|---|
| Ber. : | C 67,07 | H 5,65 | N 9,06 |
| Gef. : | 67,30 | 5,96 | 8,99 |

Beispiel 67

4-[2-(5-Chlor-2-(4-pyridyl-(2)-piperazino)-benzoylamino)-äthyl]-benzoesäure-methylester

Hergestellt aus 5-Chlor-2-[4-(2-pyridyl)-piperazino]-benzoesäure und 4-(2-Amino-äthyl)-benzoesäure-methylester-hydrochlorid in Gegenwart von Thionylchlorid und Triäthylamin analog Beispiel 37.
Ausbeute : 44,6 % der Theorie,
Schmelzpunkt des Hydrochlorids : 153-154 °C

| | | | | |
|---|---|---|---|---|
| Ber. : | C 60,58 | H 5,48 | N 10,87 | Cl 13,76 |
| Gef. : | 60,31 | 5,52 | 10,68 | 13,93 |

Beispiel 68

4-[2-(5-Chlor-2-(4-pyridyl-(2)-piperazino)-benzoylamino-äthyl]-benzoesäure

Hergestellt aus 4-[2-(5-Chlor-2-(4-pyridyl-(2)-piperazino)-benzoylamino-äthyl]-benzoesäure-methylester durch alkalische Hydrolyse analog Beispiel 2.
Ausbeute : 27 % der Theorie,
Schmelzpunkt : 120 °C (Zers.)

| | | | | |
|---|---|---|---|---|
| Ber. : | C 64,58 | H 5,42 | N 12,05 | Cl 7,63 |
| Gef. : | 64,36 | 5,49 | 11,87 | 7,48 |

Beispiel 69

4-[2-(5-Chlor-2-piperidino-benzoylamino)-1-methyl-äthyl]-benzoesäure-methylester

Hergestellt aus 5-Chlor-2-piperidino-benzoesäure und 4-(2-Amino-1-methyl-äthyl)-benzoesäure-methylester analog Beispiel 1.
Ausbeute : 42,7 % der Theorie,
Schmelzpunkt : 93-94 °C

| | | | | |
|---|---|---|---|---|
| Ber. : | C 66,57 | H 6,56 | Cl 8,54 | N 6,75 |
| Gef. : | 66,82 | 6,57 | 8,47 | 6,58 |

Beispiel 70

4-[2-(5-Chlor-2-piperidino-benzoylamino)-1-methyl-äthyl]-benzoesäure

Hergestellt aus 4-[2-(5-Chlor-2-piperidino-benzoylamino)-1-methyl-äthyl]-benzoesäure-methylester durch alkalische Hydrolyse analog Beispiel 2.
Ausbeute : 76 % der Theorie,
Schmelzpunkt : 192-194 °C

| | | | | |
|---|---|---|---|---|
| Ber. : | C 65,91 | H 6,28 | Cl 8,84 | N 6,99 |
| Gef. : | 66,00 | 6,30 | 8,77 | 6,87 |

Beispiel 71

4-[2-(5-Chlor-2-dimethylamino-benzoylamino)-1-methyl-äthyl]-benzoesäure

Hergestellt aus 4-[2-(5-Chlor-2-dimethylamino-benzoylamino)-1-methyl-äthyl]-benzoesäure-methylester durch alkalische Hydrolyse analog Beispiel 2.
Ausbeute : 86 % der Theorie,
Schmelzpunkt : 159-161 °C

| | | | | |
|---|---|---|---|---|
| Ber. : | C 63,23 | H 5,87 | Cl 9,83 | N 7,76 |
| Gef. : | 63,42 | 6,07 | 9,56 | 7,67 |

Beispiel 72

4-[2-(5-Chlor-2-dimethylamino-benzoylamino)-1-methyl-äthyl]-benzoesäure-methylester

Hergestellt aus 5-Chlor-2-dimethylamino-benzoesäure und 4-(2-Amino-1-methyl-äthyl)-benzoesäure-methylester analog Beispiel 1.
Ausbeute : 61,5 % der Theorie,
Schmelzpunkt : 79-80 °C

| | | | | |
|---|---|---|---|---|
| Ber. : | C 64,07 | H 6,18 | Cl 9,46 | N 7,47 |
| Gef. : | 64,40 | 6,52 | 9,14 | 7,20 |

Beispiel 73

4-[2-(5-Amino-2-piperidino-benzoylamino)-äthyl]-benzoesäure-äthylester

30 g (70,5 mMol) 4-[2-(5-Nitro-2-piperidino-benzoylamino)-äthyl]-benzoesäure-äthylester werden in 500 ml Methanol : Äthanol (1 : 1) und Palladium-Kohle als Katalysator, bei Raumtemperatur und einem Wasserstoffdruck von 5 bar hydriert. Nach Abtrennen des Katalysators und Abdestillieren des Lösungsmittels wird die Verbindung durch Filtration über Kieselgel mit Essigsäureäthylester als Laufmittel gereinigt.
Ausbeute : 93 % der Theorie,
Schmelzpunkt : < 20 °C

| | | | |
|---|---|---|---|
| Ber. : | C 69,84 | H 7,39 | N 10,62 |
| Gef. : | 70,10 | 7,20 | 10,43 |

Beispiel 74

4-[2-(5-Amino-2-piperidino-benzoylamino)-äthyl]-benzoesäure-dihydrochlorid

Hergestellt aus 4-[2-(5-Amino-2-piperidino-benzoylamino)-äthyl]-benzoesäure-äthylester durch alkalische Hydrolyse analog Beispiel 2. Anschließend wird das Produkt in Aceton mit isopropanolischer Salzsäure in das Dihydrochlorid überführt.

Ausbeute : 87 % der Theorie,
Schmelzpunkt : 70 °C

| | | | |
|---|---|---|---|
| Ber. : | C 57,26 | H 6,18 | N 9,54 |
| Gef. : | 57,40 | 6,30 | 9,52 |

Beispiel 75

4-[2-(5-Acetamino-2-piperidino-benzoylamino)-äthyl]-benzoesäure-äthylester

Hergestellt aus 2,5 g (6,3 mMol) 4-[2-(5-Amino-2-piperidinobenzoylamino)-äthyl]-benzoesäure-äthylester und 25 ml Essigsäureanhydrid bei Raumtemperatur. Das Reaktionsprodukt fällt aus und wird mit Äther nachgewaschen.
Ausbeute : 1,9 g (69 % der Theorie),
Schmelzpunkt : 165 °C

| | | | |
|---|---|---|---|
| Ber. : | C 68,62 | H 7,14 | N 9,60 |
| Gef. : | 68,92 | 7,09 | 9,50 |

Beispiel 76

4-[2-(5-Acetamino-2-piperidino-benzoylamino)-äthyl]-benzoesäure

Hergestellt aus 4-[2-(5-Acetamino-2-piperidino-benzoylamino)-äthyl]-benzoesäure-äthylester durch alkalische Hydrolyse analog Beispiel 2.
Ausbeute : 98 %der Theorie,
Schmelzpunkt : 212 °C

| | | | |
|---|---|---|---|
| Ber. : | C 67,46 | H 6,64 | N 10,26 |
| Gef. : | 67,00 | 6,67 | 10,04 |

Beispiel 77

4-[2-(5-Dimethylaminosulfonyl-2-piperidino-benzoylamino)-äthyl]-benzoesäure-methylester

Hergestellt aus 5-Dimethylaminosulfonyl-2-piperidino-benzoesäure und 4-(2-Äthylamino)-benzoesäure-methylester analog Beispiel 1.
Ausbeute : 77 % der Theorie,
Schmelzpunkt : 138-140 °C

| | | | | |
|---|---|---|---|---|
| Ber. : | C 60,87 | H 6,60 | N 8,87 | S 6,77 |
| Gef. : | 61,08 | 6,67 | 8,86 | 6,80 |

Beispiel 78

4-[2-(5-Dimethylaminosulfonyl-2-piperidino-benzoylamino)-äthyl]-benzoesäure

Hergestellt aus 4-[2-(5-Dimethylaminosulfonyl-2-piperidino-benzoylamino)-äthyl]-benzoesäure-methylester durch alkalische Verseifung analog Beispiel 2.
Ausbeute : 91 % der Theorie,
Schmelzpunkt : 220 °C

| | | | | |
|---|---|---|---|---|
| Ber. : | C 60,11 | H 6,36 | N 9,14 | S 6,98 |
| Gef. : | 60,30 | 6,42 | 8,96 | 6,98 |

Beispiel 79

4-[2-(5-Cyano-2-piperidino-benzoylamino)-äthyl]-benzoesäure-äthylester

Hergestellt aus 5-Cyano-2-piperidino-benzoesäure und 4-(2-Aminoäthyl)-benzoesäure-äthylester analog Beispiel 1.
Ausbeute : 76 % der Theorie,
Schmelzpunkt : 97 °C

| | | | |
|---|---|---|---|
| Ber. : | C 71,08 | H 6,71 | N 10,36 |
| Gef. : | 71,37 | 6,74 | 10,33 |

Beispiel 80

4-[2-(5-Cyano-2-piperidino-benzoylamino)-äthyl]-benzoesäure

Hergestellt aus 4-[2-(5-Cyano-2-piperidino-benzoylamino)-äthyl]-benzoesäure-äthylester durch alkalische Hydrolyse analog Beispiel 2.
Ausbeute : 40 % der Theorie,
Schmelzpunkt : 190 °C

| | | | |
|---|---|---|---|
| Ber. : | C 70,00 | H 6,14 | N 11,13 |
| Gef. : | 69,81 | 6,03 | 10,98 |

Beispiel 81

4-[2-(5-Äthoxycarbonyl-2-piperidino-benzoylamino)-äthyl]-benzoesäure-äthylester-hydrochlorid

2,5 g (6,2 mMol) 4-[2-(5-Cyano-2-piperidino-benzoylamino)-äthyl]-benzoesäure-äthylester werden in 80 ml Äthanol gelöst und mit Chlorwasserstoff gesättigt. Nach sechstägigem Stehen bei Raumtemperatur wird das Lösungsmittel eingedampft, der Rückstand in Eiswasser gelöst, mit Natronlauge auf pH 9 gestellt und mit Chloroform extrahiert. Nach Trocknung über Natriumsulfat und Abdestillieren des Lösungsmittels wird der Abdampfrückstand in Äther mit ätherischer Salzsäure in das Hydrochlorid überführt.
Ausbeute : 2,5 g (89,1 % der Theorie),
Schmelzpunkt : 86-88 °C

| | | | | |
|---|---|---|---|---|
| Ber. : | C 63,85 | H 6,80 | N 5,72 | Cl 7,24 |
| Gef. : | 63,71 | 6,69 | 5,74 | 7,11 |

Beispiel 82

4-[2-(5-Hydroxycarbonyl-2-piperidino-benzoylamino)-äthyl]-benzoesäure

Hergestellt durch alkalische Hydrolyse von 4-[2-(5-Äthoxycarbonyl-2-piperidino-benzoylamino)-äthyl]-benzoesäure-äthyl-ester-hydrochlorid analog Beispiel 2.
Ausbeute : 92 % der Theorie,
Schmelzpunkt : 242 °C

| | | | |
|---|---|---|---|
| Ber. : | C 66,65 | H 6,10 | N 7,06 |
| Gef. : | 65,96 | 6,18 | 7,23 |

Beispiel 83

4-[2-(2-(4-Hydroxy-piperidino-)-5-nitro-benzoylamino)-äthyl]-benzoesäure-methylester

a) 4-[2-(2-Chlor-5-nitro-benzoylamino)-äthyl]-benzoesäure-methylester

8 g (40 mMol) 2-Chlor-5-nitro-benzoesäure werden in 40 ml absolutem Tetrahydrofuran mit 6,8 g (44 mMol) N,N'-Carbonyldiimidazol in das Imidazolid überführt. Nach einer Reaktionszeit von 2 Stunden werden 7,9 g (44 mMol) 4-(2-Amino-äthyl)-benzoesäure-methylester bei Raumtemperatur zugesetzt und noch ca. 16 Stunden gerührt. Nach Abdestillieren des Lösungsmittels wird das Rohprodukt über eine Kieselgelsäule mit Toluol : Essigsäureäthylester (1 : 1) als Laufmittel chromatographisch gereinigt.
Ausbeute : 12 g (83 % der Theorie),
Schmelzpunkt : 163 °C

| | | | |
|---|---|---|---|
| Ber. : | C 56,28 | H 4,17 | N 7,72 |
| Gef. : | 56,58 | 4,41 | 7,82 |

b) 4-[2-(2-(4-Hydroxy-piperidino)-5-nitro-benzoylamino)-äthyl]-benzoesäure-methylester

Eine Lösung von 5 g (14 mMol) 4-[2-(2-Chlor-5-nitro-benzoylamino)-äthyl]-benzoesäure-methylester in 100 ml Äthanol wird mit 2,83 (28 mMol) 4-Hydroxy-piperidin 14 Stunden aud Rückflußtemperatur erhitzt. Nach Abdestillieren des Lösungsmittels im Rotationsverdampfer, wird der Trockenrückstand in Eiswasser gelöst, mit verdünnter Salzsäure auf pH 5 gestellt und mit Chloroform extrahiert. Nach Trocknung über Natriumsulfat, Abdestillation des Chloroforms wird der Rückstand aus Isopropanol kristallisiert.
Ausbeute : 5,5 g (92 % der Theorie),
Schmelzpunkt : 147 °C

Beispiel 84

4-[2-(5-Amino-2-(4-hydroxy-piperidino)-benzoylamino)-äthyl]-benzoesäure-methylester

5,3 g (12,4 mMol) 4-[2-(2-(4-Hydroxy-piperidino)-5-nitro-benzoylamino)-äthyl]-benzoesäure-methylester werden in 100 ml Methanol mit 10 %iger Palladium-Kohle bei Raumtemperatur und einem

Wasserstoffdruck von 1 bar hydriert.
Ausbeute : 91 % der Theorie,
Schmelzpunkt : 78 °C

## Beispiel 85

4-[2-(5-Chlor-2-(4-hydroxy-piperidino)-benzoylamino)-äthyl]-benzoesäure-methylester

20,5 mg (52 mMol) 4-[2-(5-Amino-2-(4-hydroxy-piperidino)-benzoylamino)-äthyl]-benzoesäure-methylester werden in 80 ml halbkonzentrierter eiskalter Salzsäure gelöst und mit einer Lösung von 4 g Natriumnitrit in 25 ml eiskaltem Wasser diazotiert. Diese Lösung wird zu einer Suspension von 6 g Kupferpulver und 10 ml HCl zugetropft. Nach beendeter Stickstoffentwicklung fällt ein zähes Öl aus. Dieses Öl wird mit Chloroform extrahiert und nach Trocknung über Natriumsulfat über eine Kieselgelsäule mit dem Laufmittel Essigsäureäthylester : Methanol (9 : 1) gereinigt.
Ausbeute : 30 % der Theorie,
Schmelzpunkt : < 20 °C

| | | | |
|---|---|---|---|
| Ber. : | C 63,38 | H 6,04 | N 6,72 |
| Gef. : | 63,62 | 6,21 | 6,55 |

## Beispiel 86

4-[2-(5-Chlor-2-(4-hydroxy-piperidino)-benzoylamino)-äthyl]-benzoesäure

4,5 g (11,3 mMol) 4-[2-(5-Amino-2-(4-hydroxy-piperidino)-benzoylamino)-äthyl]-benzoesäure-methylester werden in 20 ml halbkonzentrierter Salzsäure gelöst und bei 0 °C mit 0,87 g (12,4 mMol) Natriumnitrit, gelöst in 6 ml Wasser, diazotiert. Diese Lösung wird zu einer Suspension von 1,2 g Kupferpulver in 3 ml konzentrierter Salzsäure zugetropft. Nach 2-stündigem Rühren wird für ca. 20 Minuten auf 75 °C erhitzt. Die abgekühlte Lösung wird mit Chloroform extrahiert, die Chloroformlösung mit Natriumsulfat getrocknet und der Abdampfrückstand über eine Kieselgelsäule mit Chloroform : Methanol (9 : 1) als Laufmittel gereinigt.
Ausbeute : 2,4 g (52 % der Theorie),
Schmelzpunkt : 190 °C

| | | | |
|---|---|---|---|
| Ber. : | C 62,6 | H 5,75 | N 6,93 |
| Gef. : | 62,14 | 5,84 | 6,83 |

## Beispiel 87

4-[2-(5-Chlor-2-(3-hydroxy-piperidino)-benzoylamino)-äthyl]-benzoesäure-methylester

Hergestellt aus 4-[2-(5-Amino-2-(3-hydroxy-piperidino)-benzoyl-amino)-äthyl]-benzoesäure-methylester analog Beispiel 85.
Ausbeute : 30 % der Theorie,
Schmelzpunkt : 20 °C

| | | | |
|---|---|---|---|
| Ber. : | C 63,38 | H 6,04 | N 6,72 |
| Gef. : | 63,48 | 6,21 | 6,65 |

## Beispiel 88

4-[2-(5-Chlor-2-(3-hydroxy-piperidino)-benzoylamino)-äthyl]-benzoesäure-hydrat

Hergestellt aus 4-[2-(5-Amino-2-(3-hydroxy-piperidino)-benzoylamino)-äthyl]-benzoesäure-methylester analog Beispiel 86.
Ausbeute : 40 % der Theorie,
Schmelzpunkt : 100-110 °C

| | | | |
|---|---|---|---|
| Ber. : | C 59,93 | H 5,98 | N 6,65 |
| Gef. : | 60,19 | 6,08 | 6,62 |

## Beispiel 89

4-[2-(2-(3-Hydroxy-piperidino)-5-nitro-benzoylamino)-äthyl]-benzoesäure-methylester

Hergestellt aus 4-[2-(2-Chlor-5-nitro-benzoylamino)-äthyl]-benzoesäure-methylester und 3-Hydroxy-piperidin analog Beispiel 83.
Ausbeute : 43,5 % der Theorie,
Schmelzpunkt : 78-80 °C

## Beispiel 90

4-[2-(5-Amino-2-(3-hydroxy-piperidino)-benzoylamino)-äthyl]-benzoesäure-methylester

Hergestellt aus 4-[2-(3-Hydroxy-piperidino)-5-nitro-benzoylamino)-äthyl]-benzoesäure-methylester durch katalytische Hydrierung analog Beispiel 84.

Ausbeute : 90 % der Theorie,
Schmelzpunkt : 82 °C

## Beispiel 91

4-[2-(2-Piperidino-5-propyloxy-benzoylamino)-äthyl]-benzoesäure-äthylester

Zu einer Lösung von 2 g (7,6 mMol) 2-Piperidino-5-propyloxybenzoesäure in 60 ml absolutem Tetrahydrofuran werden 1,3 g (8 mMol) N,N'-Carbonyl-diimidazol zugesetzt und anschließend unter Ausschluß von Feuchtigkeit etwa 10-14 Stunden auf Rückflußtemperatur erhitzt. Nachdem sich das Imidazolid fast quantitativ gebildet hat, wird die Lösung mit 1,5 g (8 mMol) 4-(2-Amino-äthyl)-benzoesäure-äthylester versetzt und weitere 4-6 Stunden zum Sieden erhitzt. Nach Abdestillieren des Tetrahydrofurans am Rotationsverdampfer wird der rohe Ester über eine Kieselgelsäule mit Toluol/Essigester (9 : 1) als Fließmittel chromatographisch gereinigt. Die Fraktionen, die den gereinigten Ester enthalten, werden vereinigt und das Lösungsmittel abdestilliert.

Ausbeute : 2,4 g (72 % der Theorie),
Schmelzpunkt : < 20 °C

| | | | |
|---|---|---|---|
| Ber. : | C 71,20 | H 7,81 | N 6,38 |
| Gef. : | 71,30 | 8,02 | 6,54 |

## Beispiel 92

4-[2-(2-Piperidino-5-propyloxy-benzoylamino)-äthyl]-benzoesäure-hydrochlorid

1,8 g (4,1 mMol) 4-[2-(2-Piperidino-5-propyloxy-benzoylamino)-äthyl]-benzoesäure-äthylester werden in einer Mischung aus 15 ml Methanol und 15 ml Dioxan gelöst. Bei Raumtemperatur wird 1 ml 30 %ige Natronlauge, verdünnt mit 30 ml Wasser, unter Rühren so langsam zu der Esterlösung zugetropft, daß es zu keiner bleibenden Ausfällung des Esters kommt. Die Zugabe ist nach ca. 2 Stunden beendet. Nach einigen Stunden Nachrühren werden die organischen Lösungsmittel am Rotationsverdampfer abdestilliert, die wässrige Phase mit Chloroform ausgeschüttelt und die wässrige Phase mit 2 N Salzsäure auf pH 4 gestellt. Nach Extraktion mit Chloroform, Trocknung der Chloroformphase und Abdestillieren des Chloroforms wird aus einer Lösung in Aceton das Hydrochlorid mit isopropanolischer Salzsäure ausgefällt.

Ausbeute : 1,65 g (90 % der Theorie),
Schmelzpunkt : 236 °C

| | | | | |
|---|---|---|---|---|
| Ber. : | C 64,48 | H 7,00 | N 6,26 | Cl 7,93 |
| Gef. : | 64,24 | 7,06 | 6,17 | 8,13 |

## Beispiel 93

4-[2-(5-Isopropyloxy-2-piperidino-benzoylamino)-äthyl]-benzoesäure-äthylester

Hergestellt aus 5-Isopropyloxy-2-piperidino-benzoesäure und 4-(2-Amino-äthyl)-benzoesäure-äthylester analog Beispiel 91.

Ausbeute : 58 % der Theorie,
Schmelzpunkt : 70-72 °C

| | | | |
|---|---|---|---|
| Ber. : | C 71,20 | H 7,81 | N 6,38 |
| Gef. : | 71,10 | 7,74 | 6,40 |

## Beispiel 94

4-[2-(5-Isopropyloxy-2-piperidino-benzoylamino)-äthyl]-benzoesäure-hydrochlorid

Hergestellt aus 4-[2-(5-Isopropyloxy-2-piperidino-benzoylamino)-äthyl]-benzoesäure-äthylester durch alkalische Hydrolyse analog Beispiel 92.

Ausbeute : 82 % der Theorie,
Schmelzpunkt : 225-226 °C

| | | | | |
|---|---|---|---|---|
| Ber. : | C 64,48 | H 6,98 | N 6,26 | Cl 7,93 |
| Gef. : | 64,77 | 7,30 | 6,40 | 7,79 |

Beispiel 95

4-[2-(5-Hexyloxy-2-piperidino-benzoylamino)-äthyl]-benzoesäure-äthylester

Hergestellt aus 5-Hexyloxy-2-piperidino-benzoesäure und 4-(2-Amino-äthyl)-benzoesäure-äthylester analog Beispiel 92.
Ausbeute : 61 % der Theorie,
Schmelzpunkt : 66 °C

| | | | |
|---|---|---|---|
| Ber. : | C 72,47 | H 8,38 | N 5,82 |
| Gef. : | 72,68 | 8,29 | 5,87 |

Beispiel 96

4-[2-(5-Hexyloxy-2-piperidino-benzoylamino)-äthyl]-benzoesäure-hydrochlorid

Hergestellt aus 4-[2-(5-Hexyloxy-2-piperidino-benzoylamino)-äthyl]-benzoesäure-äthylester durch alkalische Hydrolyse analog Beispiel 92.
Ausbeute : 87 % der Theorie,
Schmelzpunkt : 152-154 °C

| | | | | |
|---|---|---|---|---|
| Ber. : | C 66,30 | H 7,62 | N 5,72 | Cl 7,24 |
| Ger. : | 66,43 | 7,98 | 5,77 | 7,26 |

Beispiel 97

4-[2-(5-Benzyloxy-2-piperidino-benzoylamino)-äthyl]-benzoesäure-äthylester

Hergestellt aus 5-Benzyloxy-2-piperidino-benzoesäure und 4-(2-Amino-äthyl)-benzoesäure-äthylester analog Beispiel 91.
Ausbeute : 55 % der Theorie,
Schmelzpunkt : 120 °C

| | | | |
|---|---|---|---|
| Ber. : | C 74,04 | H 7,04 | N 5,75 |
| Gef. : | 73,90 | 7,14 | 6,03 |

Beispiel 98

4-[2-(5-Benzyloxy-2-piperidino-benzoylamino)-äthyl]-benzoesäure

Hergestellt aus 4-[2-(5-Benzyloxy-2-piperidino-benzoylamino)-äthyl]-benzoesäure-äthylester durch alkalische Hydrolyse analog Beispiel 92.
Ausbeute : 86 % der Theorie,
Schmelzpunkt : 176 °C

| | | | |
|---|---|---|---|
| Ber. : | C 73,34 | H 6,59 | N 6,10 |
| Gef. : | 73,34 | 6,76 | 6,13 |

Beispiel 99

4-[2-(5-Chlor-2-piperidino-benzoylamino)-2-methyl-äthyl]-benzoesäure-methylester

Hergestellt aus 5-Chlor-2-piperidino-benzoesäure und 4-(2-Aminopropyl)-benzoesäure-methylester analog Beispiel 91.
Ausbeute : 54 % der Theorie,
Schmelzpunkt : < 20 °C.

Beispiel 100

4-[2-(5-Chlor-2-piperidino-benzoylamino)-2-methyl-äthyl]-benzoesäure

Hergestellt aus 4-[2-(5-Chlor-2-piperidino-benzoylamino)-2-methyl-äthyl]-benzoesäure-methylester durch alkalische Hydrolyse analog Beispiel 92.
Ausbeute : 49 % der Theorie,
Schmelzpunkt : 142-145 °C

| | | | | |
|---|---|---|---|---|
| Ber. : | C 65,90 | H 6,28 | Cl 8,84 | N 6,98 |
| Gef. : | 65,85 | 6,45 | 8,83 | 6,99 |

Beispiel 101

4-[2-(3-Chlor-2-piperidino-benzoylamino)-äthyl]-benzoesäure-methylester

Hergestellt aus 3-Chlor-2-piperidino-benzoesäure und 4-(2-Amino-äthyl)-benzoesäure-methylester analog Beispiel 91.
Ausbeute : 63 % der Theorie,
Schmelzpunkt : 92-94 °C
Ber. : C 65,91 H 6,28 Cl 8,85 N 6,99
Gef. : 65,71 6,19 9,07 6,93

Beispiel 102

4-[2-(3-Chlor-2-piperidino-benzoylamino)-äthyl]-benzoesäure

Hergestellt aus 4-[2-(3-Chlor-2-piperidino-benzoylamino)-äthyl]-benzoesäure-methylester durch alkalische Hydrolyse analog Beispiel 92.
Ausbeute : 57 % der Theorie,
Schmelzpunkt : 155-158 °C
Ber. : C 65,19 H 5,99 Cl 9,17 N 7,24
Gef. : 64,96 5,99 9,38 7,50

Beispiel 103

4-[2-(4-Chlor-2-piperidino-benzoylamino)-äthyl]-benzoesäure-äthylester

Hergestellt aus 4-Chlor-2-piperidino-benzoesäure und 4-(2-Amino-äthyl)-benzoesäure-äthylester analog Beispiel 91.
Ausbeute : 71 % der Theorie,
Schmelzpunkt : < 20 °C
Ber. : C 66,58 H 6,56 N 8,54 Cl 6,75
Gef. : 66,58 6,57 8,42 6,99

Beispiel 104

4-[2-(4-Chlor-2-piperidino-benzoylamino)-äthyl]-benzoesäure

Hergestellt aus 4-[2-(4-Chlor-2-piperidino-benzoylamino)-äthyl]-benzoesäure-äthylester durch alkalische Verseifung analog Beispiel 92.
Ausbeute : 66,7 % der Theorie,
Schmelzpunkt : 164-166 °C
Ber. : C 65,20 H 5,99 N 9,16 Cl 7,24
Gef. : 65,31 5,96 9,25 7,48

Beispiel 105

4-[2-(5-Brom-2-(2-methyl-piperidino)-benzoylamino)-äthyl]-benzoesäure-äthylester

Hergestellt aus 5-Brom-2-(2-methyl-piperidino)-benzoesäure und 4-(2-Amino-äthyl)-benzoesäure-äthylester analog Beispiel 91.
Ausbeute : 74 % der Theorie,
Schmelzpunkt : 98 °C
Ber. : C 60,89 H 6,18 N 5,92
Gef. : 60,99 6,43 5,78

Beispiel 106

4-[2-(5-Brom-2-(2-methyl-piperidino)-benzoylamino)-äthyl]-benzoesäure

Hergestellt aus 4-[2-(5-Brom-2-(2-methyl-piperidino)-benzoylamino)-äthyl]-benzoesäure-äthylester durch alkalische Hydrolyse analog Beispiel 92.
Ausbeute : 73 % der Theorie,
Schmelzpunkt : 183 °C
Ber. : C 65,91 H 6,29 N 6,99
Gef. : 65,70 6,35 6,80

Beispiel 107

4-[2-(5-Chlor-2-(3,5-trans-dimethyl-piperidino)-benzoylamino)-äthyl]-benzoesäure-methylester

Hergestellt aus 5-Chlor-2-(3,5-trans-dimethyl-piperidino)-benzoesäure und 4-(2-Amino-äthyl)-benzoesäure-äthylester analog Beispiel 91.
Ausbeute : 75 % der Theorie,
Schmelzpunkt : 105-107 °C

| | | | | |
|---|---|---|---|---|
| Ber. : | C 67,20 | H 6,81 | N 6,53 | Cl 8,27 |
| Gef. : | 67,40 | 6,92 | 6,47 | 8,26 |

Beispiel 108

4-[2-(5-Chlor-2-(3,5-trans-dimethyl-piperidino)-benzoylamino)-äthyl]-benzoesäure

Hergestellt aus 4-[2-(5-Chlor-2-(3,5-trans-dimethyl-piperidino)-benzoylamino)-äthyl]-benzoesäure-methylester durch alkalische Hydrolyse analog Beispiel 92.
Ausbeute : 86 % der Theorie,
Schmelzpunkt : 164-167 °C

| | | | | |
|---|---|---|---|---|
| Ber. : | C 66,58 | H 6,56 | N 6,75 | Cl 8,55 |
| Gef. : | 66,80 | 6,71 | 6,65 | 8,63 |

Beispiel 109

4-[2-(5-Brom-2-(3,5-cis-dimethyl-piperidino)-benzoylamino)-äthyl]-benzoesäure-methylester

Hergestellt aus 5-Brom-2-(3,5-cis-dimethyl-piperidino)-benzoesäure und 4-(2-Amino-äthyl)-benzoesäure-methylester analog Beispiel 91.
Ausbeute : 82 % der Theorie,
Schmelzpunkt : 142-144 °C

| | | | | |
|---|---|---|---|---|
| Ber. : | C 60,89 | H 6,17 | N 5,92 | Cl 16,88 |
| Gef. : | 60,81 | 6,08 | 5,85 | 16,72 |

Beispiel 110

4-[2-(5-Brom-2-(3,5-cis-dimethyl-piperidino)-benzoylamino)-äthyl]-benzoesäure

Hergestellt aus 4-[2-(5-Brom-2-(3,5-cis-dimethyl-piperidino)-benzoylamino)-äthyl]-benzoesäure-methylester durch alkalische Hydrolyse analog Beispiel 92.
Ausbeute : 88 % der Theorie,
Schmelzpunkt : 184-185 °C

| | | | | |
|---|---|---|---|---|
| Ber. : | C 60,13 | H 5,92 | N 6,10 | Cl 17,40 |
| Gef. : | 59,98 | 5,87 | 6,02 | 17,30 |

Beispiel 111

4-[2-(5-Methoxy-2-(3,5-cis-dimethyl-piperidino)-benzoylamino)-äthyl]-benzoesäure-äthylester

Hergestellt aus 5-Methoxy-2-(3,5-cis-dimethyl-piperidino)-benzoesäure und 2-(Amino-äthyl)-benzoesäure-äthylester analog Beispiel 91.
Ausbeute : 14 % der Theorie,
Schmelzpunkt : 97-99 °C

| | | | |
|---|---|---|---|
| Ber. : | C 71,21 | H 7,81 | N 6,39 |
| Gef. : | 71,29 | 7,78 | 6,16 |

Beispiel 112

4-[2-(5-Methoxy-2-(3,5-cis-dimethyl-piperidino)-benzoylamino)-äthyl]-benzoesäure

Hergestellt aus 4-[2-(5-Methoxy-2-(3,5-cis-dimethyl-piperidino)-benzoylamino)-äthyl]-benzoesäure-äthylester durch alkalische Hydrolyse analog Beispiel 92.
Ausbeute : 53 % der Theorie,
Schmelzpunkt : 200-203 °C

| | | | |
|---|---|---|---|
| Ber. : | C 70,22 | H 7,37 | N 6,82 |
| Gef. : | 70,22 | 7,38 | 6,82 |

Beispiel 113

4-[2-(5-Chlor-2-(3,3,5,5-tetramethyl-piperidino)-benzoylamino)-äthyl]-benzoesäure-äthylester

Hergestellt aus 5-Chlor-2-(3,3,5,5-tetramethyl-piperidino)-benzoesäure und 4-(2-Amino-äthyl)-benzoesäure-äthylester analog Beispiel 91.
Ausbeute : 34 % der Theorie,
Schmelzpunkt : 108 °C

| | | | | |
|---|---|---|---|---|
| Ber. : | C 68,85 | H 7,49 | N 5,94 | Cl 7,52 |
| Gef. : | 68,75 | 7,37 | 5,79 | 7,69 |

Beispiel 114

4-[2-(5-Chlor-2-(3,3,5,5-tetramethyl-piperidino)-benzoylamino)-äthyl]-benzoesäure

Hergestellt aus 4-[2-(5-Chlor-2-(3,3,5,5-tetramethyl-piperidino)-benzoylamino)-äthyl]-benzoesäure-äthylester durch alkalische Hydrolyse analog Beispiel 92.
Ausbeute : 99 % der Theorie,
Schmelzpunkt : 170-172 °C

| | | | | |
|---|---|---|---|---|
| Ber. : | C 67,78 | H 7,05 | N 6,32 | Cl 8,00 |
| Gef. : | 67,60 | 7,09 | 6,13 | 7,95 |

Beispiel 115

4-[2-(5-Chlor-2-(piperidon-(2)-yl-(1))-benzoylamino)-äthyl]-benzoesäure-äthylester

Hergestellt aus 5-Chlor-2-(piperidon-(2)-yl-(1))-benzoesäure und 4-(2-Amino-äthyl)-benzoesäure-äthylester analog Beispiel 91.
Ausbeute : 42 % der Theorie,
Schmelzpunkt : 118 °C

| | | | |
|---|---|---|---|
| Ber. : | C 64,40 | H 5,88 | N 6,53 |
| Gef. : | 64,32 | 5,87 | 6,58 |

Beispiel 116

4-[2-(5-Chlor-2-(piperidon-(2)-yl-(1))-benzoylamino)-äthyl]-benzoesäure-hydrat

Hergestellt aus 4-[2-(5-Chlor-2-(piperidon-(2)-yl-(1))-benzoylamino)-äthyl]-benzoesäure-äthylester durch alkalische Hydrolyse analog Beispiel 92.
Ausbeute : 64 % der Theorie,
Schmelzpunkt : 190-193 °C

| | | | |
|---|---|---|---|
| Ber. : | C 60,48 | H 5,56 | N 6,72 |
| Gef. : | 60,74 | 5,47 | 6,96 |

Beispiel 117

4-[2-(5-Brom-2-(4-methoxy-piperidino)-benzoylamino)-äthyl]-benzoesäure-äthylester

Hergestellt aus 5-Brom-2-(4-methoxy-piperidino)-benzoesäure und 4-(2-Amino-äthyl)-benzoesäure-äthylester analog Beispiel 91.
Ausbeute : 82 % der Theorie,
Schmelzpunkt : < 20 °C

| | | | |
|---|---|---|---|
| Ber. : | C 58,90 | H 5,97 | N 5,72 |
| Gef. : | 59,25 | 6,08 | 5,27 |

Beispiel 118

4-[2-(5-Brom-2-(4-methoxy-piperidino)-benzoylamino)-äthyl]-benzoesäure

Hergestellt aus 4-[2-(5-Brom-2-(4-methoxy-piperidino)-benzoylamino)-äthyl]-benzoesäure-äthylester durch alkalische Hydrolyse analog Beispiel 92.
Ausbeute : 65 % der Theorie,
Schmelzpunkt : 186 °C

| | | | |
|---|---|---|---|
| Ber. : | C 57,28 | H 5,46 | N 6,07 |
| Gef. : | 56,40 | 5,46 | 5,85 |

Beispiel 119

4-[2-(5-Chlor-2-(2,6-dimethyl-morpholino)-benzoylamino)-äthyl]-benzoesäure-äthylester

Hergestellt aus 5-Chlor-2-(2,6-dimethyl-morpholino)-benzoesäure und 4-(2-Amino-äthyl)-benzoesäureäthylester analog Beispiel 91.
Ausbeute : 52 % der Theorie,
Schmelzpunkt : 111-112 °C
Ber. : C 64,78 H 6,57 N 6,30
Gef. : 64,72 6,64 6,24

Beispiel 120

4-[2-(5-Chlor-2-(2,6-dimethyl-morpholino)-benzoylamino)-äthyl]-benzoesäure

Hergestellt aus 4-[2-(5-Chlor-2-(2,6-dimethyl-morpholino)-benzoylamino)-äthyl]-benzoesäure-äthylester durch alkalische Hydrolyse analog Beispiel 92.
Ausbeute : 86 % der Theorie,
Schmelzpunkt : 232 °C
Ber. : C 63,38 H 6,04 N 6,72
Gef. : 63,38 6,28 6,69

Beispiel 121

4-[2-(5-Chlor-2-(2,6-dimethyl-thiomorpholino)-benzoylamino)-äthyl]-benzoesäure-äthylester

Hergestellt aus 5-Chlor-2-(2,6-dimethyl-thiomorpholino)-benzoesäure und 4-(2-Amino-äthyl)-benzoesäure-äthylester analog Beispiel 91.
Ausbeute : 71 % der Theorie,
Schmelzpunkt : < 20 °C
Ber. : C 62,53 H 6,34 N 6,08
Gef. : 62,60 6,52 6,08

Beispiel 122

4-[2-(5-Chlor-2-(2,6-dimethyl-thiomorpholino)-benzoylamino)-äthyl]-benzoesäure

Hergestellt aus 4-[2-(5-Chlor-2-(2,6-dimethyl-thiomorpholino)-benzoylamino)-äthyl]-benzoesäure-äthylester durch alkalische Hydrolyse analog Beispiel 92.
Ausbeute : 51 % der Theorie,
Schmelzpunkt : 183-185 °C
Ber. : C 61,03 H 5,82 N 6,47
Gef. : 60,62 6,01 6,30

Beispiel 123

4-[2-(5-Chlor-2-(thiomorpholino-1,1-dioxyd)-benzoylamino)-äthyl]-benzoesäure-äthylester

Hergestellt aus 5-Chlor-2-(thiomorpholino-1,1-dioxyd)-benzoesäure und 4-(2-Amino-äthyl)-benzoesäure-äthylester analog Beispiel 91.
Ausbeute : 40 % der Theorie,
Schmelzpunkt : 158 °C
Ber. : C 56,83 H 5,42 N 6,02
Gef. : 56,78 5,78 6,15

Beispiel 124

4-[2-(2-Hexamethylenimino-5-methoxy-benzoylamino)-äthyl]-benzoesäure-äthylester

Hergestellt aus 2-Hexamethylenimino-5-methoxy-benzoesäure und 4-(2-Amino-äthyl)-benzoesäure-äthylester analog Beispiel 91.
Ausbeute : 46 % der Theorie,
Schmelzpunkt : 92 °C
Ber. : C 70,73 H 7,60 N 6,60
Gef. : 70,46 7,53 6,72

Beispiel 125

4-[2-(2-Hexamethylenimino-5-methoxy-benzoylamino)-äthyl]-benzoesäure-hydrochlorid

Hergestellt aus 4-[2-(2-Hexamethylenimino-5-methoxy-benzoyl-amino)-äthyl]-benzoesäure-äthylester durch alkalische Hydrolyse analog Beispiel 92.
Ausbeute : 92 % der Theorie,
Schmelzpunkt : 126 °C

| | | | | |
|---|---|---|---|---|
| Ber. : | C 63,30 | H 6,75 | N 6,46 | Cl 8,18 |
| Gef. : | 63,80 | 7,24 | 6,57 | 7,62 |

Beispiel 126

4-[2-(2-Heptamethylenimino-5-methoxy-benzoylamino)-äthyl]-benzoesäure-äthylester

Hergestellt aus 2-Heptamethylenimino-5-methoxy-benzoesäure und 4-(2-Amino-äthyl)-benzoesäure-äthylester analog Beispiel 91.
Ausbeute : 30 % der Theorie,
Schmelzpunkt : < 20 °C

Beispiel 127

4-[2-(2-Heptamethylenimino-5-methoxy-benzoylamino)-äthyl]-benzoesäure-hydrochlorid

Hergestellt aus 4-[2-(2-Heptamethylenimino-5-methoxy-benzoylamino)-äthyl]-benzoesäure-äthylester durch alkalische Verseifung analog Beispiel 92.
Ausbeute : 97 % der Theorie,
Schmelzpunkt : 110-112 °C

| | | | |
|---|---|---|---|
| Ber. : | C 59,68 | H 6,88 | N 5,80 |
| Gef. : | 60,80 | 6,87 | 5,63 |

Beispiel 128

4-[2-(2-Heptamethylenimino-5-isopropyloxy-benzoylamino)-äthyl]-benzoesäure-äthylester

Hergestellt aus 2-Heptamethylenimino-5-isopropyloxy-benzoesäure und 4-(2-Amino-äthyl)-benzoesäure-äthylester analog Beispiel 92.
Ausbeute : 62 % der Theorie,
Schmelzpunkt : < 20 °C

| | | | |
|---|---|---|---|
| Ber. : | C 72,07 | H 8,20 | N 6,00 |
| Gef. : | 72,20 | 8,16 | 5,90 |

Beispiel 129

4-[2-(2-Heptamethylenimino-5-isopropyloxy-benzoylamino)-äthyl]-benzoesäure-hydrochlorid

Hergestellt aus 4-[2-(2-Heptamethylenimino-5-isopropyloxy-benzoylamino)-äthyl]-benzoesäure-äthylester durch alkalische Hydrolyse analog Beispiel 92.
Ausbeute : 91 % der Theorie,
Schmelzpunkt : 202 °C

| | | | | |
|---|---|---|---|---|
| Ber. : | C 65,73 | H 7,42 | N 5,90 | Cl 7,46 |
| Gef. : | 65,85 | 7,58 | 5,82 | 7,23 |

Beispiel 130

4-[2-(5-Brom-2-heptamethylenimino-benzoylamino)-äthyl]-benzoesäure-äthylester

Hergestellt aus 5-Brom-2-heptamethylenimino-benzoesäure und 4-(2-Amino-äthyl)-benzoesäure-äthylester analog Beispiel 91.
Ausbeute : 42,5 % der Theorie,
Schmelzpunkt : 81 °C

| | | | | |
|---|---|---|---|---|
| Ber. : | C 61,80 | H 6,50 | N 5,78 | Br 16,40 |
| Gef. : | 61,60 | 6,40 | 5,74 | 16,40 |

Beispiel 131

4-[2-(5-Brom-2-heptamethylenimino-benzoylamino)-äthyl]-benzoesäure

Hergestellt aus 4-[2-(5-Brom-2-heptamethylenimino-benzoylamino)-äthyl]-benzoesäure-äthylester durch alkalische Hydrolyse analog Beispiel 92.
Ausbeute : 94 % der Theorie,
Schmelzpunkt : 189 °C

| | | | | |
|---|---|---|---|---|
| Ber. : | C 60,13 | H 5,92 | N 6,10 | Br 17,39 |
| Gef. : | 59,97 | 6,01 | 6,05 | 17,51 |

Beispiel 132

4-[2-(2-(3-Aza-bicyclo [3,2,2] nonan-3-yl)-5-chlor-benzoylamino)-äthyl]-benzoesäure-äthylester

Hergestellt aus 2-(3-Aza-bicyclo [3,2,2] nonan-3-yl)-5-chlor-benzoesäure und 4-(2-Amino-äthyl)-benzoesäure-äthylester analog Beispiel 91.
Ausbeute : 82 % der Theorie,
Schmelzpunkt : 114 °C

| | | | |
|---|---|---|---|
| Ber. : | C 68,63 | H 6,87 | N 6,16 |
| Gef. : | 68,78 | 7,08 | 6,16 |

Beispiel 133

4-[2-(2-(3-Aza-bicyclo [3,2,2] nonan-3-yl)-5-chlor-benzoylamino)-äthyl]-benzoesäure-hydrochlorid

Hergestellt aus 4-[2-(2-(3-Aza-bicyclo [3,2,2] nonan-3-yl)-5-chlor-benzoylamino)-äthyl]-benzoesäure-äthylester durch alkalische Hydrolyse analog Beispiel 92.
Ausbeute : 98 % der Theorie,
Schmelzpunkt : 158 °C

| | | | | |
|---|---|---|---|---|
| Ber. : | C 62,20 | H 6,03 | N 6,05 | Cl 15,30 |
| Gef. : | 62,76 | 6,37 | 6,07 | 15,25 |

Beispiel 134

4-[2-(5-Chlor-2-octamethylenimino-benzoylamino)-äthyl]-benzoesäure-äthylester

Hergestellt aus 5-Chlor-2-octamethylenimino-benzoesäure und 4-(2-Amino-äthyl)-benzoesäure-äthylester analog Beispiel 91.
Ausbeute : 41 % der Theorie,
Schmelzpunkt : < 20 °C

| | | | |
|---|---|---|---|
| Ber. : | C 68,33 | H 7,28 | N 6,13 |
| Gef. : | 68,15 | 7,10 | 6,09 |

Beispiel 135

4-[2-(5-Chlor-2-octamethylenimino-benzoylamino)-äthyl]-benzoesäure

Hergestellt aus 4-[2-(5-Chlor-2-octamethylenimino-benzoylamino)-äthyl]-benzoesäure-äthylester durch alkalische Hydrolyse analog Beispiel 92.
Ausbeute : 94 % der Theorie,
Schmelzpunkt : 172 °C

| | | | |
|---|---|---|---|
| Ber. : | C 67,20 | H 6,81 | N 6,53 |
| Gef. : | 66,90 | 6,76 | 6,39 |

Beispiel 136

4-[2-(5-Chlor-2-nonamethylenimino-benzoylamino)-äthyl]-benzoesäure-äthylester

Hergestellt aus 5-Chlor-2-nonamethylenimino-benzoesäure und 4-(2-Amino-äthyl)-benzoesäure-äthylester analog Beispiel 91.
Ausbeute : 24 % der Theorie,
Schmelzpunkt : < 20 °C

| | | | | |
|---|---|---|---|---|
| Ber. : | C 69,14 | H 7,09 | N 5,97 | Cl 7,56 |
| Gef. : | 69,38 | 7,28 | 6,13 | 7,88 |

Beispiel 137

4-[2-(5-Chlor-2-nonamethylenimino-benzoylamino)-äthyl]-benzoesäure

Hergestellt aus 4-[2-(5-Chlor-2-nonamethylenimino-benzoylamino)-äthyl]-benzoesäure-äthylester durch alkalische Hydrolyse analog Beispiel 92.
Ausbeute : 82 % der Theorie,
Schmelzpunkt : 173-174 °C
Ber. : C 68,00 H 6,63 N 6,35 Cl 8,04
Gef. : 67,62 6,78 6,23 7,60

Beispiel 138

4-[2-(5-Chlor-2-decamethylenimino-benzoylamino)-äthyl]-benzoesäure-äthylester

Hergestellt aus 5-Chlor-2-decamethylenimino-benzoesäure und 4-(2-Amino-äthyl)-benzoesäure-äthylester analog Beispiel 91.
Ausbeute : 65 % der Theorie,
Schmelzpunkt : < 20 °C
Ber. : C 69,33 H 7,69 N 5,78
Gef. : 69,29 7,64 5,92

Beispiel 139

4-[2-(5-Chlor-2-decamethylenimino-benzoylamino)-äthyl]-benzoesäure

Hergestellt aus 4-[2-(5-Chlor-2-decamethylenimino-benzoylamino)-äthyl]-benzoesäure-äthylester durch alkalische Hydrolyse analog Beispiel 92.
Ausbeute : 58 % der Theorie,
Schmelzpunkt : 177 °C
Ber. : C 68,33 H 7,28 N 6,13
Gef. : 68,43 7,28 6,41

Beispiel 140

4-[2-(5-Chlor-2-undecamethylenimino-benzoylamino)-äthyl]-benzoesäure-äthylester

Hergestellt aus 5-Chlor-2-undecamethylenimino-benzoesäure und 4-(2-Amino-äthyl)-benzoesäure-äthylester analog Beispiel 91.
Ausbeute : 71 % der Theorie,
Schmelzpunkt : 80 °C
Ber. : C 69,79 H 7,88 N 5,61
Gef. : 69,35 7,66 5,84

Beispiel 141

4-[2-(5-Chlor-2-undecamethylenimino-benzoylamino)-äthyl]-benzoesäure

Hergestellt aus 4-[2-(5-Chlor-2-undecamethylenimino-benzoylamino)-äthyl]-benzoesäure-äthylester durch alkalische Hydrolyse analog Beispiel 92.
Ausbeute : 77 % der Theorie,
Schmelzpunkt : 218-220 °C
Ber. : C 68,84 H 7,49 N 5,95
Gef. : 68,34 7,21 6,19

Beispiel 142

4-[2-(5-Chlor-2-dodecamethylenimino-benzoylamino)-äthyl]-benzoesäure-äthylester

Hergestellt aus 5-Chlor-2-dodecamethylenimino-benzoesäure und 4-(2-Amino-äthyl)-benzoesäure-äthylester analog Beispiel 92.
Ausbeute : 53 % der Theorie,
Schmelzpunkt : < 20 °C
Ber. : C 70,22 H 8,05 N 5,46
Gef. : 70,56 7,77 5,71

Beispiel 143

4-[2-(5-Chlor-2-dodecamethylenimino-benzoylamino)-äthyl]-benzoesäure

Hergestellt aus 4-[2-(5-Chlor-2-dodecamethylenimino-benzoylamino)-äthyl]-benzoesäure-äthylester durch alkalische Verseifung analog Beispiel 92.
Ausbeute : 33 % der Theorie,
Schmelzpunkt : 185-187 °C
Ber. : C 69,33 H 7,69 N 5,78
Gef. : 69,17 7,54 5,95

Beispiel 144

4-[2-(5-Chlor-2-(N-methyl-anilino)-benzoylamino)-äthyl]-benzoesäure-äthylester

Hergestellt aus 5-Chlor-2-(N-methyl-anilino)-benzoesäure und 4-(2-Amino-äthyl)-benzoesäure-äthylester analog Beispiel 91.
Ausbeute : 70 % der Theorie,
Schmelzpunkt : < 20 °C

Beispiel 145

4-[2-(5-Chlor-2-(N-methyl-anilino)-benzoylamino)-äthyl]-benzoesäure

Hergestellt aus 4-[2-(5-Chlor-2-(N-methyl-anilino)-benzoylamino)-äthyl]-benzoesäure-äthylester durch alkalische Hydrolyse analog Beispiel 92.
Ausbeute : 65 % der Theorie,
Schmelzpunkt : 186-188 °C
Ber. : C 67,56 H 5,80 N 6,85
Gef. : 67,81 5,66 6,87

Beispiel 146

4-[2-(2-(N-Äthyl-cyclohexylamino)-5-chlor-benzoylamino)-äthyl]-benzoesäure-äthylester

Hergestellt aus 2-(N-Äthyl-cyclohexylamino)-5-chlor-benzoesäure und 4-(2-Amino-äthyl)-benzoesäure-äthylester analog Beispiel 91.
Ausbeute : 41 % der Theorie,
Schmelzpunkt : 94 °C
Ber. : C 68,33 H 7,28 N 6,13
Gef. : 68,00 7,10 6,03

Beispiel 147

4-[2-(2-(N-Äthyl-cyclohexylamino)-5-chlor-benzoylamino)-äthyl]-benzoesäure

Hergestellt aus 4-[2-(2-(N-Äthyl-cyclohexylamino)-5-chlor-benzoylamino)-äthyl]-benzoesäure-äthylester durch alkalische Hydrolyse analog Beispiel 92.
Ausbeute : 63 % der Theorie,
Schmelzpunkt : 163 °C
Ber. : C 67,20 H 6,81 N 6,53
Gef. : 67,25 6,67 6,45

Beispiel 148

4-[2-(2-N-Butyl-cyclohexylamino)-5-chlor-benzoylamino)-äthyl]-benzoesäure-äthylester

Hergestellt aus 2-(N-Butyl-cyclohexylamino)-5-chlor-benzoesäure und 4-(2-Amino-äthyl)-benzoesäure-äthylester analog Beispiel 91.
Ausbeute : 46 % der Theorie,
Schmelzpunkt : 77 °C
Ber. : C 69,33 H 7,60 N 5,78
Gef. : 69,12 7,69 5,78

Beispiel 149

4-[2-(2-(N-Butyl-cyclohexylamino)-5-chlor-benzoylamino)-äthyl]-benzoesäure

Hergestellt aus 4-[2-(2-(N-Butyl-cyclohexylamino)-5-chlor-benzoylamino)-äthyl]-benzoesäure-äthylester durch alkalische Hydrolyse analog Beispiel 92.
Ausbeute : 79 % der Theorie,
Schmelzpunkt : 85 °C
Ber. : C 68,33 H 7,28 N 6,13
Gef. : 68,44 7,18 6,40

Beispiel 150

4-[2-(5-Chlor-2-(N-isobutyl-cyclohexylamino)-benzoylamino)-äthyl]-benzoesäure-äthylester

Hergestellt aus 5-Chlor-2-(N-isobutyl-cyclohexylamino)-benzoesäure und 4-(2-Amino-äthyl)-benzoesäure-äthylester analog Beispiel 91.
Ausbeute : 46 % der Theorie,
Schmelzpunkt : 83 °C
Ber. : C 69,33 H 7,69 N 5,78
Gef. : 69,17 7,54 5,66

Beispiel 151

4-[2-(5-Chlor-2-(N-isobutyl-cyclohexylamino)-benzoylamino)-äthyl]-benzoesäure

Hergestellt aus 4-[2-(5-Chlor-2-(N-isobutyl-cyclohexylamino)-benzoylamino)-äthyl]-benzoesäure-äthylester durch alkalische Verseifung analog Beispiel 92.
Ausbeute : 62 % der Theorie,
Schmelzpunkt : 129 °C
Ber. : C 68,33 H 7,28 N 6,13
Gef. : 68,30 7,18 6,17

Beispiel 152

4-[2-(5-Chlor-2-dimethylamino-benzoylamino)-2-methyl-äthyl]-benzoesäure-methylester

Hergestellt aus 5-Chlor-2-dimethylamino-benzoesäure und 4-(2-Amino-propyl)-benzoesäure-methylester analog Beispiel 91.
Ausbeute : 34 % der Theorie,
Schmelzpunkt : < 20 °C

Beispiel 153

4-[2-(5-Chlor-2-dimethylamino-benzoylamino)-2-methyl-äthyl]-benzoesäure

Hergestellt aus 4-[2-(5-Chlor-2-dimethylamino-benzoylamino)-2-methyl-äthyl]-benzoesäure-methylester durch alkalische Hydrolyse analog Beispiel 92.
Ausbeute : 49 % der Theorie,
Schmelzpunkt : 142-145 °C
Ber. : C 63,22 H 5,86 N 7,76
Gef. : 62,90 5,98 7,54

Beispiel 154

4-[2-(3-Chlor-2-dimethylamino-benzoylamino)-äthyl]-benzoesäure-methylester

Hergestellt aus 3-Chlor-2-dimethylamino-benzoesäure und 4-(2-Amino-äthyl)-benzoesäure-methylester analog Beispiel 91.
Ausbeute : 76 % der Theorie,
Schmelzpunkt : < 20 °C

Beispiel 155

4-[2-(3-Chlor-2-dimethylamino-benzoylamino)-äthyl]-benzoesäure

Hergestellt aus 4-[2-(3-Chlor-2-dimethylamino-benzoylamino)-äthyl]-benzoesäure-methylester durch alkalische Hydrolyse analog Beispiel 92.
Ausbeute : 76 % der Theorie,
Schmelzpunkt : 175-178 °C
Ber. : C 62,33 H 5,52 N 8,08 Cl 10,22
Gef. : 62,60 5,56 8,26 10,27

Beispiel 156

4-[2-(2-Dimethylamino-5-methoxy-benzoylamino)-äthyl]-benzoesäure-methylester

Hergestellt aus 2-Dimethylamino-5-methoxy-benzoesäure und 4-(2-Amino-äthyl)-benzoesäure-methylester analog Beispiel 91.
Ausbeute : 81 % der Theorie,
Schmelzpunkt : < 20 °C

Beispiel 157

4-[2-(2-Dimethylamino-5-methoxy-benzoylamino)-äthyl]-benzoesäure

Hergestellt aus 4-[2-(2-Dimethylamino-5-methoxy-benzoylamino)-äthyl]-benzoesäure-methylester durch alkalische Hydrolyse analog Beispiel 92.
Ausbeute : 71 % der Theorie,
Schmelzpunkt : 147-150 °C
Ber. : C 66,65 H 6,48 N 8,18
Gef. : 66,85 6,35 8,12

Beispiel 158

4-[2-(4-Chlor-2-dimethylamino-benzoylamino)-äthyl]-benzoesäure-äthylester

3,55 g (10 mMol) 4-[2-(4-Amino-2-dimethylamino-benzoylamino)-äthyl]-benzoesäure-äthylester (Schmelzpunkt : 135-136 °C, hergestellt durch Hydrierung von 4-[2-(2-Dimethylamino-4-nitro-benzoylamino)-äthyl]-benzoesäure-äthylester mit Palladium/Kohle) werden in 4 ml konz. Salzsäure und ca. 5 g Eis durch tropfenweise Zugabe einer Lösung von 820 mg (11,6 mMol) Natriumnitrit in 5 ml Wasser diazotiert. Nach 30 Minuten wird die Diazoniumsalzlösung bei Raumtemperatur zu einer Suspension von 1,2 g Kupferbronze in 1 ml konz. Salzsäure getropft. Nach Stehen über Nacht wird mit Wasser verdünnt und mit Chloroform extrahiert. Die eingeengten Chloroformextrakte werden an Kieselgel säulenchromatographisch gereinigt (Fließmittel : Toluol/Essigester = 1 : 1 ; $R_f$ = 0,75).
Ausbeute : 27 % der Theorie,
Schmelzpunkt : 97-99 °C
Ber. : C 64,08 H 6,18 N 9,46 Cl 7,47
Gef. : 64,37 6,42 9,32 7,44

Beispiel 159

4-[2-(4-Chlor-2-dimethylamino-benzoylamino)-äthyl]-benzoesäure

Hergestellt durch alkalische Verseifung von 4-[2-(4-Chlor-2-dimethylamino-benzoylamino)-äthyl]-benzoesäure-äthylester analog Beispiel 92.
Ausbeute : 95 % der Theorie,
Schmelzpunkt : 163-165 °C
Ber. : C 62,34 H 5,52 N 10,22 Cl 8,08
Gef. : 61,92 5,55 10,60 8,15

Beispiel 160

4-[2-(2-Dimethylamino-4-nitro-benzoylamino)-äthyl]-benzoesäure-äthylester

Hergestellt analog Beispiel 91 aus 2-Dimethylamino-4-nitro-benzoesäure und 4-(2-Amino-äthyl)-benzoesäure-äthylester.
Ausbeute : 95 % der Theorie,
Schmelzpunkt : 145 °C
Ber. : C 62,33 H 6,01 N 10,90
Gef. : 62,73 6,00 11,09

Beispiel 161

4-[2-(6-Chlor-2-dimethylamino-benzoylamino)-äthyl]-benzoesäure-methylester

Hergestellt aus 6-Chlor-2-dimethylamino-benzoesäure und 4-(2-Amino-äthyl)-benzoesäure-methylester analog Beispiel 91.

Ausbeute : 25 % der Theorie,
Schmelzpunkt : 115-117 °C

| | | | | |
|---|---|---|---|---|
| Ber. : | C 63,24 | H 5,87 | N 7,76 | Cl 9,83 |
| Gef. : | 63,33 | 5,73 | 7,90 | 9,92 |

Beispiel 162

4-[2-(6-Chlor-2-dimethylamino-benzoylamino)-äthyl]-benzoesäure

Hergestellt aus 4-[2-(6-Chlor-2-dimethylamino-benzoylamino)-äthyl]-benzoesäure-methylester durch alkalische Hydrolyse analog Beispiel 92.

Ausbeute : 35 % der Theorie,
Schmelzpunkt : 155-158 °C

| | | | | |
|---|---|---|---|---|
| Ber. : | C 62,34 | H 5,52 | N 8,08 | Cl 10,22 |
| Gef. : | 62,98 | 5,73 | 7,92 | 10,11 |

Beispiel 163

4-[2-[2-(4-Benzyl-piperazino)-5-chlor-benzoylamino]-äthyl]-benzoesäure-äthylester

Hergestellt aus 2-(4-Benzyl-piperazino)-5-chlor-benzoesäure und 4-(2-Amino-äthyl)-benzoesäure-äthylester analog Beispiel 91.

Ausbeute : 39 % der Theorie,
schmelzpunkt : < 20 °C

| | | | | |
|---|---|---|---|---|
| Ber. : | C 68,83 | H 6,37 | N 7,00 | Cl 8,30 |
| Gef. : | 68,97 | 6,52 | 6,93 | 8,21 |

Beispiel 164

4-[2-[2-Piperazino-benzoylamino]-äthyl]-benzoesäure-äthylester

Hergestellt aus 4-[2-[2-(4-Benzyl-piperazino)-5-chlor-benzoylamino]-äthyl]-benzoesäure-äthylester durch katalytische Hydrierung mit Palladium/Kohle bei Raumtemperatur und einem Wasserstoffdruck von 1 bar.

Ausbeute : 60 % der Theorie,
Schmelzpunkt : < 20 °C

| | | | |
|---|---|---|---|
| Ber. : | C 69,27 | H 7,13 | N 11,01 |
| Gef. : | 69,22 | 7,11 | 10,90 |

Beispiel 165

4-[2-(5-Chlor-2-(decahydro-isochinolin-2-yl)-benzoylamino)-äthyl]-benzoesäure-äthylester

In 30 ml absolutem Tetrahydrofuran werden 2,5 g (8,5 mMol) 5-Chlor-2-(decahydro-isochinolin-2-yl)-benzoesäure mit 1,4 g (8,6 mMol) Carbonyldiimidazol 8 Stunden zum Sieden erhitzt. Zu dem gebildeten Imidazolid werden 1,64 g (8,6 mMol) 4-(2-Amino-äthyl)-benzoesäure-äthylester zugesetzt und das Reaktionsgemisch wird weitere 12 Stunden auf Rückflußtemperatur erwärmt. Nach Abdestillieren des Lösungsmittels wird der Ester über eine Kieselgelsäule mit dem Fließmittel Toluol/Essigester (9 : 1) chromatographisch gereinigt.

Ausbeute : 2,7 g (68 % der Theorie),
Schmelzpunkt : < 20 °C.

| | | | | |
|---|---|---|---|---|
| Ber. : | C 69,14 | H 7,09 | N 5,97 | Cl 7,55 |
| Gef. : | 69,43 | 6,98 | 6,10 | 7,76 |

Beispiel 166

4-[2-(5-Chlor-2-(decahydro-isochinolin-2-yl)-benzoylamino)-äthyl]-benzoesäure-hydrochlorid

1,9 g (4,1 mMol) 4-[2-(5-Chlor-2-(decahydro-isochinolin-2-yl)-benzoylamino)-äthyl]-benzoesäure-

äthylester werden in 40 ml einer Mischung von Dioxan und Methanol (1 : 1) gelöst. Nach Zugabe von 5 ml 30 %iger Natronlauge, verdünnt mit 20 ml Wasser, wird bei Raumtemperatur hydrolysiert. Nach einigen Stunden werden die organischen Lösungsmittel abdestilliert. Nach Extraktion mit Chloroform wird die wässrige Phase mit 2N Salzsäure auf pH 4-5 gestellt und die Säure, die bei diesem pH ausgefällt, mit Chloroform extrahiert. Nach Trocknung und Abdestillieren des Chloroforms wird in Aceton das Hydrochlorid mit isopropanolischer Salzsäure ausgefällt.

Ausbeute : 1,5 g (77 % der Theorie),
Schmelzpunkt : 226 °C.

| | | | | |
|---|---|---|---|---|
| Ber. : | C 62,88 | H 6,33 | N 5,86 | Cl 14,85 |
| Gef. : | 62,60 | 6,36 | 5,93 | 14,76 |

Beispiel 167

4-[2-(5-Chlor-2-(decahydro-3-benzazepin-3-yl)-benzoylamino)-äthyl]-benzoesäure-äthylester

Hergestellt aus 5-Chlor-2-(decahydro-3-benzazepin-3-yl)-benzoesäure und 4-(2-Amino-äthyl)-benzoesäure-äthylester analog Beispiel 165.

Ausbeute : 97 % der Theorie,
Schmelzpunkt : < 20 °C.

| | | | | |
|---|---|---|---|---|
| Ber. : | C 69,62 | H 7,30 | N 5,80 | Cl 7,33 |
| Gef. : | 69,88 | 7,22 | 5,63 | 7,46 |

Beispiel 168

4-[2-(5-Chlor-2-(decahydro-3-benzazepin-3-yl)-benzoylamino)-äthyl]-benzoesäure-hydrochlorid

Hergestellt aus 4-[2-(5-Chlor-2-(decahydro-3-benzazepin-3-yl)-benzoylamino)-äthyl]-benzoesäure-äthylester durch alkalische Hydrolyse analog Beispiel 166.

Ausbeute : 84 % der Theorie,
Schmelzpunkt : 216 °C

| | | | | |
|---|---|---|---|---|
| Ber. : | C 63,53 | H 6,56 | N 5,69 | Cl 14,42 |
| Gef. : | 63,51 | 6,59 | 5,76 | 14,35 |

Beispiel 169

4-[2-(5-Brom-2-(decahydro-3-benzazepin-3-yl)-benzoylamino)-äthyl]-benzoesäure-äthylester

Hergestellt aus 5-Brom-2-(decahydro-3-benzazepin-3-yl)-benzoesäure und 4-(2-Amino-äthyl)-benzoesäure-äthylester analog Beispiel 165.

Ausbeute : 98 % der Theorie,
Schmelzpunkt : < 20 °C.

| | | | | |
|---|---|---|---|---|
| Ber. : | C 63,75 | H 6,68 | N 5,31 | Br 15,14 |
| Gef. : | 64,06 | 6,56 | 5,16 | 15,00 |

Beispiel 170

4-[2-(5-Brom-2-(decahydro-3-benzazepin-3-yl)-benzoylamino)-äthyl]-benzoesäure-hydrochlorid

Hergestellt aus 4-[2-(5-Brom-2-(decahydro-3-benzazepin-3-yl)-benzoylamino)-äthyl]-benzoesäure-äthylester durch alkalische Hydrolyse analog Beispiel 166.

Ausbeute : 84 % der Theorie,
Schmelzpunkt : 216 °C.

| | | | | | |
|---|---|---|---|---|---|
| Ber. : | C 58,26 | H 6,01 | N 5,22 | Cl 6,61 | Br 14,91 |
| Gef. : | 58,22 | 5,85 | 5,34 | 6,65 | 15,00 |

Beispiel 171

4-[2-(5-Chlor-2-(octahydro-isoindol-2-yl)-benzoylamino)-äthyl]-benzoesäure-äthylester

Hergestellt aus 5-Chlor-2-(octahydro-isoindol-2-yl)-benzoesäure und 4-(2-Amino-äthyl)-benzoesäure-äthylester analog Beispiel 165.

Ausbeute : 72 % der Theorie,
Schmelzpunkt : < 20 °C.

| | | | | |
|---|---|---|---|---|
| Ber. : | C 68,63 | H 6,86 | N 6,15 | Cl 7,79 |
| Gef. : | 68,72 | 6,92 | 6,20 | 7,94 |

Beispiel 172

4-[2-(5-Chlor-2-(octahydro-isoindol-2-yl)-benzoylamino)-äthyl]-benzoesäure-hydrochlorid

Hergestellt aus 4-[2-(5-Chlor-2-(octahydro-isoindol-2-yl)-benzoylamino)-äthyl]-benzoesäure-äthylester durch alkalische Hydrolyse analog Beispiel 166.
Ausbeute : 83 % der Theorie,
Schmelzpunkt : 214 °C.

| | | | | |
|---|---|---|---|---|
| Ber. : | C 62,20 | H 6,09 | N 6,04 | Cl 15,32 |
| Gef. : | 62,45 | 6,23 | 6,13 | 15,70 |

Beispiel 173

4-[2-(5-Brom-2-octamethylenimino-benzoylamino)-äthyl]-benzoesäure-äthylester

Hergestellt aus 5-Brom-2-octamethylenimino-benzoesäure und 4-(2-Amino-äthyl)-benzoesäure-äthylester analog Beispiel 165.
Ausbeute : 84 % der Theorie,
Schmelzpunkt : < 20 °C.

| | | | | |
|---|---|---|---|---|
| Ber. : | C 62,27 | H 6,63 | N 5,58 | Br 15,93 |
| Gef. : | 62,40 | 6,66 | 5,53 | 15,96 |

Beispiel 174

4-[2-(5-Brom-2-octamethylenimino-benzoylamino)-äthyl]-benzoesäure-hydrochlorid

Hergestellt aus 4-[2-(5-Brom-2-octamethylenimino-benzoylamino)-äthyl]-benzoesäure-äthylester durch alkalische Hydrolyse analog Beispiel 166.
Ausbeute : 90 % der Theorie,
Schmelzpunkt : 164 °C.

| | | | | | |
|---|---|---|---|---|---|
| Ber. : | C 56,53 | H 5,92 | N 5,49 | Br 15,67 | Cl 6,95 |
| Gef. : | 56,82 | 5,96 | 5,47 | 14,85 | 6,59 |

Beispiel 175

4-[2-(5-Cyano-2-octamethylenimino-benzoylamino)-äthyl]-benzoesäure-äthylester

Hergestellt aus 5-Cyano-2-octamethylenimino-benzoesäure und 4-(2-Amino-äthyl)-benzoesäure-äthylester analog Beispiel 165.
Ausbeute : 30 % der Theorie,
Schmelzpunkt : 94 °C

| | | | |
|---|---|---|---|
| Ber. : | C 72,45 | H 7,43 | N 9,38 |
| Gef. : | 72,50 | 7,50 | 9,41 |

Beispiel 176

4-[2-(5-Cyano-2-octamethylenimino-benzoylamino)-äthyl]-benzoesäure

Hergestellt aus 4-[2-(5-Cyano-2-octamethylimino-benzoylamino)-äthyl]-benzoesäure-äthylester durch alkalische Hydrolyse analog Beispiel 166.
Ausbeute : 87 % der Theorie,
Schmelzpunkt : 186 °C

| | | | |
|---|---|---|---|
| Ber. : | C 71,57 | H 6,96 | N 10,01 |
| Gef. : | 71,50 | 7,14 | 9,66 |

Beispiel 177

4-[2-(2-Octamethylenimino-5-nitro-benzoylamino)-äthyl]-benzoesäure-äthylester

Hergestellt aus 2-Octamethylenimino-5-nitro-benzoesäure und 4-(2-Amino-äthyl)-benzoesäure-äthylester analog Beispiel 165.
Ausbeute : 50 % der Theorie,
Schmelzpunkt : 116 °C

| | | | |
|---|---|---|---|
| Ber. : | C 66,79 | H 7,11 | N 8,98 |
| Gef. : | 67,00 | 7,17 | 9,04 |

Beispiel 178

4-[2-(2-Octamethylenimino-5-nitro-benzoylamino)-äthyl]-benzoesäure-hydrochlorid

Hergestellt aus 4-[2-(2-Octamethylenimino-5-nitro-benzoylamino)-äthyl]-benzoesäure-äthylester durch alkalische Hydrolyse analog Beispiel 166.

Ausbeute : 93 % der Theorie,
Schmelzpunkt : 148 °C

| | | | | |
|---|---|---|---|---|
| Ber. : | C 62,06 | H 6,74 | N 9,24 | Cl 4,53 |
| Gef. : | 62,46 | 6,46 | 9,10 | 4,76 |

Beispiel 179

4-[2-(5-Amino-2-octamethylenimino-benzoylamino)-äthyl]-benzoesäure-äthylester-dihydrochlorid

Hergestellt aus 4-[2-(2-Octamethylenimino-5-nitro-benzoylamino)-äthyl]-benzoesäure-äthylester durch katalytische Hydrierung in methanolischer Lösung bei einem Wasserstoffdruck von 5 bar bei Raumtemperatur mit 10 %iger Palladiumkohle als Katalysator.

Ausbeute : 79 % der Theorie,
Schmelzpunkt : 162 °C

| | | | | |
|---|---|---|---|---|
| Ber. : | C 61,16 | H 7,30 | N 8,22 | Cl 13,90 |
| Gef. : | 61,50 | 7,84 | 8,54 | 13,50 |

Beispiel 180

4-[2-(5-Amino-2-octamethylenimino-benzoylamino)-äthyl]-benzoesäure-hydrochlorid

Hergestellt aus 4-[2-(5-Amino-2-octamethylenimino-benzoylamino)-äthyl]-benzoesäure-äthylester-hydrochlorid durch alkalische Hydrolyse analog Beispiel 166.

Ausbeute : 50 % der Theorie,
Schmelzpunkt : 130 °C

| | | | | |
|---|---|---|---|---|
| Ber. : | C 64,62 | H 7,23 | N 9,42 | Cl 7,94 |
| Gef. : | 63,90 | 7,10 | 9,20 | 7,74 |

Beispiel 181

4-[2-(2-Octamethylenimino-5-methoxy-benzoylamino)-äthyl]-benzoesäure-äthylester

Hergestellt aus 2-Octamethylenimino-5-methoxy-benzoesäure und 4-(2-Amino-äthyl)-benzoesäure-äthylester analog Beispiel 165.

Ausbeute : 80 % der Theorie,
Schmelzpunkt : < 20 °C

| | | | |
|---|---|---|---|
| Ber. : | C 71,65 | H 8,01 | N 6,18 |
| Gef. : | 71,65 | 8,15 | 6,25 |

Beispiel 182

4-[2-(2-Octamethylenimino-5-methoxy-benzoylamino)-äthyl]-benzoesäure-hydrochlorid

Hergestellt aus 4-[2-(2-Octamethylenimino-5-methoxy-benzoyl-amino)-äthyl]-benzoesäure-äthylester durch alkalische Hydrolyse analog Beispiel 166.

Ausbeute : 83 % der Theorie,
Schmelzpunkt : 216 °C

| | | | | |
|---|---|---|---|---|
| Ber. : | C 65,13 | H 7,21 | N 6,07 | Cl 7,68 |
| Gef. : | 64,53 | 7,39 | 5,96 | 7,70 |

Beispiel 183

4-[2-(5-Äthoxy-2-octamethylenimino-benzoylamino)-äthyl]-benzoesäure-äthylester

Hergestellt aus 5-Äthoxy-2-octamethylenimino-benzoesäure und 4-(2-Amino-äthyl)-benzoesäure-äthylester analog Beispiel 165.

Ausbeute : 66 % der Theorie,
Schmelzpunkt : < 20 °C

| | | | |
|---|---|---|---|
| Ber. : | C 72,07 | H 8,20 | N 6,00 |
| Gef. : | 72,25 | 8,21 | 6,06 |

Beispiel 184

4-[2-(5-Äthoxy-2-octamethylenimino-benzoylamino)-äthyl]-benzoesäure-hydrochlorid

Hergestellt aus 4-[2-(5-Äthoxy-2-octamethylenimino-benzoylamino)-äthyl]-benzoesäure-äthylester durch alkalische Hydrolyse analog Beispiel 166.

Ausbeute : 98 % der Theorie,
Schmelzpunkt : 172 °C

| | | | | |
|---|---|---|---|---|
| Ber. : | C 65,73 | H 7,42 | N 5,89 | Cl 7,46 |
| Gef. : | 65,50 | 7,20 | 5,79 | 7,19 |

Beispiel 185

4-[2-(5-Isopropyloxy-2-octamethylenimino-benzoylamino)-äthyl]-benzoesäure-äthylester

Hergestellt aus 5-Isopropyloxy-2-octamethylenimino-benzoesäure und 4-(2-Amino-äthyl)-benzoesäure-äthylester analog Beispiel 165.

Ausbeute : 87 % der Theorie,
Schmelzpunkt : < 20 °C

| | | | |
|---|---|---|---|
| Ber. : | C 72,47 | H 8,38 | N 5,82 |
| Gef. : | 73,04 | 8,44 | 5,76 |

Beispiel 186

4-[2-(5-Isopropyloxy-2-octamethylenimino-benzoylamino)-äthyl]-benzoesäure-hydrochlorid

Hergestellt aus 4-[2-(5-Isopropyloxy-2-octamethylenimino-benzoylamino)-äthyl]-benzoesäure-äthylester durch alkalische Hydrolyse analog Beispiel 166.

Ausbeute : 93 % der Theorie,
Schmelzpunkt : 152 °C

| | | | | |
|---|---|---|---|---|
| Ber. : | C 66,30 | H 7,62 | N 5,72 | Cl 7,24 |
| Gef. : | 66,40 | 7,50 | 5,54 | 7,11 |

Beispiel 187

4-[2-(5-Butyl-(2)-oxy-2-octamethylenimino-benzoylamino)-äthyl]-benzoesäure-äthylester

Hergestellt aus 5-Butyl-(2)-oxy-2-octamethylenimino-benzoesäure und 4-(2-Amino-äthyl)-benzoesäure-äthylester analog Beispiel 165.

Ausbeute : 64 % der Theorie,
Schmelzpunkt : < 20 °C

| | | | |
|---|---|---|---|
| Ber. : | C 72,84 | H 8,56 | N 5,66 |
| Gef. : | 72,58 | 8,48 | 5,27 |

Beispiel 188

4-[2-(5-Butyl-(2)-oxy-2-octamethylenimino-benzoylamino)-äthyl]-benzoesäure-hydrochlorid

Hergestellt aus 4-[2-(5-Butyl-(2)-oxy-2-octamethylenimino-benzoylamino)-äthyl]-benzoesäure-äthylester durch alkalische Hydrolyse analog Beispiel 166.

Ausbeute : 88 % der Theorie,
Schmelzpunkt : 142 °C

| | | | | |
|---|---|---|---|---|
| Ber. : | C 66,84 | H 7,81 | N 5,56 | Cl 7,04 |
| Gef. : | 66,40 | 7,84 | 5,20 | 6,71 |

Beispiel 189

4-[2-(5-Chlor-2-(4-isopropyl-piperidino)-benzoylamino)-äthyl]-benzoesäure-äthylester

Hergestellt aus 5-Chlor-2-(4-isopropyl-piperidino)-benzoesäure und 4-(2-Amino-äthyl)-benzoesäure-äthylester analog Beispiel 165.

Ausbeute : 72 % der Theorie,
Schmelzpunkt : < 20 °C

| | | | | |
|---|---|---|---|---|
| Ber. : | C 68,03 | H 7,69 | N 6,10 | Cl 7,73 |
| Gef. : | 68,20 | 7,62 | 6,20 | 7,41 |

Beispiel 190

4-[2-(5-Chlor-2-(4-isopropyl-piperidino)-benzoylamino)-äthyl]-benzoesäure

Hergestellt aus 4-[2-(5-Chlor-2-(4-isopropyl-piperidino)-benzoylamino)-äthyl]-benzoesäure-äthylester durch alkalische Hydrolyse analog Beispiel 166.
Ausbeute : 75 % der Theorie,
Schmelzpunkt : 164 °C

| | | | | |
|---|---|---|---|---|
| Ber. : | C 67,20 | H 6,81 | N 6,53 | Cl 8,27 |
| Gef. : | 67,40 | 6,94 | 6,74 | 8,41 |

Beispiel 191

4-[2-(5-Chlor-2-(4-tert. butyl-piperidino)-benzoylamino)-äthyl]-benzoesäure-äthylester

Hergestellt aus 5-Chlor-2-(4-tert. butyl-piperidino)-benzoesäure und 4-(2-Amino-äthyl)-benzoesäure-äthylester analog Beispiel 165.
Ausbeute : 63 % der Theorie,
Schmelzpunkt : 103 °C

| | | | | |
|---|---|---|---|---|
| Ber. : | C 68,84 | H 7,49 | N 5,95 | Cl 7,53 |
| Gef. : | 69,10 | 7,60 | 6,20 | 7,90 |

Beispiel 192

4-[2-(5-Chlor-2-(4-tert. butyl-piperidino)-benzoylamino)-äthyl]-benzoesäure

Hergestellt aus 4-[2-(5-Chlor-2-(4-tert. butyl-piperidino)-benzoylamino)-äthyl]-benzoesäure-äthylester analog Beispiel 166.
Ausbeute : 87 % der Theorie,
Schmelzpunkt : 160 °C

| | | | | |
|---|---|---|---|---|
| Ber. : | C 67,78 | H 7,05 | N 6,33 | Cl 8,00 |
| Gef. : | 67,93 | 7,21 | 6,50 | 8,20 |

Beispiel 193

4-[2-(5-Chlor-2-(1,4-dioxa-8-aza-spiro[4,6]undecan-8-yl)-benzoylamino)-äthyl]-benzoesäure-äthylester

Hergestellt aus 5-Chlor-2-(1,4-dioxa-8-aza-spiro[4,6]undecan-8-yl)-benzoesäure und 4-(2-Amino-äthyl)-benzoesäure-äthylester analog Beispiel 165.
Ausbeute : 43 % der Theorie,
Schmelzpunkt : < 20 °C

| | | | | |
|---|---|---|---|---|
| Ber. : | C 64,12 | H 6,42 | N 5,75 | Cl 7,28 |
| Gef. : | 64,40 | 6,31 | 6,01 | 7,62 |

Beispiel 194

4-[2-(5-Chlor-2-(1,4-dioxa-8-aza-spiro[4,6]undecan-8-yl)-benzoylamino)-äthyl]-benzoesäure

Hergestellt aus 4-[2-(5-Chlor-2-(1,4-dioxa-8-aza-spiro[4,6]-undecan-8-yl)-benzoylamino)-äthyl]-benzoesäure-äthylester durch alkalische Hydrolyse analog Beispiel 166.
Ausbeute : 71 % der Theorie,
Schmelzpunkt : 185 °C

| | | | | |
|---|---|---|---|---|
| Ber. : | C 62,81 | H 5,93 | N 6,10 | Cl 7,72 |
| Gef. : | 63,02 | 6,05 | 6,11 | 7,98 |

Beispiel 195

4-[2-(2-Diallylamino-5-chlor-benzoylamino)-äthyl]-benzoesäureäthylester

Hergestellt aus 2-Diallylamino-5-chlor-benzoesäure und 4-(2-Amino-äthyl)-benzoesäure-äthylester analog Beispiel 165.
Ausbeute : 48 % der Theorie,
Schmelzpunkt : < 20 °C

| | | | |
|---|---|---|---|
| Ber : | C 67,52 | H 6,29 | N 6,55 |
| Gef. : | 67,64 | 6,38 | 6,56 |

Beispiel 196

4-[2-(2-Diallylamino-5-chlor-benzoylamino)-äthyl]-benzoesäure

Hergestellt aus 4-[2-(2-Diallylamino-5-chlor-benzoylamino)-äthyl]-benzoesäure-äthylester durch alkalische Hydrolyse analog Beispiel 166.
Ausbeute : 75 % der Theorie,
Schmelzpunkt : 130 °C
Ber. : C 66,24 H 5,81 N 7,02
Gef. : 66,50 5,83 6,92

Beispiel 197

4-[2-(5-Nitro-2,4-dipiperidino-benzoylamino)-äthyl]-benzoesäureäthylester

Hergestellt aus 5-Nitro-2,4-dipiperidino-benzoesäure und 4-(2-Amino-äthyl)-benzoesäure-äthylester analog Beispiel 165.
Ausbeute : 40 % der Theorie,
Schmelzpunkt : < 20 °C
Ber : C 66,12 H 7,13 N 11,01
Gef. : 66,13 7,09 11,05

Beispiel 198

4-[2-(5-Nitro-2,4-dipiperidino-benzoylamino)-äthyl]-benzoesäure

Hergestellt aus 4-[2-(5-Nitro-2,4-dipiperidino-benzoylamino)-äthyl]-benzoesäure-äthylester durch alkalische Hydrolyse analog Beispiel 166.
Ausbeute : 95 % der Theorie,
Schmelzpunkt : 208 °C
Ber. : C 64,98 H 6,71 N 11,65
Gef. : 64,40 6,72 11,03

Beispiel 199

4-[2-(5-Amino-2,4-dipiperidino-benzoylamino)-äthyl]-benzoesäureäthylester

1,4 g (2,8 mMol) 4-[2-(5-Nitro-2,4-dipiperidino-benzoylamino)-äthyl]-benzoesäure-äthylester werden in 100 ml Äthanol gelöst und bei Raumtemperatur und einem Wasserstoffdruck von 5 bar mit 10 %iger Palladiumkohle als Katalysator hydriert. Nach Abfiltrieren des Katalysators und Abdestillieren des Methanols erhält man beim Kristallisieren aus Aceton 0,8 g (60 % der Theorie) vom Schmelzpunkt 172 °C.
Ber : C 70,26 H 8,00 N 11,70
Gef. : 70,39 8,19 11,72

Beispiel 200

4-[2-(5-Amino-2,4-dipiperidino-benzoylamino)-äthyl]-benzoesäurehydrochlorid

Hergestellt aus 4-[2-(5-Amino-2,4-dipiperidino-benzoylamino)-äthyl]-benzoesäure-äthylester durch alkalische Hydrolyse analog Beispiel 166.
Ausbeute : 95 % der Theorie,
Schmelzpunkt : 271 °C
Ber. : C 64,11 H 7,24 N 11,50 Cl 7,27
Gef. : 64,40 7,27 11,33 7,50

Beispiel 201

4-[2-(2-(N-Adamantyl-(1)-N-methyl-amino)-5-chlor-benzoylamino)-äthyl]-benzoesäure-äthylester

Hergestellt aus 2-(N-Adamantyl-(1)-N-methyl-amino)-5-chlor-benzoesäure und 4-(2-Amino-äthyl)-benzoesäure-äthylester analog Beispiel 165.
Ausbeute : 0,85 g (34 % der Theorie),
Schmelzpunkt : < 20 °C
Ber. : C 70,36 H 7,13 N 5,66 Cl 7,16
Gef. : 70,05 7,08 5,46 7,07

Beispiel 202

4-[2-(2-Adamantyl-(1)-N-methyl-amino)-5-chlor-benzoylamino)-äthyl]-benzoesäure

Hergestellt aus 4-[2-(2-(N-Adamantyl-(1)-N-methyl-amino)-5-chlor-benzoylamino)-äthyl]-benzoesäure-äthylester durch alkalische Hydrolyse analog Beispiel 166.
Ausbeute : 0,33 g (64 % der Theorie),
Schmelzpunkt : 207 °C

| | | | | |
|---|---|---|---|---|
| Ber. : | C 69,44 | H 6,69 | N 6,00 | Cl 7,59 |
| Gef. : | 69,13 | 6,44 | 6,02 | 7,93 |

Beispiel 203

4-[2-(5-Chlor-2-(1,2,3,6-tetrahydro-pyridino)-benzoylamino)-äthyl]-benzoesäure-äthylester

Hergestellt aus 5-Chlor-2-(1,2,3,6-tetrahydro-pyridino)-benzoesäure und 4-(2-Amino-äthyl)-benzoesäure-äthylester analog Beispiel 165.
Ausbeute : 4,7 g (88 % der Theorie),
Schmelzpunkt : < 20 °C

| | | | | |
|---|---|---|---|---|
| Ber. : | C 66,90 | H 6,10 | N 6,78 | Cl 8,59 |
| Gef. : | 67,60 | 6,21 | 7,02 | 8,54 |

Beispiel 204

4-[2-(5-Chlor-2-(1,2,3,6-tetrahydro-pyridino)-benzoylamino)-äthyl]-benzoesäure

Hergestellt aus 4-[2-(5-Chlor-2-(1,2,3,6-tetrahydro-pyridino)-benzoylamino)-äthyl]-benzoesäure-äthylester durch alkalische Hydrolyse analog Beispiel 166.
Ausbeute : 2,27 g (73,7 % der Theorie),
Schmelzpunkt : 181-182 °C

| | | | | |
|---|---|---|---|---|
| Ber. : | C 65,54 | H 5,50 | N 7,28 | Cl 9,21 |
| Gef. : | 65,50 | 5,49 | 7,32 | 9,12 |

Beispiel 205

4-[2-(5-Chlor-2-(N-isobutyl-N-propyl-amino)-benzoylamino)-äthyl]-benzoesäure-äthylester

Hergestellt aus 5-Chlor-2-(N-isobutyl-N-propyl-amino)-benzoesäure und 4-(2-Amino-äthyl)-benzoesäure-äthylester analog Beispiel 165.
Ausbeute : 5,8 g (86,8 % der Theorie),
Schmelzpunkt : < 20 °C

| | | | | |
|---|---|---|---|---|
| Ber. : | C 67,48 | H 7,47 | N 6,30 | Cl 7,97 |
| Gef. : | 67,70 | 7,63 | 6,26 | 7,96 |

Beispiel 206

4-[2-(5-Chlor-2-(N-isobutyl-N-propyl-amino)-benzoylamino)-äthyl]-benzoesäure

Hergestellt aus 4-[2-(5-Chlor-2-(N-isobutyl-N-propyl-amino)-benzoylamino)-äthyl]-benzoesäure-äthylester durch alkalische Hydrolyse analog Beispiel 166.
Ausbeute : 2,7 g (79 % der Theorie),
Schmelzpunkt : 128 °C

| | | | | |
|---|---|---|---|---|
| Ber. : | C 66,25 | H 7,01 | N 6,72 | Cl 8,50 |
| Gef. : | 66,60 | 7,08 | 6,66 | 8,64 |

Beispiel 207

4-[2-(2-N,N-Diäthylamino-3-methyl-benzoylamino)-äthyl]-benzoesäure-methylester

Hergestellt aus 2-N,N-Diäthylamino-3-methyl-benzoesäure und 4-(2-Amino-äthyl)-benzoesäure-methylester analog Beispiel 165.
Ausbeute : 57 % der Theorie,
Schmelzpunkt : < 20 °C

| | | | |
|---|---|---|---|
| Ber. : | C 71,71 | H 7,66 | N 7,61 |
| Gef. : | 71,71 | 7,83 | 7,55 |

Beispiel 208

4-[2-(2-N,N-Diäthylamino-3-methyl-benzoylamino)-äthyl]-benzoesäure

Hergestellt aus 4-[2-(2-N,N-Diäthylamino-3-methyl-benzoylamino)-äthyl]-benzoesäure-methylester durch alkalische Verseifung analog Beispiel 166.

Ausbeute : 63,1 % der Theorie,
Schmelzpunkt : 150-152 °C
Ber. : C 71,15 H 7,39 N 7,91
Gef. : 71,01 7,38 8,13

Beispiel 209

4-[2-(5-Chlor-2-(4-(2-furoyl)-piperazino)-benzoylamino)-äthyl]-benzoesäure-methylester

Hergestellt analog Beispiel 165 aus 5-Chlor-2-(4-(2-furoyl)-piperazino)-benzoesäure und 4-(2-Amino-äthyl)-benzoesäure-methylester.

Ausbeute : 88,4 % der Theorie,
Schmelzpunkt : 93-95 °C
Ber. : C 62,97 H 5,28 N 8,47 Cl 7,15
Gef. : 62,88 5,30 8,36 7,32

Beispiel 210

4-[2-(5-Chlor-2-(4-(2-furoyl)-piperazino)-benzoylamino)-äthyl]-benzoesäure

Hergestellt aus 4-[2-(5-Chlor-2-(4-(2-furoyl)-piperazino)-benzoylamino)-äthyl]-benzoesäure-methylester durch alkalische Verseifung analog Beispiel 166.

Ausbeute : 26,4 % der Theorie,
Schmelzpunkt : 187-188 °C
Ber. : C 62,31 H 5,02 N 8,72 Cl 7,36
Gef. : 62,08 4,95 8,56 7,61

Beispiel 211

4-[2-(5-Chlor-2-(N-methyl-N-benzylamino)-benzoylamino)-äthyl]-benzoesäure-äthylester

Hergestellt aus 5-Chlor-2-(N-methyl-N-benzylamino)-benzoesäure und 4-(2-Amino-äthyl)-benzoesäure-äthylester analog Beispiel 165.

Ausbeute : 47 % der Theorie,
Schmelzpunkt : 93-95 °C
Ber. : C 69,25 H 6,03 N 6,21 Cl 7,86
Gef. : 69,50 6,35 6,31 7,90

Beispiel 212

4-[2-(5-Chlor-2-(N-methyl-N-benzylamino)-benzoylamino)-äthyl]-benzoesäure

Hergestellt aus 4-[2-(5-Chlor-2-(N-methyl-N-benzylamino)-benzoylamino)-äthyl]-benzoesäure-äthylester durch alkalische Verseifung analog Beispiel 166.

Ausbeute : 64,2 % der Theorie,
Schmelzpunkt : 127-128 °C
Ber. : C 68,16 H 5,48 N 6,62 Cl 8,38
Gef. : 68,01 5,59 6,81 8,54

Beispiel 213

4-[2-(2-(4-Äthoxycarbonyl-piperazino)-5-chlor-benzoylamino)-äthyl]-benzoesäure-äthylester

Hergestellt aus 2-(4-Äthoxycarbonyl-piperazino)-5-chlor-benzoesäure und 4-(2-Amino-äthyl)-benzoesäure-äthylester analog Beispiel 165.

Ausbeute : 71,2 % der Theorie,
Schmelzpunkt : < 20 °C
Ber. : C 61,53 H 6,20 N 8,61 Cl 7,26
Gef. : 61,78 6,30 8,23 7,21

Beispiel 214

4-[2-(2-(4-Äthoxycarbonyl-piperazino)-5-chlor-benzoylamino)-äthyl]-benzoesäure

Hergestellt aus 4-[2-(2-(4-Äthoxycarbonyl-piperazino)-5-chlor-benzoylamino)-äthyl]-benzoesäure-äthylester durch alkalische Verseifung analog Beispiel 166.
Ausbeute : 93 % der Theorie,
Schmelzpunkt : 168-170 °C

| | | | | |
|---|---|---|---|---|
| Ber. : | C 60,06 | H 5,70 | N 9,14 | Cl 7,71 |
| Gef. : | 59,93 | 5,91 | 9,20 | 7,97 |

Beispiel 215

4-[2-(5-Äthyl-2-piperidino-benzoylamino)-äthyl]-benzoesäure-äthylester

Hergestellt aus 6-Äthyl-1-(5-Brom-pentyl)-4H-3,1-benzoxazin-2,4-(1H)-dion und 4-(2-Amino-äthyl)-benzoesäure-äthylester analog Beispiel 217.
Ausbeute : 55,6 % der Theorie,
Schmelzpunkt : < 20 °C

| | | | |
|---|---|---|---|
| Ber. : | C 73,50 | H 7,90 | N 6,86 |
| Gef. : | 73,26 | 7,88 | 6,97 |

Beispiel 216

4-[2-(5-Äthyl-2-piperidino-benzoylamino)-äthyl]-benzoesäure

Hergestellt aus 4-[2-(5-Äthyl-2-piperidino-benzoylamino)-äthyl]-benzoesäure-äthylester durch alkalische Verseifung analog Beispiel 166.
Ausbeute : 84,5 % der Theorie,
Schmelzpunkt : 177 °C

| | | | |
|---|---|---|---|
| Ber. : | C 72,60 | H 7,42 | N 7,36 |
| Gef. : | 72,59 | 7,28 | 7,16 |

Beispiel 217

4-[2-(5-Methyl-2-piperidino-benzoylamino)-äthyl]-benzoesäure-äthylester

10,6 g (32,5 mMol) 1-(5-Brom-pentyl)-6-methyl-4H-3,1-benzoxazin-2,4-(1H)-dion [hergestellt aus 1,5-Dibrompentan und 6-Methyl-4H-3,1-benzoxazin-2,4-(1H)-dion] werden in 100 ml absolutem Dioxan mit 19,3 g (0,1 Mol) 4-(2-Amino-äthyl)-benzoesäure-äthylester und 13 g N-Äthyl-diisopropylamin 4 Tage bei Raumtemperatur gerührt. Dann wird eingeengt, mit Wasser versetzt und mit Chloroform extrahiert. Die getrockneten Chloroformextrakte werden eingeengt und an Kieselgel mit Toluol/Essigester 10 : 1 als Fließmittel chromatographiert.
Ausbeute : 6,85 g (53,5 % der Theorie),
Schmelzpunkt : < 20 °C

| | | | |
|---|---|---|---|
| Ber. : | C 73,07 | H 7,66 | N 7,10 |
| Gef. : | 72,62 | 7,15 | 7,12 |

Beispiel 218

4-[2-(5-Methyl-2-piperidino-benzoylamino)-äthyl]-benzoesäure

Hergestellt aus 4-[2-(5-Methyl-2-piperidino-benzoylamino)-äthyl]-benzoesäure-äthylester durch alkalische Verseifung analog Beispiel 166.
Ausbeute : 80 % der Theorie,
Schmelzpunkt : 199-200 °C

| | | | |
|---|---|---|---|
| Ber. : | C 72,11 | H 7,15 | N 7,64 |
| Gef. : | 72,10 | 6,95 | 7,70 |

Beispiel 219

4-[2-(5-Chlor-2-(4-p-chlorphenyl-piperazino)-benzoylamino)-äthyl]-benzoesäure-äthylester

Hergestellt aus 5-Chlor-2-(4-p-chlorphenyl-piperazino)-benzoesäure und 4-(2-Amino-äthyl)-benzoesäure-äthylester analog Beispiel 165.

Ausbeute : 64,1 % der Theorie,
Schmelzpunkt : 153-155 °C
Ber. : C 63,88 H 5,55 N 7,98 Cl 13,47
Gef. : 63,77 5,47 7,93 13,40

Beispiel 220

4-[2-(5-Chlor-2-(4-methyl-piperazino)-benzoylamino)-äthyl]-benzoesäure-methylester

Hergestellt aus 5-Chlor-2-(4-methyl-piperazino)-benzoesäure-hydrochlorid und 4-(2-Amino-äthyl)-benzoesäure-methylester analog Beispiel 165.
Ausbeute : 52 % der Theorie,
Schmelzpunkt : < 20 °C
Ber. : C 63,53 H 6,30 N 10,11 Cl 8,52
Gef. : 63,62 6,05 10,23 8,28

Beispiel 221

4-[2-(5-Chlor-2-piperidino-benzoylamino)-2-methyl-propyl]-benzoesäure-äthylester

Hergestellt aus 5-Chlor-2-piperidino-benzoesäure und 4-(2-Amino-2-methyl-propyl)-benzoesäure-äthylester-hydrochlorid (Schmelzpunkt : 199 °C) analog Beispiel 165.
Ausbeute : 51 % der Theorie,
Schmelzpunkt : < 20 °C
Ber. : C 67,78 H 7,05 N 6,32 Cl 8,00
Gef. : 67,75 6,91 6,05 7,87

Beispiel 222

4-[2-(5-Chlor-2-piperidino-benzoylamino)-2-methyl-propyl]-benzoesäure

Hergestellt aus 4-[2-(5-Chlor-2-piperidino-benzoylamino)-2-methylpropyl]-benzoesäure-äthylester durch alkalische Verseifung analog Beispiel 166.
Ausbeute : 62 % der Theorie,
Schmelzpunkt : 208 °C
Ber. : C 66,58 H 6,56 N 6,75 Cl 8,55
Gef. : 66,90 6,71 6,50 8,52

Beispiel 223

4-[2-(5-Chlor-2-(1,2,3,4,5,6,7,8-octahydro-isochinolin-2-yl)-benzoylamino)-äthyl]-benzoesäure-äthylester

Hergestellt aus 5-Chlor-2-(1,2,3,4,5,6,7,8-octahydro-isochinolin-2-yl)-benzoesäure und 4-(2-Amino-äthyl)-benzoesäure-äthylester analog Beispiel 165.
Ausbeute : 58 % der Theorie,
Schmelzpunkt : < 20 °C
Ber. : C 69,44 H 6,68 N 5,99 Cl 7,59
Gef. : 69,62 6,72 6,10 7,72

Beispiel 224

4-[2-(5-Chlor-2-(1,2,3,4,5,6,7,8-octahydro-isochinolin-2-yl)-benzoylamino)-äthyl]-benzoesäure-hydrochlorid

Hergestellt aus 4-[2-(5-Chlor-2-(1,2,3,4,5,6,7,8-octahydro-isochinolin-2-yl)-benzoylamino)-äthyl]-benzoesäure-äthylester durch alkalische Hydrolyse analog Beispiel 166.
Ausbeute : 82 % der Theorie,
Schmelzpunkt : 220 °C
Ber. : C 63,15 H 5,93 N 5,89 Cl 14,93
Gef. : 63,45 6,09 6,02 15,90

Beispiel 225

4-[2-(5-Chlor-2-octamethylenimino-benzoylamino)-äthyl]-benzoesäure-methylester

Hergestellt aus 5-Chlor-2-octamethylenimino-benzoesäure und 4-(2-Amino-äthyl)-benzoesäure-

methylester analog Beispiel 165.

Ausbeute : 83 % der Theorie,
Schmelzpunkt : < 20 °C

| | | | | |
|---|---|---|---|---|
| Ber. : | C 67,78 | H 7,05 | N 6,32 | Cl 8,00 |
| Gef. : | 67,96 | 7,21 | 6,54 | 8,20 |

Beispiel 226

4-[2-(5-Chlor-2-octamethylenimino-benzoylamino)-äthyl]-benzoesäure-äthylester

Hergestellt aus 4-[2-(5-Chlor-2-octamethylenimino-benzoylamino)-äthyl]-benzoesäure und absolutem Äthanol mit stöchiometrischen Mengen Thionylchlorid.

Ausbeute : 86 % der Theorie,
Schmelzpunkt : < 20 °C

| | | | | |
|---|---|---|---|---|
| Ber. : | C 68,33 | H 7,27 | N 6,13 | Cl 7,75 |
| Gef. : | 68,21 | 7,40 | 6,20 | 7,62 |

Beispiel 227

4-[2-(5-Chlor-2-octamethylenimino-benzoylamino)-äthyl]-benzoesäure-cyclohexylester

3 g (7 mMol) 4-[2-(5-Chlor-2-octamethylenimino-benzoylamino)-äthyl]-benzoesäure werden in 30 ml absolutem Pyridin mit 1,2 g (7,4 mMol) Carbonyldiimidazol bei Raumtemperatur in das Imidazolid überführt. Nach Zugabe von 1,48 g (14,8 mMol) Cyclohexanol wird 2 bis 3 Stunden auf Siedetemperatur erhitzt. Nach Abdestillieren der Lösungsmittel wird das Rohprodukt chromatographisch über eine Kieselgelsäule mit Toluol-Essigsäureäthylester als Fließmittel gereinigt.

Ausbeute : 2,7 g (75 % der Theorie),
Schmelzpunkt : 88 °C

| | | | | |
|---|---|---|---|---|
| Ber. : | C 70,50 | H 7,70 | N 5,48 | Cl 6,93 |
| Gef. : | 70,60 | 8,23 | 5,26 | 6,69 |

Beispiel 228

4-[2-(5-Chlor-2-octamethylenimino-benzoylamino)-äthyl]-benzoesäure-tert. butylester

4,3 g (0,01 Mol) 4-[2-(5-Chlor-2-octamethylenimino-benzoylamino)-äthyl]-benzoesäure werden in 150 ml Essigsäure-tert. butylester suspendiert, mit 1,43 g (0,011 Mol) 70 %iger Perchlorsäure versetzt und 24 Stunden bei Raumtemperatur gerührt. Das Reaktionsgemisch wird in 500 ml Chloroform aufgenommen. Die Chloroformlösung wird sorgfältig mit Wasser ausgeschüttelt. Das aus der Chloroformphase nach Trocknung über Natriumsulfat gewonnene Rohprodukt wird über eine Kieselgelsäule mit Toluol-Essigsäureäthylester als Fließmittel chromatographisch gereinigt.

Ausbeute : 3 g (62 % der Theorie),
Schmelzpunkt : < 20 °C

| | | | | |
|---|---|---|---|---|
| Ber. : | C 69,33 | H 7,68 | N 5,77 | Cl 7,30 |
| Gef. : | 69,64 | 7,78 | 5,78 | 7,40 |

Analog den Beispielen 225 bis 227 werden folgende Verbindungen hergestellt :

4-[2-(5-Chlor-2-octamethylenimino-benzoylamino)-äthyl]-benzoesäure-propylester
Ausbeute : 70 % der Theorie, Schmelzpunkt : < 20 °C.
4-[2-(5-Chlor-2-octamethylenimino-benzoylamino)-äthyl]-benzoesäure-isopropylester
Ausbeute : 84 % der Theorie, Schmelzpunkt : < 20 °C.
4-[2-(5-Chlor-2-octamethylenimino-benzoylamino)-äthyl]-benzoesäure-butylester
Ausbeute : 90 % der Theorie, Schmelzpunkt : < 20 °C.
4-[2-(5-Chlor-2-octamethylenimino-benzoylamino)-äthyl]-benzoesäure-isobutylester
Ausbeute : 74 % der Theorie, Schmelzpunkt : < 20 °C.
4-[2-(5-Chlor-2-octamethylenimino-benzoylamino)-äthyl]-benzoesäure-hexylester
Ausbeute : 63 % der Theorie, Schmelzpunkt : < 20 °C.
4-[2-(5-Chlor-2-octamethylenimino-benzoylamino)-äthyl]-benzoesäure-benzylester
Ausbeute : 83 % der Theorie, Schmelzpunkt : < 20 °C.

Beispiel 229

4-[2-(5-Chlor-2-octamethylenimino-benzoylamino)-äthyl]-benzyl-alkohol

4,57 g (0,01 Mol) 4-[2-(5-Chlor-2-octamethylenimino-benzoylamino)-äthyl]-benzoesäure-äthylester

werden in 100 ml absolutem Ather gelöst und zu einer Suspension von 0,72 g (0,011 Mol) Lithiumaluminiumhydrid in 30 ml absolutem Äther zugefügt und eine Stunde auf Siedetemperatur erhitzt. Nach dem Erkalten werden 15 ml Wasser vorsichtig zugesetzt. Nach Filtration wird die Ätherphase über Natriumsulfat getrocknet. Das Rohprodukt wird chromatographisch über eine Kieselgelsäule mit Toluol/Essigsäureäthylester = 1 : 1 als Fließmittel gereinigt.

Ausbeute : 3,5 g (84 % der Theorie),
Schmelzpunkt : < 20 °C

| | | | | |
|---|---|---|---|---|
| Ber. : | C 69,46 | H 7,52 | N 6,75 | Cl 8,54 |
| Gef. : | 69,32 | 7,58 | 6,75 | 8,80 |

Beispiel 230

4-[2-(5-Chlor-2-octamethylenimino-benzoylamino)-äthyl]-benzylmalonsäure-diäthylester

2,58 g (5,5 mMol) 4-[2-(5-Chlor-2-octamethylenimino-benzoylamino)-äthyl]-benzylchlorid-hydrochlorid (hergestellt aus 2,3 g (5,5 mMol) 4-[2-(5-Chlor-2-octamethylenimino-benzoylamino)-äthyl]-benzylalkohol und Thionylchlorid in Chloroform) werden in 25 ml absolutem Äthanol gelöst und zu einer Lösung von 3,2 g (20 mMol) Malonsäurediäthylester und 20 mMol Natriumäthylat in absolutem Äthanol getropft. Anschließend erhitzt man 4 Stunden zum Rückfluß, engt dann ein, säuert mit verdünnter Salzsäure an und extrahiert mit Chloroform. Nach dem Einengen der Extrakte wird an Kieselgel mit Toluol/Essigester = 10 : 1 säulenchromatographisch gereinigt.

Ausbeute : 1,7 g (60,7 % der Theorie),
Schmelzpunkt : 80-82 °C

| | | | | |
|---|---|---|---|---|
| Ber. : | C 66,83 | H 7,42 | N 6,36 | Cl 5,03 |
| Gef. : | 66,83 | 7,51 | 6,62 | 5,07 |

Beispiel 231

[2-(5-Chlor-2-octamethylenimino-benzoylamino)-äthyl]-benzol

Hergestellt aus 5-Chlor-2-octamethylenimino-benzoesäure und 2-Phenyl-äthylamin analog Beispiel 165.

Ausbeute : 69 % der Theorie,
Schmelzpunkt : 66 °C

| | | | | |
|---|---|---|---|---|
| Ber. : | C 71,76 | H 7,59 | N 7,17 | Cl 9,39 |
| Gef. : | 72,00 | 7,65 | 7,27 | 9,21 |

Beispiel 232

4-[2-(5-Chlor-2-piperidino-benzoylamino)-äthyl]-acetophenon

2 g (15 mMol) Aluminiumchlorid werden in 10 ml Dichloräthan unter Kühlung mit 0,6 g (7,6 mMol) Acetylchlorid versetzt. Nach anschließender Zugabe von 1 g (2,92 mMol) [2-(5-Chlor-2-piperidino-benzoylamino)-äthyl]-benzol wird 3 Stunden bei 40-45 °C gerührt. Dann wird eingeengt und der Rückstand mit eiskalter verdünnter Salzsäure versetzt. Nach Extraktion mit eingedampft und zur Reinigung an Kieselgel im Fließmittel Chloroform/Essigester = 10 : 1 chromatographiert.

Ausbeute : 0,42 g (37,4 % der Theorie),
Schmelzpunkt : 61-63 °C

| | | | | |
|---|---|---|---|---|
| Ber. : | C 68,65 | H 6,55 | N 7,28 | Cl 9,01 |
| Gef. : | 68,73 | 6,76 | 7,33 | 9,16 |

Beispiel 233

4-[2-(5-Chlor-2-octamethylenimino-benzoylamino)-äthyl]-acetophenon-hydrochlorid

Hergestellt analog Beispiel 232 durch Umsetzung von [2-(5-Chlor-2-octamethylenimino-benzoylamino)-äthyl]-benzol mit Acetylchlorid in Gegenwart von Aluminiumchlorid.

Ausbeute : 41 % der Theorie,
Schmelzpunkt : 160 °C

| | | | | |
|---|---|---|---|---|
| Ber. : | C 64,79 | H 6,96 | N 6,04 | Cl 15,30 |
| Gef. : | 64,91 | 7,05 | 5,98 | 15,11 |

Beispiel 234

4-[2-(5-Chlor-2-piperidino-benzoylamino)-äthyl]-phenylessigsäure

4,86 g (10 mMol) 4-[2-(5-Chlor-2-piperidino-benzoylamino)-äthyl]-phenyl-thioessigsäure-morpholid (Schmelzpunkt: < 20 °C, hergestellt aus 4-[2-(5-Chlor-2-piperidino-benzoylamino)-äthyl]-acetophenon durch Willgerodt-Reaktion mit Schwefel und Morpholin) werden in 50 ml Äthanol mit 2 g (50 mMol) Natriumhydroxid 2 Tage gekocht. Dann wird eingeengt, mit Wasser versetzt und mit Äther extrahiert. Die wässrige Phase wird anschließend angesäuert, der gebildete Niederschlag abgesaugt und aus Acetonitril umkristallisiert.

Ausbeute: 0,96 g (24 % der Theorie),
Schmelzpunkt: 151 °C.

| | | | | |
|---|---|---|---|---|
| Ber.: | C 65,91 | H 6,28 | N 6,99 | Cl 8,84 |
| Gef.: | 65,61 | 6,34 | 7,18 | 8,77 |

## Beispiel 235

4-[2-(5-Chlor-2-octamethylenimino-benzoylamino)-äthyl]-benzaldehyd

Zu einer im Bad von 160-170 °C gerührten Suspension von 0,35 g feingepulvertem wasserfreiem Natriumcarbonat in 3,5 ml Äthylenglykol gibt man 0,35 g (0,59 mMol) $N^1$-4-(2-(5-Chlor-2-octamethylenimino-benzoylamino)-äthyl)-benzoyl-$N^2$-tosyl-hydrazin vom Schmelzpunkt 153-158 °C, hergestellt aus 4-[2-(5-Chlor-2-octamethylenimino-benzoylamino)-äthyl]-benzoesäure und Tosylhydrazin mit Carbonyldiimidazol in Tetrahydrofuran. Nach 1,5 Minuten (Gasentwicklung beendet) entfernt man das Heizbad und versetzt einige Minuten später mit viel Eis. Man extrahiert zweimal mit Äther, trocknet und filtriert die vereinigten Extrakte und dampft sie im Vakuum ein. Das erhaltene hellgelbe harzige Öl reinigt man durch Säulenchromatographie an Kieselgel mit dem Laufmittel Chloroform/Aceton = 20 : 1.

Ausbeute: 66 % der Theorie,
Schmelzpunkt: < 20 °C.
Ber.: Molpeak m/e = 412/414 (1 Cl)
Gef.: Molpeak m/e = 412/414 (1 Cl)
Schmelzpunkt des Hydrochlorids × 0,5 $H_2O$ : 156 °C.

| | | | | |
|---|---|---|---|---|
| Ber.: | C 62,88 | H 6,82 | N 6,11 | Cl 15,46 |
| Gef.: | 62,85 | 7,11 | 6,05 | 15,43 |

## Beispiel 236

4-[2-(5-Chlor-2-octamethylenimino-benzoylamino)-äthyl]-benzaldehyd

Man schüttelt die Lösung von 0,50 g (1,2 mMol) 4-[2-(5-Chlor-2-octamethylenimino-benzoylamino)-äthyl]-benzylalkohol in 75 ml absolutem Aceton mit 7,5 g aktivem Mangandioxid zwei Stunden bei Raumtemperatur, filtriert über eine Celite-Schicht auf einer G4-Fritte und dampft das Filtrat im Vakuum ein. Das erhaltene bräunliche zähe Öl reinigt man durch Säulenchromatographie an Kieselgel (Chloroform/Aceton = 20 : 1).

Ausbeute: 5 % der Theorie,
Schmelzpunkt: < 20 °C.
Ber.: Molpeak m/e = 412/414 (1 Cl)
Gef.: Molpeak m/e = 412/414 (1 Cl)

## Beispiel 237

4-[2-(5-Chlor-2-octamethylenimino-benzoylamino)-äthyl]-benzaldehyd-diäthylacetal

Eine Mischung von 0,23 g (0,56 mMol) 4-[2-(5-Chlor-2-octamethylenimino-benzoylamino)-äthyl]-benzaldehyd, 0,20 ml (1,2 mMol) Orthoameisensäure-triäthylester, 0,02 g Ammoniumchlorid und 0,2 ml wasserfreiem Äthanol erhitzt man 30 Minuten bei 90 °C. Nach Abkühlen gießt man in 2N-Ammoniak ein und extrahiert mit Äther. Den über Natriumsulfat getrockneten Extrakt dampft man im Vakuum ein und reinigt den Eindampfrückstand durch Säulenchromatographie an Kieselgel (Toluol/Aceton = 10 : 1).

Ausbeute: 23 % der Theorie,
Schmelzpunkt: < 20 °C.
Ber.: Molpeak m/e = 486/488
Gef.: Molpeak m/e = 486/488

## Beispiel 238

4-[2-(5-Chlor-2-octamethylenimino-benzoylamino)-äthyl]-zimtsäure

1 g (2,4 mMol) 4-[2-(5-Chlor-2-octamethylenimino-benzoylamino)-äthyl]-benzaldehyd und 1 g (10 mMol) Malonsäure werden in 10 ml absolutem Pyridin nach Zusatz von 0,5 ml Piperidin eine Stunde

auf 100 °C erhitzt. Dann gibt man auf Eis/verdünnte Salzsäure und saugt den gebildeten Niederschlag ab. Zur Reinigung wird an Kieselgel mit Toluol/Essigester = 1 : 1 als Fließmittel chromatographiert.

Ausbeute : 21 % der Theorie,
Schmelzpunkt : 90 °C.

| | | | | |
|---|---|---|---|---|
| Ber. : | C 68,63 | H 6,87 | N 6,16 | Cl 7,79 |
| Gef. : | 68,69 | 6,82 | 6,10 | 7,83 |

Beispiel 239

4-[2-(5-Chlor-2-piperidino-benzoylamino)-äthyl]-benzoylessigsäureäthylester

Hergestellt durch Friedel-Crafts-Acylierung von [2-(5-Chlor-2-piperidino-benzoylamino)-äthyl]-benzol mit Malonsäureäthylesterchlorid analog Beispiel 232.

Ausbeute : 21 % der Theorie,
Schmelzpunkt : < 20 °C.
Ber. : m/e 456/458 (1 Cl)
Gef. : m/e 456/458 (1 Cl)

Beispiel 240

4-[2-(5-Chlor-2-octamethylenimino-benzoylamino)-äthyl]-nitrobenzol

Hergestellt aus 5-Chlor-2-octamethylenimino-benzoesäure und 4-(2-Aminoäthyl)-nitrobenzol analog Beispiel 165.

Ausbeute : 85 % der Theorie,
Schmelzpunkt : 102 °C.

| | | | | |
|---|---|---|---|---|
| Ber. : | C 64,25 | H 6,56 | N 9,77 | Cl 8,24 |
| Gef. : | 64,45 | 6,57 | 9,73 | 8,24 |

Beispiel 241

4-[2-(5-Chlor-2-octamethylenimino-benzoylamino)-äthyl]-anilin

Hergestellt aus 4-[2-(5-Chlor-2-octamethylenimino-benzoylamino)-äthyl]-nitrobenzol durch Reduktion mit Zinn(II)-chlorid in Salzsäure.

Ausbeute : 74 % der Theorie,
Schmelzpunkt : 87 °C.

| | | | | |
|---|---|---|---|---|
| Ber. : | C 69,07 | H 7,56 | N 10,50 | Cl 8,86 |
| Gef. : | 69,10 | 7,77 | 10,61 | 9,10 |

Beispiel 242

4-[2-(5-Chlor-2-octamethylenimino-benzoylamino)-äthyl]-toluol-hydrochlorid

Hergestellt aus 5-Chlor-2-octamethylenimino-benzoesäure und 4-(2-Amino-äthyl)-toluol analog Beispiel 165.

Ausbeute : 69 % der Theorie,
Schmelzpunkt : 167-171 °C.

| | | | | |
|---|---|---|---|---|
| Ber. : | C 66,20 | H 7,41 | N 6,44 | Cl 16,29 |
| Gef. : | 66,78 | 7,39 | 6,87 | 15,90 |

Beispiel 243

4-[2-(5-Chlor-2-octamethylenimino-benzoylamino)-äthyl]-chlorbenzol

Hergestellt aus 5-Chlor-2-octamethylenimino-benzoesäure und 4-(2-Amino-äthyl)-chlorbenzol analog Beispiel 165.

Ausbeute : 66 % der Theorie,
Schmelzpunkt : 58 °C.

| | | | |
|---|---|---|---|
| Ber. : | C 65,87 | H 6,73 | N 6,68 |
| Gef. : | 65,99 | 6,55 | 6,51 |

Beispiel 244

4-[2-(5-Chlor-2-octamethylenimino-benzoylamino)-äthyl]-benzoesäurenitril

4,36 g (0,01 Mol) 4-[2-(5-Chlor-2-octamethylenimino-benzoylamino)-äthyl]-anilin werden in 3,8 ml konzentrierter Salzsäure gelöst, mit 28 ml Wasser verdünnt und bei 0 °C mit einer Lösung von 0,76 g (0,011 Mol) Natriumnitrit in 3 ml Wasser bei tropfenweiser Zugabe diazotiert. Nach halbstündigem Nachrühren wird mit Natriumcarbonat pH 6 eingestellt. Diese Diazoniumsalzlösung wird zu einer frisch bereiteten Lösung von Trinatriumtetracyano-kupfer(I)-Komplex bei 0 °C zugetropft.

[Diese Komplexlösung wird aus 3,2 g (0,0 128 Mol) Kupfersulfat × 5 $H_2O$ und 0,87 g Natriumchlorid gelöst in 10 ml Wasser hergestellt. Nach Reduktion zum Kupfer(I)-chlorid mit einer Lösung von 0,66 g Natriumhydrogensulfat und 0,44 g Natriumhydroxyd in 5 ml Wasser wird das gewaschene Kupfer(I)-chlorid zu einer Lösung von 1,7 g Natriumcyanid in 30 ml Wasser zugesetzt].

Nach abgeschlossener Stickstoffentwicklung wird das Reaktionsgemisch eine Stunde auf 70 °C erwärmt. Nach dem Erkalten wird bei pH 8 mit Chloroform extrahiert. Das aus den Chloroformextrakten erhaltene Rohprodukt wird über eine Kieselgelsäule gereinigt (Fließmittel : Toluol/Äthylacetat = 8 : 2).

Ausbeute : 38 % der Theorie,
Schmelzpunkt : 102 °C.

| | | | | |
|---|---|---|---|---|
| Ber. : | C 70,31 | H 6,88 | N 10,25 | Cl 8,64 |
| Gef. : | 70,50 | 6,59 | 10,45 | 8,92 |

## Beispiel 245

4-[2-(5-Chlor-2-octamethylenimino-benzoylamino)-äthyl]-ortho-benzoesäure-äthylester

Äquimolare Mengen von 4-[2-(5-Chlor-2-octamethylenimino-benzoylamino)-äthyl]-benzoesäurenitril und absolutes Äthanol werden in Äther mit Chlorwasserstoff gesättigt. Das Reaktionsgemisch wird 4 Tage bei 4 °C stehengelassen, wobei sich der Iminoäther als Hydrochlorid teils ölig teils kristallin abscheidet. Nach Abdekantieren des Lösungsmittels, Nachwaschen mit Äther wird ein Überschuß von absolutem Äthanol bei 4 °C zugesetzt und das Reaktionsgemisch 2 Tage bei dieser Temperatur belassen. Nach Abdestillieren des Lösungsmittels wird das Rohprodukt über eine Kieselgelsäule mit Toluol als Fließmittel chromatographisch gereinigt.

Ausbeute : 30 % der Theorie,
Schmelzpunkt : < 20 °C.

| | | | | |
|---|---|---|---|---|
| Ber. : | C 67,84 | H 8,16 | N 5,27 | Cl 6,67 |
| Gef. : | 67,60 | 8,05 | 5,14 | 6,43 |

## Beispiel 246

4-[2-(5-Chlor-2-octamethylenimino-benzoylamino)-äthyl]-benzoesäuremorpholid

2 g (4,7 mMol) 4-[2-(5-Chlor-2-octamethylenimino-benzoylamino)-äthyl]-benzoesäure werden in 20 ml absolutem Pyridin gelöst und bei Raumtemperatur durch Zugabe von 0,83 g (5,1 mMol) Carbonyldiimidazol quantitativ in das Imidazolid überführt. Nach Zusatz von 0,41 g Morpholin wird 6 Stunden auf Rückflußtemperatur erwärmt. Anschließend wird Pyridin abdestilliert und der Eindampfrückstand über eine Kieselgelsäule mit dem Fließmittel Toluol/Essigsäureäthylester = 6 : 4 chromatographisch gereinigt.

Ausbeute : 1,8 g (76,5 % der Theorie),
Schmelzpunkt : < 20 °C.

| | | | |
|---|---|---|---|
| Ber. : | C 67,52 | H 7,29 | N 8,44 |
| Gef. : | 67,01 | 7,35 | 8,17 |

## Beispiel 247

4-[2-(5-Chlor-2-octamethylenimino-benzoylamino)-äthyl]-benzoesäure-piperidid

2 g (4,7 mMol) 4-[2-(5-Chlor-2-octamethylenimino-benzoylamino)-äthyl]-benzoesäure werden in 30 ml absolutem Toluol suspendiert und mit 0,65 ml Triäthylamin in das Salz überführt. Nach Abkühlung auf − 10 °C werden 0,5 g (4,7 mMol) Chlorameisensäureathylester zugesetzt und 30 Minuten nachgerührt. Zu dem gemischten Anhydrid werden 0,4 g (4,7 mMol) Piperidin zugesetzt. Nach 2 Stunden werden die Lösungsmittel abdestilliert und das Rohprodukt über eine Kieselgelsäule mit dem Fließmittel Essigsäureäthylester chromatographisch gereinigt.

Ausbeute : 2 g (86 % der Theorie)
Schmelzpunkt : < 20 °C.

| | | | |
|---|---|---|---|
| Ber. : | C 70,21 | H 7,72 | N 8,47 |
| Gef. : | 70,42 | 7,83 | 8,51 |

Analog den Beispielen 246 und 247 werden folgende Verbindungen hergestellt :

4-[2-(5-Chlor-2-octamethylenimino-benzoylamino)-äthyl]-benzoesäureamid

Ausbeute : 75 % der Theorie,
Schmelzpunkt : 122 °C.

| | | | |
|---|---|---|---|
| Ber. : | C 67,35 | H 7,07 | N 9,82 |
| Gef. : | 67,95 | 7,48 | 9,78 |

4-[2-(5-Chlor-2-octamethylenimino-benzoylamino)-äthyl]-benzoesäure-dipropylamid
Ausbeute : 75 % der Theorie,
Schmelzpunkt : < 20 °C.

| | | | |
|---|---|---|---|
| Ber. : | C 70,35 | H 8,27 | N 8,21 |
| Gef. : | 69,95 | 8,04 | 7,94 |

4-[2-(5-Chlor-2-octamethylenimino-benzoylamino)-äthyl]-benzoesäure-diallylamid
Ausbeute : 70 % der Theorie,
Schmelzpunkt : < 20 °C.

| | | | |
|---|---|---|---|
| Ber. : | C 70,90 | H 7,54 | N 8,27 |
| Gef. : | 70,23 | 7,30 | 7,98 |

4-[2-(5-Chlor-2-octamethylenimino-benzoylamino)-äthyl]-benzoesäure-thiomorpholid
Ausbeute : 70 % der Theorie,
Schmelzpunkt : < 20 °C.

| | | | |
|---|---|---|---|
| Ber. : | C 65,41 | H 7,06 | N 8,17 |
| Gef. : | 65,54 | 7,14 | 7,93 |

4-[2-(5-Chlor-2-octamethylenimino-benzoylamino)-äthyl]-benzoesäure-N-methyl-piperazid
Ausbeute : 75 % der Theorie,
Schmelzpunkt : < 20 °C.

| | | | |
|---|---|---|---|
| Ber. : | C 68,15 | H 7,69 | N 10,96 |
| Gef. : | 68,23 | 7,73 | 11,08 |

4-[2-(5-Chlor-2-octamethylenimino-benzoylamino)-äthyl]-benzoesäure-N-äthyl-N-cyclohexylamid
Ausbeute : 58 % der Theorie,
Schmelzpunkt : < 20 °C

| | | | |
|---|---|---|---|
| Ber. : | C 71,42 | H 8,24 | N 7,81 |
| Gef. : | 71,60 | 8,30 | 7,57 |

4-[2-(5-Chlor-2-octamethylenimino-benzoylamino)-äthyl]-benzoesäure-isopropylamid
Ausbeute : 54 % der Theorie,
Schmelzpunkt : 171 °C

| | | | |
|---|---|---|---|
| Ber. : | C 68,99 | H 7,72 | N 8,94 |
| Gef. : | 69,34 | 7,52 | 8,74 |

4-[2-(5-Chlor-2-octamethylenimino-benzoylamino)-äthyl]-benzoesäure-butylamid
Ausbeute : 53 % der Theorie, Schmelzpunkt : 163 °C.

| | | | |
|---|---|---|---|
| Ber. : | C 69,47 | H 7,91 | N 8,68 |
| Gef. : | 69,53 | 7,95 | 8,72 |

## Beispiel 248

$N^1$-(1-(4-Äthoxycarbonyl-phenyl)-äthyl)-$N^2$-(5-chlor-2-piperidino-benzoyl)-hydrazin

Man versetzt 2,4 g (10 mMol) 5-Chlor-2-piperidino-benzoesäure in 15 ml wasserfreiem Tetrahydrofuran mit 1,62 g (10 mMol) N,N-Carbonyldiimidazol, rührt 5 Minuten bei 20 °C und 45 Minuten unter Rückfluß. Zu der abgekühlten Lösung gibt man bei 20 °C eine Lösung von 2,08 g (10 mMol) 4-(1-Hydrazin-äthyl)-benzoesäureäthylester [frisch hergestellt aus der entsprechenden Menge 4-(1-Hydrazino-äthyl)-benzoesäure-äthylester-hydrochlorid vom Schmelzpunkt 98-100 °C durch Versetzen mit der stöchiometrischen Menge wässriger Natronlauge unter Eiskühlung, Extraktion mit Chloroform und Eindampfen des getrockneten Chloroform-Extraktes bei 30 °C im Vakuum] in 9 ml wasserfreiem Tetrahydrofuran. Nach Rühren über Nacht bei Raumtemperatur dampft man im Vakuum ein und verteilt zwischen Wasser und Chloroform. Die vereinigten Chloroform-Extrakte trocknet und filtriert man und dampft sie im Vakuum ein. Den rotbraunen öligen Eindampfrückstand reinigt man durch Säulenchromatographie an Kieselgel (Toluol/Aceton = 8/1).

Ausbeute : 41,9 % der Theorie,
Schmelzpunkt : < 20 °C (hellgelbes Öl).
Ber. : Molpeak m/e = 429/431 (1 Cl)
Gef. : Molpeak m/e = 429/431 (1 Cl)

Beispiel 249

$N^1$-(1-(4-Äthoxycarbonyl-phenyl)-äthyl)-$N^2$-(5-chlor-2-dimethylamino-benzoyl)-hydrazin

Hergestellt aus 5-Chlor-2-dimethylamino-benzoesäure, Carbonyldiimidazol und 4-(1-Hydrazino-äthyl)-benzoesäure-äthylester in wasserfreiem Tetrahydrofuran analog Beispiel 248.
Ausbeute : 18 % der Theorie,
Schmelzpunkt : < 20 °C.
Ber. : Molpeak m/e = 389/391 (1 Cl)
Gef. : Molpeak m/e = 389/391 (1 Cl)

Beispiel 250

$N^1$-(1-(4-Carboxyphenyl)-äthyl)-$N^2$-(5-chlor-2-piperidino-benzoyl)-hydrazin

Man rührt 1,8 g (4,2 mMol) $N^1$-(1-(4-Äthoxycarbonyl-phenyl)-äthyl)-$N^2$-(5-chlor-2-piperidino-benzoyl)-hydrazin und 0,20 g (5 mMol) Natriumhydroxid in 8 ml Äthanol und 8 ml Wasser 5 Stunden bei 60 °C. Nach Abdampfen des Äthanols im Vakuum wird mit 2N Salzsäure auf pH 6 eingestellt und mit Äthylacetat mehrfach extrahiert. Die vereinigten Extrakte wäscht man mit Wasser, trocknet sie über Natriumsulfat, filtriert sie und dampft im Vakuum ein. Den festen Eindampfrückstand kristallisiert man aus Methanol um.
Ausbeute : 45,8 % der Theorie,
Schmelzpunkt : 212-215 °C.

| | | | |
|---|---|---|---|
| Ber. : | C 62,76 | H 6,02 | N 10,45 |
| Gef. : | 62,90 | 6,07 | 10,44 |

Beispiel 251

$N^1$-(1-(4-Carboxyphenyl)-äthyl)-$N^2$-(5-chlor-2-dimethylamino-benzoyl)-hydrazin

Hergestellt aus $N^1$-(1-(4-Äthoxycarbonyl-phenyl)-äthyl)-$N^2$-(5-chlor-2-dimethylamino-benzoyl)-hydrazin durch Verseifung mit Natriumhydroxid analog Beispiel 250.
Ausbeute : 34,4 % der Theorie,
Schmelzpunkt : 210-212 °C.

| | | | |
|---|---|---|---|
| Ber. : | C 59,75 | H 5,57 | H 11,61 |
| Gef. : | 59,32 | 5,60 | 11,41 |

Beispiel 252

4-[1-(5-Chlor-2-octamethylenimino-benzoylaminoxy)-äthyl]-benzoesäure-methylester

Man rührt 2 g (7,1 mMol) 5-Chlor-2-octamethylenimino-benzoesäure und 1,2 g (7,4 mMol) Carbonyldiimidazol in 10 ml wasserfreiem Pyridin 40 Minuten bei 20 °C. Dann gibt man 1,4 g (7,2 mMol) 4-(1-Aminoxy-äthyl)-benzoesäure-methylester [frisch hergestellt analog Beispiel 248 aus 4-(1-Aminoxy-äthyl)-benzoesäure-methylester-hydrochlorid vom Schmelzpunkt 158-160 °C] hinzu und rührt über Nacht bei 100 °C. Man engt im Vakuum zur Trockne ein und reinigt den Eindampfrückstand durch Säulenchromatographie an Kieselgel (Toluol/Essigester = 9 : 1).
Ausbeute : 41 % der Theorie,
Schmelzpunkt : < 20 °C.

Beispiel 253

3-(5-Chlor-2-octamethylenimino-benzoylaminoxy-methyl)-benzoesäure-methylester

Hergestellt aus 5-Chlor-2-octamethylenimino-benzoesäure, Carbonyl-diimidazol und 4-(Aminoxy-methyl)-benzoesäure-methylester [frisch hergestellt aus dem Hydrochlorid vom Schmelzpunkt 252-255 °C] in wasserfreiem Pyridin analog Beispiel 252.
Ausbeute : 38 % der Theorie,
Schmelzpunkt : < 20 °C.
Ber. : Molpeak m/e = 444/446 (1 Cl)
Gef. : Molpeak m/e = 444/446 (1 Cl)

Beispiel 254

3-[1-(5-Chlor-2-dimethylamino-benzoylaminoxy)-äthyl]-benzoesäure-methylester

Hergestellt aus 5-Chlor-2-dimethylamino-benzoesäure, Carbonyldiimidazol und 4-(1-Aminoxy-äthyl)-benzoesäure-methylester analog Beispiel 252 aber in wasserfreiem Tetrahydrofuran bei 20 °C und 16 Stunden Reaktionszeit.

Ausbeute : 59 % der Theorie,
Schmelzpunkt : < 20 °C.

Beispiel 255

4-[1-(5-Chlor-2-(cis-3,5-dimethyl-piperidino)-benzoylaminoxy)-äthyl]-benzoesäure-methylester

Hergestellt aus 5-Chlor-2-(cis-3,5-dimethyl-piperidino)-benzoesäure, Carbonyldiimidazol und 4-(1-Aminoxy-äthyl)-benzoesäure-methylester analog Beispiel 252.

Ausbeute : 85 % der Theorie,
Schmelzpunkt : < 20 °C.

Beispiel 256

4-[1-(5-Chlor-2-piperidino-benzoylaminoxy)-äthyl]-benzoesäure-methylester

Man rührt 7,55 g (25,8 mMol) 5-Chlor-2-piperidino-benzhydroxamsäure-Kaliumsalz (Schmelzpunkt : 153 °C (Zers.)) und 6,30 g 4-(1-Brom-äthyl)-benzoesäure-methylester in 20 ml Dimethylformamid 18 Stunden bei 20 °C, versetzt dann mit der dreifachen Menge Wasser und extrahiert mit Äther. Den getrockneten und filtrierten Äther-Extrakt dampft man im Vakuum ein. Den Eindampfrückstand reinigt man durch Säulenchromatographie an Kieselgel (Cyclohexan/Äthylacetat = 1/1).

Ausbeute : 69,2 % der Theorie,
Schmelzpunkt : < 20 °C.

| | | | | |
|---|---|---|---|---|
| Ber. : | C 63,37 | H 6,04 | Cl 8,50 | N 6,72 |
| Gef. : | 63,54 | 6,17 | 8,49 | 6,63 |

Beispiel 257

4-(5-Chlor-2-dimethylamino-benzoylaminoxy-methyl)-benzoesäure

Zu 4,8 g (19 mMol) 5-Chlor-2-dimethylamino-benzhydroxamsäure-Kaliumsalz (Schmelzpunkt : 140 °C (Zers.)) und 1,06 g (19 mMol) Kaliumhydroxid in 50 ml Äthanol/Wasser (1/1) gibt man 4,2 g (19 mMol) 4-Brommethyl-benzoesäure und erhitzt 6 Stunden zum Rückfluß. Man dampft im Vakuum ein und löst den Eindampfrückstand in Wasser unter Zusatz von Kalilauge. Nach Extraktion mit Äthylacetat stellt man die wässrige Phase auf pH 7 und extrahiert erneut mit Äthylacetat. Diesen Äthylacetat-Extrakt trocknet und filtriert man und dampft ihn im Vakuum ein. Den Eindampfrückstand reinigt man durch zweimalige Säulenchromatographie an Kieselgel (Chloroform/Methanol = 9/1 und 4/1). Die einheitlichen Fraktionen vereinigt man, dampft sie ein und verteilt den Eindampfrückstand zwischen Wasser und Äthylacetat. Aus dem Äthylacetat-Extrakt erhält man durch Eindampfen im Vakuum ein fast farbloses Öl, das beim Verreiben mit Äther kristallisiert.

Ausbeute : 1,5 % der Theorie,
Schmelzpunkt : 160-161 °C.

| | | | |
|---|---|---|---|
| Ber. : | C 58,53 | H 4,91 | N 8,03 |
| Gef. : | 58,49 | 5,10 | 7,88 |

Beispiel 258

4-[1-(5-Chlor-2-piperidino-benzoylaminoxy)-äthyl]-benzoesäure

Man erhitzt eine Lösung von 6,0 g (14,4 mMol) 4-[1-(5-Chlor-2-piperidino-benzoylaminoxy)-äthyl]-benzoesäure-methylester und 1,15 g (28,8 mMol) Natriumhydroxid in 100 ml Äthanol 6 Stunden bei 50-60 °C. Nach Eindampfen im Vakuum verteilt man zwischen verdünnter Salzsäure und Chloroform. Den getrockneten und filtrierten Chloroform-Extrakt dampft man im Vakuum ein und kristallisiert den Eindampfrückstand aus einem Chloroform/Methanol-Gemisch unter Zusatz von Petroläther um.

Ausbeute : 44,8 % der Theorie,
Schmelzpunkt : 201-203 °C.

| | | | | |
|---|---|---|---|---|
| Ber. : | C 62,61 | H 5,76 | Cl 8,80 | N 6,96 |
| Gef. : | 62,68 | 5,67 | 8,76 | 6,96 |

Beispiel 259

4-[1-(5-Chlor-2-dimethylamino-benzoylaminoxy)-äthyl]-benzoesäure

Hergestellt aus 4-[1-(5-Chlor-2-dimethylamino-benzoylaminoxy)-äthyl]-benzoesäure-methylester durch Verseifen mit Natriumhydroxid in Äthanol/Wasser analog Beispiel 248 ; es wurde jedoch mit Äthylacetat extrahiert und aus Äthanol umkristallisiert.

Ausbeute : 65,7 % der Theorie,
Schmelzpunkt : 202-205 °C.

| | | | | |
|---|---|---|---|---|
| Ber. : | C 59,58 | H 5,28 | Cl 9,77 | N 7,72 |
| Gef. : | 59,70 | 5,34 | 10,00 | 7,90 |

Beispiel 260

4-[1-(5-Chlor-2-(cis-3,5-dimethyl-piperidino)-benzoylaminoxy)-äthyl]-benzoesäure

Hergestellt aus 4-[1-(5-Chlor-2-(cis-3,5-dimethyl-piperidino)-benzoylaminoxy)-äthyl]-benzoesäure-methylester durch Verseifen mit Natriumhydroxid in Äthanol/Wasser analog Beispiel 248.

Ausbeute : 63,8 % der Theorie,
Schmelzpunkt : 205-208 °C.

| | | | | |
|---|---|---|---|---|
| Ber. : | C 64,10 | H 6,32 | Cl 8,23 | N 6,50 |
| Gef. : | 64,40 | 6,66 | 8,44 | 6,50 |

Beispiel 261

4-(5-Chlor-2-octamethylenimino-benzoylaminoxy-methyl)-benzoesäure

Hergestellt aus 4-(5-Chlor-2-octamethylenimino-benzoylaminoxymethyl)-benzoesäure-methylester durch Verseifen mit Natriumhydroxid in Äthanol/Wasser analog Beispiel 248.

Ausbeute : 43,1 % der Theorie,
Schmelzpunkt : 135-138 °C (aus Äther).

| | | | | |
|---|---|---|---|---|
| Ber. : | C 64,10 | H 6,32 | Cl 8,23 | N 6,50 |
| Gef. : | 64,29 | 6,29 | 8,33 | 6,73 |

Beispiel 262

4-[1-(5-Chlor-2-octamethylenimino-benzoylaminoxy)-äthyl]-benzoesäure

Hergestellt aus 4-[1-(5-Chlor-2-octamethylenimino-benzoylaminoxy)-äthyl]-benzoesäure-methylester durch Verseifen mit Natriumhydroxid in Äthanol/Wasser analog Beispiel 248.

Ausbeute : 69 % der Theorie,
Schmelzpunkt : 187-190 °C.

| | | | | |
|---|---|---|---|---|
| Ber. : | C 64,78 | H 6,57 | Cl 7,97 | N 6,30 |
| Gef. : | 64,60 | 6,58 | 7,88 | 6,16 |

Beispiel 263

3-[4-[2-(5-Chlor-2-piperidino-benzoylamino)-äthyl]-phenyl]-propionsäure-äthylester

1,2 g (5 mMol) 5-Chlor-2-piperidino-benzoesäure, 1,5 g (5,8 mMol) 3-[4-(2-Amino-äthyl)-phenyl]-propionsäure-äthylester-hydrochlorid und 1,84 g (7 mMol) Triphenylphosphin werden in 30 ml absolutem Acetonitril vorgelegt und nacheinander mit 0,5 ml (5 mMol) Tetrachlorkohlenstoff und 2,45 ml (17,5 mMol) Triäthylamin versetzt. Nachdem man 20 Stunden bei Raumtemperatur gerührt hat, wird vom Niederschlag abfiltriert und das Filtrat eingeengt. Der Einengungsrückstand wird an Kieselgel in Toluol/Essigester = 5 : 1 säulenchromatographisch gereinigt.

Ausbeute : 1,2 g (54,5 % der Theorie),
Schmelzpunkt : < 20 °C.

| | | | | |
|---|---|---|---|---|
| Ber. : | C 67,78 | H 7,05 | N 6,32 | Cl 8,00 |
| Gef. : | 67,33 | 6,91 | 6,18 | 8,09 |

Beispiel 264

3-[4-[2-(5-Chlor-2-piperidino-benzoylamino)-äthyl]-phenyl]-propionsäure

Hergestellt durch alkalische Verseifung von 3-[4-[2-(5-Chlor-2-piperidino-benzoylamino)-äthyl]-phenyl]-propionsäure-äthylester analog Beispiel 166.

Ausbeute : 80 % der Theorie,
Schmelzpunkt : 139 °C.

| | | | | |
|---|---|---|---|---|
| Ber. : | C 66,57 | H 6,56 | N 6,75 | Cl 8,54 |
| Gef. : | 66,51 | 6,62 | 6,60 | 8,40 |

Beispiel 265

3-[4-[2-(5-Chlor-2-octamethylenimino-benzoylamino)-äthyl]-phenyl]-propionsäure-äthylester

Hergestellt aus 5-Chlor-2-octamethylenimino-benzoesäure und 3-[4-(2-Amino-äthyl)-phenyl]-propionsäure-äthylester-hydrochlorid analog Beispiel 263.
Ausbeute : 76 % der Theorie,
Schmelzpunkt : < 20 °C.

| | | | | |
|---|---|---|---|---|
| Ber. : | C 69,33 | H 7,69 | N 5,78 | Cl 7,31 |
| Gef. : | 69,22 | 7,59 | 5,66 | 7,26 |

Beispiel 266

3-[4-[2-(5-Chlor-2-octamethylenimino-benzoylamino)-äthyl]-phenyl]-propionsäure

Hergestellt aus 3-[4-[2-(5-Chlor-2-octamethylenimino-benzoylamino)-äthyl]-phenyl]-propionsäure-äthylester durch alkalische Verseifung analog Beispiel 166.
Ausbeute : 79 % der Theorie,
Schmelzpunkt : 92-94 °C.

| | | | | |
|---|---|---|---|---|
| Ber. : | C 68,33 | H 7,28 | N 6,13 | Cl 7,76 |
| Gef. : | 68,54 | 7,38 | 6,28 | 7,81 |

Beispiel 267

4-[2-(2-Piperidino-5-propyl-benzoylamino)-äthyl]-benzoesäure-äthylester

Hergestellt aus 1-(5-Brom-pentyl)-6-propyl-4H-3,1-benzoxazin-2,4-(1H)-dion und 4-(2-Amino-äthyl)-benzoesäure-äthylester analog Beispiel 217.
Ausbeute : 41 % der Theorie,
Schmelzpunkt : < 20 °C.

| | | | |
|---|---|---|---|
| Ber. : | C 73,90 | H 8,11 | N 6,63 |
| Gef. : | 73,45 | 7,92 | 6,42 |

Beispiel 268

4-[2-(5-Butyl-2-piperidino-benzoylamino)-äthyl]-benzoesäure-äthylester

Hergestellt aus 6-Butyl-1-(5-brom-pentyl)-4H-3,1-benzoxazin-2,4-(1H)-dion und 4-(2-Amino-äthyl)-benzoesäure-äthylester analog Beispiel 217.
Ausbeute : 59 % der Theorie,
Schmelzpunkt : < 20 °C.

| | | | |
|---|---|---|---|
| Ber. : | C 74,28 | H 8,31 | N 6,42 |
| Gef. : | 73,90 | 8,05 | 6,13 |

Beispiel 269

4-[2-(4-Chlor-5-nitro-2-piperidino-benzoylamino)-äthyl]-benzoesäure-äthylester

Hergestellt aus 4-Chlor-5-nitro-2-piperidino-benzoesäure und 4-(2-Amino-äthyl)-benzoesäure-äthylester analog Beispiel 165.
Ausbeute : 20 % der Theorie,
Schmelzpunkt : < 20 °C.

| | | | | |
|---|---|---|---|---|
| Ber. : | C 60,06 | H 5,70 | N 9,13 | Cl 7,70 |
| Gef. : | 60,20 | 5,78 | 9,25 | 7,85 |

Beispiel 270

4-[2-(5-Chlor-2-octamethylenimino-benzoylamino)-äthyl]-benzoesäure

Ein Gemisch von 0,45 g (1 mMol) 4-[2-(5-Chlor-2-octamethylenimino-benzoylamino)-äthyl]-benzaldehyd-hydrochlorid, 5 ml 0,5 N-Natronlauge und 0,30 g (1,3 mMol) Silberoxid erhitzt man unter starkem Rühren 45 Minuten auf dem Dampfbad. Nach Erkalten säuert man mit 2N-Schwefelsäure an und extrahiert mit Äther. Die organische Phase trocknet, filtriert man und dampft sie im Vakuum ein. Den Eindampfrückstand reinigt man durch Säulenchromatographie an Kieselgel (Chloroform/Methanol = 10/1).

Ausbeute : 34 % der Theorie,
Schmelzpunkt : 172 °C.

Beispiel 271

4-[2-(5-Chlor-2-octamethylenimino-benzoylamino)-äthyl]-benzoesäure-Natriumsalz

5 g (11,7 mMol) 4-[2-(5-Chlor-2-octamethylenimino-benzoylamino)-äthyl]-benzoesäure werden in 20 ml absolutem Tetrahydrofuran gelöst und mit einer Natriumäthylatlösung, hergestellt aus 0,27 g (11,7 mMol) Natrium und 10 ml Äthanol, versetzt, dabei fällt das Natriumsalz aus. Nach Zugabe von 80 ml Äther wird eine Stunde nachgerührt, abgesaugt und bei 80 °C im Umlufttrockenschrank getrocknet.

Ausbeute : 4,5 g (85 % der Theorie),
Schmelzpunkt : 290 °C.

| | | | |
|---|---|---|---|
| Ber. : | C 63,92 | H 6,26 | N 6,21 |
| Gef. : | 63,90 | 6,35 | 6,18 |

Beispiel 272

4-[2-(5-Chlor-2-octamethylenimino-benzoylamino)-äthyl]-benzoesäure-hydrochlorid

5 g (11,7 mMol) 4-[2-(5-Chlor-2-octamethylenimino-benzoylamino)-äthyl]-benzoesäure werden in 150 ml Aceton heiß gelöst und filtriert. Mit isopropanolischer Salzsäure wird das Hydrochlorid ausgefällt.

Ausbeute : 5 g (92 % der Theorie),
Schmelzpunkt : 205 °C.

| | | | | |
|---|---|---|---|---|
| Ber. : | C 61,93 | H 6,50 | N 6,01 | Cl 15,23 |
| Gef. : | 62,10 | 6,86 | 6,24 | 14,85 |

Beispiel 273

4-[2-(5-Äthyl-2-octamethylenimino-benzoylamino)-äthyl]-benzoesäure-äthylester

Hergestellt aus 5-Äthyl-2-octamethylenimino-benzoesäure und 4-(2-Amino-äthyl)-benzoesäure-äthylester analog Beispiel 165.

Ausbeute : 78 % der Theorie,
Schmelzpunkt : < 20 °C.

| | | | |
|---|---|---|---|
| Ber. : | C 74,63 | H 8,50 | N 6,22 |
| Gef. : | 74,41 | 8,10 | 6,01 |

Beispiel 274

4-[2-(5-Äthyl-2-octamethylenimino-benzoylamino)-äthyl]-benzoesäure

Hergestellt aus 4-[2-(5-Äthyl-2-octamethylenimino-benzoylamino)-äthyl]-benzoesäure-äthylester durch alkalische Verseifung analog Beispiel 166.

Ausbeute : 85 % der Theorie,
Schmelzpunkt : 145 °C.

| | | | |
|---|---|---|---|
| Ber. : | C 73,90 | H 8,11 | N 6,63 |
| Gef. : | 74,15 | 8,15 | 6,42 |

Beispiel 275

4-[2-(5-Chlor-2-octamethylenimino-benzoylamino)-äthyl]-benzoesäure-äthylester

5,6 g (0,02 Mol) 5-Chlor-2-octamethylenimino-benzoesäure werden in 50 ml absolutem Toluol mit 2 g (0,02 Mol) Triäthylamin versetzt und bei − 5 °C mit 2,2 g (0,02 Mol) Chlorameisensäure-äthylester in das gemischte Anhydrid übergeführt. Nach halbstündigem Rühren wird eine 4-(2-Amino-äthyl)-benzoesäure-äthylester-Lösung in 30 ml Chloroform zugesetzt, die aus 4,59 (0,02 Mol) 4-(2-Amino-äthyl)-benzoesäure-äthylester-hydrochlorid und der äquivalenten Menge 2 g (0,02 Mol) Triäthylamin hergestellt wurde. Nach dreistündigem Rühren bei Raumtemperatur wird mit verdünnter Salzsäure versetzt, die organischen Phasen abgetrennt und über Natriumsulfat getrocknet. Nach Abdestillieren der Lösungsmittel wird auf einer Kieselgelsäule mit dem Fließmittel Toluol/Essigsäureäthylester = 9 : 1 der als Nebenprodukt gebildete 4-(2-Äthoxycarbonylamino-äthyl)-benzoesäure-äthylester abgetrennt.

Ausbeute : 1,6 g (18 % der Theorie),
Schmelzpunkt : < 20 °C.

| | | | |
|---|---|---|---|
| Ber. : | C 68,33 | H 7,28 | N 6,13 |
| Gef. : | 68,62 | 7,25 | 5,90 |

Beispiel 276

4-[2-(5-Chlor-2-octamethylenimino-benzoylamino)-äthyl]-benzoesäure

5,6 g (0,02 Mol) 5-Chlor-2-octamethylenimino-benzoesäure werden in 50 ml absolutem Toluol mit 2 g (0,02 Mol) Triäthylamin versetzt und bei − 5 °C mit 2,2 g (0,02 Mol) Chlorameisensäure-äthylester in das gemischte Anhydrid übergeführt. Nach halbstündigem Nachrühren wird eine Lösung von 3,3 g (0,02 Mol) 4-(2-Amino-äthyl)-benzoesäure in 20 ml 1 N Natronlauge zugesetzt und bei Raumtemperatur 4 Stunden lang gerührt. Anschließend werden 30 ml Wasser zugesetzt, die organische Phase abgetrennt und nochmals mit Chloroform ausgeschüttelt. Aus der wässrigen Phase wird durch Ansäuern mit 1N Salzsäure auf pH 5,5 die Säure ausgefällt und aus Essigsäureäthylester umkristallisiert.

Ausbeute : 1,2 g (14 % der Theorie),
Schmelzpunkt : 172 °C.

| | | | |
|---|---|---|---|
| Ber. : | C 67,20 | H 6,81 | N 6,53 |
| Gef. : | 66,92 | 6,77 | 6,43 |

Beispiel 277

4-[2-(5-Chlor-2-octamethylenimino-benzoylamino)-äthyl]-benzoesäure

8,87 g (12,6 mMol) Kupfer(II)-di-[5-chlor-2-octamethylenimino-benzoat]-dihydrochlorid-Komplex [hergestellt aus 5-Amino-2-octamethylen-benzoesäure über das Diazoniumsalz mit Kupfer(I)-chlorid, Schmelzpunkt : 177-178 °C] werden in 25 ml absolutem Pyridin gelöst und unter Eiskühlung mit 3 g (25,3 mMol) Thionylchlorid tropfenweise versetzt, so daß die Temperatur bei 20-30 °C bleibt. Nach halbstündigem Nachrühren werden 4,9 g (25,3 mMol) 4-(2-Amino-äthyl)-benzoesäure-äthylester, gelöst in 5 ml absolutem Pyridin, zugesetzt und 3 Stunden auf 70 °C erwärmt. Nach Abdestillieren des Pyridins wird der Rückstand in einem Gemisch von 50 ml Methanol und 50 ml Dioxan gelöst und nach Zusatz von 4,6 g Kaliumhydroxid, gelöst in 90 ml Wasser, 16 Stunden bei Raumtemperatur gerührt. Anschließend werden die Lösungsmittel abdestilliert, der Eindampfrückstand in 140 ml Wasser gelöst, durch Einstellen des pH auf 5,5 das Produkt ausgefällt und aus Essigsäureäthylester umkristallisiert.

Ausbeute : 4,1 g (38 % der Theorie),
Schmelzpunkt : 172 °C.

| | | | | |
|---|---|---|---|---|
| Ber. : | C 67,20 | H 6,81 | N 6,52 | Cl 8,26 |
| Gef. : | 61,10 | 6,72 | 6,45 | 8,20 |

Beispiel 278

4-[2-(5-Chlor-2-octamethylenimino-benzoylamino)-äthyl]-benzoesäure-äthylester

10,5 g (15 mMol) Kupfer(II)-di-[5-chlor-2-octamethylenimino-benzoat]-dihydrochlorid-Komplex [hergestellt aus 5-Amino-2-octamethylenimino-benzoesäure über das Diazoniumsalz und Kupfer(I)-chlorid, Schmelzpunkt : 177-178 °C] werden in 25 ml absolutem Pyridin mit 5,4 g (33 mMol) Carbonyldiimidazol versetzt, wobei Kohlendioxidentwicklung auftritt. Nach halbstündigem Rühren bei Raumtemperatur werden 6,8 g (35 mMol) 4-(2-Amino-äthyl)-benzoesäure-äthylester, gelöst in 5 ml absolutem Pyridin, zugesetzt und anschließend 3 Stunden auf 70 °C erhitzt. Nach Abdestillieren des Pyridins wird der Rückstand in Wasser gelöst, bei pH 4 mit Chloroform ausgeschüttelt und die Chloroformauszüge über Natriumsulfat getrocknet. Nach dem Einengen wird der Rückstand über eine Kieselgelsäule mit dem Fließmittel Toluol/Essigsäure-äthylester chromatographisch gereinigt.

Ausbeute : 10 g (66 % der Theorie),
Schmelzpunkt : < 20 °C.

| | | | | |
|---|---|---|---|---|
| Ber. : | C 68,33 | H 7,28 | N 6,13 | Cl 7,75 |
| Gef. : | 68,30 | 7,22 | 5,91 | 7,66 |

Beispiel 279

4-[2-(5-Chlor-2-octamethylenimino-benzoylamino)-äthyl]-benzoesäure

8,4 g (0,02 Mol) 4-[2-(5-Chlor-2-octamethylenimino-benzoylamino)-äthyl]-chlorbenzol (Schmelzpunkt : 58 °C) werden in 80 ml Tetrahydrofuran gelöst, auf − 60 °C gekühlt und unter Stickstoff mit 18 ml (0,04 Mol) einer vorgekühlten 2,3 molaren Lösung von n-Butyl-Lithium in Hexan versetzt. Dieses Reaktionsgemisch wird nach 15 Minuten in ca. 10 g fein zerriebenes Kohlendioxid unter Stickstoff eingetragen. Nach dem Erwärmen auf Raumtemperatur wird eingedampft und der Abdampfrückstand in Wasser gelöst. Das Produkt wird durch Einstellen des pH auf 5,5 in Wasser ausgefällt, aus Essigsäureäthylester umkristallisiert und anschliessend chromatographisch gereinigt.

Ausbeute : 1 g (12 % der Theorie),

Schmelzpunkt : 172 °C.
Ber. : C 67,20 H 6,81 N 6,52 Cl 8,26
Gef. : 67,00 6,72 6,46 8,19

Beispiel 280

4-[2-(5-Chlor-2-octamethylenamino-benzoylamino)-äthyl]-benzoesäure

Zu einer Lösung von 11,5 g (0,03 Mol) 2-(5-Chlor-2-octamethylenimino-benzoylamino)-äthylbenzol in 50 ml Schwefelkohlenstoff werden bei 0-5 °C 7,6 g (0,06 Mol) Oxalylchlorid zugetropft und anschließend 8 g (0,06 Mol) Aluminiumchlorid eingetragen. Nach einstündigem Rühren werden nochmals die gleichen Mengen Oxalylchlorid und Aluminiumchlorid zugesetzt, und anschließend 2 bis 3 Stunden auf 50 °C erhitzt. Nach dem Versetzen der abgekühlten Lösung mit Eiswasser und Salzsäure wird mit Chloroform extrahiert. Der getrocknete Chloroformeindampfrückstand wird aus Essigsäureäthylester unter Verwendung von Aktivkohle zweimal umkristallisiert.
Ausbeute : 2,4 g (19 % der Theorie),
Schmelzpunkt : 172 °C
Ber. : C 67,20 H 6,81 N 6,52 Cl 8,26
Gef. : 67,11 6,45 6,45 8,19

Beispiel 281

4-[2-(5-Chlor-2-piperidino-benzoylamino)-äthyl]-benzoesäure

Zu einer gerührten Natriumhypobromit-Lösung [hergestellt aus 0,92 g (23 mMol) Natriumhydroxid, gelöst in 4,5 ml Wasser, und 0,36 ml (7 mMol) Brom unter Eiskühlung] tropft man innerhalb 15 Minuten bei 35-40 °C die Lösung von 0,60 g (1,56 mMol) 4-[2-(5-Chlor-2-piperidino-benzoylamino)-äthyl]-acetophenon in 6 ml Dioxan. Nach 40 Minuten bei 35-40 °C gibt man wässrige Natriumhydrogensulfit-Lösung und Wasser zu und dampft das Gemisch im Vakuum ein. Den Rückstand löst man in Wasser, säuert die Lösung mit 2N Salzsäure unter Kühlung an und nimmt den ausgefallenen Niederschlag in einem Äther/Äthylacetat-Gemisch auf. Die organische Phase trocknet man über Natriumsulfat, filtriert sie und dampft sie im Vakuum ein. Den fast farblosen festen Rückstand (0,45 g) kristallisiert man aus heißem Aceton um, filtriert und trocknet bei 110 °C/30 Torr.
Ausbeute : 0,06 g (10 % der Theorie),
Schmelzpunkt : 201-203 °C
Ber. : C 65,19 H 5,99 Cl 9,16 N 7,24
Gef. : 65,53 5,91 9,32 7,10

Beispiel 282

4-[2-(5-Chlor-2-octamethylenimino-benzoylamino)-äthyl]-benzoesäure

Hergestellt analog Beispiel 281 aus 4-[2-(5-Chlor-2-octamethylenimino-benzoylamino)-äthyl]-acetophenon mit Natriumhypobromit-Lösung.
Ausbeute : 11 % der Theorie,
Schmelzpunkt : 171-172 °C
Ber. : C 67,19 H 6,81 Cl 8,26 N 6,53
Gef. : 67,50 6,75 8,53 6,24

Beispiel 283

4-[2-(5-Chlor-2-piperidino-benzoylamino)-äthyl]-benzoesäure-äthylester

0,82 g (0,002 Mol) 4-[2-(2-Brom-5-chlor-benzoylamino)-äthyl]-benzoesäure-äthylester [Schmelzpunkt : 116-118 °C, hergestellt durch Umsetzung von 2-Brom-5-chlor-benzoesäure mit 4-(2-Aminoäthyl)-benzoesäure-äthylester analog Verfahren a)] werden mit 0,85 g (0,01 Mol) Piperidin und 1 Spatelspitze Kupferpulver eine Stunde unter Rühren auf 100 °C erhitzt. Nach dem Erkalten wird das Reaktionsgemisch mit Essigsäure angesäuert und 3 mal mit Chloroform extrahiert. Die vereinigten Chloroformphasen werden über Natriumsulfat getrocknet. Nach Abdestillieren des Lösungsmittels wird der verbleibende Rückstand auf einer Kieselgelsäule gereinigt (Laufmittel : Chloroform/Essigester = 19 : 1).
Ausbeute : 0,49 g (48 % der Theorie),
Ber. : Molpeak m/e : 414/416 (1 Cl)
Gef. : Molpeak m/e : 414/416 (1 Cl)

Beispiel 284

4-[2-(5-Chlor-2-piperidino-benzoylamino)-äthyl]-benzoesäure-äthylester

0,48 g (0,002 Mol) 5-Chlor-2-piperidino-benzoesäureamid (Schmelzpunkt : 140-142 °C, hergestellt aus 5-Chlor-2-piperidino-benzoesäure durch Umsetzung mit Carbonyldiimidazol und Ammoniak) werden mit 0,09 g (0,002 Mol) 55 %igem Natriumhydrid unter Rühren in 5 ml absolutem Toluol 10 Minuten auf ca. 60 °C erwärmt. Nach beendeter Wasserstoffentwicklung wird auf Raumtemperatur abgekühlt und die Lösung von 0,51 g (0,002 Mol) 4-(2-Brom-äthyl)-benzoesäure-äthylester ($M^+$ = 256/258 m/e 1 Br, hergestellt aus 4-(2-Hydroxyäthyl-benzoesäure-äthylester mit Thionylbromid) in 2 ml absolutem Toluol zugetropft. Danach wird noch 6 Stunden zum Rückfluß erhitzt. Nach Abkühlung der Reaktionsmischung wird mit wässrigem Äthanol versetzt und mit Chloroform mehrmals extrahiert. Die vereinigten Chloroformextrakte werden über Natriumsulfat getrocknet und das Lösungsmittel abdestilliert. Der Rückstand wird auf einer Kieselgelsäule gereinigt (Laufmittel : Chloroform/Essigester = 19 : 1).

Ausbeute : 0,2 g (25 % der Theorie),
Ber. : Molpeak m/e = 414/416 (1 Cl)
Gef. : Molpeak m/e = 414/416 (1 Cl)

Beispiel 285

4-[2-(5-Chlor-2-octamethylenimino-benzoylamino)-äthyl]-benzoesäure-äthylester

8,2 g (0,02 Mol) 4-[2-(5-Chlor-2-octamethylenimino-benzoylamino)-äthyl]-benzoesäurenitril (Schmelzpunkt : 102 °C) werden in 80 ml Äthanol gelöst und mit Chlorwasserstoff gesättigt. Nach 24 Stunden wird eine Stunde auf 50 °C erwärmt und die Lösungsmittel abdestilliert. Der Abdampfrückstand wird in Eiswasser gelöst, auf pH 9 gestellt und mit Chloroform ausgeschüttelt. Nach Einengen der organischen Phase wird der Rückstand über eine Kieselgelsäule chromatographisch gereinigt.

Ausbeute : 6,4 g (70 % der Theorie),
Schmelzpunkt : < 20 °C

| | | | | |
|---|---|---|---|---|
| Ber. : | C 68,33 | H 7,27 | N 6,12 | Cl 7,75 |
| Gef. : | 68,32 | 7,29 | 6,12 | 7,90 |

Beispiel A'

Tabletten mit 5 mg 4-[2-(5-Chlor-2-(3,5-dimethyl-piperidino)-benzoylamino)-äthyl]-benzoesäure

Zusammensetzung :

1 Tablette enthält :

| | | |
|---|---|---|
| Wirksubstanz | (1) | 5,0 mg |
| Maisstärke | (2) | 62,0 mg |
| Milchzucker | (3) | 48,0 mg |
| Polyvinylpyrrolidon | (4) | 4,0 mg |
| Magnesiumstearat | (5) | 1,0 mg |
| | | 120,0 mg |

Herstellungsverfahren :

1, 2, 3 und 4 werden gemischt und mit Wasser befeuchtet. Die feuchte Mischung wird durch ein Sieb mit 1,5 mm-Maschenweite gedrückt und bei ca. 45 °C getrocknet. Das trockene Granulat wird durch ein Sieb mit 1,0 mm-Maschenweite geschlagen und mit 5 vermischt. Die fertige Mischung preßt man auf einer Tablettenpresse mit Stempeln von 7 mm Durchmesser, die mit einer Teilkerbe versehen sind, zu Tabletten.

Tablettengewicht : 120 mg.

Beispiel B'

Dragées mit 2,5 mg 4-[2-(5-Chlor-2-octamethylenimino-benzoylamino)-äthyl]-benzoesäure

1 Dragéekern enthält :

| | | |
|---|---|---|
| Wirksubstanz | (1) | 2,5 mg |
| Kartoffelstärke | (2) | 44,0 mg |
| Milchzucker | (3) | 30,0 mg |
| Polyvinylpyrrolidon | (4) | 3,0 mg |
| Magnesiumstearat | (5) | 0,5 mg |
| | | 80,0 mg |

Herstellungsverfahren :

1, 2, 3 und 4 werden gut gemischt und mit Wasser befeuchtet. Die feuchte Masse drückt man durch ein Sieb mit 1 mm Maschenweite, trocknet bei ca. 45 °C und schlägt das Granulat anschließend durch dasselbe Sieb. Nach dem Zumischen von 5 werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichen aus Zucker und Talkum besteht. Die fertigen Dragées werden mit Wachs poliert.

Dragéegewicht : 120 mg

Beispiel C'

Tabletten mit 10 mg 4-[2-(5-Chlor-2-hexamethylenimino-benzoylamino)-äthyl]-benzoesäure

Zusammensetzung :

1 Tablette enthält :

| | |
|---|---|
| Wirksubstanz | 10,0 mg |
| Milchzucker, pulv. | 70,0 mg |
| Maisstärke | 31,0 mg |
| Polyvinylpyrrolidon | 8,0 mg |
| Magnesiumstearat | 1,0 mg |
| | 120,0 mg |

Herstellungsverfahren

Die Mischung aus der Wirksubstanz, Milchzucker und Maisstärke wird mit einer 20 %igen Lösung von Polyvinylpyrrolidon in Wasser befeuchtet. Die feuchte Masse wird durch ein Sieb mit 1,5 mm-Maschenweite granuliert und bei 45 °C getrocknet. Das getrocknete Granulat wird durch ein Sieb mit 1 mm-Maschenweite gerieben und mit Magnesiumstearat homogen vermischt.

Tablettengewicht : 120 mg

Stempel : 7 mm ∅ mit Teilkerbe

Beispiel D'

Dragées mit 5 mg 4-[2-(5-Chlor-2-hexamethylenimino-benzoylamino)-äthyl]-benzoesäure

1 Dragéekern enthält :

| | |
|---|---|
| Wirksubstanz | 5,0 mg |
| Calciumphosphat, sekundär | 70,0 mg |
| Maisstärke | 50,0 mg |
| Polyvinylpyrrolidon | 4,0 mg |
| Magnesiumstearat | 1,0 mg |
| | 130,0 mg |

Herstellungsverfahren

Die Mischung aus der Wirksubstanz, Calciumphosphat und Maisstärke wird mit einer 15 %igen Lösung von Polyvinylpyrrolidon in Wasser befeuchtet. Die feuchte Masse wird durch ein Sieb mit 1 mm-Maschenweite geschlagen, bei 45 °C getrocknet und anschliessend durch dasselbe Sieb gerieben. Nach dem Vermischen mit der angegebenen Menge Magnesiumstearat werden daraus Dragéekerne gepreßt.

Kerngewicht : 130 mg

Stempel : 7 mm ∅

Auf die so hergestellen Dragéekerne wird auf bekannte Art eine Schicht aus Zucker und Talkum aufgetragen. Die fertigen Dragées werden mit Wachs poliert.

Dragéegewicht : 180 mg

Beispiel E'

Tabletten mit 10 mg 4-[2-(5-Chlor-2-piperidino-benzoylamino)-äthyl]-benzoesäure

Zusammensetzung :

1 Tablette enthält :

| | |
|---|---:|
| Wirksubstanz | 10,0 mg |
| Milchzucker, pulv. | 70,0 mg |
| Maisstärke | 31,0 mg |
| Polyvinylpyrrolidon | 8,0 mg |
| Magnesiumstearat | 1,0 mg |
| | 120,0 mg |

Herstellungsverfahren

siehe analog Beispiel C'.

Beispiel F'

Dragées zu 5 mg 4-[2-(5-Chlor-2-piperidino-benzoylamino)-äthyl]-benzoesäure

1 Dragéekern enthält :

| | |
|---|---:|
| Wirksubstanz | 5,0 mg |
| Calciumphosphat, sekundär | 70,0 mg |
| Maisstärke | 50,0 mg |
| Polyvinylpyrrolidon | 4,0 mg |
| Magnesiumstearat | 1,0 mg |
| | 130,0 mg |

Herstellungsverfahren

siehe analog Beispiel D'.

**Ansprüche** (für die Vertragsstaaten : BE, CH, DE, FR, GB, IT, LI, LU, NL, SE)

1. Carbonsäureamide der allgemeinen Formel I

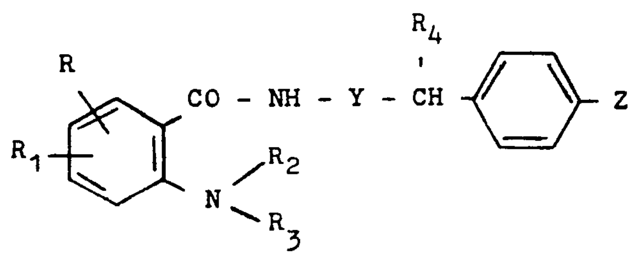

(I)

in der

R ein Wasserstoff-, Chlor- oder Bromatom oder eine cyclische Alkyleniminogruppe mit 4 bis 7 Kohlenstoffatomen im Iminoring,

$R_1$ ein Wasserstoff-, Fluor-, Chlor- oder Bromatom, eine Alkyl- oder Alkoxygruppe mit jeweils 1 bis 6 Kohlenstoffatomen, eine durch eine Phenylgruppe substituierte Alkoxygruppe mit 1 bis 3 Kohlenstoffatomen, eine Hydroxy-, Nitro-, Amino-, Cyano- oder Carboxygruppe, eine Alkanoylamino-, Alkoxycarbonyl- oder Dialkylamidosulfonylgruppe, wobei der Alkylteil jeweils 1 bis 3 Kohlenstoffatome enthalten kann,

$R_2$ und $R_3$, die gleich oder verschieden sein können, Alkylgruppen mit 1 bis 7 Kohlenstoffatomen, Alkenylgruppen mit 3 bis 7 Kohlenstoffatomen, Cycloalkylgruppen mit 3 bis 7 Kohlenstoffatomen, durch eine Phenylgruppe substituierte Alkylgruppen mit 1 bis 3 Kohlenstoffatomen, Phenyl- oder Adamantylgruppen oder

$R_2$ und $R_3$ zusammen mit dem dazwischenliegenden Stickstoffatom eine unverzweigte Alkyleniminogruppe mit 3 bis 12 Kohlenstoffatomen im Iminoring, eine unverzweigte Alkyleniminogruppe mit 4 bis 6 Kohlenstoffatomen im Iminoring, der durch eine oder zwei Alkylgruppen mit jeweils 1 bis 4 Kohlenstoffatomen, eine Alkoxygruppe mit 1 bis 3 Kohlenstoffatomen, eine Hydroxy-, Phenyl-, Carboxy- oder Alkoxycarbonylgruppe mit insgesamt 2 bis 4 Kohlenstoffatomen substituiert ist und/oder in dem eine Methylengruppe durch eine Iminogruppe, welche durch eine Alkylgruppe mit 1 bis 3 Kohlenstoffatomen, eine Alkoxycarbonylgruppe mit insgesamt 2 bis 4 Kohlenstoffatomen, eine Phenyl-, Halogenphenyl-, Benzyl-, Pyridyl- oder Furoylgruppe substituiert sein kann, durch ein Sauerstoff- oder Schwefelatom, durch eine Carbonyl-, Sulfoxid- oder Sulfonylgruppe ersetzt ist, eine gesättigte oder teilweise ungesättigte Azabicycloalkylgruppe mit 7 bis 10 Kohlenstoffatomen, eine in 3- und 5-Stellung durch insgesamt 3 oder 4 Alkylgruppen mit jeweils 1 bis 3 Kohlenstoffatomen substituierte Piperidinogruppe, eine 1,4-Dioxa-8-aza-spiroalkylgruppe mit 6 bis 9 Kohlenstoffatomen, eine Pyrrolyl- oder Tetrahydro-pyridinogruppe,

$R_4$ ein Wasserstoffatom oder eine Alkylgruppe mit 1 bis 3 Kohlenstoffatomen,

Y ein Sauerstoffatom, eine Iminogruppe oder eine gegebenenfalls durch eine oder zwei Alkylgruppen mit jeweils 1 bis 3 Kohlenstoffatomen substituierte Methylengruppe,

Z ein Wasserstoff- oder Halogenatom, eine Carboxy-, Cyano-, Formyl-, Hydroxymethyl-, Hydroxycarbonyläthylen-, Nitro-, Amino-, Allyloxycarbonyl-, Phenoxycarbonyl-, Benzyloxycarbonyl- oder Phenyläthoxycarbonylgruppe, eine Alkoxycarbonylgruppe mit insgesamt 2 bis 8 Kohlenstoffatomen, eine Cycloalkoxycarbonylgruppe mit insgesamt 4 bis 8 Kohlenstoffatomen eine gegebenenfalls durch 2 oder 3 Alkoxygruppen, durch eine Carboxy-, Alkoxycarbonyl- oder Äthylendioxygruppe substituierte Methylgruppe, wobei die Alkoxygruppe jeweils 1 bis 3 Kohlenstoffatome enthalten kann, eine gegebenenfalls durch eine Carboxygruppe oder Alkoxycarbonylgruppe mit insgesamt 2 bis 4 Kohlenstoffatomen substituierte Acetylgruppe, eine durch eine oder zwei Alkoxycarbonylgruppen mit insgesamt je 2 bis 4 Kohlenstoffatomen oder durch zwei Carboxygruppen substituierte Äthyl- oder Äthylengruppe, eine gegebenenfalls durch eine Alkylgruppe mit 1 bis 7 Kohlenstoffatomen, eine Cycloalkylgruppe mit 3 bis 7 Kohlenstoffatomen und/oder Alkenylgruppe mit 3 bis 7 Kohlenstoffatomen mono- oder disubstituierte Aminocarbonylgruppe, eine Piperidinocarbonyl-, Morpholinocarbonyl-, Thiomorpholinocarbonyl- oder N-Alkyl-piperazinocarbonylgruppe, wobei die Alkylgruppe 1 bis 3 Kohlenstoffatome enthalten kann, oder auch eine durch eine Carboxygruppe substituierte Äthylgruppe, wenn die Reste $R_2$ und $R_3$ zusammen mit dem dazwischenliegenden Stickstoffatom einen der eingangs erwähnten cyclischen Iminoreste darstellen, bedeuten, und deren physiologisch verträgliche Salze mit anorganischen oder organischen Säuren und auch Basen, wenn Z eine Carboxygruppe darstellt oder enthält.

2. Carbonsäureamide der allgemeinen Formel I gemäß Anspruch 1, in der

R ein Wasserstoffatom,

$R_1$ ein Wasserstoff-, Fluor-, Chlor- oder Bromatom, eine Alkoxygruppe mit 1 bis 6 Kohlenstoffatomen, eine Alkylgruppe mit 1 bis 4 Kohlenstoffatomen, eine Alkoxycarbonylgruppe mit insgesamt 2 bis 4 Kohlenstoffatomen, eine Hydroxy-, Cyano-, Carboxy-, Nitro-, Amino-, Acetamido-, Dimethylaminosulfonyl- oder Benzyloxygruppe,

$R_2$ eine Alkylgruppe mit 1 bis 6 Kohlenstoffatomen, eine Cyclohexyl-, Phenyl-, Benzyl-, Adamantyl- oder Allylgruppe,

$R_3$ eine Alkylgruppe mit 1 bis 6 Kohlenstoffatomen oder eine Allygruppe oder

$R_2$ und $R_3$ zusammen mit dem dazwischenliegenden Stickstoffatom eine unverzweigte Alkyleniminogruppe mit 4 bis 12 Kohlenstoffatomen im Iminoring, eine durch eine Alkylgruppe mit 1 bis 4 Kohlenstoffatomen, eine Phenyl-, Hydroxy-, Methoxy-, Carboxy- oder Alkoxycarbonylgruppe mit insgesamt 2 bis 4 Kohlenstoffatomen substituierte Piperidinogruppe, eine in 3- und 5-Stellung je durch eine Alkylgruppe mit 1 bis 3 Kohlenstoffatomen substituierte Piperidinogruppe, eine in 3- und 5-Stellung je durch zwei Alkylgruppen mit jeweils 1 bis 3 Kohlenstoffatomen substituierte Piperidinogruppe, eine gegebenenfalls durch eine oder zwei Methylgruppen substituierte Morpholino-, Thiomorpholino-, 1-Oxido-thiomorpholino- oder 1,1-Dioxido-thiomorpholinogruppe, eine gegebenenfalls in 4-Stellung durch eine Methyl-, Benzyl-, Phenyl-, Chlorphenyl-, Pyridyl-, Furoyl- oder Alkoxycarbonylgruppe mit insgesamt 2 bis 4 Kohlenstoffatomen substituierte Piperazinogruppe, eine Pyrrol-yl-, Piperidon-(2)-yl-(1)-, 1,2,3,6-Tetrahydro-pyridino-, 1,4-Dioxa-8-aza-spiro[4,5]decan-8-yl, 1,4-Dioxa-8-aza-spiro[4,6]undecan-8-yl-, Octahydro-isoindol-2-yl-, 1,2,3,4-Tetrahydro-isochinolin-2-yl-, 1,2,3,4,5,6,7,8-Octahydro-isochinolin-2-yl, Decahydro-isochinolin-2-yl-, 1,2,4,5-Tetrahydro-3-benzazepin-3-yl-, Decahydro-3-benzazepin-3-yl- oder 3-Aza-bicyclononan-3-yl-gruppe,

$R_4$ ein Wasserstoffatom oder eine Methylgruppe,

Y eine Methylen-, Methyl-methylen- oder Dimethyl-methylengruppe, eine NH-Gruppe oder ein Sauerstoffatom und

Z eine Carboxy-, Cyano-, Formyl- oder Hydroxymethylgruppe, eine Alkoxycarbonylgruppe mit insgesamt 2 bis 7 Kohlenstoffatomen, eine Cyclohexyloxycarbonyl-, Benzyloxycarbonyl-, Diäthoxymethyl-, Hydroxycarbonylmethyl-, Bis-2,2-äthoxycarbonyl-äthyl-, 2-Hydroxycarbonyl-äthylen- oder 2-Äthoxycarbonyl-äthylgruppe, eine gegebenenfalls durch eine Hydroxycarbonyl- oder Äthoxycarbonylgruppe substituierte Acetylgruppe oder auch eine 2-Hydroxycarbonyl-äthylgruppe, wenn die Reste $R_2$ und $R_3$ zusammen mit dem dazwischenliegenden Stickstoffatom einen der eingangs erwähnten cyclischen Iminoreste darstellen, bedeuten, und deren physiologisch verträgliche Salze mit anorganischen oder organischen Säuren und auch Basen, wenn Z eine Carboxygruppe darstellt oder enthält.

3. Carbonsäureamide der allgemeinen Formel I gemäß Anspruch 1, in der

$R_1$ sich in Position 5 des Phenylkernes befindet und

R, $R_1$ bis $R_4$, Y und Z wie im Anspruch 2 definiert sind, und deren physiologisch verträgliche Salze mit anorganischen oder organischen Säuren oder auch Basen, wenn Z eine Carboxygruppe darstellt oder enthält.

4. Carbonsäureamide der allgemeinen Formel I gemäß Anspruch 1, in der

R ein Wasserstoffatom,

$R_1$ in 5-Position ein Wasserstoff-, Fluor-, Chlor- oder Bromatom, eine Alkyl- oder Alkoxygruppe mit jeweils 1 bis 3 Kohlenstoffatomen, eine Carboxy-, Cyano- oder Nitrogruppe,

$R_2$ und $R_3$ zusammen mit dem dazwischenliegenden Stickstoffatom eine N,N-Dialkylamino- oder N-Alkyl-cyclohexylaminogruppe mit jeweils 1 bis 3 Kohlenstoffatomen im Alkylteil, eine Pyrrolidino-, Piperidino-, Hexamethylenimino-, Heptamethylenimino-, Octamethylenimino- oder Nonamethyleniminogruppe, eine durch eine Alkylgruppe mit 1 bis 4 Kohlenstoffatomen, durch eine Methoxy- oder Phenylgruppe substituierte Piperidinogruppe, eine in 3- und 5-Stellung jeweils durch eine oder zwei Methyl- oder Äthylgruppen substituierte Piperidinogruppe, ein gegebenenfalls in 2- und 6-Stellung je durch eine Methylgruppe substituierte Morpholino- oder Thiomorpholinogruppe, eine 1,4-Dioxa-8-aza-spiro[4,5]decan-8-yl-, 1,4-Dioxa-8-aza-spiro[4,6]undecan-yl-, Octahydro-isoindol-2-yl-, 1,2,3,4-Tetrahydro-isochinolin-2-yl-, 1,2,3,4,5,6,7,8-Octahydro-isochinolin-2-yl, Decahydro-isochinolin-2-yl-, 1,2,4,5-Tetrahydro-3H-3-benzazepin-3-yl-, Decahydro-3H-3-benzazepin-3-yl-, 3-Aza-bicylco[3,2,2]nonan-3-yl- oder N-Methyl-adamantyl-(1)-amino-Gruppe,

$R_4$ ein Wasserstoffatom oder eine Methylgruppe,

Y eine Methylen-, Methyl-methylen-, Dimethyl-methylen- oder NH-Gruppe oder ein Sauerstoffatom,

Z eine Alkoxycarbonylgruppe mit insgesamt 2 bis 4 Kohlenstoffatomen, eine Carboxy-, Formyl- oder Hydroxymethylgruppe bedeuten, und deren physiologisch verträgliche Salze mit anorganischen oder organischen Säuren und auch Basen, wenn Z die Carboxygruppe darstellt.

5. Carbonsäureamide der allgemeinen Formel I gemäß Anspruch 3, in der

R, $R_4$, Y und Z wie im Anspruch 3 definiert sind,

$R_1$ ein Chlor- oder Bromatom, eine Methyl-, Äthyl- oder Methoxygruppe,

$R_2$ und $R_3$ zusammen mit dem dazwischenliegenden Stickstoffatom eine Pyrrolidino-, Piperidino-, Methyl-piperidino-, 4-Methoxy-piperidino-, 4-Phenyl-piperidino-, Hexamethylenimino-, Heptamethylenimino-, Octamethylenimino-, Nonamethylenimino-, 3,5-Dimethyl-piperidino-, Morpholino-, 2,6-Dimethylmorpholino-, Thiomorpholino-, 2,6-Dimethylthiomorpholino-, 1,4-Dioxa-8-aza-spiro[4,5]decan-8-yl-, 1,2,3,4-Tetrahydro-isochinolin-2-yl-, Decahydro-isochinolin-2-yl-, 1,2,4,5-Tetrahydro-3H-3-benzazepin-3-yl-, Decahydro-3H-3-benzazepin-3-yl-, Octahydro-isoindol-2-yl- oder N-Methyl-adamantyl-(1)-amino-gruppe oder eine in 4-Stellung durch eine Alkylgruppe mit 2 bis 4 Kohlenstoffatomen substituierte Piperidinogruppe bedeuten, und deren physiologisch verträgliche Salze mit anorganischen oder organischen Säuren und auch Basen, wenn Z die Carboxygruppe darstellt.

6. 4-[2-(5-Chlor-2-piperidino-benzoylamino)-äthyl]-benzoesäure, deren Alkylester mit 1 bis 3 Kohlenstoffatomen und deren Säureadditionssalze.

7. 4-[2-(5-Chlor-2-nonamethylenimino-benzoylamino)-äthyl]-benzoesäure, deren Alkylester mit 1 bis 3 Kohlenstoffatomen und deren Säureadditionssalze.

8. 4-[2-(5-Chlor-2-(1,4-dioxa-8-aza-spiro[4,5]decan-8-yl)-benzoylamino)-äthyl]-benzoesäure, deren Alkylester mit 1 bis 3 Kohlenstoffatomen und deren Säureadditionssalze.

9. 4-[2-(5-Chlor-2-octamethylenimino-benzoylamino)-äthyl]-benzoesäure, deren Alkylester mit 1 bis 3 Kohlenstoffatomen und deren Säureadditionssalze.

10. 4-[2-(5-Brom-octamethylenimino-benzoylamino)-äthyl]-benzoesäure, deren Alkylester mit 1 bis 3 Kohlenstoffatomen und deren Säureadditionssalze.

11. 4-[2-(5-Chlor-2-(cis-3,5-dimethylpiperidino)-benzoylamino)-äthyl]-benzoesäure, deren Alkylester mit 1 bis 3 Kohlenstoffatomen und deren Säureadditionssalze.

12. Arzneimittel, enthaltend eine Verbindung gemäß den Ansprüchen 1-11 neben einem oder mehreren inerten Trägerstoffen und/oder Verdünnungsmitteln.

13. Arzneimittel gemäß Anspruch 12 zur Behandlung des Diabetes mellitus.

14. Verfahren zur Herstellung von neuen Carbonsäureamiden der allgemeinen Formel I gemäß den Ansprüchen 1 bis 11, sowie von deren physiologisch verträglichen Salzen mit anorganischen oder organischen Säuren und auch Basen, wenn Z eine Carboxygruppe darstellt oder enthält, dadurch gekennzeichnet, daß

a) eine Aminobenzoesäure der allgemeinen Formel II

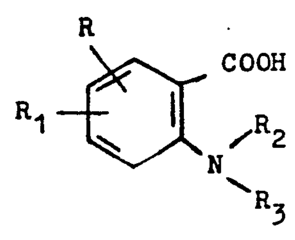

(II)

in der R, $R_1$, $R_2$ und $R_3$ wie eingangs definiert sind, oder deren gegebenenfalls im Reaktionsgemisch hergestelltes reaktionsfähiges Derivat mit einem Amin der allgemeinen Formel III

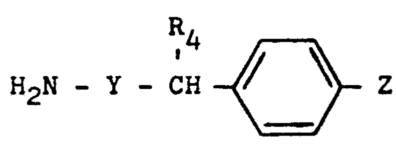

(III)

in der $R_4$, Y und Z wie eingangs definiert sind, oder mit einem gegebenenfalls im Reaktionsgemisch gebildeten N-aktivierten Amin der allgemeinen Formel III, wenn eine Aminobenzoesäure der allgemeinen Formel II eingesetzt wird und wenn Z in einem N-aktivierten Amin der allgemeinen Formel III keine Carboxy- oder Aminogruppe enthält, umgesetzt wird oder

b) zur Herstellung von Verbindungen der allgemeinen Formel I, in der $R_1$ mit Ausnahme der Hydroxy- und Aminogruppe wie eingangs definiert ist und Y eine gegebenenfalls durch eine oder zwei Alkylgruppen mit jeweils 1 bis 3 Kohlenstoffatomen substituierte Methylengruppe darstellt, eine Verbindung der allgemeinen Formel IV

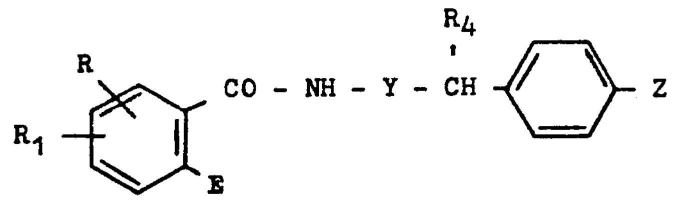

(IV)

in der

R, $R_4$ und Z wie eingangs definiert sind,

$R_1$ und Y die oben erwähnten Bedeutungen besitzen und E ein Halogenatom darstellt, mit einem Amin der allgemeinen Formel V

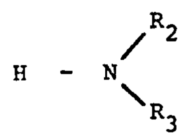

(V)

in der $R_2$ und $R_3$ wie eingangs definiert sind, umgesetzt wird oder

c) zur Herstellung von Verbindungen der allgemeinen Formel I, in der Z eine Carboxygruppe und Y keine NH-Gruppe darstellt, eine Verbindung der allgemeinen Formel VI

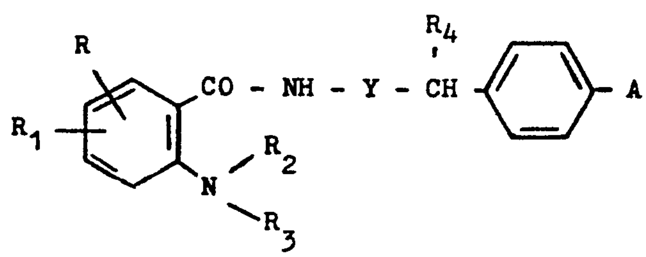

(VI)

in der

R und $R_1$ bis $R_4$ wie eingangs definiert sind,

Y mit Ausnahme der NH-Gruppe wie eingangs definiert ist und

A eine durch Oxidation in eine Carboxygruppe überführbare Gruppe bedeutet, oxydiert wird oder

d) zur Herstellung von Verbindungen der allgemeinen Formel I, in der Z eine Carboxygruppe darstellt, eine Verbindung der allgemeinen Formel VII

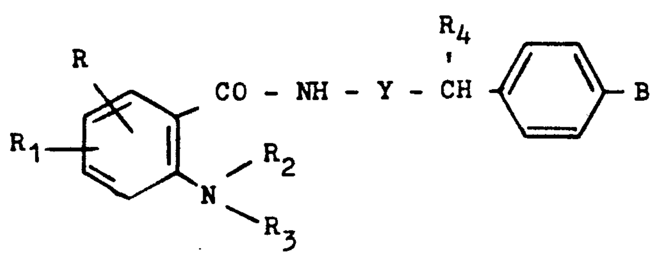

(VII)

in der R, $R_1$ bis $R_4$ und Y wie eingangs definiert sind und B eine durch Hydrolyse in eine Carboxygruppe überführbare Gruppe darstellt, hydrolysiert wird oder

e) zur Herstellung von Verbindungen der allgemeinen Formel I, in der $R_2$ eine Alkylgruppe mit 1 bis 7 Kohlenstoffatomen, eine Cycloalkylgruppe mit 3 bis 7 Kohlenstoffatomen, eine durch eine Phenylgruppe substituierte Alkylgruppe mit 1 bis 3 Kohlenstoffatomen oder eine Alkenylgruppe mit 3 bis 7 Kohlenstoffatomen, $R_3$ eine Alkylgruppe mit 1 bis 7 Kohlenstoffatomen, eine Cycloalkylgruppe mit 3 bis

7 Kohlenstoffatomen, eine Alkenylgruppe mit 3 bis 7 Kohlenstoffatomen, eine durch eine Phenylgruppe substituierte Alkylgruppe mit 1 bis 3 Kohlenstoffatomen oder eine Adamantylgruppe oder $R_2$ und $R_3$ mit dem dazwischenliegenden Stickstoffatom einen 5- bis 7-gliedrigen Alkyleniminoring, eine durch eine Alkylgruppe mit 1 bis 4 Kohlenstoffatomen substituierte Piperidinogruppe oder eine in 3- und 5-Stellung jeweils durch eine Alkylgruppe mit 1 bis 3 Kohlenstoffatomen substituierte Piperidinogruppe und Y eine gegebenenfalls durch eine oder zwei Alkylgruppen mit jeweils 1 bis 3 Kohlenstoffatomen substituierte Methylengruppe bedeuten, eine gegebenenfalls im Reaktionsgemisch gebildete Verbindung der allgemeinen Formel VIII

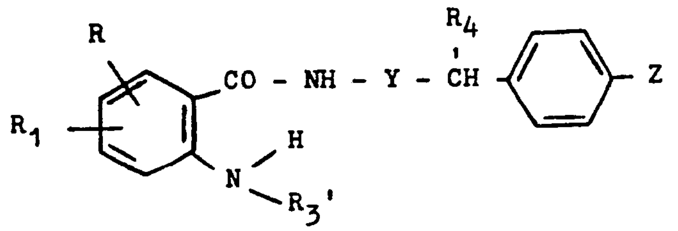

(VIII)

in der

R, $R_1$, $R_4$ und Z wie eingangs definiert sind,

$R_3'$ ein Wasserstoffatom darstellt oder die für $R_3$ vorstehend erwähnten Bedeutungen besitzt und

Y die oben erwähnten Bedeutungen besitzt, mit einer Verbindung der allgemeinen Formel IX

$$R_2'—G \qquad (IX)$$

in der

$R_2'$ die für $R_2$ eingangs erwähnten Bedeutungen besitzt oder zusammen mit dem Rest $R_3'$ der Formel VIII eine geradkettige Alkylengruppe mit 4 bis 6 Kohlenstoffatomen, eine durch eine Alkylgruppe mit 1 bis 4 Kohlenstoffatomen substituierte n-Pentylengruppe oder eine in 2- und 4-Stellung jeweils durch eine Alkylgruppe mit 1 bis 3 Kohlenstoffatomen substituierte n-Pentylengruppe darstellt und

G eine nukleophile Austrittsgruppe wie ein Halogenatom oder eine Sulfonyloxygruppe bedeutet, umgesetzt wird oder

f) zur Herstellung von Verbindungen der allgemeinen Formel I, in der Y die NH-Gruppe oder ein Sauerstoffatom darstellt, eine Verbindung der allgemeinen Formel X

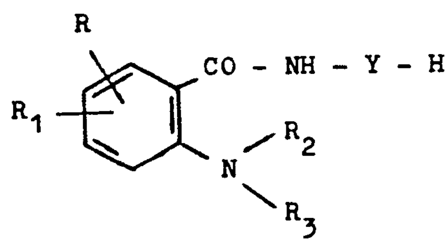

(X)

in der

R und $R_1$ bis $R_3$ wie eingangs definiert sind und

Y die oben erwähnten Bedeutungen besitzt, oder deren Alkalisalz mit einem Phenylderivat der allgemeinen Formel XI

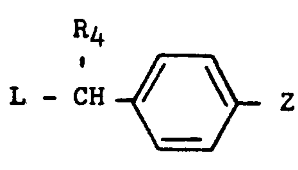

(XI)

in der

$R_4$ und Z wie eingangs definiert sind und

L eine nukleophile Austrittsgruppe wie eine Halogenatom oder eine Sulfonyloxygruppe darstellt, umgesetzt wird oder

g) zur Herstellung von Verbindungen der allgemeinen Formel I, in der $R_1$ mit Ausnahme der Hydroxy-, Carboxy-, Amino- oder Alkanoylaminogruppe wie eingangs definiert ist und Y eine gegebenenfalls durch eine oder zwei Alkylgruppen mit jeweils 1 bis 3 Kohlenstoffatomen substituierte Methylengruppe darstellt, ein Amid der allgemeinen Formel XII

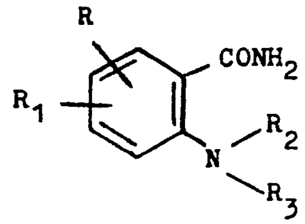

(XII)

in der

R, $R_2$ und $R_3$ wie eingangs definiert sind und

$R_1$ die oben erwähnten Bedeutungen besitzt, oder dessen Alkalisalz mit einer Verbindung der allgemeinen Formel XIII

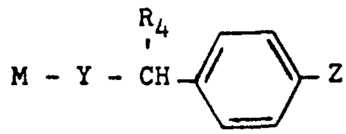

(XIII)

in der

$R_4$ und Z wie eingangs definiert sind,

Y die oben erwähnten Bedeutungen besitzt und

M eine nukleophile Austrittsgruppe wie ein Halogenatom oder eine Sulfonyloxygruppe darstellt, umgesetzt wird oder

h) zur Herstellung von Verbindungen der allgemeinen Formel I, in der $R_1$ ein Wasserstoff-, Fluor-, Chlor- oder Bromatom, eine Alkyl- oder Alkoxygruppe mit jeweils 1 bis 6 Kohlenstoffatomen, eine durch eine Phenylgruppe substituierte Alkoxygruppe mit 1 bis 3 Kohlenstoffatomen, eine Hydroxy-, Nitro-, Carboxy- oder Alkanoylaminogruppe, Y eine gegebenenfalls durch eine oder zwei Alkylgruppen mit jeweils 1 bis 3 Kohlenstoffatomen substituierte Methylengruppe und Z eine Carboxygruppe bedeuten, eine Verbindung der allgemeinen Formel XIV

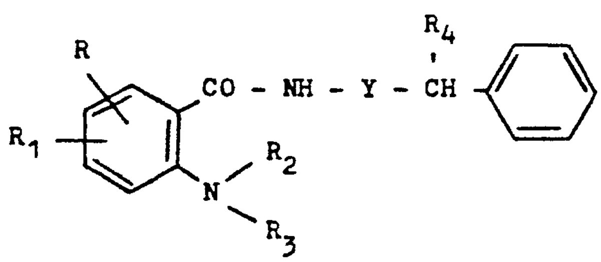

(XIV)

in der

R und $R_2$ bis $R_4$ wie eingangs definiert sind,

$R_1$ und Y die oben erwähnten Bedeutungen besitzen, mit einem Oxalylhalogenid oder Phosgen in Gegenwart einer Lewis-Säure acyliert wird oder

i) zur Herstellung von Verbindungen der allgemeinen Formel I, in der $R_1$ ein Wasserstoff-, Fluor-, Chlor- oder Bromatom, eine Alkyl- oder Alkoxygruppe mit jeweils 1 bis 6 Kohlenstoffatomen, eine durch eine Phenylgruppe substituierte Alkoxygruppe mit 1 bis 3 Kohlenstoffatomen, eine Nitro-, Carboxy-, Alkanoylamino- oder Alkoxycarbonylgruppe mit jeweils 1 bis 3 Kohlenstoffatomen im Alkylteil, Y eine gegebenenfalls durch eine oder zwei Alkylgruppen mit jeweils 1 bis 3 Kohlenstoffatomen substituierte Methylengruppe und Z eine Carboxygruppe bedeuten, eine Verbindung der allgemeinen Formel XV

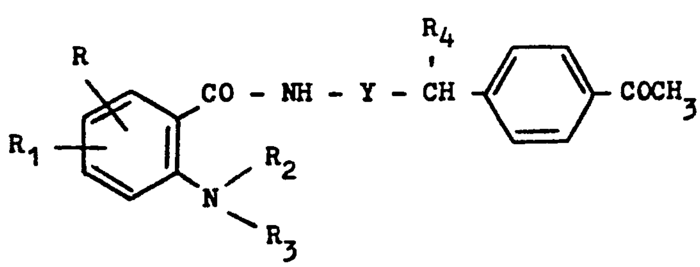

(XV)

in der

R und $R_2$ bis $R_4$ wie eingangs definiert sind,

$R_1$ und Y die oben erwähnten Bedeutungen besitzen, mit einem gegebenenfalls im Reaktionsgemisch hergestellten Hypohalogenit umgesetzt wird

und gewünschtenfalls anschließend eine so erhaltene Verbindung der allgemeinen Formel I, in der Z eine Carboxygruppe darstellt, mittels Veresterung bzw. Amidierung in eine entsprechende Verbindung der allgemeinen Formel I, in der Z eine veresterte Carboxygruppe oder eine gegebenenfalls durch eine Alkylgruppe mit 1 bis 7 Kohlenstoffatomen, eine Cycloalkylgruppe mit 3 bis 7 Kohlenstoffatomen und/oder Alkenylgruppe mit 3 bis 7 Kohlenstoffatomen mono- oder disubstituierte Aminocarbonylgruppe, eine Piperidinocarbonyl-, Morpholinocarbonyl-, Thiomorpholinocarbonyl- oder N-Alkylpiperazinocarbonylgruppe darstellt, übergeführt wird und/oder

eine erhaltene Verbindung der allgemeinen Formel I, in der $R_1$ und/oder Z eine Nitrogruppe darstellt, mittels Reduktion in eine entsprechende Verbindung der allgemeinen Formel I, in der $R_1$ und/oder Z eine Aminogruppe darstellt, übergeführt wird und/oder

eine erhaltene Verbindung der allgemeinen Formel I, in der $R_1$ und/oder Z eine Aminogruppe darstellt, über ein entsprechendes Diazoniumsalz in eine entsprechende Verbindung der allgemeinen Formel I, in der $R_1$ die Hydroxy- oder Cyangruppe, ein Fluor-, Chlor- oder Bromatom und/oder Z ein Chlor- oder Bromatom oder die Cyangruppe darstellen, übergeführt wird, wobei eine so gegebenenfalls erhaltene Verbindung der allgemeinen Formel I, in der $R_1$ die Hydroxygruppe darstellt, mittels Alkylierung in eine entsprechende Verbindung der allgemeinen Formel I, in der $R_1$ eine Alkoxygruppe mit 1 bis 6 Kohlenstoffatomen oder eine durch eine Phenylgruppe substituierte Alkoxygruppe mit 1 bis 3 Kohlenstoffatomen darstellt, übergeführt werden kann, und/oder

eine erhaltene Verbindung der allgemeinen Formel I, in der $R_1$ eine Aminogruppe darstellt, mittels Acylierung in eine entsprechende Verbindung der allgemeinen Formel I, in der $R_1$ eine Alkanoylaminogruppe mit 1 bis 3 Kohlenstoffatomen im Alkanoylteil darstellt, übergeführt wird und/oder

eine erhaltene Verbindung der allgemeinen Formel I, in der $R_1$ eine Alkoxycarbonylgruppe und/oder $R_2$ und $R_3$ zusammen mit dem dazwischenliegenden Stickstoffatom eine durch eine Alkoxycarbonylgruppe substituierte Alkyleniminogruppe mit 4 bis 6 Kohlenstoffatomen darstellt, wobei der Alkoxyteil jeweils 1 bis 3 Kohlenstoffatome enthalten kann, mittels Hydrolyse in eine entsprechende Verbindung der allgemeinen Formel I, in der $R_1$ die Carboxygruppe und/oder $R_2$ und $R_3$ zusammen mit dem dazwischenliegenden Stickstoffatom eine durch eine Carboxygruppe substituierte Alkyleniminogruppe mit 4 bis 6 Kohlenstoffatomen im Iminoring darstellt, übergeführt wird und/oder

eine erhaltene Verbindung der allgemeinen Formel I, in der $R_2$ und $R_3$ zusammen mit dem dazwischenliegenden Stickstoffatom eine N-Benzylpiperazinogruppe darstellt, mittels Entbenzylierung in eine entsprechende Verbindung der allgemeinen Formel I, in der $R_2$ und $R_3$ zusammen mit dem dazwischenliegenden Stickstoffatom eine Piperazinogruppe darstellt, übergeführt wird und/oder

eine erhaltene Verbindung der allgemeinen Formel I, in der $R_1$ ein Chlor- oder Bromatom darstellt, mittels Enthalogenierung in eine entsprechende Verbindung der allgemeinen Formel I, in der $R_1$ ein Wasserstoffatom darstellt, übergeführt wird und/oder

ein Carbonsäureamid der allgemeinen Formel I, in der Z eine gegebenenfalls veresterte Carboxygruppe darstellt, mittels Reduktion mit einem Metallhydrid in eine entsprechende Verbindung der allgemeinen Formel I, in der Z die Hydroxymethylgruppe darstellt, übergeführt wird und/oder

eine erhaltene Verbindung der allgemeinen Formel I, in der Z die Hydroxymethylgruppe darstellt, mittels Oxidation in eine entsprechende Verbindung der allgemeinen Formel I, in der Z die Formylgruppe darstellt, übergeführt wird und/oder

eine erhaltene Verbindung der allgemeinen Formel I, in der Z die Hydroxymethylgruppe darstellt, mittels Halogenierung und anschließender Umsetzung mit einem Malonsäureester in eine entsprechende Verbindung der allgemeinen Formel I, in der Z eine durch zwei Alkoxycarbonylgruppen substituierte Äthylgruppe darstellt, übergeführt wird und/oder

eine erhaltene Verbindung der allgemeinen Formel I, in der Z die Formylgruppe darstellt, mittels Acetalisierung in eine entsprechende Verbindung der allgemeinen Formel I, in der Z eine Dialkoxymethylgruppe darstellt, übergeführt wird und/oder

eine erhaltene Verbindung der allgemeinen Formel I, in der Z die Formylgruppe darstellt, mittels Kondensation und gegebenenfalls anschließender Hydrolyse in eine entsprechende Verbindung der allgemeinen Formel I, in der Z eine durch eine Hydroxycarbonyl- oder Alkoxycarbonylgruppe substituierte Äthylengruppe darstellt, übergeführt wird und/oder

eine erhaltene Verbindung der allgemeinen Formel I, in der Z ein Wasserstoffatom darstellt, mittels Friedel-Crafts-Acylierung in eine entsprechende Verbindung der allgemeinen Formel I, in der Z eine gegebenenfalls durch eine Alkoxycarbonylgruppe substituierte Acetylgruppe darstellt, übergeführt wird und/oder

eine erhaltene Verbindung der allgemeinen Formel I, in der Z eine Nitrilgruppe darstellt, mittels Alkoholyse über einen entsprechenden Iminoester in eine entsprechende Verbindung der allgemeinen Formel I, in der Z eine Trialkoxymethylgruppe darstellt, übergeführt wird und/oder

eine erhaltene Verbindung der allgemeinen Formel I, in der Z eine Trialkoxymethylgruppe darstellt, mittels Hydrolyse in eine entsprechende Verbindung der allgemeinen Formel I, in der Z eine Alkoxycarbonylgruppe darstellt, übergeführt wird und/oder

eine erhaltene Verbindung der allgemeinen Formel I, in der Z die Acetylgruppe darstellt, durch Erhitzen mit einem Amin und Schwefel und anschließend in Gegenwart einer anorganischen Base in eine entsprechende Verbindung der allgemeinen Formel I, in der Z die Hydroxycarbonylmethylgruppe darstellt, übergeführt wird und/oder

eine erhaltene Verbindung der allgemeinen Formel I, in der Z die Carboxygruppe darstellt, mittels Überführung in ein Sulfonsäurehydrazid und anschließende Disproportionierung in eine entsprechende Verbindung der allgemeinen Formel I, in der Z die Formylgruppe darstellt, übergeführt wird und/oder

eine erhaltene Verbindung der allgemeinen Formel I, in der $R_1$ ein Wasserstoff-, Fluor-, Chlor- oder Bromatom, eine Alkyl- oder Alkoxygruppe mit jeweils 1 bis 6 Kohlenstoffatomen oder eine durch eine Phenylgruppe substituierte Alkoxygruppe mit 1 bis 3 Kohlenstoffatomen, Y eine gegebenenfalls durch eine oder zwei Alkylgruppen mit jeweils 1 bis 3 Kohlenstoffatomen substituierte Methylengruppe und Z ein Chlor- oder Bromatom bedeuten, über ihre entsprechende metallorganische Verbindung mittels Kohlendioxid in eine entsprechende Verbindung der allgemeinen Formel I, in der Z die Carboxygruppe darstellt, übergeführt wird und/oder

eine erhaltene Verbindung der allgemeinen Formel I in ihre physiologisch verträglichen Salze mit anorganischen oder organischen Säuren und auch Basen, wenn Z eine Carboxygruppe enthält, übergeführt wird.

**Ansprüche** (für den Vertragsstaat AT)

1. Verfahren zur Herstellung von neuen Carbonsäureamiden der allgemeinen Formel I

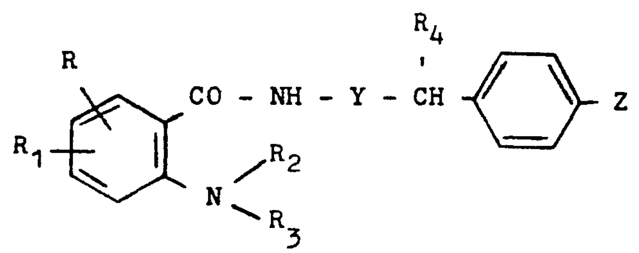

(I)

in der

R ein Wasserstoff-, Chlor- oder Bromatom oder eine cyclische Alkyleniminogruppe mit 4 bis 7 Kohlenstoffatomen im Iminoring,

$R_1$ ein Wasserstoff-, Fluor-, Chlor- oder Bromatom, eine Alkyl- oder Alkoxygruppe mit jeweils 1 bis 6 Kohlenstoffatomen, eine durch eine Phenylgruppe substituierte Alkoxygruppe mit 1 bis 3 Kohlenstoffatomen, eine Hydroxy-, Nitro-, Amino-, Cyano- oder Carboxygruppe, eine Alkanoylamino-, Alkoxycarbonyl- oder Dialkylamidosulfonylgruppe, wobei der Alkylteil jeweils 1 bis 3 Kohlenstoffatome enthalten kann,

$R_2$ und $R_3$, die gleich oder verschieden sein können, Alkylgruppen mit 1 bis 7 Kohlenstoffatomen, Alkenylgruppen mit 3 bis 7 Kohlenstoffatomen, Cycloalkylgruppen mit 3 bis 7 Kohlenstoffatomen, durch eine Phenylgruppe substituierte Alkylgruppen mit 1 bis 3 Kohlenstoffatomen, Phenyl- oder Adamantylgruppen oder

$R_2$ und $R_3$ zusammen mit dem dazwischenliegenden Stickstoffatom eine unverzweigte Alkyleniminogruppe mit 3 bis 12 Kohlenstoffatomen im Iminoring, eine unverzweigte Alkyleniminogruppe mit 4 bis 6 Kohlenstoffatomen im Iminoring, der durch eine oder zwei Alkylgruppen mit jeweils 1 bis 4 Kohlenstoffatomen, eine Alkoxygruppe mit 1 bis 3 Kohlenstoffatomen, eine Hydroxy-, Phenyl-, Carboxy- oder Alkoxycarbonylgruppe mit insgesamt 2 bis 4 Kohlenstoffatomen substituiert ist und/oder in dem eine Methylengruppe durch eine Iminogruppe, welche durch eine Alkylgruppe mit 1 bis 3 Kohlenstoffatomen, eine Alkoxycarbonylgruppe mit insgesamt 2 bis 4 Kohlenstoffatomen, eine Phenyl-, Halogenphenyl-, Benzyl-, Pyridyl- oder Furoylgruppe substituiert sein kann, durch ein Sauerstoff- oder Schwefelatom, durch eine Carbonyl-, Sulfoxid- oder Sulfonylgruppe ersetzt ist, eine gesättigte oder teilweise ungesättigte Azabicycloalkylgruppe mit 7 bis 10 Kohlenstoffatomen, eine in 3- und 5-Stellung durch insgesamt 3 oder 4 Alkylgruppen mit jeweils 1 bis 3 Kohlenstoffatomen substituierte Piperidinogruppe, eine 1,4-Dioxa-8-aza-spiroalkylgruppe mit 6 bis 9 Kohlenstoffatomen, eine Pyrrolyl- oder Tetrahydro-pyridinogruppe,

$R_4$ ein Wasserstoffatom oder eine Alkylgruppe mit 1 bis 3 Kohlenstoffatomen,

Y ein Sauerstoffatom, eine Iminogruppe oder eine gegebenenfalls durch eine oder zwei Alkylgruppen mit jeweils 1 bis 3 Kohlenstoffatomen substituierte Methylengruppe,

Z ein Wasserstoff- oder Halogenatom, eine Carboxy-, Cyano-, Formyl-, Hydromethyl-, Hydroxycarbonyläthylen-, Nitro-, Amino-, Allyloxycarbonyl-, Phenoxycarbonyl-, Benzyloxycarbonyl- oder Phenyläthoxycarbonylgruppe, eine Alkoxycarbonylgruppe mit insgesamt 2 bis 8 Kohlenstoffatomen, eine Cycloalkoxycarbonylgruppe mit insgesamt 4 bis 8 Kohlenstoffatomen, eine gegebenenfalls durch 2 oder 3 Alkoxygruppen, durch eine Carboxy-, Alkoxycarbonyl- oder Äthylendioxygruppe substituierte Methylgruppe,

wobei die Alkoxygruppe jeweils 1 bis 3 Kohlenstoffatome enthalten kann, eine gegebenenfalls durch eine Carboxygruppe oder Alkoxycarbonylgruppe mit insgesamt 2 bis 4 Kohlenstoffatomen substituierte Acetylgruppe, eine durch eine oder zwei Alkoxycarbonylgruppen mit insgesamt je 2 bis 4 Kohlenstoffatomen oder durch zwei Carboxygruppen substituierte Äthyl- oder Äthylengruppe, eine gegebenenfalls durch eine Alkylgruppe mit 1 bis 7 Kohlenstoffatomen, ine Cycloalkylgruppe mit 3 bis 7 Kohlenstoffatomen und/oder Alkenylgruppe mit 3 bis 7 Kohlenstoffatomen mono- oder disubstituierte Aminocarbonylgruppe, eine Piperidinocarbonyl-, Morpholinocarbonyl-, Thiomorpholinocarbonyl- oder N-Alkyl-piperazinocarbonylgruppe, wobei die Alkylgruppe 1 bis 3 Kohlenstoffatome enthalten kann, oder auch eine durch eine Carboxygruppe substituierte Äthylgruppe, wenn die Reste $R_2$ und $R_3$ zusammen mit dem dazwischenliegenden Stickstoffatom einen der eingangs erwähnten cyclischen Iminoreste darstellen, bedeuten, und deren physiologisch verträgliche Salze mit anorganischen oder organischen Säuren und auch Basen, wenn Z eine Carboxygruppe darstellt oder enthält, dadurch gekennzeichnet, daß

a) eine Aminobenzoesäure der allgemeinen Formel II

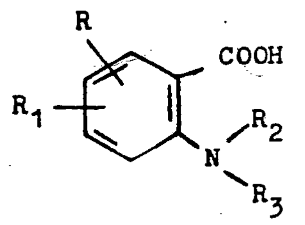

(II)

in der R, $R_1$, $R_2$ und $R_3$ wie eingangs definiert sind, oder deren gegebenenfalls im Reaktionsgemisch hergestelltes reaktionsfähiges Derivat mit einem Amin der allgemeinen Formel III

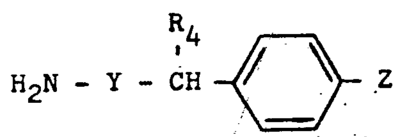

(III)

in der $R_4$, Y und Z wie eingangs definiert sind, oder mit einem gegebenenfalls im Reaktionsgemisch gebildeten N-aktivierten Amin der allgemeinen Formel III, wenn eine Aminobenzoesäure der allgemeinen Formel II eingesetzt wird und wenn Z in einem N-aktivierten Amin der allgemeinen Formel III keine Carboxy- oder Aminogruppe enthält, umgesetzt wird oder

b) zur Herstellung von Verbindungen der allgemeinen Formel I, in der $R_1$ mit Ausnahme der Hydroxy- und Aminogruppe wie eingangs definiert ist und Y eine gegebenenfalls durch eine oder zwei Alkylgruppen mit jeweils 1 bis 3 Kohlenstoffatomen substituierte Methylengruppe darstellt, eine Verbindung der allgemeinen Formel IV

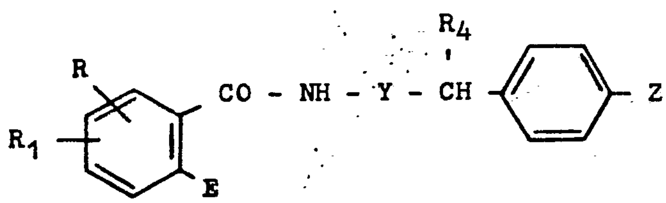

(IV)

in der

R, $R_4$ und Z wie eingangs definiert sind,

$R_1$ und Y die oben erwähnten Bedeutungen besitzen und

E ein Halogenatom darstellt, mit einem Amin der allgemeinen Formel V

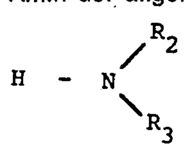

(V)

in der $R_2$ und $R_3$ wie eingangs definiert sind, umgesetzt wird oder

c) zur Herstellung von Verbindungen der allgemeinen Formel I, in der Z eine Carboxygruppe und Y keine NH-Gruppe darstellt, eine Verbindung der allgemeinen Formel VI

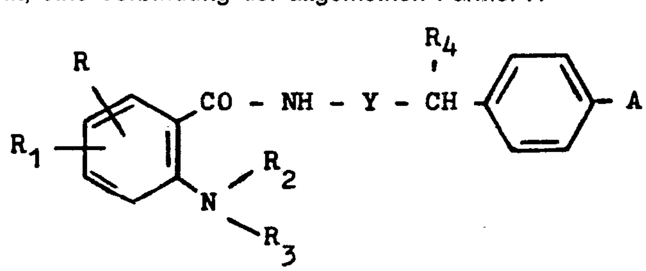

(VI)

in der

R und $R_1$ bis $R_4$ wie eingangs definiert sind,

Y mit Ausnahme der NH-Gruppe wie eingangs definiert ist und

A eine durch Oxidation in eine Carboxygruppe überführbare gruppe bedeutet, oxydiert wird oder

d) zur Herstellung von Verbindungen der allgemeinen Formel I, in der Z eine Carboxygruppe darstellt, eine Verbindung der allgemeinen Formel VII

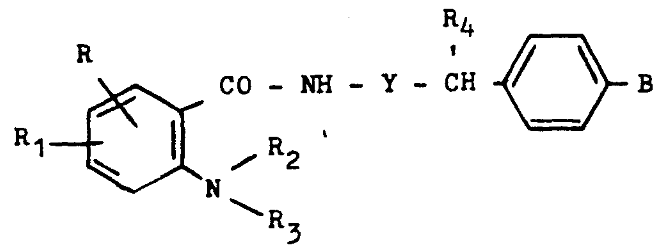

(VII)

in der

R, $R_1$ bis $R_4$ und Y wie eingangs definiert sind und

B eine durch Hydrolyse in eine Carboxygruppe überführbare Gruppe darstellt, hydrolysiert wird oder

e) zur Herstellung von Verbindungen der allgemeinen Formel I, in der $R_2$ eine Alkylgruppe mit 1 bis 7 Kohlenstoffatomen, eine Cycloalkylgruppe mit 3 bis 7 Kohlenstoffatomen, eine durch eine Phenylgruppe substituierte Alkylgruppe mit 1 bis 3 Kohlenstoffatomen oder eine Alkenylgruppe mit 3 bis 7 Kohlenstoffatomen, $R_3$ eine Alkylgruppe mit 1 bis 7 Kohlenstoffatomen, eine Cycloalkylgruppe mit 3 bis 7 Kohlenstoffatomen, eine Alkenylgruppe mit 3 bis 7 Kohlenstoffatomen, eine durch eine Phenylgruppe substituierte Alkylgruppe mit 1 bis 3 Kohlenstoffatomen oder eine Adamantylgruppe oder $R_2$ und $R_3$ mit dem dazwischenliegenden Stickstoffatom einen 5- bis 7-gliedrigen Alkyleniminoring, eine durch eine Alkylgruppe mit 1 bis 4 Kohlenstoffatomen substituierte Piperidinogruppe oder eine in 3- und 5-Stellung jeweils durch eine Alkylgruppe mit 1 bis 3 Kohlenstoffatomen substituierte Piperidinogruppe und Y eine gegebenenfalls durch eine oder zwei Alkylgruppen mit jeweils 1 bis 3 Kohlenstoffatomen substituierte Methylengruppe bedeuten, eine gegebenenfalls im Reaktionsgemisch gebildete Verbindung der allgemeinen Formel VIII

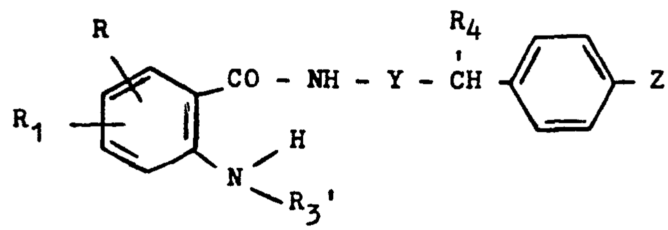

(VIII)

in der

R, $R_1$, $R_4$ und Z wie eingangs definiert sind,

$R_3'$ ein Wasserstoffatom darstellt oder die für $R_3$ vorstehend erwähnten Bedeutungen besitzt, und

Y die oben erwähnten Bedeutungen besitzt mit einer Verbindung der allgemeinen Formel IX

$$R_2'\text{—}G \qquad (IX)$$

in der

$R_2'$ die für $R_2$ eingangs erwähnten Bedeutungen besitzt oder zusammen mit dem Rest $R_3'$ der Formel VIII eine geradkettige Alkylengruppe mit 4 bis 6 Kohlenstoffatomen, eine durch eine Alkylgruppe mit 1 bis 4 Kohlenstoffatomen substituierte n-Pentylengruppe oder eine in 2- und 4-Stellung jeweils durch eine Alkylgruppe mit 1 bis 3 Kohlenstoffatomen substituierte n-Pentylengruppe darstellt und

G eine nukleophile Austrittsgruppe wie ein Halogenatom oder eine Sulfonyloxygruppe bedeutet, umgesetzt wird oder

f) zur Herstellung von Verbindungen der allgemeinen Formel I, in der Y die NH-Gruppe oder ein Sauerstoffatom darstellt, eine Verbindung der allgemeinen Formel X

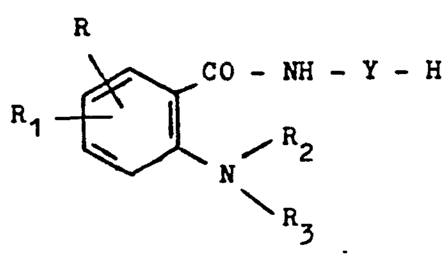

(X)

in der

R und $R_1$ bis $R_3$ wie eingangs definiert sind und

Y die oben erwähnten Bedeutungen besitzt, oder deren Alkalisalz mit einem Phenylderivat der allgemeinen Formel XI

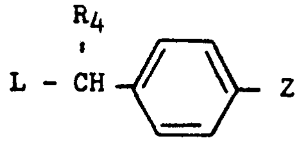

(XI)

in der

$R_4$ und Z wie eingangs definiert sind und

L eine nukleophile Austrittsgruppe wie ein Halogenatom oder eine Sulfonyloxygruppe darstellt, umgesetzt wird

g) zur Herstellung von Verbindungen der allgemeinen Formel I, in der $R_1$ mit Ausnahme der Hydroxy-, Carboxy-, Amino- oder Alkanoylaminogruppe wie eingangs definiert ist und Y eine gegebenenfalls durch eine oder zwei Alkylgruppen mit jeweils 1 bis 3 Kohlenstoffatomen substituierte Methylengruppe darstellt, ein Amid der allgemeinen Formel XII

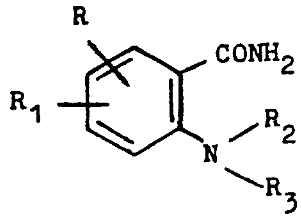

(XII)

in der

R, $R_2$ und $R_3$ wie eingangs definiert sind und

$R_1$ die oben erwähnten Bedeutungen besitzt, oder dessen Alkalisalz mit einer Verbindung der allgemeinen Formel XIII

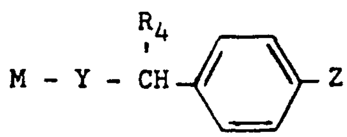

(XIII)

in der

$R_4$ und Z wie eingangs definiert sind,

Y die oben erwähnten Bedeutungen besitzt und

M eine nukleophile Austrittsgruppe wie ein Halogenatom oder eine Sulfonyloxygruppe darstellt, umgesetzt wird oder

h) zur Herstellung von Verbindungen der allgemeinen Formel I, in der $R_1$ ein Wasserstoff-, Fluor-, Chlor- oder Bromatom, eine Alkyl- oder Alkoxygruppe mit jeweils 1 bis 6 Kohlenstoffatomen, eine durch eine Phenylgruppe substituierte Alkoxygruppe mit 1 bis 3 Kohlenstoffatomen, eine Hydroxy-, Nitro-, Carboxy- oder Alkanoylaminogruppe, Y eine gegebenenfalls durch eine oder zwei Alkylgruppen mit jeweils 1 bis 3 Kohlenstoffatomen substituierte Methylengruppe und Z eine Carboxygruppe bedeuten, eine Verbindung der allgemeinen Formel XIV

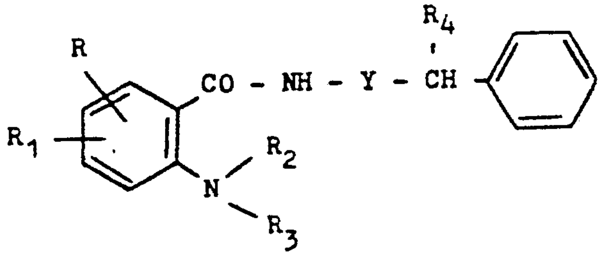

(XIV)

in der

R und $R_2$ bis $R_4$ wie eingangs definiert sind,

$R_1$ und Y die oben erwähnten Bedeutungen besitzen, mit einem Oxalylhalogenid oder Phosgen in

Gegenwart einer Lewis-Säure acyliert wird oder

i) zur Herstellung von Verbindungen der allgemeinen Formel I, in der $R_1$ ein Wasserstoff-, Fluor-, Chlor- oder Bromatom, eine Alkyl- oder Alkoxygruppe mit jeweils 1 bis 6 Kohlenstoffatomen, eine durch eine Phenylgruppe substituierte Alkoxygruppe mit 1 bis 3 Kohlenstoffatomen, eine Nitro-, Carboxy-, Alkanoylamino- oder Alkoxycarbonylgruppe mit jeweils 1 bis 3 Kohlenstoffatomen im Alkylteil, Y eine gegebenenfalls durch eine oder zwei Alkylgruppen mit jeweils 1 bis 3 Kohlenstoffatomen substituierte Methylengruppe und Z eine Carboxygruppe bedeuten, eine Verbindung der allgemeinen Formel XV

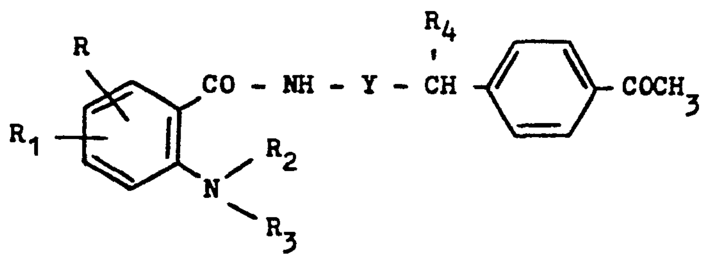

(XV)

in der

R und $R_2$ bis $R_4$ wie eingangs definiert sind,

$R_1$ und Y die oben erwähnten Bedeutungen besitzen, mit einem gegebenenfalls im Reaktionsgemisch hergestellten Hypohalogenit umgesetzt wird

und gewünschtenfalls anschließend eine so erhaltene Verbindung der allgemeinen Formel I, in der Z eine Carboxygruppe darstellt, mittels Veresterung bzw. Amidierung in eine entsprechende Verbindung der allgemeinen Formel I, in der Z eine veresterte Carboxygruppe oder eine gegebenenfalls durch eine Alkylgruppe mit 1 bis 7 Kohlenstoffatomen, eine Cycloalkylgruppe mit 3 bis 7 Kohlenstoffatomen und/oder Alkenylgruppe mit 3 bis 7 Kohlenstoffatomen mono- oder disubstituierte Aminocarbonylgruppe, eine Piperidinocarbonyl-, Morpholinocarbonyl-, Thiomorpholinocarbonyl- oder N-Alkylpiperazinocarbonylgruppe darstellt, übergeführt wird und/oder

eine erhaltene Verbindung der allgemeinen Formel I, in der $R_1$ und/oder Z eine Nitrogruppe darstellt, mittels Reduktion in eine entsprechende Verbindung der allgemeinen Formel I, in der $R_1$ und/oder Z eine Aminogruppe darstellt, übergeführt wird und/oder

eine erhaltene Verbindung der allgemeinen Formel I, in der $R_1$ und/oder Z eine Aminogruppe darstellt, über ein entsprechendes Diazoniumsalz in eine entsprechende Verbindung der allgemeinen Formel I, in der $R_1$ die Hydroxy- oder Cyangruppe, ein Fluor-, Chlor- oder Bromatom und/oder Z ein Chlor- oder Bromatom oder die Cyangruppe darstellen, übergeführt wird, wobei eine so gegebenenfalls erhaltene Verbindung der allgemeinen Formel I, in der $R_1$ die Hydroxygruppe darstellt, mittels Alkylierung in eine entsprechende Verbindung der allgemeinen Formel I, in der $R_1$ eine Alkoxygruppe mit 1 bis 6 Kohlenstoffatomen oder eine durch eine Phenylgruppe substituierte Alkoxygruppe mit 1 bis 3 Kohlenstoffatomen darstellt, übergeführt werden kann, und/oder

eine erhaltene Verbindung der allgemeinen Formel I, in der $R_1$ eine Aminogruppe darstellt, mittels Acylierung in eine entsprechende Verbindung der allgemeinen Formel I, in der $R_1$ eine Alkanoylaminogruppe mit 1 bis 3 Kohlenstoffatomen im Alkanoylteil darstellt, übergeführt wird und/oder

eine erhaltene Verbindung der allgemeinen Formel I, in der $R_1$ eine Alkoxycarbonylgruppe und/oder $R_2$ und $R_3$ zusammen mit dem dazwischenliegenden Stickstoffatom eine durch eine Alkoxycarbonylgruppe substituierte Alkyleniminogruppe mit 4 bis 6 Kohlenstoffatomen darstellt, wobei der Alkoxyteil jeweils 1 bis 3 Kohlenstoffatome enthalten kann, mittels Hydrolyse in eine entsprechende Verbindung der allgemeinen Formel I, in der $R_1$ die Carboxygruppe und/oder $R_2$ und $R_3$ zusammen mit dem dazwischenliegenden Stickstoffatom eine durch eine Carboxygruppe substituierte Alkyleniminogruppe mit 4 bis 6 Kohlenstoffatomen im Iminoring darstellt, übergeführt wird und/oder

eine erhaltene Verbindung der allgemeinen Formel I, in der $R_2$ und $R_3$ zusammen mit dem dazwischenliegenden Stickstoffatom eine N-Benzylpiperazinogruppe darstellt, mittels Entbenzylierung in eine entsprechende Verbindung der allgemeinen Formel I, in der $R_2$ und $R_3$ zusammen mit dem dazwischenliegenden Stickstoffatom eine Piperazinogruppe darstellt, übergeführt wird und/oder

eine erhaltene Verbindung der allgemeinen Formel I, in der $R_1$ ein Chlor- oder Bromatom darstellt, mittels Enthalogenierung in eine entsprechende Verbindung der allgemeinen Formel I, in der $R_1$ ein Wasserstoffatom darstellt, übergeführt wird und/oder

ein Carbonsäureamid der allgemeinen Formel I, in der Z eine gegebenenfalls veresterte Carboxygruppe darstellt, mittels Reduktion mit einem Metallhydrid in eine entsprechende Verbindung der allgemeinen Formel I, in der Z die Hydroxymethylgruppe darstellt, übergeführt wird und/oder

eine erhaltene Verbindung der allgemeinen Formel I, in der Z die Hydroxymethylgruppe darstellt, mittels Oxidation in eine entsprechende Verbindung der allgemeinen Formel I, in der Z die Formylgruppe darstellt, übergeführt wird und/oder

eine erhaltene Verbindung der allgemeinen Formel I, in der Z die Hydroxymethylgruppe darstellt, mittels Halogenierung und anschließender Umsetzung mit einem Malonsäureester in eine entsprechende

Verbindung der allgemeinen Formel I, in der Z eine durch zwei Alkoxycarbonylgruppen substituierte Äthylgruppe darstellt, übergeführt wird und/oder

eine erhaltene Verbindung der allgemeinen Formel I, in der Z die Formylgruppe darstellt, mittels Acetalisierung in eine entsprechende Verbindung der allgemeinen Formel I, in der Z eine Dialkoxymethylgruppe darstellt, übergeführt wird und/oder

eine erhaltene Verbindung der allgemeinen Formel I, in der Z die Formylgruppe darstellt, mittels Kondensation und gegebenenfalls anschließender Hydrolyse in eine entsprechende Verbindung der allgemeinen Formel I, in der Z eine durch eine Hydroxycarbonyl- oder Alkoxycarbonylgruppe substituierte Äthylengruppe darstellt, übergeführt wird und/oder

eine erhaltene Verbindung der allgemeinen Formel I, in der Z ein Wasserstoffatom darstellt, mittels Friedel-Crafts-Acylierung in eine entsprechende Verbindung der allgemeinen Formel I, in der Z eine gegebenenfalls durch eine Alkoxycarbonylgruppe substituierte Acetylgruppe darstellt, übergeführt wird und/oder

eine erhaltene Verbindung der allgemeinen Formel I, in der Z eine Nitrilgruppe darstellt, mittels Alkoholyse über einen entsprechenden Iminoester in eine entsprechende Verbindung der allgemeinen Formel I, in der Z eine Trialkoxymethylgruppe darstellt, übergeführt wird und/oder

eine erhaltene Verbindung der allgemeinen Formel I, in der Z eine Trialkoxymethylgruppe darstellt, mittels Hydrolyse in eine entsprechende Verbindung der allgemeinen Formel I, in der Z eine Alkoxycarbonylgruppe darstellt, übergeführt wird und/oder

eine erhaltene Verbindung der allgemeinen Formel I, in der Z die Acetylgruppe darstellt, durch Erhitzen mit einem Amin und Schwefel und anschließend in Gegenwart einer anorganischen Base in eine entsprechende Verbindung der allgemeinen Formel I, in der Z die Hydroxycarbonylmethylgruppe darstellt, übergeführt wird und/oder

eine erhaltene Verbindung der allgemeinen Formel I, in der Z die Carboxygruppe darstellt, mittels Überführung in ein Sulfonsäurehydrazid und anschließende Disproportionierung in eine entsprechende Verbindung der allgemeinen Formel I, in der Z die Formylgruppe darstellt, übergeführt wird und/oder

eine erhaltene Verbindung der allgemeinen Formel I, in der $R_1$ ein Wasserstoff-, Fluor-, Chlor- oder Bromatom, eine Alkyl- oder Alkoxygruppe mit jeweils 1 bis 6 Kohlenstoffatomen oder eine durch eine Phenylgruppe substituierte Alkoxygruppe mit 1 bis 3 Kohlenstoffatomen, Y eine gegebenenfalls durch eine oder zwei Alkylgruppen mit jeweils 1 bis 3 Kohlenstoffatomen substituierte Methylengruppe und Z ein Chlor- oder Bromatom bedeuten, über ihre entsprechende metallorganische Verbindung mittels Kohlendioxid in eine entsprechende Verbindung der allgemeinen Formel I, in der Z die Carboxygruppe darstellt, übergeführt wird und/oder

eine erhaltene Verbindung der allgemeinen Formel I in ihre physiologisch verträglichen Salze mit anorganischen oder organischen Säuren und auch Basen, wenn Z eine Carboxygruppe enthält, übergeführt wird.

2. Verfahren gemäß Anspruch 1 zur Herstellung von neuen Carbonsäureamiden der allgemeinen Formel I'

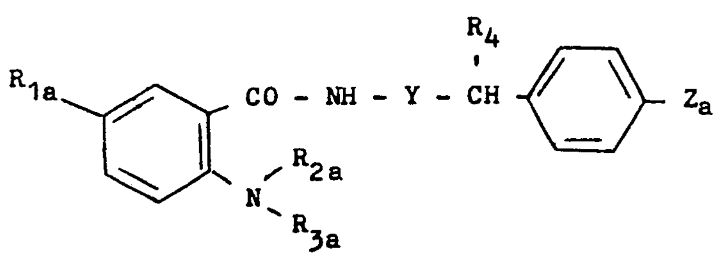

(I')

in der

$R_4$ wie im Anspruch 1 definiert ist,

$R_{1a}$ ein Wasserstoff-, Fluor-, Chlor- oder Bromatom, eine Nitro-, Amino-, Alkanoylamino-, Alkoxy-, Cyano-, Carboxy-, Alkoxycarbonyl- oder Dialkylamidosulfonylgruppe, wobei der Alkylteil jeweils 1 bis 3 Kohlenstoffatome enthalten kann,

$R_{2a}$ und $R_{3a}$, die gleich oder verschieden sein können, Alkylgruppen mit 1 bis 7 Kohlenstoffatomen oder Cycloalkylgruppen mit 3 bis 7 Kohlenstoffatomen oder

$R_{2a}$ und $R_{3a}$ zusammen mit dem dazwischenliegenden Stickstoffatom eine Iminogruppe mit 4 bis 6 Kohlenstoffatomen im Iminoring, der durch 1 oder 2 Alkylgruppen mit jeweils 1 bis 3 Kohlenstoffatomen, eine Alkoxygruppe mit 1 bis 3 Kohlenstoffatomen, eine Hydroxy-, Phenyl-, Carboxy- oder Alkoxycarbonylgruppe mit insgesamt 2 bis 4 Kohlenstoffatomen substituiert sein kann, oder in dem eine Methylengruppe des Iminoringes durch eine cyclische Ketalgruppe mit insgesamt 3 oder 4 Kohlenstoffatomen, durch ein Sauerstoff- oder Schwefelatom, durch eine Sulfoxid oder durch eine Iminogruppe, die durch eine Phenyl- oder Pyridylgruppe substituiert ist, ersetzt sein kann, eine Trimethylenimino-, Pyrrolyl-, Tetrahydroisochinolyl- oder Tetrahydro-benzazepinylgruppe und

$Z_a$ eine Carboxy- oder Alkoxycarbonylgruppe mit insgesamt 2 bis 5 Kohlenstoffatomen bedeuten,

und von deren physiologisch verträglichen Salzen mit anorganischen oder organischen Säuren oder auch Basen, wenn $Z_a$ eine Carboxygruppe darstellt, dadurch gekennzeichnet, daß

a) eine Aminobenzoesäure der allgemeinen Formel IIa

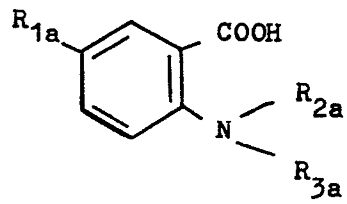

(IIa)

in der $R_{1a}$, $R_{2a}$ und $R_{3a}$ wie eingangs definiert sind, oder deren gegebenenfalls im Reaktionsgemisch hergestelltes reaktionsfähiges Derivat mit einem Phenylalkylamin der allgemeinen Formel IIIa

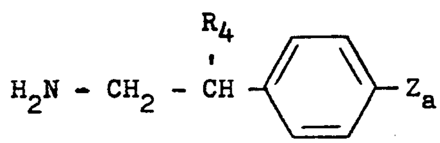

(IIIa)

in der $R_4$ und $Z_a$ wie eingangs definiert sind, oder mit dessen gegebenenfalls im Reaktionsgemisch gebildeten reaktionsfähigen Derivat, wenn eine Aminobenzoesäure der allgemeinen Formel IIa eingesetzt wird und $Z_a$ eine Alkoxycarbonylgruppe mit insgesamt 2 bis 5 Kohlenstoffatomen darstellt, umgesetzt wird oder

b) zur Herstellung einer Verbindung der allgemeinen Formel I', in der $R_{1a}$ die Nitrogruppe darstellt, ein Nitrobenzamid der allgemeinen Formel IVa

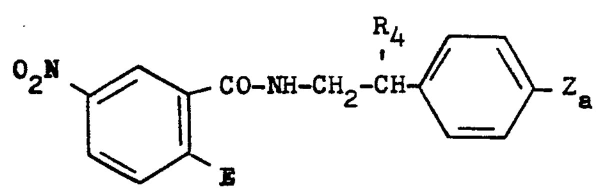

(IVa)

in der

$R_4$ und $Z_a$ wie eingangs definiert sind und

E ein Halogenatom darstellt, mit einem Amin der allgemeinen Formel Va

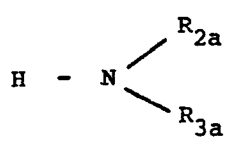

(Va)

in der $R_{2a}$ und $R_{3a}$ wie eingangs definiert sind, umgesetzt wird oder

c) zur Herstellung einer Verbindung der allgemeinen Formel I', in der $Z_a$ eine Carboxygruppe darstellt, eine Verbindung der allgemeinen Formel VIa

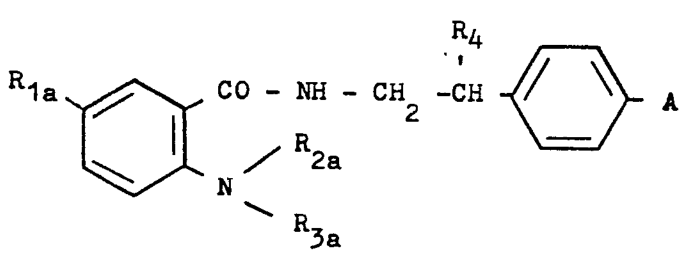

(VIa)

in der $R_4$ und A wie im Anspruch 1 und $R_{1a}$ bis $R_{3a}$ wie eingangs definiert sind, oxidiert wird oder

d) zur Herstellung einer Verbindung der allgemeinen Formel I', in der $Z_a$ eine Carboxygruppe darstellt, eine Verbindung der allgemeinen Formel VIIa

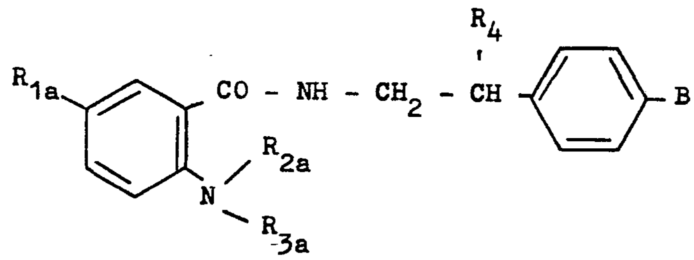

(VIIa)

in der $R_4$ und B wie im Anspruch 1 und $R_{1a}$ bis $R_{3a}$ wie eingangs definiert sind, hydrolysiert wird oder

e) zur Herstellung einer Verbindung der allgemeinen Formel I', in der $R_{2a}$ und $R_{3a}$, die gleich oder verschieden sein können, Alkylgruppen mit 1 bis 7 Kohlenstoffatomen oder Cycloalkylgruppen mit 3 bis 7 Kohlenstoffatomen oder $R_{2a}$ und $R_{3a}$ zusammen mit dem dazwischenliegenden Stickstoffatom einen 5- bis 7-gliedrigen Alkyleniminoring, eine durch eine Alkylgruppe mit 1 bis 3 Kohlenstoffatomen substituierte Piperidinogruppe oder eine in 3- und 5-Stellung jeweils durch eine Alkylgruppe mit jeweils 1 bis 3 Kohlenstoffatomen substituierte Piperidinogruppe darstellt, eine Verbindung der allgemeinen Formel VIIIa

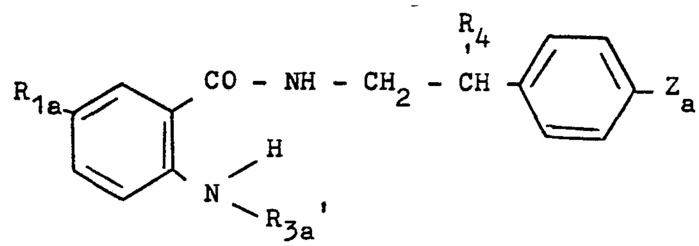

(VIIIa)

in der

$R_4$ wie im Anspruch 1, $R_{1a}$ und $Z_a$ wie eingangs definiert sind und

$R_{3a}'$ ein Wasserstoffatom darstellt oder die für $R_{3a}$ vorstehend erwähnten Bedeutungen besitzt, mit einer Verbindung der allgemeinen Formel IXa

$$R_{2a}'\text{—}G \qquad \text{(IXa)}$$

in der

$R_{2a}'$ die für $R_{2a}$ eingangs erwähnten Bedeutungen besitzt oder zusammen mit dem Rest $R_{3a}'$ der Formel VIIIa eine geradkettige Alkylengruppe mit 4 bis 6 Kohlenstoffatomen, eine durch eine Alkylgruppe mit 1 bis 3 Kohlenstoffatomen substituierte n-Pentylengruppe oder eine in 2- und 4-Stellung jeweils durch eine Alkylgruppe mit 1 bis 3 Kohlenstoffatomen substituierte n-Pentylengruppe darstellt und

G eine nukleophile Austrittsgruppe bedeutet, umgesetzt wird und gewünschtenfalls anschließend eine erhaltene Verbindung der allgemeinen Formel I', in der $Z_a$ eine Carboxygruppe darstellt, mittels Veresterung in eine entsprechende Verbindung der allgemeinen Formel I', in der $Z_a$ eine Alkoxycarbonylgruppe mit insgesamt 2 bis 5 Kohlenstoffatomen darstellt, übergeführt wird und/oder

eine erhaltene Verbindung der allgemeinen Formel I', in der $R_{1a}$ die Nitrogruppe darstellt, mittels Reduktion in eine entsprechende Verbindung der allgemeinen Formel $I_a$, in der $R_{1a}$ die Aminogruppe darstellt, übergeführt wird und/oder

eine erhaltene Verbindung der allgemeinen Formel I', in der $R_{1a}$ die Aminogruppe darstellt, über ein entsprechendes Diazoniumsalz in eine entsprechende Verbindung der allgemeinen Formel I', in der $R_{1a}$ die Cyangruppe, ein Fluor-, Chlor- oder Bromatom darstellt, übergeführt wird und/oder

eine erhaltene Verbindung der allgemeinen Formel I', in der $R_{1a}$ die Aminogruppe darstellt, mittels Acylierung in eine entsprechende Verbindung der allgemeinen Formel I', in der $R_{1a}$ eine Alkanoylaminogruppe mit 1 bis 3 Kohlenstoffatomen im Alkanoylteil darstellt, übergeführt wird und/oder

eine erhaltene Verbindung der allgemeinen Formel I', in der $R_{1a}$ die Cyangruppe darstellt, mittels Alkoholyse in eine entsprechende Verbindung der allgemeinen Formel I', in der $R_{1a}$ eine Alkoxycarbonylgruppe mit 1 bis 3 Kohlenstoffatomen im Alkoxyteil darstellt, übergeführt wird und/oder

eine erhaltene Verbindung der allgemeinen Formel I', in der $R_{1a}$ eine Alkoxycarbonylgruppe und/oder $R_{2a}$ und $R_{3a}$ zusammen mit dem dazwischenliegenden Stickstoffatom eine durch eine Alkoxycarbonylgruppe substituierte Iminogruppe mit 4 bis 6 Kohlenstoffatomen im Iminoring darstellt, wobei der Alkoxyteil jeweils 1 bis 3 Kohlenstoffatomen enthalten kann, mittels Hydrolyse in eine entsprechende Verbindung der allgemeinen Formel I', in der $R_{1a}$ die Carboxygruppe und/oder $R_{2a}$ und $R_{3a}$ zusammen mit dem dazwischenliegenden Stickstoffatom eine durch eine Carboxygruppe substituierte Iminogruppe mit 4 bis 6 Kohlenstoffatomen im Iminoring darstellt, übergeführt wird und/oder

eine erhaltene Verbindung der allgemeinen Formel I' in ihre physiologisch verträglichen Salze mit anorganischen oder organischen Säuren oder auch Basen, wenn $Z_a$ eine Carboxygruppe darstellt, übergeführt wird.

3. Verfahren gemäß Anspruch 1 zur Herstellung von neuen Carbonsäureamiden der allgemeinen Formel I''

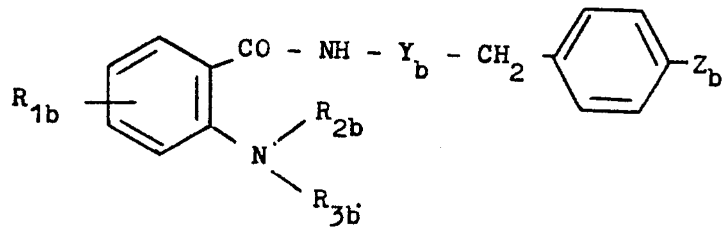

(I'')

in der

$R_{1b}$ ein Wasserstoff-, Chlor- oder Bromatom, eine Alkoxygruppe mit 1 bis 6 Kohlenstoffatomen, eine Benzyloxy-, Nitro- oder Aminogruppe,

$R_{2b}$ und $R_{3b}$, die gleich oder verschieden sein können, Alkylgruppen mit 1 bis 4 Kohlenstoffatomen, Cycloalkylgruppen mit 3 bis 7 Kohlenstoffatomen oder Phenylgruppen oder

$R_{2b}$ und $R_{3b}$ zusammen mit dem dazwischenliegenden Stickstoffatom eine unverzweigte Alkyleniminogruppe mit 5 bis 12 Kohlenstoffatomen im Iminoring, eine Piperidinogruppe, in der eine Methylengruppe durch eine Methylmethylen-, Methoxymethylen-, Carbonyl-, Imino- oder Benzyliminogruppe ersetzt ist, eine in 3- und 5-Stellung jeweils durch 1 Alkylgruppe mit 1 bis 3 Kohlenstoffatomen substituierte Piperidinogruppe, wobei die Methylengruppe in 4-Stellung gleichzeitig durch ein Sauerstoff- oder Schwefelatom, eine Sulfoxid- oder Sulfongruppe ersetzt sein kann, eine in 3- und 5-Stellung durch insgesamt 3 oder 4 Alkylgruppen mit jeweils 1 bis 3 Kohlenstoffatomen substituierte Piperidinogruppe, eine 1,1-Dioxidothiomorpholino- oder Azabicycloalkylgruppe mit 7 bis 9 Kohlenstoffatomen,

$Y_b$ eine gegebenenfalls durch eine Alkylgruppe mit 1 bis 3 Kohlenstoffatomen substituierte Methylengruppe und

$Z_b$ eine Carboxygruppe oder eine Alkoxycarbonylgruppe mit insgesamt 2 bis 5 Kohlenstoffatomen bedeuten, sowie von deren physiologisch verträglichen Salzen mit anorganischen oder organischen Säuren und auch Basen, wenn $Z_b$ eine Carboxygruppe darstellt, dadurch gekennzeichnet, daß

a) eine Aminobenzoesäure der allgemeinen Formel IIb

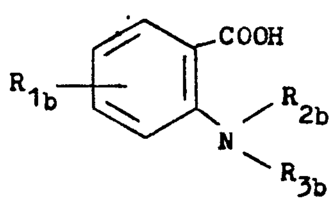

(IIb)

in der $R_{1b}$, $R_{2b}$ und $R_{3b}$ wie eingangs definiert sind, oder deren gegebenenfalls im Reaktionsgemisch hergestelltes reaktionsfähiges Derivat mit einem Phenylalkylamin der allgemeinen Formel IIIb

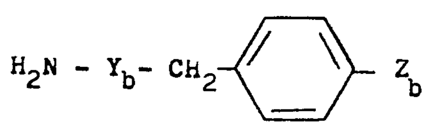

(IIIb)

in der $Z_b$ und $Y_b$ wie eingangs definiert sind, oder mit einem gegebenenfalls im Reaktionsgemisch gebildeten N-aktivierten Phenylalkylamin der allgemeinen Formel Vb, wenn eine Aminobenzoesäure der allgemeinen Formel IVb eingesetzt wird und in einem N-aktivierten Phenylalkylamin der allgemeinen Formel Vb $Z_b$ eine Alkoxycarbonylgruppe mit insgesamt 2 bis 5 Kohlenstoffatomen darstellt, umgesetzt wird oder

b) zur Herstellung einer Verbindung der allgemeinen Formel I'', in der $R_{1b}$ in 3- oder 5-Stellung die Nitrogruppe darstellt, ein Nitrobenzamid der allgemeinen Formel IVb

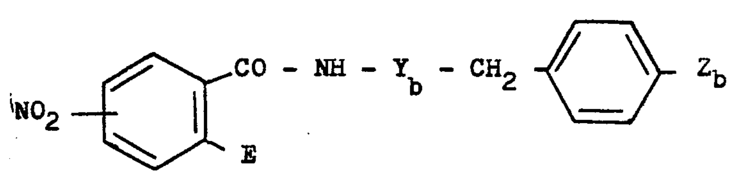

(IVb)

in der

$Z_b$ und $Y_b$ wie eingangs definiert sind und

E ein Halogenatom darstellt, mit einem Amin der allgemeinen Formel Vb

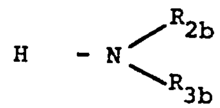

(Vb)

in der $R_{2b}$ und $R_{3b}$ wie eingangs definiert sind, umgesetzt wird oder

c) zur Herstellung einer Verbindung der allgemeinen Formel I'', in der $Z_b$ eine Carboxygruppe darstellt, eine Verbindung der allgemeinen Formel VIb

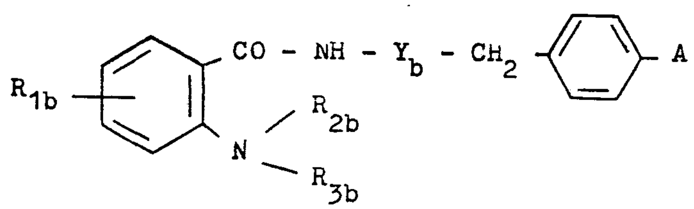

(VIb)

in der A wie im Anspruch 1, $R_{1b}$ bis $R_{3b}$ und $Y_b$ wie eingangs definiert sind, oxidiert wird oder

d) zur Herstellung einer Verbindung der allgemeinen Formel I'', in der $Z_b$ eine Carboxygruppe darstellt, eine Verbindung der allgemeinen Formel VIIb

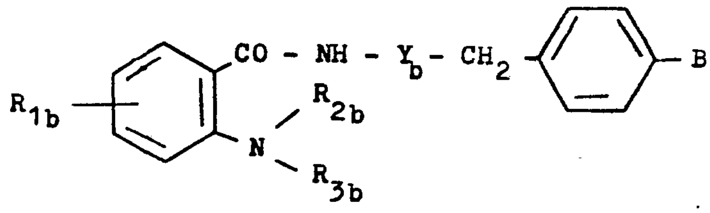

(VIIb)

in der B wie im Anspruch 1, $R_{1b}$ bis $R_{3b}$ und $Y_b$ wie eingangs definiert sind, hydrolysiert wird oder

e) zur Herstellung einer Verbindung der allgemeinen Formel I'', in der $R_{2b}$ und $R_{3b}$, die gleich oder verschieden sein können, Alkylgruppen mit 1 bis 4 Kohlenstoffatomen, Cycloalkylgruppen mit 3 bis 7 Kohlenstoffatomen oder Phenylgruppen oder $R_{2b}$ und $R_{3b}$ zusammen mit dem dazwischenliegenden Stickstoffatom einen 6- bis 7-gliedrigen Iminoring, eine Methylpiperidinogruppe oder eine in 3- und 5-Stellung durch 2 bis 4 Alkylgruppen mit jeweils 1 bis 3 Kohlenstoffatomen substituierte Piperidinogruppe darstellen, eine Verbindung der allgemeinen Formel VIIIb

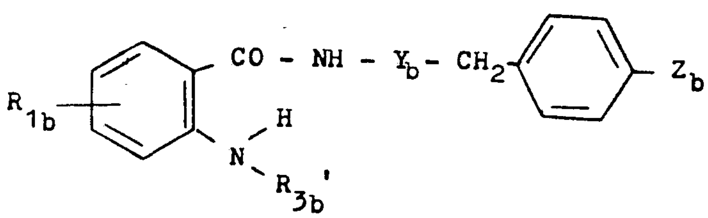

(VIIIb)

in der

$R_{1b}$, $Y_b$ und $Z_b$ wie eingangs definiert sind und

$R_{3b}'$ ein Wasserstoffatom darstellt oder die für $R_{3b}$ vorstehend erwähnten Bedeutungen besitzt, mit einer Verbindung der allgemeinen Formel IXb

$$R_{2b}'—G \qquad (IXb)$$

in der

$R_{2b}'$ die für $R_{2b}$ eingangs erwähnten Bedeutungen besitzt oder zusammen mit dem Rest $R_{3b}'$ der Formel VIIIb eine geradkettige Alkylengruppe mit 5 bis 6 Kohlenstoffatomen, eine durch eine Methylgruppe substituierte n-Pentylengruppe oder eine in 2- und 4-Stellung durch 2 bis 4 Alkylgruppen mit jeweils 1 bis 3 Kohlenstoffatomen substituierte n-Pentylengruppe darstellt und

G eine nukleophile Austrittsgruppe bedeutet, umgesetzt wird und gewünschtenfalls anschließend eine erhaltene Verbindung der allgemeinen Formel I'', in der $Z_b$ eine Carboxygruppe darstellt, mittels

Veresterung in eine entsprechende Verbindung der allgemeinen Formel I'', in der $Z_5$ eine Alkoxycarbonylgruppe mit insgesamt 2 bis 5 Kohlenstoffatomen darstellt, übergeführt wird und/oder

eine erhaltene Verbindung der allgemeinen Formel I'', in der $R_{1b}$ die Nitrogruppe darstellt, mittels Reduktion in eine entsprechende Verbindung der allgemeinen Formel I'', in der $R_{1b}$ die Aminogruppe darstellt, übergeführt wird und/oder

eine erhaltene Verbindung der allgemeinen Formel I'', in der $R_{1b}$ die Aminogruppe darstellt, über ein entsprechendes Diazoniumsalz in eine entsprechende Verbindung der allgemeinen Formel I'', in der $R_{1b}$ ein Chlor- oder Bromatom oder die Hydroxygruppe darstellt, übergeführt wird und eine so gegebenenfalls erhaltene Verbindung der allgemeinen Formel I'', in der $R_{1b}$ die Hydroxygruppe darstellt, mittels Alkylierung oder Benzylierung in eine entsprechende Verbindung der allgemeinen Formel I'', in der $R_{1b}$ eine Benzyloxy- oder Alkoxygruppe mit 1 bis 4 Kohlenstoffatomen darstellt, übergeführt wird und/oder

eine erhaltene Verbindung der allgemeinen Formel I'', in der $R_{2b}$ und $R_{3b}$ zusammen mit dem dazwischenliegenden Stickstoffatom eine N-Benzylpiperazinogruppe darstellt, mittels Entbenzylierung in eine entsprechende Verbindung der allgemeinen Formel I'', in der $R_{2b}$ und $R_{3b}$ zusammen mit dem dazwischenliegenden Stickstoffatom eine Piperazinogruppe darstellt, übergeführt wird und/oder

eine erhaltene Verbindung der allgemeinen Formel I'', in der $R_{1b}$ ein Chlor- oder Bromatom darstellt, mittels Enthalogenierung in eine entsprechende Verbindung der allgemeinen Formel I'', in der $R_{1b}$ ein Wasserstoffatom darstellt, übergeführt wird und/oder

eine erhaltene Verbindung der allgemeinen Formel I'', in ihre physiologisch verträglichen Salze mit anorganischen oder organischen Säuren und auch Basen, wenn $Z_b$ eine Carboxygruppe darstellt, übergeführt wird.

4. Verfahren gemäß Anspruch 1 zur Herstellung von neuen Carbonsäureamiden der allgemeinen Formel I'''

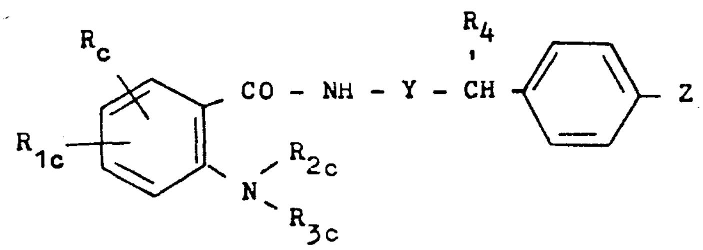

(I''')

in der

$R_4$, Y und Z wie im Anspruch 1 definiert sind,

$R_c$ ein Wasserstoff- oder Chloratom oder eine Piperidinogruppe,

$R_{1c}$ ein Wasserstoff- oder Halogenatom, eine Alkyl- oder Alkoxygruppe mit jeweils 1 bis 6 Kohlenstoffatomen, eine Hydroxy-, Amino-, Nitro- oder Cyangruppe,

$R_{2c}$ eine gegebenenfalls durch eine Phenylgruppe substituierte Alkylgruppe mit 1 bis 3 Kohlenstoffatomen oder eine Alkenylgruppe mit 3 bis 7 Kohlenstoffatomen,

$R_{3c}$ eine Alkylgruppe mit 1 bis 7 Kohlenstoffatomen, eine Alkenylgruppe mit 3 bis 7 Kohlenstoffatomen oder eine Adamantylgruppe oder

$R_{2c}$ und $R_{3c}$ zusammen mit dem dazwischenliegenden Stickstoffatom eine unverzweigte Alkyleniminogruppe mit 4 bis 12 Kohlenstoffatomen im Iminoring, eine durch eine Alkylgruppe mit 1 bis 4 Kohlenstoffatomen substituierte Piperidinogruppe, eine in 3- und in 5-Stellung jeweils durch eine Alkylgruppe mit 1 bis 3 Kohlenstoffatomen substituierte Piperidinogruppe, eine in 4-Stellung durch eine Alkylgruppe mit 1 bis 3 Kohlenstoffatomen, eine Halogenphenylgruppe, eine Carbalkoxygruppe mit insgesamt 2 bis 4 Kohlenstoffatomen oder eine Furoylgruppe substituierte Piperazinogruppe, eine Tetrahydropyridino-, Octahydroisochinolino-, Decahydro-isochinolino-, Decahydro-benzazepino-, Octahydro-isoindolo- oder 1,4-Dioxa-8-azaspiroalkylgruppe mit 7 bis 10 Kohlenstoffatomen bedeuten, sowie von deren physiologisch verträglichen Säureadditionssalzen mit anorganischen oder organischen Säuren und auch Basen, wenn Z die Carboxygruppe enthält, dadurch gekennzeichnet, daß

a) eine Aminobenzoesäure der allgemeinen Formel IIc

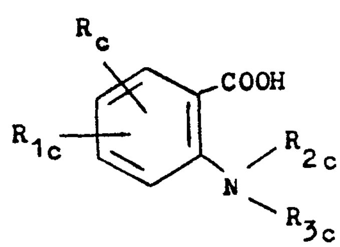

(IIc)

in der $R_c$, $R_{1c}$ bis $R_{3c}$ wie eingangs definiert sind, oder deren gegebenenfalls im Reaktionsgemisch hergestelltes reaktionsfähiges Derivat mit einem Amin der allgemeinen Formel IIIc

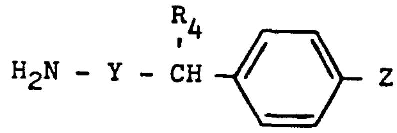

(IIIc)

in der $R_4$, Y und Z wie eingangs definiert sind, oder mit einem gegebenenfalls im Reaktionsgemisch gebildeten N-aktivierten Amin der allgemeinen Formel IIIc, wenn eine Aminobenzoesäure der allgemeinen Formel IIc eingesetzt wird und wenn Z in einem N-aktivierten Amin der allgemeinen Formel IIIc keine Carboxyl- oder Aminogruppe enthält, umgesetzt wird oder

c) zur Herstellung von Verbindungen der allgemeinen Formel I''', in der Z eine Carboxygruppe und Y keine NH-Gruppe darstellt, eine Verbindung der allgemeinen Formel VIc

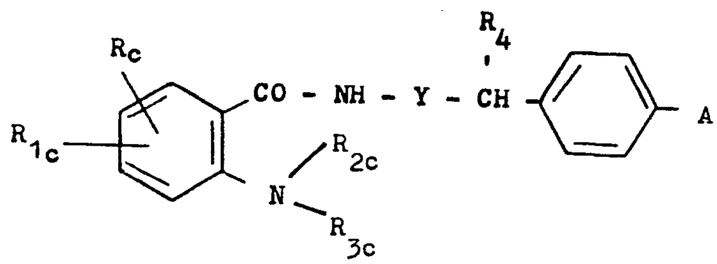

(VIc)

in der
$R_4$ und A wie im Anspruch 1, $R_c$ und $R_{1c}$ bis $R_{3c}$ und
Y wie eingangs definiert sind, oxydiert wird oder

d) zur Herstellung von Verbindungen der allgemeinen Formel I''', in der Z eine Carboxygruppe darstellt, eine Verbindung der allgemeinen Formel VIIc

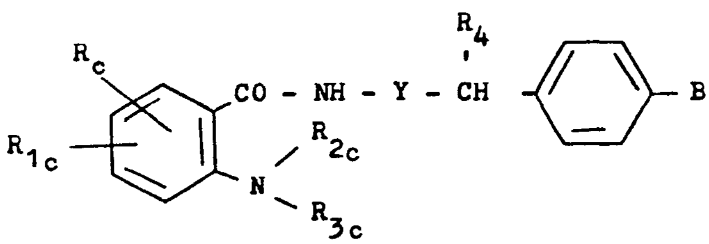

(VIIc)

in der $R_4$, B und Y wie im Anspruch 1, $R_c$ und $R_{1c}$ bis $R_{3c}$ wie eingangs definiert sind, hydrolysiert wird oder

e) zur Herstellung von Verbindungen der allgemeinen Formel I''', in der $R_{2c}$ eine gegebenenfalls durch eine Phenylgruppe substituierte Alkylgruppe mit 1 bis 3 Kohlenstoffatomen oder eine Alkenylgruppe mit 3 bis 7 Kohlenstoffatomen, $R_{3c}$ eine Alkylgruppe mit 1 bis 7 Kohlenstoffatomen, eine Alkenylgruppe mit 3 bis 7 Kohlenstoffatomen oder eine Adamantylgruppe oder $R_{2c}$ und $R_{3c}$ mit dem dazwischenliegenden Stickstoffatom einen 5- bis 7-gliedrigen Alkyleniminoring, eine durch eine Alkylgruppe mit 1 bis 4 Kohlenstoffatomen substituierte Piperidinogruppe oder eine in 3- und 5-Stellung jeweils durch eine Alkylgruppe mit 1 bis 3 Kohlenstoffatomen substituierte Piperidinogruppe und Y eine gegebenenfalls durch eine oder zwei Alkylgruppen mit jeweils 1 bis 3 Kohlenstoffatomen substituierte Methylengruppe bedeuten, eine gegebenenfalls im Reaktionsgemisch gebildete Verbindung der allgemeinen Formel VIIIc

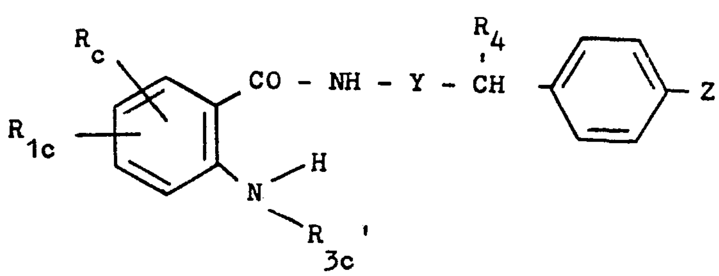

(VIIIc)

in der

$R_4$, Y und Z wie im Anspruch 1, $R_c$ und $R_{1c}$ wie eingangs definiert sind und

$R_{3c}'$ ein Wasserstoffatom darstellt oder die für $R_{3c}$ vorstehend erwähnten Bedeutungen besitzt, mit einer Verbindung der allgemeinen Formel IXc

$$R_{2c}'—G \qquad (IXc)$$

in der

G wie im Anspruch 1 definiert ist und

$R_{2c}'$ die für $R_{2c}$ eingangs erwähnten Bedeutungen besitzt oder zusammen mit dem Rest $R_{3c}'$ der Formel VIIIc eine geradkettige Alkylengruppe mit 4 bis 6 Kohlenstoffatomen, eine durch eine Alkylgruppe mit 1 bis 4 Kohlenstoffatomen substituierte n-Pentylengruppe oder eine in 2- und 4-Stellung jeweils durch eine Alkylgruppe mit 1 bis 3 Kohlenstoffatomen substituierte n-Pentylengruppe darstellt, umgesetzt wird oder

f) zur Herstellung von Verbindungen der allgemeinen Formel I‴, in der Y die NH-Gruppe oder einen Sauerstoffatom darstellt, eine Verbindung der allgemeinen Formel Xc

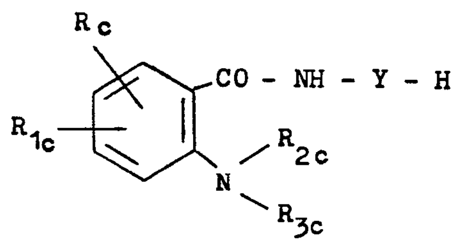

(Xc)

in der $R_c$, $R_{1c}$ bis $R_{3c}$ und Y wie eingangs definiert sind, oder deren Alkalisalz mit einem Phenylderivat der allgemeinen Formel XIc

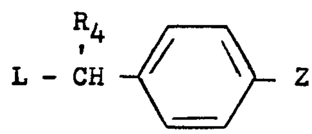

(XIc)

in der $R_4$, L und Z wie im Anspruch 1 definiert sind, umgesetzt wird und gewünschtenfalls anschließend eine so erhaltene Verbindung der allgemeinen Formel I‴, in der Z eine Carboxygruppe darstellt, mittels Veresterung bzw. Amidierung in eine entsprechende Verbindung der allgemeinen Formel I‴, in der Z eine veresterte Carboxygruppe oder eine gegebenenfalls durch eine Alkylgruppe mit 1 bis 7 Kohlenstoffatomen, eine Cycloalkylgruppe mit 3 bis 7 Kohlenstoffatomen und/oder Alkenylgruppe mit 3 bis 7 Kohlenstoffatomen mono- oder disubstituierte Aminocarbonylgruppe, eine Piperidinocarbonyl-, Morpholinocarbonyl-, Thiomorpholinocarbonyl- oder N-Alkylpiperazinocarbonylgruppe darstellt, übergeführt wird und/oder

eine erhaltene Verbindung der allgemeinen Formel I‴, in der $R_{1c}$ und/oder Z eine Nitrogruppe darstellt, mittels Reduktion in eine entsprechende Verbindung der allgemeinen Formel I‴, in der $R_{1c}$ und/oder Z eine Aminogruppe darstellt, übergeführt wird und/oder

eine erhaltene Verbindung der allgemeinen Formel I‴, in der $R_{1c}$ und/oder Z eine Aminogruppe darstellt, über ein entsprechendes Diazoniumsalz in eine entsprechende Verbindung der allgemeinen Formel I‴, in der $R_{1c}$ die Hydroxy- oder Cyangruppe, ein Chlor- oder Bromatom und/oder Z ein Chloratom oder die Cyangruppe darstellen, übergeführt wird, wobei eine so gegebenenfalls erhaltene Verbindung der allgemeinen Formel I‴, in der $R_{1c}$ die Hydroxygruppe darstellt, mittels Alkylierung in eine entsprechende Verbindung der allgemeinen Formel I‴, in der $R_{1c}$ eine Alkoxygruppe mit 1 bis 4 Kohlenstoffatomen darstellt, übergeführt werden kann, und/oder

eine erhaltene Verbindung der allgemeinen Formel I‴, in der Z die Hydroxymethylgruppe darstellt, mittels Oxidation in eine entsprechende Verbindung der allgemeinen Formel I‴, in der Z die Formylgruppe darstellt, übergeführt wird und/oder

eine erhaltene Verbindung der allgemeinen Formel I‴, in der Z die Hydroxymethylgruppe darstellt, mittels Halogenierung und anschließender Umsetzung mit einem Malonsäureester in eine entsprechende Verbindung der allgemeinen Formel I‴, in der Z eine durch zwei Alkoxycarbonylgruppen substituierte Äthylgruppe darstellt, übergeführt wird und/oder

eine erhaltene Verbindung der allgemeinen Formel I‴, in der Z die Formylgruppe darstellt, mittels Acetalisierung in eine entsprechende Verbindung der allgemeinen Formel I‴, in der Z eine Dialkoxymethylgruppe darstellt, übergeführt wird und/oder

eine erhaltene Verbindung der allgemeinen Formel I‴, in der Z die Formylgruppe darstellt, mittels

Kondensation und gegebenenfalls anschließender Hydrolyse in eine entsprechende Verbindung der allgemeinen Formel I''', in der Z eine durch eine Hydroxycarbonyl- oder Alkoxycarbonylgruppe substituierte Äthylengruppe darstellt, übergeführt wird und/oder

eine erhaltene Verbindung der allgemeinen Formel I''', in der Z ein Wasserstoffatom darstellt, mittels Friedel-Crafts-Acylierung in eine entsprechende Verbindung der allgemeinen Formel I''', in der Z eine gegebenenfalls durch eine Alkoxycarbonylgruppe substituierte Acetylgruppe darstellt, übergeführt wird und/oder

eine erhaltene Verbindung der allgemeinen Formel I''', in der Z eine Nitrilgruppe darstellt, mittels Alkoholyse über einen entsprechenden Iminoester in eine entsprechende Verbindung der allgemeinen Formel I''', in der Z eine Trialkoxymethylgruppe darstellt, übergeführt wird und/oder

eine erhaltene Verbindung der allgemeinen Formel I''', in der Z eine Trialkoxymethylgruppe darstellt, mittels Hydrolyse in eine entsprechende Verbindung der allgemeinen Formel I''', in der Z eine Alkoxycarbonylgruppe darstellt, übergeführt wird und/oder

eine erhaltene Verbindung der allgemeinen Formel I''', in der Z die Acetylgruppe darstellt, durch Erhitzen mit einem Amin und Schwefel und anschließend in Gegenwart einer anorganischen Base in eine entsprechende Verbindung der allgemeinen Formel I''', in der Z die Hydroxycarbonylmethylgruppe darstellt, übergeführt wird und/oder

eine erhaltene Verbindung der allgemeinen Formel I''', in der Z die Carboxygruppe darstellt, mittels Überführung in ein Sulfonsäurehydrazid und anschließende Disproportionierung in eine entsprechende Verbindung der allgemeinen Formel I''', in der Z die Formylgruppe darstellt, übergeführt wird und/oder

eine erhaltene Verbindung der allgemeinen Formel I''' in ihre physiologisch verträglichen Salze mit anorganischen oder organischen Säuren und auch Basen, wenn Z eine Carboxylgruppe enthält, übergeführt wird.

5. Verfahren gemäß den Ansprüchen 1 bis 4, dadurch gekennzeichnet, daß die Umsetzung in einem Lösungsmittel durchgeführt wird.

6. Verfahren gemäß den Ansprüchen 1a, 2a, 3a, 4a und 5, dadurch gekennzeichnet, daß die Umsetzung in Gegenwart eines die Säure aktivierenden, eines wasserentziehenden Mittels oder eines das Amin aktivierenden Mittels jeweils gegebenenfalls in Gegenwart einer anorganischen oder tertiären organischen Base und bei Temperaturen zwischen − 25 und 250 °C, vorzugsweise jedoch bei Temperaturen zwischen − 10 °C und der Siedetemperatur des verwendeten Lösungsmittels, durchgeführt wird.

7. Verfahren gemäß den Ansprüchen 1b, 2b, 3b und 5, dadurch gekennzeichnet, daß die Umsetzung in Gegenwart einer anorganischen oder tertiären organischen Base, in Gegenwart eines Reaktionsbeschleunigers wie Kupfer und/oder in einem Druckgefäß und bei Temperaturen zwischen 20 und 150 °C, vorzugsweise jedoch bei der Siedetemperatur des Reaktionsgemisches durchgeführt wird.

8. Verfahren gemäß den Ansprüchen 1c, 2c, 3c, 4c und 5, dadurch gekennzeichnet, daß die Umsetzung bei Temperaturen zwischen 0 und 100 °C, vorzugsweise jedoch bei Temperaturen zwischen 20 und 50 °C, durchgeführt wird.

9. Verfahren gemäß den Ansprüchen 1d, 2d, 3d, 4d und 5, dadurch gekennzeichnet, daß die Hydrolyse in Gegenwart einer Säure oder Base und bei der Siedetemperatur des Reaktionsgemisches durchgeführt wird.

10. Verfahren gemäß den Ansprüchen 1e, 2e, 3e, 4e und 5, dadurch gekennzeichnet, daß die Methylierung mit Formaldehyd in Gegenwart eines Reduktionsmittels durchgeführt wird.

**Claims** (for the Contracting States : BE, CH, DE, FR, GB, IT, LI, LU, NL, SE)

1. Carboxylic acid amides of general formula I

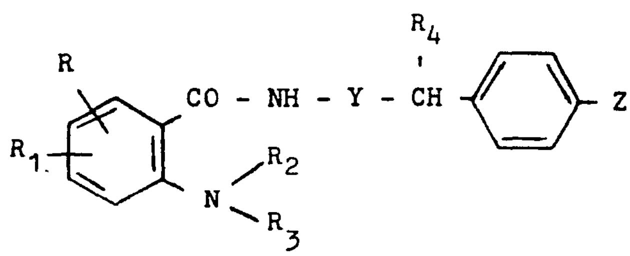

(I)

wherein

R represents a hydrogen, chlorine or bromine atom or a cyclic alkyleneimino group with 4 to 7 carbon atoms in the imino ring ;

$R_1$ represents a hydrogen, fluorine, chlorine or bromine atom ; an alkyl or alkoxy group with 1 to 6 carbon atoms ; an alkoxy group with 1 to 3 carbon atoms substituted by a phenyl group ; a hydroxy, nitro, amino, cyano or carboxy group ; an alkanoylamino, alkoxycarbonyl or dialkylamidosulphonyl group, wherein the alkyl part may contain 1 to 3 carbon atoms ;

$R_2$ and $R_3$, which may be the same or different, represent alkyl groups with 1 to 7 carbon atoms, alkenyl groups with 3 to 7 carbon atoms, cycloalkyl groups with 3 to 7 carbon atoms, alkyl groups with 1 to 3 carbon atoms substituted by a phenyl group, or phenyl or adamantyl groups, or

$R_2$ and $R_3$ together with the nitrogen atom between them represent an unbranched alkyleneimino group with 3 to 12 carbon atoms in the imino ring, an unbranched alkyleneimino group with 4 to 6 carbon atoms in the imino ring which may be substituted by one or two alkyl groups each with 1 to 4 carbon atoms, an alkoxy group with 1 to 3 carbon atoms, a hydroxy, phenyl, carboxy or alkoxycarbonyl group with a total of 2 to 4 carbon atoms and/or wherein a methylene group is replaced by an imino group (which may be substituted by an alkyl group with 1 to 3 carbon atoms, an alkoxycarbonyl group with a total of 2 to 4 carbon atoms, a phenyl, halophenyl, benzyl, pyridyl or furoyl group), by an oxygen or sulphur atom or by a carbonyl, sulphoxide or sulphonyl group ; a saturated or partially unsaturated azabicycloalkyl group with 7 to 10 carbon atoms, a piperidino group substituted in the 3- and 5-positions by a total of 3 or 4 alkyl groups each with 1 to 3 carbon atoms ; a 1,4-dioxa-8-aza-spiroalkyl group with 6 to 9 carbon atoms, or a pyrrolyl or tetrahydropyridino group ;

$R_4$ represents a hydrogen atom or an alkyl group with 1 to 3 carbon atoms ;

Y represents an oxygen atom, an imino group or a methylene group optionally substituted by one or two alkyl groups each having 1 to 3 carbon atoms,

Z represents a hydrogen or halogen atom, a carboxy, cyano, formyl, hydroxymethyl, hydroxycarbonylethylene, nitro, amino, allyloxycarbonyl, phenoxycarbonyl, benzyloxycarbonyl or phenylethoxycarbonyl group, an alkoxycarbonyl group with a total of 2 to 8 carbon atoms, a cycloalkoxycarbonyl group with a total of 4 to 8 carbon atoms, a methyl group optionally substituted by 2 or 3 alkoxy groups or by a carboxy, alkoxycarbonyl or ethylenedioxy group, wherein the alkoxy group may contain 1 to 3 carbon atoms ; an acetyl group, optionally substituted by a carboxy group or alkoxycarbonyl group with a total of 2 to 4 carbon atoms ; an ethyl or ethylene group substituted by one or two alkoxycarbonyl groups each having a total of 2 to 4 carbon atoms, or by two carboxy groups ; an aminocarbonyl group optionally mono- or di-substituted by an alkyl group with 1 to 7 carbon atoms, a cycloalkyl group with 3 to 7 carbon atoms and/or an alkenyl group with 3 to 7 carbon atoms ; a piperidinocarbonyl, morpholinocarbonyl, thiomorpholinocarbonyl or N-alkyl-piperazinocarbonyl group wherein the alkyl group may contain 1 to 3 carbon atoms ; or an ethyl group substituted by a carboxy group if the groups $R_2$ and $R_3$ together with the nitrogen atom between them represent one of the above-mentioned cyclic imino groups, and the physiologically acceptable salts thereof with inorganic or organic acids and also bases if Z represents or contains a carboxy group.

2. Carboxylic acid amines of general formula I as claimed in claim 1, wherein

R represents a hydrogen atom ;

$R_1$ represents a hydrogen, fluorine, chlorine or bromine atom ; an alkoxy group with 1 to 6 carbon atoms ; an alkyl group with 1 to 4 carbon atoms ; an alkoxycarbonyl group with a total of 2 to 4 carbon atoms ; or a hydroxy, cyano, carboxy, nitro, amino, acetamido, dimethylaminosulphonyl or benzyloxy group ;

$R_2$ represents an alkyl group with 1 to 6 carbon atoms or a cyclohexyl, phenyl, benzyl, adamantyl or allyl group,

$R_3$ represents an alkyl group with 1 to 6 carbon atoms or an allyl group, or

$R_2$ and $R_3$ together with the nitrogen atom between them represent an unbranched alkyleneimino group with 4 to 12 carbon atoms in the imino ring ; a piperidino group substituted by an alkyl group with 1 to 4 carbon atoms or by a phenyl, hydroxy, methoxy, carboxy or alkoxycarbonyl group with a total of 2 to 4 carbon atoms ; a piperidino group substituted in the 3- and 5-positions by an alkyl group with 1 to 3 carbon atoms ; a piperidino group substituted in the 3- and 5-positions by two alkyl groups each having 1 to 3 carbon atoms ; a morpholino, thiomorpholino, 1-oxidothiomorpholino or 1,1-dioxido-thiomorpholino group, each of which is optionally substituted by 1 or 2 methyl groups, a piperazino group optionally substituted in the 4-position by a methyl, benzyl, phenyl, chlorophenyl, pyridyl, furoyl or alkoxycarbonyl group with a total of 2 to 4 carbon atoms ; or a pyrrolyl, piperidon-(2)-yl-(1), 1,2,3,6-tetrahydropyridino, 1,4-dioxa-8-aza-spiro [4,5] decan-8-yl, 1,4-dioxa-8-aza-spiro [4,6]-undecan-8-yl, octahydro-isoindol-2-yl, 1,2,3,4-tetrahydro-isoquinolin-2-yl, 1,2,3,4,5,6,7,8-octahydro-isoquinolin-2-yl, decahydro-isoquinolin-2-yl, 1,2,4,5-tetrahydro-3-benzazepin-3-yl, decahydro-3-benzazepin-3-yl or 3-aza-bicyclononan-3-yl group ;

$R_4$ represents a hydrogen atom or a methyl group ;

Y represents a methylene, methyl-methylene or dimethylmethylene group, an NH group or an oxygen atom ; and

Z represents a carboxy, cyano, formyl or hydroxymethyl group ; an alkoxycarbonyl group with a total of 2 to 7 carbon atoms ; a cyclohexyloxycarbonyl, benzyloxycarbonyl, diethoxymethyl, hydroxycarbonylmethyl, bis-2,2-ethoxycarbonyl-ethyl, 2-hydroxycarbonyl-ethylene or 2-ethoxycarbonyl-ethyl group ; an acetyl group optionally substituted by a hydroxycarbonyl or ethoxy carbonyl group ; or a 2-hydroxycarbonyl-ethyl group when the groups $R_2$ and $R_3$ together with the nitrogen atom between them represent one of the above-mentioned cyclic imino groups, and the physiologically acceptable salts thereof with inorganic or organic acids and also bases if Z represents or contains a carboxy group.

3. Carboxylic acid amides of general formula I as claimed in claim 1, wherein

$R_1$ is in the 5-position of the phenyl nucleus and

R, $R_1$ to $R_4$, Y and Z are defined as in claim 2, and the physiologically acceptable salts thereof with inorganic or organic acids or also bases if Z represents or contains a carboxy group.

4. Carboxylic acid amines of general formula I as claimed in claim 1, wherein

R represents a hydrogen atom,

$R_1$ in the 5-position represents a hydrogen, fluorine, chlorine or bromine atom or an alkyl or alkoxy group each with 1 to 3 carbon atoms, or a carboxy, cyano or nitro group,

$R_2$ and $R_3$ together with the nitrogen atoms between them represent an N,N-dialkylamino or N-alkyl-cyclohexylamino group each having 1 to 3 carbon atoms in the alkyl part ; a pyrrolidino, piperidino, hexamethyleneimino, heptamethyleneimino, octamethyleneimino or nonamethyleneimino group ; a piperidino group substituted by an alkyl group with 1 to 4 carbon atoms or by a methoxy or phenyl group ; a piperidino group substituted in the 3- and 5-positions by one or two methyl or ethyl groups; a morpholino or thiomorpholino group optionally substituted in the 2- and 6-positions by a methyl group ; or a 1,4-dioxa-8-aza-spiro [4,5]-decan-8-yl, 1,4-dioxa-8-aza-spiro [4,6] undecan-yl, octahydro-isoindol-2-yl, 1,2,3,4-tetrahydro-iso-quinolin-2-yl, 1,2,3,4,5,6,7,8-octahydro-isoquinolin-2-yl, decahydro-isoquinolin-2-yl, 1,2,4,5-tetrahydro-3H-3-benzazepin-3-yl, decahydro-3H-3-benzazepin-3-yl, 3-aza-bicyclo [3,2,2]-nonan-3-yl or N-methyl-adamantyl-(1)-amino group ;

$R_4$ represents a hydrogen atom or a methyl group ;

Y represents a methylene, methyl-methylene, dimethyl-methylene or NH group or an oxygen atom ;

Z represents an alkoxycarbonyl group with a total of 2 to 4 carbon atoms, a carboxy, formyl or hydroxymethyl group, and the physiologically acceptable salts thereof with inorganic or organic acids and also bases if Z represents the carboxy group.

5. Carboxylic acid amides of general formula I as claimed in claim 3, wherein

R, $R_4$, Y and Z are defined as in claim 3,

$R_1$ represents a chlorine or bromine atom, or a methyl, ethyl or methoxy group ;

$R_2$ and $R_3$ together with the nitrogen atom between them represent a pyrrolidino, piperidino, methyl-piperidino, 4-methoxy-piperidino, 4-phenyl-piperidino, hexamethyleneimino, heptamethyleneimino, octamethyleneimino, nonamethyleneimino, 3,5-dimethyl-piperidino, morpholino, 2,6-dimethyl-morpholino, thiomorpholino, 2,6-dimethyl-thiomorpholino, 1,4-dioxa-8-aza-spiro [4,5]-decan-8-yl, 1,2,3,4-tetrahydro-isoquinolin-2-yl, decahydro-isoquinolin-2-yl, 1,2,4,5-tetrahydro-3H-3-benzazepin-3-yl, decahydro-3H-3-benzazepin-3-yl, octahydro-isoindol-2-yl or N-methyl-adamantyl-(1)-amino group ; or a piperidino group substituted in the 4-position by an alkyl group with 2 to 4 carbon atoms, and the physiologically acceptable salts thereof with inorganic or organic acids and also bases if Z represents the carboxy group.

6. 4-[2-(5-Chloro-2-piperidino-benzoylamino)-ethyl]-benzoic acid, the alkyl esters thereof with 1 to 3 carbon atoms and the acid addition salts thereof.

7. 4-[2-(5-Chloro-2-nonamethyleneimino-benzoylamino)- ethyl]-benzoic acid, the alkyl esters thereof with 1 to 3 carbon atoms and the acid addition salts thereof.

8. 4-[2-(5-Chloro-2-(1,4-dioxa-8-aza-spiro [4,5]-decan-8-yl)-benzoylamino)-ethyl]-benzoic acid, the alkyl esters thereof with 1 to 3 carbon atoms and the acid addition salts thereof.

9. 4-[2-(5-Chloro-2-octamethyleneimino-benzoylamino)-ethyl]-benzoic acid, the alkyl esters thereof with 1 to 3 carbon atoms and the acid addition salts thereof.

10. 4-[2-(5-Bromo-octamethyleneimino-benzoyl-amino)-ethyl]-benzoic acid, the alkyl esters thereof with 1 to 3 carbon atoms and the acid addition salts thereof.

11. 4-[2-(5-Chloro-2-(*cis*-3,5-dimethylpiperidino)-benzoylamino)-ethyl]-benzoic acid, the alkyl esters thereof with 1 to 3 carbon atoms and the acid addition salts thereof.

12. Pharmaceutical compositions containing a compound as claimed in claims 1 to 11 together with one or more inert carriers and/or diluents.

13. Pharmaceutical compositions as claimed in claim 12 for the treatment of diabetes mellitus.

14. Process for the preparation of new carboxylic acid amides of general formula I as claimed in claims 1 to 11, and of the physiologically acceptable salts thereof with inorganic or organic acids and also bases if Z represents or contains a carboxy group, characterised in that

a) an aminobenzoic acid of general formula II

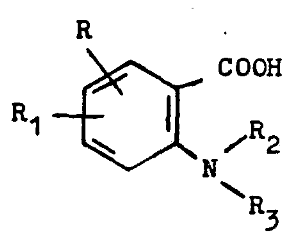

(II)

wherein R, $R_1$, $R_2$ and $R_3$ are as hereinbefore defined, or a reactive derivative thereof, optionally prepared in the reaction mixture, is reacted with an amine of general formula III

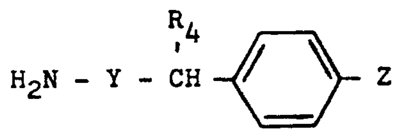

(III)

wherein $R_4$, Y and Z are as hereinbefore defined, or with an N-activated amine of general formula III optionally formed in the reaction mixture, if an aminobenzoic acid of general formula II is used and if Z in an N-activated amine of general formula III does not contain any carboxy or amino groups, or

b) in order to prepare compounds of general formula I wherein $R_1$ has the definitions given hereinbefore, with the exception of the hydroxy and amino group, and Y represents a methylene group optionally substituted by one or two alkyl groups each having 1 to 3 carbon atoms, a compound of general formula IV

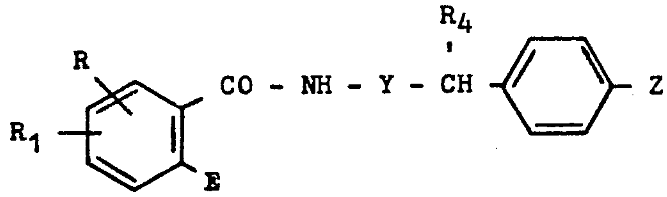

(IV)

wherein

R, $R_4$ and Z are as hereinbefore defined,

$R_1$ and Y are defined as above and

E represents a halogen atom, is reacted with an amine of general formula V

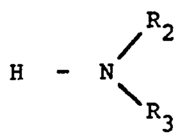

(V)

wherein $R_2$ and $R_3$ are as hereinbefore defined, or

c) in order to prepare compounds of general formula I wherein Z represents a carboxy group and Y does not represent an NH group a compound of general formula VI

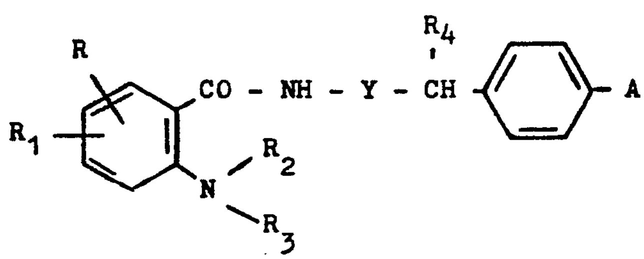

(VI)

wherein

R and $R_1$ to $R_4$ are as hereinbefore defined,

Y has the meanings given hereinbefore with the exception of the NH group and

A represents a group which can be converted into a carboxy group by oxidation, is oxidised or

d) in order to prepare compounds of general formula I wherein Z represents a carboxy group, a compound of general formula VII

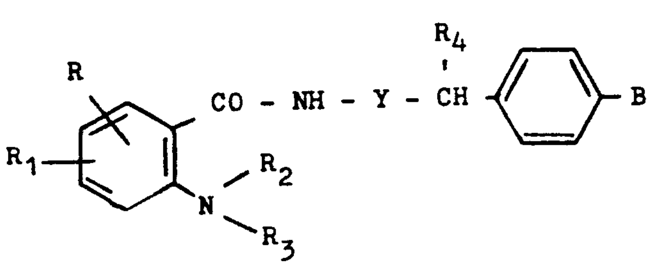

(VII)

wherein

R, $R_1$ to $R_4$ and Y are as hereinbefore defined and

B represents a group which can be converted into a carboxy group by hydrolysis, is hydrolysed, or

e) in order to prepare compounds of general formula I wherein $R_2$ represents an alkyl group with 1 to 7 carbon atoms, a cycloalkyl group with 3 to 7 carbon atoms, an alkyl group with 1 to 3 carbon atoms substituted by a phenyl group, or an alkenyl group with 3 to 7 carbon atoms, $R_3$ represents an alkyl group with 1 to 7 carbon atoms, a cycloalkyl group with 3 to 7 carbon atoms, an alkenyl group with 3 to 7 carbon atoms, an alkyl group with 1 to 3 carbon atoms substituted by a phenyl group, or an adamantyl group, or $R_2$ and $R_3$ together with the nitrogen atom between them represent a 5- to 7-membered alkyleneimino ring, a piperidino group substituted by an alkyl group with 1 to 4 carbon atoms, or a piperidino group substituted in the 3- and 5-positions by an alkyl group with 1 to 3 carbon atoms and Y represents a methylene group optionally substituted by one or two alkyl groups each with 1 to 3 carbon atoms, a compound of general formula VIII (optionally formed in the reaction mixture)

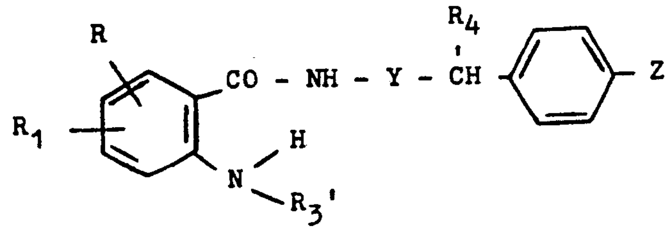

(VIII)

wherein

R, $R_1$, $R_4$ and Z are as hereinbefore defined,

$R_3'$ represents a hydrogen atom or has the meanings given for $R_3$ hereinbefore and

Y is defined as above, is reacted with a compound of general formula IX

$$R_2'—G \qquad (IX)$$

wherein

$R_2'$ has the meanings given for $R_2$ hereinbefore or together with the group $R_3'$ of formula VIII represents a straight-chained alkylene group with 4 to 6 carbon atoms, an n-pentylene group substituted by an alkyl group with 1 to 4 carbon atoms, or an n-pentylene group substituted in the 2- and 4-positions by an alkyl group with 1 to 3 carbon atoms, and

G represents a nucleophilic leaving group such as a halogen atom or a sulphonyloxy group, or

f) in order to prepare compounds of general formula I wherein Y represents the NH group or an oxygen atom, a compound of general formula X

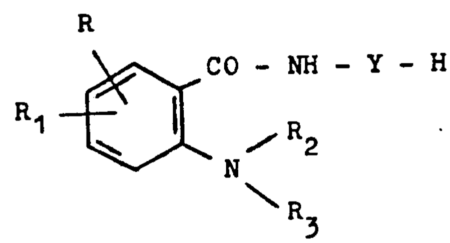

(X)

R and $R_1$ to $R_3$ are as hereinbefore defined and

Y is defined as above, or an alkali metal salt thereof, is reacted with a phenyl derivative of general formula XI

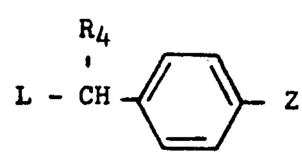

(XI)

wherein

$R_4$ and Z are hereinbefore defined and

L represents a nucleophilic leaving group such as a halogen atom or a sulphonyloxy group, or

g) in order to prepare compounds of general formula I wherein $R_1$ has the meanings given hereinbefore with the exception of the hydroxy, carboxy, amino or alkanoylamino group and Y represents a methylen group optionally substituted by one or two alkyl groups each having 1 to 3 carbon atoms, an amide of general formula XII

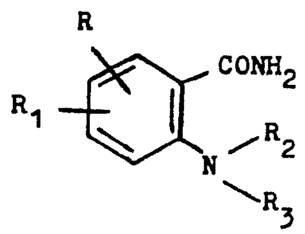

(XII)

wherein

R, $R_2$ and $R_3$ are as hereinbefore defined and

$R_1$ has the meanings given above, or an alkali metal salt thereof, is reacted with a compound of general formula XIII

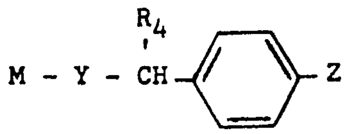

(XIII)

wherein

$R_4$ and Z are as hereinbefore defined,

Y has the meanings given above and

M represents a nucleophilic leaving group such as a halogen atom or a sulphonyloxy group, or

h) in order to prepare compounds of general formula I wherein $R_1$ represents a hydrogen, fluorine, chlorine or bromine atom or an alkyl or alkoxy group each having 1 to 6 carbon atoms, an alkoxy group with 1 to 3 carbon atoms substituted by a phenyl group, or a hydroxy, nitro, carboxy or alkanoylamino group, Y represents a methylene group optionally substituted by one or two alkyl groups each having 1 to 3 carbon atoms, and Z represents a carboxy group, a compound of general formula XIV

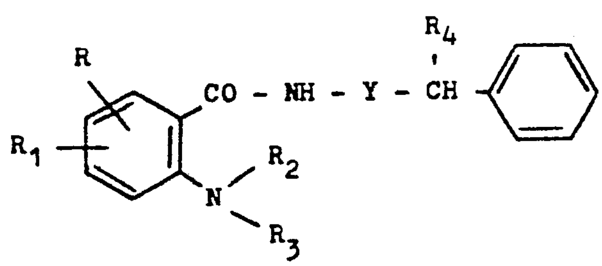

(XIV)

wherein

R and $R_2$ to $R_4$ are as hereinbefore defined,

$R_1$ and Y have the meanings given above, is acylated with an oxalyl halide or phosgene in the presence of a Lewis acid or

i) in order to prepare compounds of general formula I wherein $R_1$ represents a hydrogen, fluorine, chlorine or bromine atom, an alkyl or alkoxy group each with 1 to 6 carbon atoms, an alkoxy group with 1 to 3 carbon atoms substituted by a phenyl group, or a nitro, carboxy, alkanoylamino or alkoxycarbonyl group each having 1 to 3 carbon atoms in the alkyl part, Y represents a methylene group optionally substituted by one or two alkyl groups each with 1 to 3 carbon atoms, and Z represents a carboxy group, a compound of general formula XV

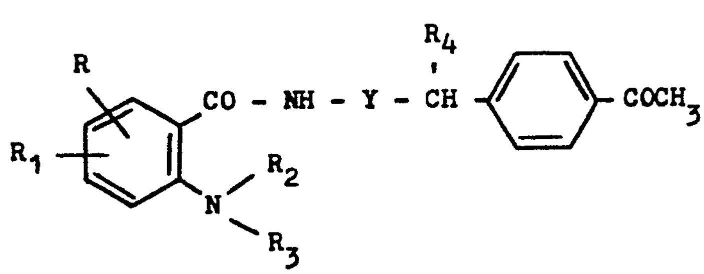

(XV)

wherein

R and $R_2$ to $R_4$ are as hereinbefore defined, and

$R_1$ and Y have the meanings given above, is reacted with a hypohalite optionally prepared in the reaction mixture

and, if desired, a compound of general formula I thus obtained wherein Z represents a carboxy group is subsequently converted by esterification or amidation into a corresponding compound of general formula I wherein Z represents an esterified carboxy group or an aminocarbonyl group optionally mono- or disubstituted by an alkyl group with 1 to 7 carbon atoms or by a cycloalkyl group with 3 to 7 carbon atoms and/or by an alkenyl group with 3 to 7 carbon atoms, or into a piperidinocarbonyl, morpholinocarbonyl, thiomorpholinocarbonyl or N-alkylpiperazinocarbonyl group, and/or

a compound of general formula I obtained wherein $R_1$ and/or Z represents a nitro group is converted by reduction into a corresponding compound of general formula I wherein $R_1$ and/or Z represents an amino group, and/or

a compound of general formula I obtained wherein $R_1$ and/or Z represents an amino group is converted, via a corresponding diazonium salt, into a corresponding compound of general formula I wherein $R_1$ represents the hydroxy or cyano group or a fluorine, chlorine or bromine atom and/or Z represents a chlorine or bromine atom or the cyano group, whilst a compound of general formula I optionally obtained in this way wherein $R_1$ represents a hydroxy group may be converted by alkylation into a corresponding compound of general formula I wherein $R_1$ represents an alkoxy group with 1 to 6 carbon atoms or an alkoxy group with 1 to 3 carbon atoms substituted by a phenyl group, and/or

a compound of general formula I obtained wherein $R_1$ represents an amino group is converted by acylation into a corresponding compound of general formula I wherein $R_1$ represents an alkanoylamino group with 1 to 3 carbon atoms in the alkanoyl part, and/or

a compound of general formula I obtained wherein $R_1$ represents an alkoxycarbonyl group and/or $R_2$ and $R_3$ together with the nitrogen atom between them represent an alkyleneimino group with 4 to 6 carbon atoms substituted by an alkoxycarbonyl group, whilst the alkoxy part may contain 1 to 3 carbon atoms, is converted by hydrolysis into a corresponding compound of general formula I wherein $R_1$ represents a carboxy group and/ or $R_2$ and $R_3$ together with the nitrogen atom between them represent an alkyleneimino group with 4 to 6 carbon atoms in the imino ring substituted by a carboxy group, and/or

a compound of general formula I obtained wherein $R_2$ and $R_3$ together with the nitrogen atom between them represents an N-benzylpiperazino group is converted by debenzylation into a corresponding compound of general formula I wherein $R_2$ and $R_3$ together with the nitrogen atom between them represent a piperazino group, and/or

a compound of general formula I obtained wherein $R_1$ represents a chlorine or bromine atom is converted by dehalogenation into a corresponding compound of general formula I wherein $R_1$ represents a hydrogen atom, and/or

a carboxylic acid amine of general formula I wherein Z represents an optionally esterified carboxy group is converted by reduction with a metal hydride into a corresponding compound of general formula I wherein Z represents the hydroxymethyl group, and/or

a compound of general formula I obtained wherein Z represents a hydroxymethyl group is converted by oxidation into a corresponding compound of general formula I wherein Z represents a formyl group, and/or

a compound of general formula I obtained wherein Z represents a hydroxymethyl group is converted by halogenation and subsequent reaction with a malonic acid ester into a corresponding compound of general formula I wherein Z represents an ethyl group substituted by two alkoxycarbonyl groups, and/or

a compound of general formula I obtained wherein Z represents the formyl group is converted by acetalisation into a corresponding compound of general formula I wherein Z represents a dialkoxymethyl group, and/or

a compound of general formula I obtained wherein Z represents a formyl group is converted by condensation and optional subsequent hydrolysis into a corresponding compound of general formula I wherein Z represents an ethylene group substituted by a hydroxycarbonyl or alkoxycarbonyl group, and/or

a compound of general formula I obtained wherein Z represents a hydrogen atom is converted by Friedel-Crafts acylation into a corresponding compound of general formula I wherein Z represents an acetyl group optionally substituted by an alkoxycarbonyl group, and/or

a compound of general formula I obtained wherein Z represents a nitrile group is converted by alcoholysis, via a corresponding imino ester, into a corresponding compound of general formula I wherein Z represents a trialkoxymethyl group, and/or

a compound of general formula I obtained wherein Z represents a trialkoxymethyl group is converted by hydrolysis into a corresponding compound of general formula I wherein Z represents an alkoxycarbonyl group, and/or

a compound of general formula I obtained wherein Z represents the acetyl group is converted, by heating the acetyl group is converted, by heating with an amine and sulphur and subsequently in the presence of an inorganic base, into a corresponding compound of general formula I wherein Z represents a hydroxycarbonylmethyl group, and/or

a compound of general formula I obtained wherein Z represents a carboxy group is converted, by conversion into a sulphonic acid hydrazide and subsequent disproportionation, into a corresponding compound of general formula I wherein Z represents a formyl group, and/or

a compound of general formula I obtained wherein $R_1$ represents a hydrogen, fluorine, chlorine or

bromine atom, an alkyl or alkoxy group each with 1 to 6 carbon atoms or an alkoxy group with 1 to 3 carbon atoms substituted by a phenyl group and Y represents a methylene group optionally substituted by one or two alkyl groups each having 1 to 3 carbon atoms and Z represents a chlorine or bromine atom, is converted, via its corresponding organometallic compound, by means of carbon dioxide, into a corresponding compound of general formula I wherein Z represents a carboxy group, and/or

a compound of general formula I obtained is converted into the physiologically acceptable salts thereof with inorganic or organic acids and also bases when Z contains a carboxy group.

**Claims** (for the Contracting State AT)

1. Process for the preparation of new carboxylic acid amides of general formula I

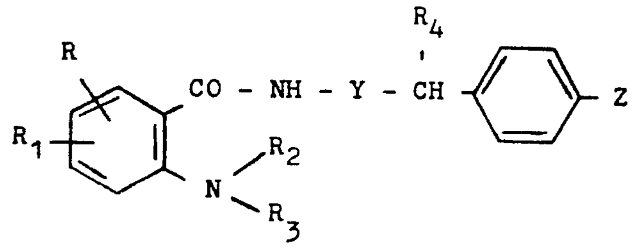

(I)

wherein

R represents a hydrogen, chlorine or bromine atom or a cyclic alkyleneimino group with 4 to 7 carbon atoms in the imino ring ;

$R_1$ represents a hydrogen, fluorine, chlorine or bromine atom ; an alkyl or alkoxy group with 1 to 6 carbon atoms ; an alkoxy group with 1 to 3 carbon atoms substituted by a phenyl group ; a hydroxy, nitro, amino, cyano or carboxy group ; an alkanoylamino, alkoxycarbonyl or dialkylamido-sulphonyl group, wherein the alkyl part may contain 1 to 3 carbon atoms ;

$R_2$ and $R_3$, which may be the same or different, represent alkyl groups with 1 to carbon atoms, alkenyl groups with 3 to 7 carbon atoms, cycloalkyl groups with 3 to 7 carbon atoms, alkyl groups with 1 to 3 carbon atoms substituted by a phenyl group, or phenyl or adamantyl groups, or

$R_2$ and $R_3$ together with the nitrogen atom between them represent an unbranched alkyleneimino group with 3 to 12 carbon atoms in the imino ring, an unbranched alkyleneimino group with 4 to 6 carbon atoms in the imino ring which may be substituted by one or two alkyl groups each with 1 to 4 carbon atoms, an alkoxy group with 1 to 3 carbon atoms, a hydroxy, phenyl, carboxy or alkoxycarbonyl group with a total of 2 to 4 carbon atoms and/or wherein a methylene group is replaced by an imino group (which may be substituted by an alkyl group with 1 to 3 carbon atoms, an alkoxycarbonyl group with a total of 2 to 4 carbon atoms, a phenyl, halophenyl, benzyl, pyridyl or furoyl group), by an oxygen or sulphur atom or by a carbonyl, sulphoxide or sulphonyl group ; with 7 to 10 carbon atoms, a piperidino group substituted in the 3- and 5-positions by a total of 3 or 4 -alkyl groups each with 1 to 3 carbon atoms ; a 1,4-dioxa-8-aza-spiroalkyl group with 6 to 9 carbon atoms, or a pyrrolyl or tetrahydropyridino group ;

$R_4$ represents a hydrogen atom or an alkyl group with 1 to 3 carbon atoms ;

Y represents an oxygen atom, an imino group or a methylene group optionally substituted by one or two alkyl groups each having 1 to 3 carbon atoms,

Z represents a hydrogen or halogen atom, a carboxy, cyano, formyl, hydroxymethyl, hydroxycarbonylethylene, nitro, amino, allyloxycarbonyl, phenoxycarbonyl, benzyloxycarbonyl or phenylethoxycarbonyl group, an alkoxycarbonyl group with a total of 2 to 8 carbon atoms, a cycloalkoxycarbonyl group with a total of 4 to 8 carbon atoms, a methyl group optionally substituted by 2 or 3 alkoxy groups or by a carboxy, alkoxycarbonyl or ethylene-dioxy group, wherein the alkoxy group may contain 1 to 3 carbon atoms ; an acetyl group, optionally substituted by a carboxy group or alkoxycarbonyl group with a total of 2 to 4 carbon atoms ; an ethyl or ethylene group substituted by 1 or two alkoxycarbonyl groups each having a total of 2 to 4 carbon atoms, or by two carboxy groups ; an aminocarbonyl group optionally mono- or di-substituted by an alkyl group with 1 to 7 carbon atoms, a cycloalkyl group with 3 to 7 carbon atoms, a cycloalkyl group with 3 to 7 carbon atoms and/or an alkenyl group with 3 to 7 carbon atoms ; a piperidinocarbonyl, morpholinocarbonyl, thiomorpholinocarbonyl or N-alkyl-piperazinocarbonyl group wherein the alkyl group may contain 1 to 3 carbon atoms ; or an ethyl group substituted by a carboxy group if the groups $R_2$ and $R_3$ together with the nitrogen atom between them represent one of the above-mentioned cyclic imino groups, and the physiologically acceptable salts thereof with inorganic or organic acids and also bases if Z represents or contains a carboxy group, characterised in that

a) an aminobenzoic acid of general formula II

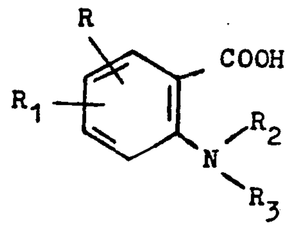

(II)

wherein R, $R_1$,$R_2$ and $R_3$ are as hereinbefore defined, or a reactive derivative thereof, optionally prepared in the reaction mixture, is reacted with an amine of general formula III

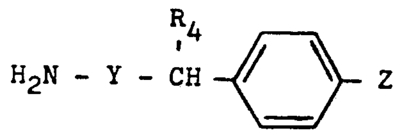

(III)

wherein $R_4$, Y and Z are as hereinbefore defined, or with an N-activated amine of general formula III optionally formed in the reaction mixture, if an aminobenzoic acid of general formula II is used and if Z in an N-activated amine of general formula III does not contain any carboxy or amino groups, or

b) in order to prepare compounds of general formula I wherein $R_1$ has the definitions given hereinbefore, with the exception of the hydroxy and amino group, and Y represents a methylene group optionally substituted by one or two alkyl groups each having 1 to 3 carbon atoms, a compound of general formula IV

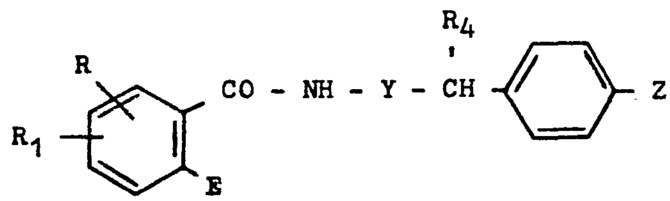

(IV)

wherein

R, $R_4$ and Z are as hereinbefore defined,

$R_1$ and Y are defined as above and

E represents a halogen atom, is reacted with an amine of general formula V

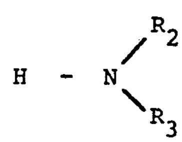

(V)

wherein $R_2$ and $R_3$ are as hereinbefore defined, or

c) in order to prepare compounds of general formula I wherein Z represents a carboxy group and Y does not represent an NH group a compound of general formula VI

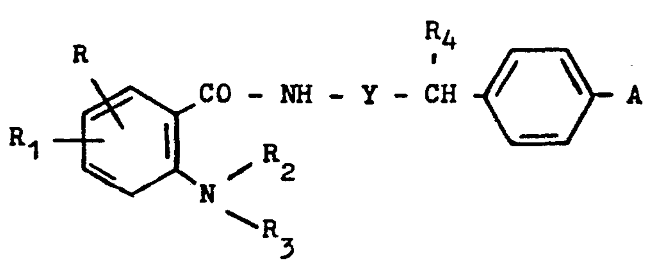

(VI)

wherein

R and $R_1$ to $R_4$ are as hereinbefore defined,

Y has the meanings given hereinbefore with the exception of the NH group and

A represents a group which can be converted into a carboxy group by oxidation, is oxidised or

d) in order to prepare compounds of general formula I wherein Z represents a carboxy group, a compound of general formula VII

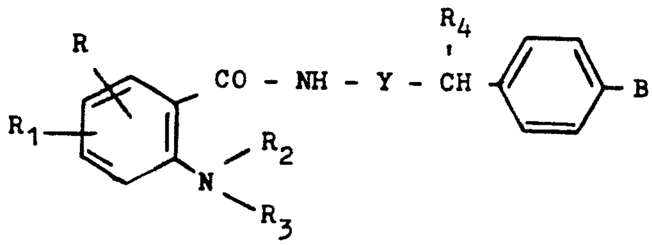

(VII)

wherein

R, $R_1$ to $R_4$ and Y are as hereinbefore defined and

B represents a group which can be converted into a carboxy group by hydrolysis, is hydrolysed, or

e) in order to prepare compounds of general formula I wherein $R_2$ represents an alkyl group with 1 to 7 carbon atoms, a cycloalkyl group with 3 to 7 carbon atoms, an alkyl group with 1 to 3 carbon atoms substituted by a phenyl group, or an alkenyl group with 3 to 7 carbon atoms, $R_3$ represents an alkyl group with 1 to 7 carbon atoms, a cycloalkyl group with 3 to 7 carbon atoms, an alkenyl group with 3 to 7 carbon atoms, an alkyl group with 1 to 3 carbon atoms substituted by a phenyl group, or an adamantyl group, or $R_2$ and $R_3$ together with the nitrogen atom between them represent a 5- to 7-membered alkyleneimino ring, a piperidino group substituted by an alkyl group with 1 to 4 carbon atoms, or a piperidino group substituted in the 3- and 5-positions by an alkyl group with 1 to 3 carbon atoms and Y represents a methylene group optionally substituted by one or two alkyl groups each with 1 to 3 carbon atoms, a compound of general formula VIII (optionally formed in the reaction mixture)

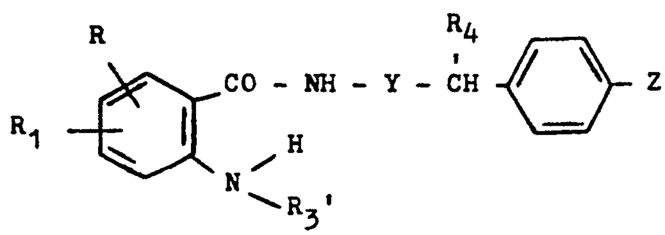

(VIII)

wherein

R, $R_1$, $R_4$ and Z are as hereinbefore defined,

$R_3'$ represents a hydrogen atom or has the meanings giver for $R_3$ hereinbefore and

Y is defined as above, is reacted with a compound of general formula IX

$$R_2'\text{—}G \qquad \text{(IX)}$$

wherein

$R_2'$ has the meanings given for $R_2$ hereinbefore or together with the group $R_3'$ of formula VIII represents a straight-chained alkylene group with 4 to 6 carbon atoms, an n-pentylene group substituted by an alkyl group with 1 to 4 carbon atoms, or an n-pentylene group substituted in the 2- and 4-positions by an alkyl group with 1 to 3 carbon atoms, and

G represents a nucleophilic leaving group such as a halogen atom or a sulphonyloxy group, or

f) in order to prepare compounds of general formula I wherein Y represents the NH group or an oxygen atom, a compound of general formula X

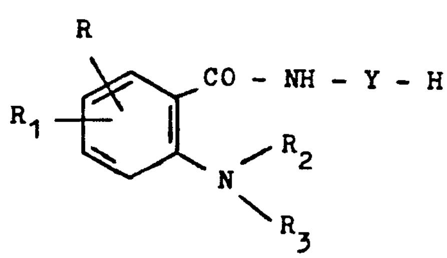

(X)

wherein,

R and $R_1$ to $R_3$ are as hereinbefore defined and

Y is defined as above, or an alkali metal salt thereof, is reacted with a phenyl derivative of general formula XI

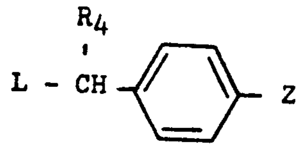

(XI)

wherein

$R_4$ and Z are hereinbefore defined and

L represents a nucleophilic leaving group such as a halogen atom or a sulphonyloxy group, or

g) in order to prepare compounds of general formula I wherein $R_1$ has the meanings given hereinbefore with the exception of the hydroxy, carboxy, amino or alkanoylamino group and Y represents a methylene group optionally substituted by one or two alkyl groups each having 1 to 3 carbon atoms, an amide of general formula XII

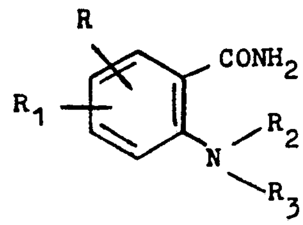

(XII)

wherein

R, $R_2$ and $R_3$ are as hereinbefore defined and

$R_1$ has the meanings given above, or an alkali metal salt thereof, is reacted with a compound of general formula XIII

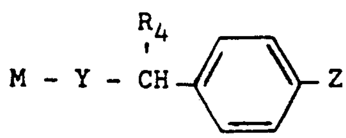

(XIII)

wherein

$R_4$ and Z are as hereinbefore defined,

Y has the meanings given above and

M represents a nucleophilic leaving group such as a halogen atom or a sulphonyloxy group, or

h) in order to prepare compounds of general formula I wherein $R_1$ represents a hydrogen, fluorine, chlorine or bromine atom or an alkyl or alkoxy group each having 1 to 6 carbon atoms, an alkoxy group with 1 to 3 carbon atoms substituted by a phenyl group, or a hydroxy, nitro, carboxy or alkanoylamino group, Y represents a methylene group optionally substituted by one or two alkyl each having 1 to 3 carbon atoms, and Z represents a carboxy group, a compound of general formula XIV

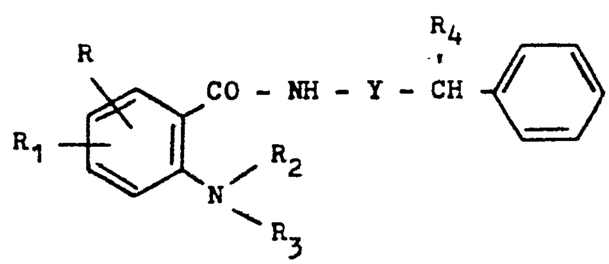

(XIV)

wherein

R and $R_2$ to $R_4$ are as hereinbefore defined,

$R_1$ and Y have the meanings given above, is acylated with an oxalyl halide or phosgene in the presence of a Lewis acid or

i) in order to prepare compounds of general formula I wherein $R_1$ represents a hydrogen, fluorine, chlorine or bromine atom, an alkyl or alkoxy group each with 1 to 6 carbon atoms, an alkoxy group with 1 to 3 carbon atoms substituted by a phenyl group, or a nitro, carboxy, alkanoylamino or alkoxycarbonyl group each having 1 to 3 carbon atoms in the alkyl part, Y represents a methylene group optionally substituted by one or two alkyl groups each with 1 to 3 carbon atoms, and Z represents a carboxy group, a compound of general formula XV

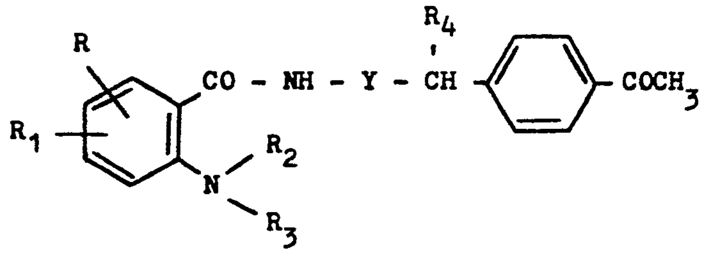

(XV)

wherein

R and $R_2$ to $R_4$ are as hereinbefore defined, and

$R_1$ and Y have the meanings given above, is reacted with a hypohalite optionally prepared in the reaction mixture and, if desired, a compound of general formula I thus obtained wherein Z represents a carboxy group is subsequently converted by esterification or amidation into a corresponding compound of general formula I wherein Z represents an esterified carboxy group or an aminocarbonyl group optionally mono- or disubstituted by an alkyl group with 1 to 7 carbon atoms or by a cycloalkyl group with 3 to 7 carbon atoms and/or by an alkenyl group with 3 to 7 carbon atoms, or into a piperidinocarbonyl, morpholinocarbonyl, thiomorpholinocarbonyl or N-alkylpiperazinocarbonyl group, and/or

a compound of general formula I obtained wherein $R_1$ and/or Z represents a nitro group is converted by reduction into a corresponding compound of general formula I wherein $R_1$ and/or Z represents an amino group, and/or

a compound of general formula I obtained wherein $R_1$ and/or Z represents an amino group is converted, via a corresponding diazonium alt, into a corresponding compound of general formula I wherein $R_1$ represents the hydroxy or cyano group or a fluorine, chlorine or bromine atom and/or Z represents a chlorine or bromine atom or the cyano group, whilst a compound of general formula I optionally obtained in this way wherein $R_1$ represents a hydroxy group may be converted by alkylation into a corresponding compound of general formula I wherein $R_1$ represents an alkoxy group with 1 to 6 carbon atoms or an alkoxy group with 1 to 3 carbon atoms substituted by a phenyl group, and/or

a compound of general formula I obtained wherein $R_1$ represents an amino group is converted by acylation into a corresponding compound of general formula I wherein $R_1$ represents an alkanoylamino group with 1 to 3 carbon atoms in the alkanoyl part, and/or

a compound of general formula I obtained wherein $R_1$ represents an alkoxycarbonyl group and/or $R_2$ and $R_3$ together with the nitrogen atom between them represent an alkyleneimino group with 4 to 6 carbon atoms substituted by an alkoxycarbonyl group, whilst the alkoxy part may contain 1 to 3 carbon atoms, is converted by hydrolysis into a corresponding compound of general formula I wherein $R_1$ represents a carboxy group and/or $R_2$ and $R_3$ together with the nitrogen atom between them represent an alkyleneimino group with 4 to 6 carbon atoms in the imino ring substituted by a carboxy group, and/or

a compound of general formula I obtained wherein $R_2$ and $R_3$ together with the nitrogen atom between them represent an N-benzylpiperazino group is converted by debenzylation into a corresponding compound of general formula I wherein $R_2$ and $R_3$ together with the nitrogen atom between them represent a piperazino group, and/or

a compound of general formula I obtained wherein $R_1$ represents a chlorine or bromine atom is converted by dehalogenation into a corresponding compound of general formula I wherein $R_1$ represents a hydrogen atom, and/or

a carboxylic acid amide of general formula I wherein Z represents an optionally esterified carboxy group is converted by reduction with a metal hydride into a corresponding compound of general formula I wherein Z represents the hydroxymethyl group, and/or

a compound of general formula I obtained wherein Z represents a hydroxymethyl group is converted by oxidation into a corresponding compound of general formula I wherein Z represents a formyl group, and/or

a compound of general formula I obtained wherein Z represents a hydroxymethyl group is converted by halogenation and subsequent reaction with a malonic acid ester into a corresponding compound of general formula I wherein Z represents an ethyl group substituted by two alkoxycarbonyl groups, and/or

a compound of general formula I obtained wherein Z represents the formyl group is converted by acetalisation into a corresponding compound of general formula I wherein Z represents a dialkoxymethyl group, and/or

a compound of general formula I obtained wherein Z represents a formyl group is converted by condensation and optional subsequent hydrolysis into a corresponding compound of general formula I wherein Z represents an ethylene group substituted by a hydroxycarbonyl or alkoxycarbonyl group, and/or

a compound of general formula I obtained wherein Z represents a hydrogen atom is converted by Friedel-Crafts acylation into a corresponding compound of general formula I wherein Z represents an acetyl group optionally substituted by an alkoxycarbonyl group, and/or

a compound of general formula I obtained wherein Z represents a nitrile group is converted by alcoholysis, via a corresponding imino ester, into a corresponding compound of general formula I wherein Z represents a trialkoxymethyl group, and/or

a compound of general formula I obtained wherein Z represents a trialkoxymethyl group is converted by hydrolysis into a corresponding compound of general formula I wherein Z represents an alkoxycarbonyl group, and/or

a compound of general formula I obtained wherein Z represents the acetyl group is converted, by heating with an amine and sulphur and subsequently in the presence of an inorganic base, into a corresponding compound of general formula I wherein Z represents a hydroxycarbonylmethyl group, and/or

a compound of general formula I obtained wherein Z represents a carboxy group is converted, by conversion into a sulphonic acid hydrazide and subsequent disproportionation, into a corresponding compound of general formula I wherein Z represents a formyl group, and/or

a compound of general formula I obtained wherein $R_1$ represents a hydrogen, fluorine, chlorine or bromine atom, an alkyl or alkoxy group each with 1 to 6 carbon atoms or an alkoxy group with 1 to 3 carbon atoms substituted by a phenyl group and Y represents a methylene group optionally substituted by one or two alkyl groups each having 1 to 3 carbon atoms and Z represents a chlorine or bromine atom, is converted, via its corresponding organometallic compound, by means of carbon dioxide, into a corresponding compound of general formula I wherein Z represents a carboxy group, and/or

a compound of general formula I obtained is converted into the physiologically acceptable salts thereof with inorganic or organic acids and also bases when Z contains a carboxy group.

2. Process as claimed in claim 1 for the preparation of new carboxylic acid amides of general formula I'.

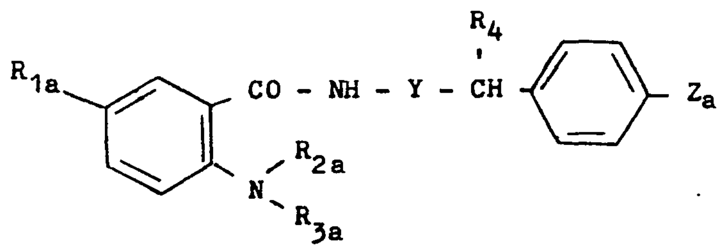

(I')

wherein

$R_4$ is defined as in claim 1,

$R_{1a}$ represents a hydrogen, fluorine, chlorine or bromine atom, a nitro, amino, alkanoylamino, alkoxy, cyano, carboxy, alkoxycarbonyl or dialkylamidosulphonyl group, wherein the alkyl moiety may contain 1 to 3 carbon atoms,

$R_{2a}$ and $R_{3a}$, which may be the same or different, represent alkyl groups with 1 to 7 carbon atoms or cycloalkyl groups with 3 to 7 carbon atoms or

$R_{2a}$ and $R_{3a}$ together with the nitrogen atom between them represent an imino group with 4 to 6 carbon atoms in the imino ring, which may be substituted by 1 or 2 alkyl groups each with 1 to 3 carbon atoms, or by an alkoxy group with 1 to 3 carbon atoms or by a hydroxy, phenyl, carboxy or alkoxycarbonyl group with a total of 2 to 4 carbon atoms ; or wherein a methylene group of the imino ring may be replaced by a cyclic ketal group with a total of 3 or 4 carbon atoms, or by an oxygen or sulphur atom or by a sulphoxide or an imino group, which is substituted by a phenyl or pyridyl group ; a trimethyleneimino, pyrrolyl, tetrahydro-isoquinolyl or tetrahydrobenzazepinyl group and

$Z_a$ represents a carboxy or alkoxycarbonyl group with a total of 2 to 5 carbon atoms, and of the physiologically acceptable salts thereof with inorganic or organic acids or also bases if $Z_a$ represents a carboxy group, characterised in that

a) an aminobenzoic acid of general formula IIa

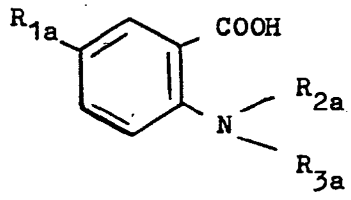

(IIa)

wherein $R_{1a}$, $R_{2a}$ and $R_{3a}$ are as hereinbefore defined, or a reactive derivative thereof optionally prepared in the reaction mixture, is reacted with a phenylalkylamine of general formula IIIa

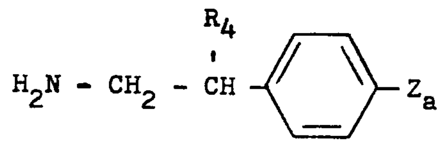

(IIIa)

wherein $R_4$ and $Z_4$ are as hereinbefore defined, or with a reactive derivative thereof optionally formed in the reaction mixture, if an aminobenzoic acid of general formula IIa is used and $Z_a$ represents an alkoxycarbonyl group with a total of 2 to 5 carbon atoms, or

b) in order to prepare a compound of general formula I' wherein $R_{1a}$ represents a nitro group, a nitrobenzamide of general formula IVa

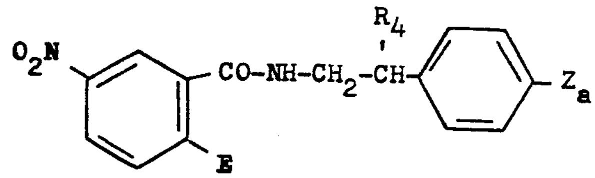

(IVa)

wherein

$R_4$ and $Z_a$ are as hereinbefore defined and

E represents a halogen atom, is reacted with an amine of general formula Va

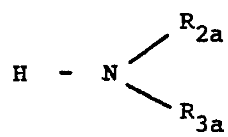

(Va)

wherein $R_{2a}$ and $R_{3a}$ are as hereinbefore defined, or

c) in order to prepare a compound of general formula I' wherein $Z_a$ represents a carboxy group, a compound of general formula VIa

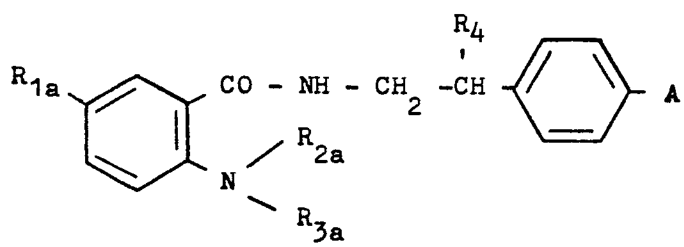

(VIa)

wherein $R_4$ and A are defined as in claim 1 and $R_{1a}$ to $R_{3a}$ are as hereinbefore defined, is oxidised, or

d) in order to prepare a compound of general formula I' wherein $Z_a$ represents a carboxy group, a compound of general formula VIIa

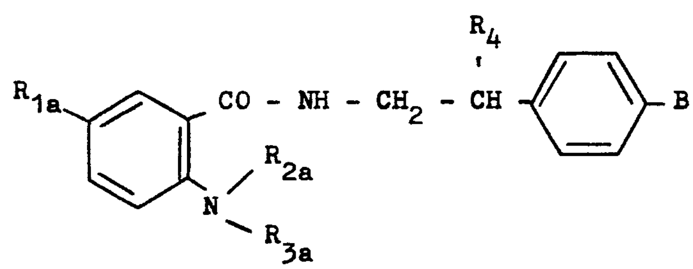

(VIIa)

wherein $R_4$ and B are defined as in claim 1 and $R_{1a}$ to $R_{3a}$ are as hereinbefore defined, is hydrolysed, or

e) in order to prepare a compound of general formula I' wherein $R_{2a}$ and $R_{3a}'$ which may be the same or different, represent alkyl groups with 1 to 7 carbon atoms or cycloalkyl groups with 1 to 7 carbon atoms or cycloalkyl groups with 3 to 7 carbon atoms or $R_{2a}$ and $R_{3a}$ together with the nitrogen atom between them represent a 5- to 7-membered alkyleneimino ring, a piperidino group substituted by an alkyl group with 1 to 3 carbon atoms or a piperidino group substituted in the 3- and 5-positions by an alkyl group with 1 to 3 carbon atoms, a compound of general formula VIIIa

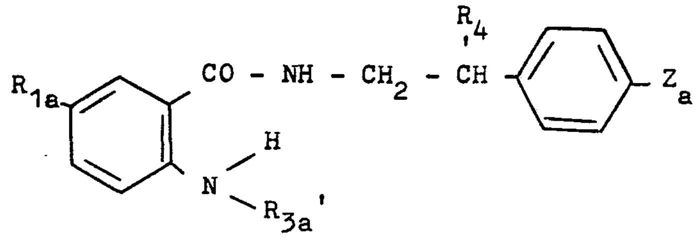

(VIIIa)

wherein

$R_4$ is defined as in claim 1 and $R_{1a}$ and $Z_a$ are as hereinbefore defined and

$R_{3a}'$ represents a hydrogen atom or has the meanings given hereinbefore for $R_{3a}'$ is reacted with a compound of general formula IXa

$$R_{2a}'—G \qquad (IXa)$$

wherein

$R_{2a}'$ has the meanings given hereinbefore for $R_{2a}$ or together with the group $R_{3a}'$ of formula VIIIa represents a straight-chained alkylene group with 4 to 6 carbon atoms, an n-pentylene group substituted by an alkyl group with 1 to 3 carbon atoms or an n-pentylene group substituted in the 2- and 4-positions by an alkyl group with 1 to 3 carbon atoms, and

G represents a nucleophilic leaving group,

and subsequently, if desired, a compound of general formula I′ obtained wherein $Z_a$ represents a carboxy group is converted by esterification into a corresponding compound of general formula I′ wherein $Z_a$ represents an alkoxycarbonyl group with a total of 2 to 5 carbon atoms, and/or

a compound of general formula I′ obtained wherein $R_{1a}$ represents a nitro group is converted by reduction into a corresponding compound of general formula $I_a$ wherein $R_{1a}$ represents an amino group, and/or

a compound of general formula I′ obtained wherein $R_{1a}$ represents an amino group is converted via a corresponding diazonium salt into a corresponding compound of general formula I′ wherein $R_{1a}$ represents a cyano group or a fluorine, chlorine or bromine atom, and/or

a compound of general formula I′ obtained wherein $R_{1a}$ represents an amino group is converted by acylation into a corresponding compound of general formula I′ wherein $R_{1a}$ represents an alkanoylamino group with 1 to 3 carbon atoms in the alkanoyl part, and/or

a compound of general formula I′ obtained wherein $R_{1a}$ represents the cyano group is converted by alcoholysis into a corresponding compound of general formula I′ wherein $R_{1a}$ represents an alkoxycarbonyl group with 1 to 3 carbon atoms in the alkoxy part, and/or

a compound of general formula I′ obtained wherein $R_{1a}$ represents an alkoxycarbonyl group and/or $R_{2a}$ and $R_{3a}$ together with the nitrogen atom between them represent an imino group with 4 to 6 carbon atoms in the imino ring, substituted by an alkoxycarbonyl group, whilst the alkoxy part may contain 1 to 3 carbon atoms, is converted by hydrolysis into a corresponding compound of general formula I′ wherein $R_{1a}$ represents the carboxy group and/or $R_{2a}$ and $R_{3a}$ together with the nitrogen atom between them represent an imino group with 4 to 6 carbon atoms in the imino ring substituted by a carboxy group, and/or

a compound of general formula I′ obtained is converted into the physiologically acceptable salts thereof with inorganic and organic acids or also bases if $Z_a$ represents a carboxy group.

3. Process as claimed in claim 1 for the preparation of new carboxylic acid amides of general formula I″

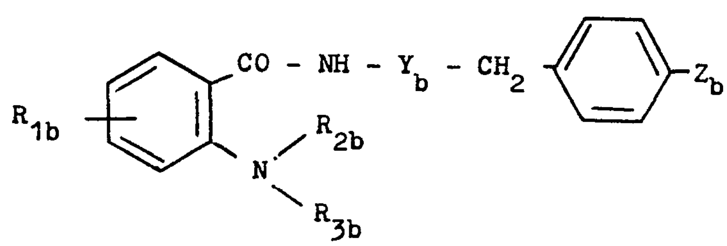

(I″)

wherein

$R_{1b}$ represents a hydrogen, chlorine or bromine atom, an alkoxy group with 1 to 6 carbon atoms or a benzyloxy, nitro or amino group,

$R_{2b}$ and $R_{3b}'$, which may be the same or different, represent alkyl groups with 1 to 4 carbon atoms, cycloalkyl groups with 3 to 7 carbon atoms or phenyl groups or

$R_{2b}$ and $R_{3b}$ together with the nitrogen atom between represent an unbranched alkyleneimino group with 5 to 12 carbon atoms in the imino ring, a piperidino group wherein a methylene group is replaced by a methylmethylene, methoxymethylene, carbonyl, imino or benzylimino group ; a piperidino group substituted in the 3- and 5-positions by an alkyl group with 1 to 3 carbon atoms, whilst the methylene group may simultaneously be substituted in the 4-position by an oxygen or sulphur atom or by a sulphoxide or sulphone group ; a piperidino group substituted in the 3- and 5-positions by a total of 3 or 4 alkyl groups each having 1 to 3 carbon atoms ; or a 1,1-dioxidothiomorpholino or azabicycloalkyl group with 7 to 9 carbon atoms,

$Y_b$ represents a methylene group optionally substituted by an alkyl group with 1 to 3 carbon atoms, and

$Z_b$ represents a carboxy group or an alkoxycarbonyl group with a total of 2 to 5 carbon atoms, and of the physiologically acceptable salts thereof with inorganic or organic acids and also bases if $Z_b$ represents a carboxy group, characterised in that

a) an aminobenzoic acid of general formula IIb

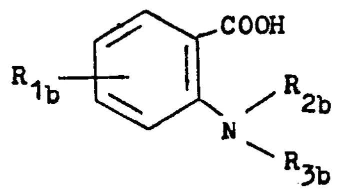

(IIb)

wherein $R_{1b}$, $R_{2b}$ and $R_{3b}$ are as hereinbefore defined, or a reactive derivative thereof optionally prepared in the reaction mixture, is reacted with a phenylalkylamine of general formula IIIb

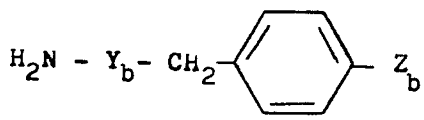

(IIIb)

wherein $Z_b$ and $Y_b$ are as hereinbefore defined, or with an N-activated phenylalkylamine of general formula Vb optionally formed in the reaction mixture, if an aminobenzoic acid of general formula IVb is used and in an N-activated phenylalkylamine of general formula Vb $Z_b$ represents an alkoxycarbonyl group with a total of 2 to 5 carbon atoms, or

b) in order to prepare a compound of general formula I'' wherein $R_{1b}$ in the 3- or 5-positions represents the nitro group, a nitrobenzamide of general formula IVb

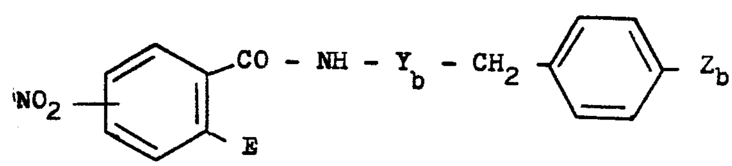

(IVb)

wherein

$Z_b$ and $Y_b$ are as hereinbefore defined and

E represents a halogen atom, is reacted with an amine of general formula Vb

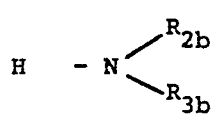

(Vb)

wherein $R_{2b}$ and $R_{3b}$ are as hereinbefore defined, or

c) in order to prepare a compound of general formula I'' wherein $Z_b$ represents a carboxy group, a compound of general formula VIb

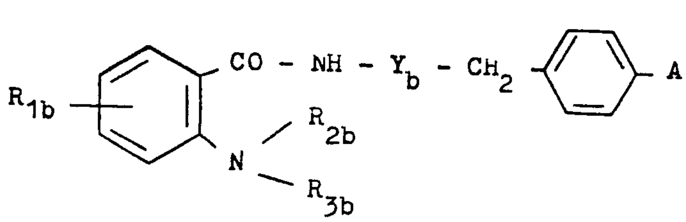

(VIb)

wherein A is defined as in claim 1 and $R_{1b}$ to $R_{3b}$ and $Y_b$ are as hereinbefore defined, is oxidised, or

d) in order to prepare a compound of general formula I″ wherein $Z_b$ represents a carboxy group, a compound of general formula VIIb

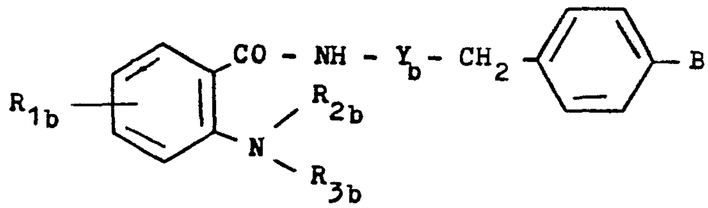

(VIIb)

wherein B is defined as in claim 1 and $R_{1b}$ to $R_{3b}$ and $Y_b$ are as hereinbefore defined, is hydrolysed, or

e) in order to prepare a compound of general formula I″ wherein $R_{2b}$ and $R_{3b}$, which may be the same or different, represent alkyl groups with 1 to 4 carbon atoms, cycloalkyl groups with 3 to 7 carbon atoms or phenyl groups or $R_{2b}$ and $R_{3b}$ together with the nitrogen atom between them represent a 6- to 7-membered imino ring, a methylpiperidino group or a piperidino group substituted in the 3- and 5-positions by 2 to 4 alkyl groups each having 1 to 3 carbon atoms, a compound of general formula VIIIb

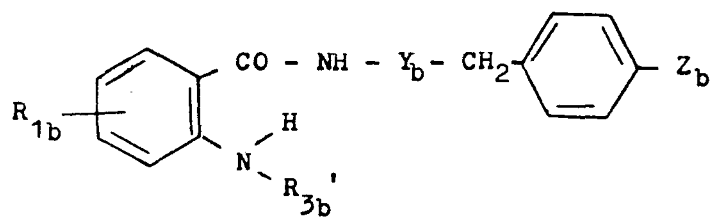

(VIIIb)

wherein

$R_{1b}$, $Y_b$ and $Z_b$ are as hereinbefore defined and

$R_{3b}'$ represents a hydrogen atom or has the meanings given hereinbefore for $R_{3b}'$ is reacted with a compound of general formula IXb

$$R_{2b}'\text{—}G \qquad \text{(IXb)}$$

wherein

$R_{2b}'$ has the meanings given for $R_{2b}$ hereinbefore or together with the group $R_{3b}'$ of formula VIIIb represents a straight-chained alkylene group with 5 to 6 carbon atoms, an n-pentylene group substituted by a methyl group or an n-pentylene group substituted in the 2- and 4-positions by 2 to 4 alkyl groups each having 1 to 3 carbon atoms, and

G represents a nucleophilic leaving group,

and subsequently, if desired, a compound of general formula I″ obtained wherein $Z_b$ represents a carboxy group is converted by esterification into a corresponding compound of general formula I″ wherein $Z_5$ represents an alkoxycarbonyl group with a total of 2 to 5 carbon atoms, and/or

a compound of general formula I″ obtained wherein $R_{1b}$ represents the nitro group is converted by reduction into a corresponding compound of general formula I″ wherein $R_{1b}$ represents an amino group, and/or

a compound of general formula I″ obtained wherein $R_{1b}$ represents an amino group is converted, via a corresponding diazonium salt, into a corresponding compound of general formula I″ wherein $R_{1b}$ represents a chlorine or bromine atom or a hydroxy group, and a compound of general formula I″ thus obtained wherein $R_{1b}$ represents a hydroxy group is converted by alkylation or benzylation into a corresponding compound of general formula I″ wherein $R_{1b}$ represents a benzyloxy or alkoxy group with 1 to 4 carbon atoms, and/or

a compound of general formula I″ obtained wherein $R_{2b}$ and $R_{3b}$ together with the hydrogen atom between them represent an n-benzylpiperazino group is converted by debenzylation into a corresponding compound of general formula I″ wherein $R_{2b}$ and $R_{3b}$ together with the nitrogen atom between them represent a piperazino group, and/or

a compound of general formula I″ obtained wherein $R_{1b}$ represents a chlorine or bromine atom is converted by dehalogenation into a corresponding compound of general formula I″ wherein $R_{1b}$ represents a hydrogen atom, and/or

a compound of general formula I″ obtained is converted into the physiologically acceptable salts thereof with inorganic or organic acids and also bases if $Z_b$ represents a carboxy group.

4. Process as claimed in claim 1 for the preparation of new carboxylic amides of general formula I‴

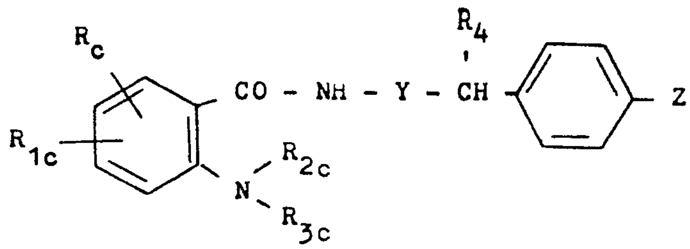

(I''')

wherein

$R_4$, Y and Z are defined as in claim 1,

$R_c$ represents a hydrogen or chlorine atom or a piperidino group,

$R_{1c}$ represents a hydrogen or halogen atom, an alkyl or alkoxy group each with 1 to 6 carbon atoms or a hydroxy, amino, nitro or cyano group,

$R_{2c}$ represents an alkyl group with 1 to 3 carbon atoms optionally substituted by a phenyl group, or an alkenyl group with 3 to 7 carbon atoms,

$R_{3c}$ represents an alkyl group with 1 to 7 carbon atoms, an alkenyl group with 3 to 7 carbon atoms or an adamantyl group, or

$R_{2c}$ and $R_{3c}$ together with the nitrogen atom between them represent an unbranched alkylenemino group with 4 to 12 carbon atoms in the imino ring ; a piperidino group substituted by an alkyl group with 1 to 4 carbon atoms ; a piperidino group substituted in the 3- and 5-positions by an alkyl group with 1 to 3 carbon atoms ; a piperazino group substituted in the 4-position by an alkyl group with 1 to 3 carbon atoms or by a halophenyl group or by a carbalkoxy group with a total of 2 to 4 carbon atoms or by a furoyl group ; a tetrahydropyridino, octahydroisoquinolino, decahydro-isoquinolino, decahydro-benzazepino, octahydro-isoindolo or 1,4-dioxa-8-azaspiroalkyl group with 7 to 10 carbon atoms, and of the physiologically acceptable acid addition salts thereof with inorganic or organic acids and also bases if Z contains a carboxy group, characterised in that

a) an aminobenzoic acid of general formula IIc

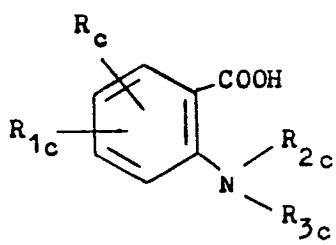

(IIc)

wherein $R_c$, $R_{1c}$ to $R_{3c}$ are as hereinbefore defined, or a reactive derivative thereof optionally prepared in the reaction mixture, is reacted with an amine of general formula IIIc

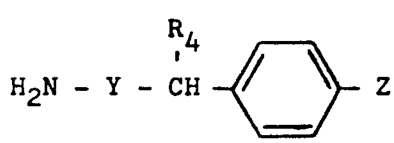

(IIIc)

wherein $R_4$, Y and Z are as hereinbefore defined, or with an N-activated amine of general formula IIIc optionally formed in the reaction mixture, if an aminobenzoic acid of general formula IIc is used and if Z in an N-activated amine of general formula IIIc does not contain a carboxyl or amino group, or

c) in order to prepare compounds of general formula I''', wherein Z represents a carboxy group and Y does not represent an NH group, a compound of general formula VIc

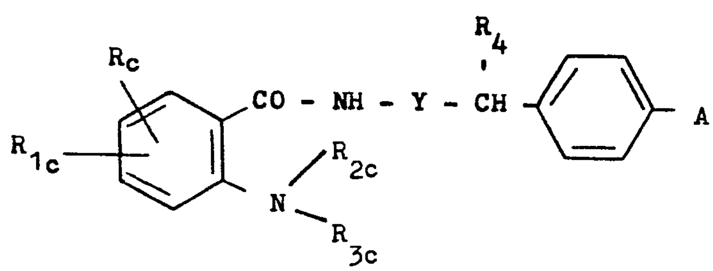

(VIc)

wherein $R_4$ and A are defined as in claim 1 and $R_c$ and $R_{1c}$ to $R_{3c}$ and Y are as hereinbefore defined, is oxidised, or

d) in order to prepare compounds of general formula I‴ wherein Z represents a carboxy group, a compound of general formula VIIc

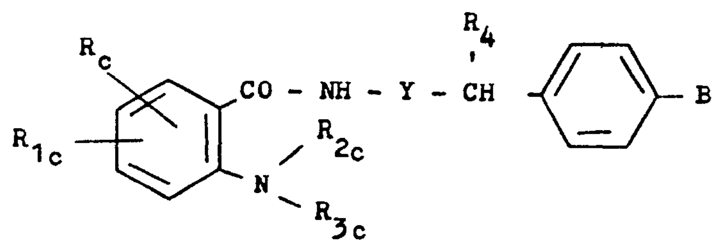

(VIIc)

wherein

$R_4$, B and Y are defined as in claim 1 and $R_c$ and

$R_{1c}$ to $R_{3c}$ are as hereinbefore defined, is hydrolysed, or

e) in order to prepare compounds of general formula I‴ wherein $R_{2c}$ represents an alkyl group with 1 to 3 carbon atoms optionally substituted by a phenyl group, or an alkenyl group with 3 to 7 carbon atoms, $R_{3c}$ represents an alkyl group with 1 to 7 carbon atoms, an alkenyl group with 3 to 7 carbon atoms or an adamantyl group, or $R_{2c}$ and $R_{3c}$ together with the nitrogen atom between them represent a 5- to 7-membered alkyleneimino ring, a piperidino group substituted by an alkyl group with 1 to 4 carbon atoms, or a piperidino group substituted in the 3- and 5-positions by an alkyl group with 1 to 3 carbon atoms, and Y represents a methylene group optionally substituted by one or two alkyl groups each having 1 to 3 carbon atoms, a compound of general formula VIIIc

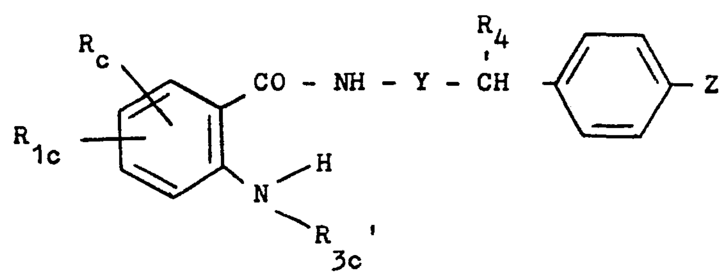

(VIIIc)

optionally formed in the reaction mixture, wherein

$R_4$, Y and Z are defined as in claim 1, $R_c$ and $R_{1c}$ are as hereinbefore defined and

$R_{3c}'$ represents a hydrogen atom or has the meanings given for $R_{3c}$ hereinbefore, is reacted with a compound of general formula IXc

$$R_{2c}'—G \qquad \text{(IXc)}$$

wherein

G is defined as in claim 1 and

$R_{2c}'$ has the meanings given for $R_{2c}$ hereinbefore or, together with the group $R_{3c}'$ of formula VIIIc, represents a straight-chained alkylene group with 4 to 6 carbon atoms, an n-pentylene group substituted by an alkyl group with 1 to 4 carbon atoms, or an n-pentylene group substituted in the 2- and 4-positions by an alkyl group with 1 to 3 carbon atoms, or

f) in order to prepare compounds of general formula I‴ wherein Y represents an NH group or an oxygen atom, a compound of general formula Xc

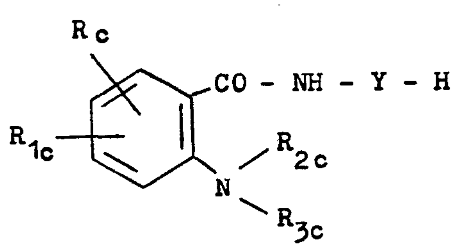

(Xc)

wherein $R_c$, $R_{1c}$ to $R_{3c}$ and Y are as hereinbefore defined, or an alkali metal salt thereof, is reacted with a phenyl derivative of general formula XIc

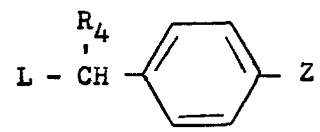

(XIc)

wherein $R_4$, L and Z are defined as in claim 1,

and subsequently, if desired, a compound of general formula I''' thus obtained wherein Z represents a carboxy group, is converted by esterification or amidation into a corresponding compound of general formula I''' wherein Z represents an esterified carboxy group or an aminocarbonyl group optionally mono- or disubstituted by an alkyl group with 1 to 7 carbon atoms, a cycloalkyl group with 3 to 7 carbon atoms and/or an alkenyl group with 3 to 7 carbon atoms ; a piperidinocarbonyl, morpholinocarbonyl, thiomorpholinocarbonyl or N-alkylpiperazinocarbonyl group, and/or

a compound of general formula I''' obtained wherein $R_{1c}$ and/or Z represents a nitro group is converted by reduction into a corresponding compound of general formula I''' wherein $R_{1c}$ and/or Z represents an amino group, and/or

a compound of general formula I''' obtained wherein $R_{1c}$ and/or Z represents an amino group is converted, via a corresponding diazonium salt, into a corresponding compound of general formula I''' wherein $R_{1c}$ represents a hydroxy or cyano group or a chlorine or bromine atom and/or Z represents a chlorine atom or a cyano group, whilst a compound of general formula I''' optionally obtained wherein $R_{1c}$ represents the hydroxy group may be converted by alkylation into a corresponding compound of general formula I''' wherein $R_{1c}$ represents an alkoxy group with 1 to 4 carbon atoms, and/or

a compound of general formula I''' obtained wherein Z represents a hydroxymethyl group is converted by oxidation into a corresponding compound of general formula I''' wherein Z represents the formyl group, and/or

a compound of general formula I''' obtained wherein Z represents a hydroxymethyl group is converted by halogenation and subsequent reaction with a malonic acid ester into a corresponding compound of general formula I''' wherein Z represents an ethyl group substituted by two alkoxycarbonyl groups, and/or

a compound of general formula I''' obtained wherein Z represents a formyl group is converted by acetalisation into a corresponding compound of general formula I''' wherein Z represents dialkoxymethyl group, and/or

a compound of general formula I''' obtained wherein Z represents a formyl group is converted, by condensation and optional subsequent hydrolysis, into a corresponding compound of general formula I''' wherein Z represents an ethylene group substituted by a hydroxycarbonyl or alkoxycarbonyl group, and/or

a compound of general formula I''' obtained wherein Z represents a hydrogen atom is converted by Friedel-Crafts acylation into a corresponding compound of general formula I''' wherein Z represents an acetyl group optionally substituted by an alkoxycarbonyl group, and/or

a compound of general formula I''' obtained wherein Z represents a nitrile group is converted by alcoholysis via a corresponding imino ester into a corresponding compound of general formula I''' wherein Z represents a trialkoxymethyl group, and/or

a compound of general formula I''' obtained wherein Z represents a trialkoxymethyl group is converted by hydrolysis into a corresponding compound of general formula I''' wherein Z represents an alkoxycarbonyl group, and/or

a compound of general formula I''' obtained wherein Z represents an acetyl group is converted by heating with an amine and sulphur and subsequently in the presence of an inorganic base into a corresponding compound of general formula I''' wherein Z represents a hydroxycarbonylmethyl group, and/or

a compound of general formula I''' wherein Z represents a carboxy group is converted, by conversion into a sulphonic acid hydracide and subsequent disproportionation, into a corresponding compound of general formula I''' wherein Z represents a formyl group, and/or

a compound of general formula I''' obtained is converted into the physiologically acceptable salts thereof with inorganic or organic acids and also bases if Z contains a carboxyl group.

5. Process as claimed in claims 1 to 4, characterised in that the reaction is carried out in a solvent.

6. Process as claimed in claims 1a, 2a, 3a, 4a and 5, characterised in that the reaction is carried out in the presence of an acid-activating agent, a dehydrating agent or an amine-activating agent, optionally in the presence of an inorganic or tertiary organic base and at temperatures of between − 25 and 250 °C, preferably at temperatures of between − 10 °C and the boiling temperature of the solvent used.

7. Process as claimed in claim 1b, 2b, 3b and 5, characterised in that the reaction is carried out in the presence of an inorganic or tertiary organic base, in the presence of a reaction accelerator such as copper and/or in a pressure vessel and at temperatures of between 20 and 150 °C, preferably at the boiling temperature of the reaction mixture.

8. Process as claimed in claims 1c, 2c, 3c, 4c and 5, characterised in that the reaction is carried out at temperatures of between 0 and 100 °C, but preferably at temperatures of between 20 and 50 °C.

9. Process as claimed in claims 1d, 2d, 3d, 4d and 5, characterised in that the hydrolysis is carried out in the presence of an acid or base and at the boiling temperature of the reaction mixture.

10. Process as claimed in claims 1e, 2e, 3e, 4e, and 5, characterised in that the methylation is carried out with formaldehyde in the presence of a reducing agent.

**Revendications** (pour les états contractants ; BE, CH, DE, FR, GB, IT, LI, LU, NL, SE.)

1. Carboxamides de formule générale I

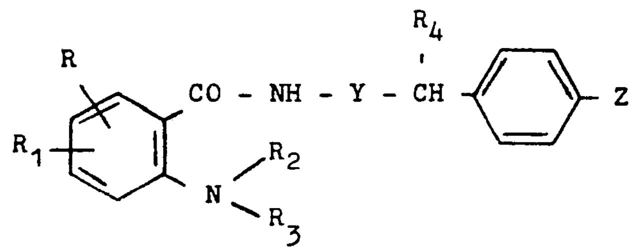

(I)

dans laquelle

R représente un atome d'hydrogène, de chlore ou de brome, ou un groupe alcoylèneimino cyclique avec 4 à 7 atomes de carbone dans le cycle imino,

$R_1$ représente un atome d'hydrogène, de fluor, de chlore ou de brome, un groupe alcoyle ou alcoxy avec à chaque fois 1 à 6 atomes de carbone, un groupe alcoxy avec 1 à 3 atomes de carbone substitué par un groupe phényle, un groupe hydroxy, nitro, amino, cyano ou carboxy, un groupe alcanoylamino, alcoxycarbonyle ou dialcoylamidosulfonyle, la partie alcoyle pouvant à chaque fois contenir 1 à 3 atomes de carbone,

$R_2$ et $R_3$, qui peuvent être identiques ou différents, représentent des groupes alcoyle avec 1 à 7 atomes de carbone, des groupes alcènyle avec 3 à 7 atomes de carbone, des groupes cycloalcoyle avec 3 à 7 atomes de carbone, des groupes alcoyle avec 1 à 3 atomes de carbone substitués par un groupe phényle, des groupes phényle ou adamantyle ou

$R_2$ et $R_3$ représentent ensemble avec l'atome d'azote intermédiaire un groupe alcoylèneimino non ramifié avec 3 à 12 atomes de carbone dans le cycle imino, un groupe alcoylèneimino non ramifié avec 4 à 6 atomes de carbone dans le cycle imino qui est substitué par un ou deux groupes alcoyle avec à chaque fois 1 à 4 atomes de carbone, un groupe alcoxy avec 1 à 3 atomes de carbone, un groupe hydroxy, phényle, carboxy ou alcoxycarbonyle avec en tout 2 à 4 atomes de carbone et/ou dans lequel un groupe méthylène est remplacé par un groupe imino, lequel peut être substitué par un groupe alcoyle avec 1 à 3 atomes de carbone, un groupe alcoxycarbonyle avec en tout 2 à 4 atomes de carbone, un groupe phényle, halogénophényle, benzyle, pyridyle ou furoyle, ou est remplacé par un atome d'oxygène ou de soufre, par un groupe carbonyle, sulfoxyde ou sulfonyle, ou représentent ensemble un groupe azabicycloalcoyle saturé ou partiellement non saturé avec 7 à 10 atomes de carbone, un groupe pipéridino substitué en positions 3 et 5 par en tout 3 ou 4 groupes alcoyle avec chacun 1 à 3 atomes de carbone, ou représentent un groupe 1,4-dioxa-8-aza-spiro-alcoyle avec 6 à 9 atomes de carbone, un groupe pyrrolyle ou tétra-hydropyridino,

$R_4$ représente un atome d'hydrogène ou un groupe alcoyle avec 1 à 3 atomes de carbone,

Y représente un atome d'oxygène, un groupe imino ou un groupe méthylène éventuellement substitué par un ou deux groupes alcoyle avec chacun 1 à 3 atomes de carbone, et

Z représente un atome d'hydrogène ou d'halogène, un groupe carboxy, cyano, formyle, hydroxyméthyle, hydroxycarbonyléthylène, nitro, amino, allyloxycarbonyle, phénoxycarbonyle, benzyloxycarbonyle ou phényléthoxycarbonyle, un groupe alcoxycarbonyle avec en tout 2 à 8 atomes de carbone, un groupe cycloalcoxycarbonyle avec en tout 4 à 8 atomes de carbone, un groupe méthyle éventuellement substitué par deux ou trois groupes alcoxy, par un groupe carboxy, alcoxycarbonyle ou éthylènedioxy, le groupe alcoxy pouvant dans chaque cas contenir 1 à 3 atomes de carbone, un groupe acétyle éventuellement substitué par un groupe carboxy ou alcoxycarbonyle avec en tout 2 à 4 atomes de carbone, un groupe éthyle ou éthylène substitué par un ou deux groupes alcoxycarbonyle avec en tout à chaque fois 2 à 4 atomes de carbone, ou par deux groupes carboxy, un groupe aminocarbonyle éventuellement mono- ou di-substitué par un groupe alcoyle avec 1 à 7 atomes de carbone, un groupe cycloalcoyle avec 3 à 7 atomes de carbone et/ou alcényle avec 3 à 7 atomes de carbone, ou représente un groupe pipéridinocarbonyle, morpholinocarbonyle, thiomorpholinocarbonyle ou N-alcoyl-pipérazinocarbonyle, le groupe alcoyle pouvant contenir 1 à 3 atomes de carbone, ou également un groupe éthyle substitué par un groupe carboxy lorsque les radicaux $R_2$ et $R_3$ représentent ensemble avec l'atome d'azote intermédiaire l'un des radicaux imino cycliques mentionnés au début, et leurs sels physiologiquement supportables avec des acides minéraux ou organiques et également des bases, lorsque Z représente ou contient un groupe carboxy.

2. Carboxamides de formule générale I selon la revendication 1, dans laquelle

R représente un atome d'hydrogène,

$R_1$ représente un atome d'hydrogène, de fluor, de chlore ou de brome, un groupe alcoxy avec 1 à 6 atomes de carbone, un groupe alcoyle avec 1 à 4 atomes de carbone, un groupe alcoxycarbonyle avec en tout 2 à 4 atomes de carbone, un groupe hydroxy, cyano, carboxy, nitro, amino, acétamido, diméthylaminosulfonyle ou benzyloxy,

$R_2$ représente un groupe alcoyle avec 1 à 6 atomes de carbone, un groupe cyclohexyle, phényle, benzyle, adamantyle ou allyle,

$R_3$ représente un groupe alcoyle avec 1 à 6 atomes de carbone ou un groupe allyle ou

$R_2$ et $R_3$ représentent ensemble avec l'atome d'azote intermédiaire un groupe alcoylèneimino non ramifié avec 4 à 12 atomes de carbone dans le cycle imino, un groupe pipéridino substitué par un groupe alcoyle avec 1 à 4 atomes de carbone, un groupe phényle, hydroxy, méthoxy, carboxy ou alcoxycarbonyle avec en tout 2 à 4 atomes de carbone, ou représentent un groupe pipéridino substitué en positions 3 et 5 à chaque fois par un groupe alcoyle avec 1 à 3 atomes de carbone, ou représentent un groupe pipéridino substitué en positions 3 et 5 à chaque fois par deux groupes alcoyle avec chacun 1 à 3 atomes de carbone, ou représentent un groupe morpholino thiomorpholino, 1-oxydo-thiomorpholino ou 1,1-dioxydo-thiomorpholino, éventuellement substitué par un ou deux groupes méthyle, ou représentent un groupe pipéridino éventuellement substitué en position 4 par un groupe méthyle, benzyle, phényle, chlorophényle, pyridyle, furoyle ou alcoxycarbonyle avec en tout 2 à 4 atomes de carbone, ou représentent un groupe pyrrolyle, pipéridone-(2)-yle-(1), 1,2,3,6-tétrahydropyridino, 1,4-dioxa-8-aza-spiro-[4,5] décane-8-yle, 1,4-dioxa-8-aza-spiro [4,6] undécane-8-yle, octahydro-isoindol-2-yle, 1,2,3,4-tétrahydro-isoquinoléine-2-yle, 1,2,3,4,5,6,7,8-octahydro-isoquinoléine-2-yle, décahydro-isoquinoléine-2-yle, 1,2,4,5-tétrahydro-3-benzazépine-3-yle, décahydro-3-benzazépine-3-yle ou 3-aza-bicyclo-nonane-3-yle,

$R_4$ représente un atome d'hydrogène ou un groupe méthyle,

Y représente un groupe méthylène, méthyl-méthylène ou diméthylméthylène, un groupe NH ou un atome d'oxygène et

Z représente un groupe carboxy, cyano, formyle ou hydroxyméthyle, un groupe alcoxycarbonyle avec en tout 2 à 7 atomes de carbone, un groupe cyclohexyloxycarbonyle, benzyloxycarbonyle, diéthoxyméthyle, hydroxycarbonylméthyle, bis-2,2-éthoxycarbonyl-éthyle, 2-hydroxy-carbonyl-éthylène ou 2-éthoxycarbonyl-éthyle, un groupe acétyle éventuellement substitué par un groupe hydroxycarbonyle ou éthoxycarbonyle ou également un groupe 2-hydroxycarbonyl-éthyle, lorsque les radicaux $R_2$ et $R_3$ représentent ensemble avec l'atome d'azote intermédiaire l'un des radicaux imino cycliques mentionnés au début, et leurs sels physiologiquement supportables avec des acides minéraux ou organiques et aussi des bases, lorsque Z représente ou contient un groupe carboxy.

3. Carboxamides de formule générale I selon la revendication 1, dans laquelle

$R_1$ se trouve en position 5 du noyau phényle et

R, $R_1$ à $R_4$, Y et Z sont définis comme à la revendication 2, et leurs sels physiologiquement supportables avec des acides minéraux ou organiques et également des bases, lorsque Z représente ou contient un groupe carboxy.

4. Carboxamides de formule générale I selon la revendication 1, dans laquelle

R représente un atome d'hydrogène,

$R_1$, en position 5, représente un atome d'hydrogène, de fluor, de chlore ou de brome, un groupe alcoyle ou alcoxy avec à chaque fois 1 à 3 atomes de carbone, un groupe carboxy, cyano ou nitro,

$R_2$ et $R_3$ représentent ensemble avec l'atome d'azote intermédiaire un groupe N,N-dialcoylamino ou N-alcoyl-cyclohexylamino avec à chaque fois 1 à 3 atomes de carbone dans la partie alcoyle, un groupe pyrrolidino, pipéridino, hexaméthylèneimino, heptaméthylèneimino, octaméthylèneimino ou nonaméthylèneimino, un groupe pipéridino substitué par un groupe alcoyle avec 1 à 4 atomes de carbone, par un groupe méthoxy ou phényle, ou représentent un groupe pipéridino substitué en positions 3 et 5 à chaque fois par un ou deux groupes méthyle ou éthyle, ou représentent un groupe morpholino ou thiomorpholino éventuellement substitué en positions 2 et 6 à chaque fois par un groupe méthyle, ou représentent un groupe 1,4-dioxo-8-aza-spiro [4,5] décane-8-yle, 1,4-dioxa-8-aza-spiro [4,6] undécane-yle, octahydro-isoindol-2-yle, 1,2,3,4-tétrahydro-isoquinoléine-2-yle, 1,2,3,4,5,6,7,8-octahydro-isoquinoléine-2-yle, décahydro-isoquinoléine-2-yle, 1,2,4,5-tétrahydro-3H-3-benzazépine-3-yle, décahydro-3H-3-benzazépine-3-yle, 3-aza-bicyclo [3,2,2]-nonane-3-yle ou N-méthyl-adamantyl-(1)-amino,

$R_4$ représente un atome d'hydrogène ou un groupe méthyle,

Y représente un groupe méthylène, méthyl-méthylène, diméthyl-méthylène ou NH ou un atome d'oxygène, et

Z représente un groupe alcoxycarbonyle avec en tout 2 à 4 atomes de carbone, un groupe carboxy, formyle ou hydroxyméthyle, et leurs sels physiologiquement supportables avec des acides minéraux ou organiques et également des bases, lorsque Z représente le groupe carboxy.

5. Carboxamides de formule générale I selon la revendication 3, dans laquelle

R, $R_4$, Y et Z sont définis comme dans la revendication 3,

$R_1$ représente un atome de chlore ou de brome, un groupe méthyle, éthyle ou méthoxy,

$R_2$ et $R_3$ représentent ensemble avec l'atome d'azote intermédiaire un groupe pyrrolidino, pipéridino, méthyl-pipéridino, 4-méthoxy-pipéridino, 4-phényl-pipéridino, hexaméthylèneimino, heptaméthylèneimino, octaméthylèneimino, nonaméthylèneimino, 3,5-diméthyl-pipéridino, morpholino, 2,6-diméthyl-

morpholino, thiomorpholino, 2,6-diméthylthiomorpholino, 1,4-dioxa-8-aza-spiro [4,5] décane-8-yle, 1,2,3,4-tétrahydro-isoquinoléine-2-yle, décahydro-isoquinoléine-2-yle, 1,2,4,5-tétrahydro-3H-3-benzazépine-3-yle, décahydro-3H-3-benzazépine-3-yle, octahydro-isoindol-2-yle ou N-méthyl-adamantyl-(1)-amino ou un groupe pipéridino substitué en position 4 par un groupe alcoyle avec 2 à 4 atomes de carbone, et leurs sels physiologiquement supportables avec des acides minéraux ou organiques et également des bases, lorsque Z représente le groupe carboxy.

6. L'acide 4-[2-(5-chloro-2-pipéridino-benzoylamino)-éthyl]-benzoïque, ses esters d'alcoyle avec 1 à 3 atomes de carbone et ses sels d'addition d'acides.

7. L'acide 4-[2-(5-chloro-2-nonaméthylèneimino-benzoylamino)-éthyl]-benzoïque, ses esters d'alcoyle avec 1 à 3 atomes de carbone et ses sels d'addition d'acides.

8. L'acide 4-[2-(5-chloro-2-(1,4-dioxa-8-aza-spiro [4,5] décane-8-yl)-benzoylamino)-éthyl]-benzoïque, ses esters d'alcoyle avec 1 à 3 atomes de carbone et ses sels d'addition d'acides.

9. L'acide 4-[2-(5-chloro-2-octaméthylèneimino-benzoylamino)-éthyl]-benzoïque, ses esters d'alcoyle avec 1 à 3 atomes de carbone et ses sels d'addition d'acides.

10. L'acide 4-[2-(5-bromo-octaméthylèneimino-benzoylamino)-éthyl]-benzoïque, ses esters d'alcoyle avec 1 à 3 atomes de carbone et ses sels d'addition d'acides.

11. L'acide 4-[2-(5-chloro-2-(cis-3,5-diméthylpipéridino)-benzoylamino)-éthyl]-benzoïque, ses esters d'alcoyle avec 1 à 3 atomes de carbone et ses sels d'addition d'acides.

12. Médicament contenant un composé selon les revendications 1-11 conjointement à un ou plusieurs excipients et/ou diluants inertes.

13. Médicament selon la revendication 12 pour le traitement du diabète sucré.

14. Procédé de préparation de nouveaux carboxamides de formule générale I selon les revendications 1 à 11, ainsi que de leurs sels physiologiquement supportables avec des acides minéraux ou organiques ou également des bases, lorsque Z représente ou contient un groupe carboxy, caractérisé en ce que :

a) on fait réagir un acide aminobenzoïque de formule générale II

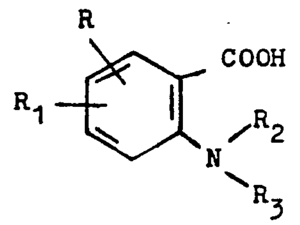

(II)

dans laquelle R, $R_1$, $R_2$ et $R_3$ sont définis comme au début, ou son dérivé réactif éventuellement préparé dans le mélange réactionnel avec une amine de formule générale III

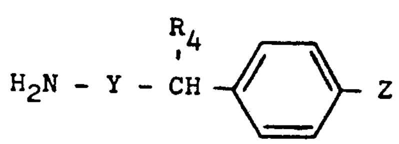

(III)

dans laquelle $R_4$, Y et Z sont définis comme au début, ou avec une amine N-activée de formule générale III éventuellement formée dans le mélange réactionnel lorsqu'on utilise un acide aminobenzoïque de formule générale II et lorsque Z dans une amine N-activée de formule générale III ne contient pas de groupe carboxy ou amino, ou

b) pour la préparation de composés de formule générale I dans laquelle $R_1$ est défini comme au début à l'exception du groupe hydroxy et du groupe amino et Y représente un groupe méthylène éventuellement substitué par un ou deux groupes alcoyle avec chacun 1 à 3 atomes de carbone, on fait réagir un composé de formule générale IV

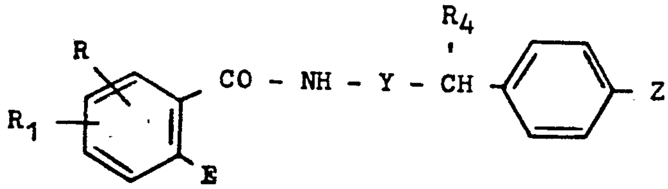

(IV)

dans laquelle

R, $R_4$ et Z sont définis comme au début,

$R_1$ et Y possèdent les significations indiquées plus haut et

E représente un atome d'halogène, avec une amine de formule générale V

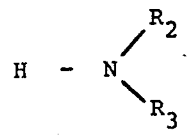

(V)

dans laquelle $R_2$ et $R_3$ sont définis comme au début, ou

c) pour la préparation de composés de formule générale I dans laquelle Z représente un groupe carboxy et Y ne représente pas le groupe NH, on oxyde un composé de formule générale VI

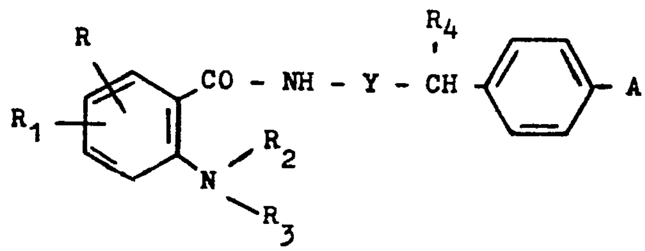

(VI)

dans laquelle

R et $R_1$ à $R_4$ sont définis comme au début,

Y est défini comme au début à l'exception du groupe NH et

A représente un groupe transformable par oxydation en un groupe carboxy, ou

d) pour la préparation de composés de formule générale I dans laquelle Z représente un groupe carboxy, on hydrolyse un composé de formule générale VII

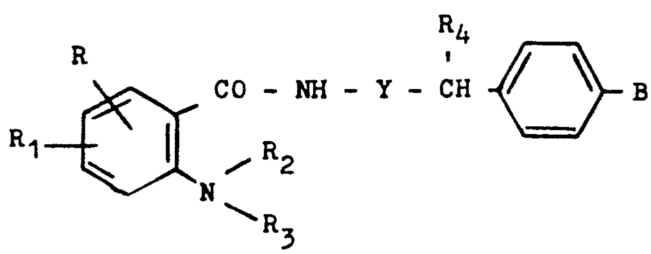

(VII)

dans laquelle R, $R_1$ à $R_4$ et Y sont définis comme au début et B représente un groupe transformable en un groupe carboxy par hydrolyse, ou

e) pour la préparation de composés de formule générale I dans laquelle $R_2$ représente un groupe alcoyle avec 1 à 7 atomes de carbone, un groupe cycloalcoyle avec 3 à 7 atomes de carbone, un groupe alcoyle avec 1 à 3 atomes de carbone substitué un groupe phényle ou un groupe alcényle avec 3 à 7 atomes de carbone, $R_3$ représente un groupe alcoyle avec 1 à 7 atomes de carbone, un groupe cycloalcoyle avec 3 à 7 atomes de carbone, un groupe alcényle avec 3 à 7 atomes de carbone, un groupe alcoyle avec 1 à 3 atomes de carbone substitué par un groupe phényle ou un groupe adamantyle ou $R_2$ et $R_3$ représentent avec l'atome d'azote intermédiaire un cycle alcoylèneimino à 5 à 7 membres, un groupe pipéridino substitué par un groupe alcoyle avec 1 à 4 atomes de carbone ou un groupe pipéridino substitué en position 3 et 5 à chaque fois par un groupe alcoyle avec 1 à 3 atomes de carbone et Y représente un groupe méthylène éventuellement substitué par un ou deux groupes alcoyle avec chacun 1 à 3 atomes de carbone, on fait réagir un composé éventuellement formé dans le mélange réactionnel, de formule générale VIII

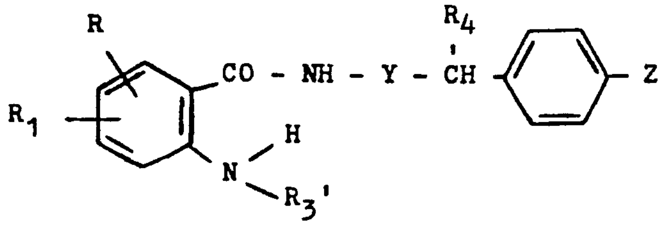

(VIII)

dans laquelle

R, $R_1$, $R_4$ et Z sont définis comme au début,

$R_3'$ représente un atome d'hydrogène ou possède les significations mentionnées plus haut pour $R_3$ et

Y possède les significations mentionnées plus haut, avec un composé de formule générale IX

$R_2'$—G (IX)

dans laquelle

$R_2'$ possède les significations mentionnées au début pour $R_2$ ou représente ensemble avec le radical $R_3'$ de la formule VIII un groupe alcoylène rectiligne avec 4 à 6 atomes de carbone, un groupe n-pentylène substitué par un groupe alcoyle avec 1 à 4 atomes de carbone, ou un groupe n-pentylène substitué en position 2 et 4 à chaque fois par un groupe alcoyle avec 1 à 3 atomes de carbone et

G représente un groupe nucléophile partant tel qu'un atome d'halogène ou un groupe sulfonyloxy, ou

f) pour la préparation de composés de formule générale I dans laquelle Y représente le groupe NH ou un atome d'oxygène, on fait réagir un composé de formule générale X

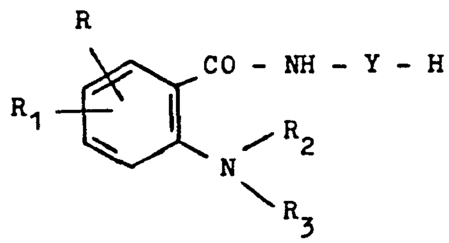

(X)

dans laquelle

R et $R_1$ à $R_3$ sont définis comme au début et

Y possède les significations mentionnées plus haut, ou son sel alcalin, avec un dérivé phénylé de formule générale XI

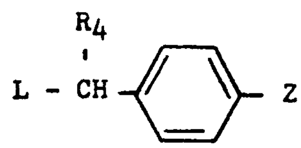

(XI)

dans laquelle

$R_4$ et Z sont définis comme au début et

L représente un groupe partant nucléophile tel qu'un atome d'halogène ou un groupe sulfonyloxy, ou

g) pour la préparation de composés de formule générale I dans laquelle $R_1$ est défini comme au début à l'exception du groupe hydroxy, carboxy, amino ou alcanoylamino et Y représente un groupe méthylène éventuellement substitué par un ou deux groupes alcoyle avec chacun 1 à 3 atomes de carbone, on fait réagir un amide de formule générale XII

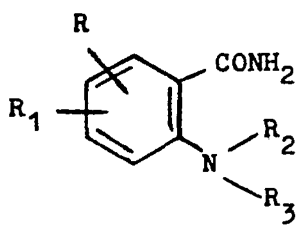

(XII)

dans laquelle

R, $R_2$ et $R_3$ sont définis comme au début et

$R_1$ possède les significations mentionnées plus haut, ou son sel alcalin, avec un composé de formule générale XIII

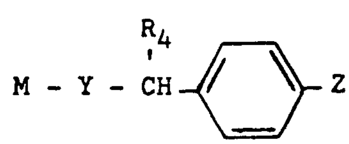

(XIII)

dans laquelle

$R_4$ et Z sont définis comme au début,

Y possède les significations mentionnées plus haut et

M représente un groupe partant nucléophile tel qu'un atome d'halogène ou un groupe sulfonyloxy, ou

h) pour la préparation de composés de formule générale I dans laquelle $R_1$ représente un atome d'hydrogène, de fluor, de chlore ou de brome, un groupe alcoyle ou alcoxy avec chacun 1 à 6 atomes de carbone, un groupe alcoxy avec 1 à 3 atomes de carbone substitué par un groupe phényle, un groupe hydroxy, nitro, carboxy ou alcanoylamino, Y représente un groupe méthylène éventuellement substitué par un ou deux groupes alcoyle avec chacun 1 à 3 atomes de carbone et Z représente un groupe carboxy, on acyle un composé de formule générale XIV

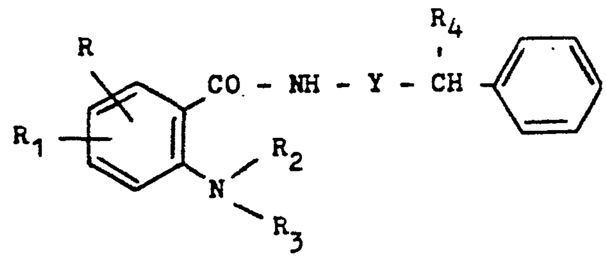

(XIV)

dans laquelle

R et $R_2$ à $R_4$ sont définis comme au début,

$R_1$ et Y possèdent les significations mentionnées plus haut, au moyen d'un halogénure d'oxalyle ou de phosgène en présence d'un acide de Lewis ou

i) pour la préparation de composés de formule générale I, dans laquelle $R_1$ représente un atome d'hydrogène, de fluor, de chlore ou de brome, un groupe alcoyle ou alcoxy avec chacun 1 à 6 atomes de carbone, un groupe alcoxy avec 1 à 3 atomes de carbone substitué par un groupe phényle, un groupe nitro, carboxy, alcanoylamino ou alcoxycarbonyle avec chacun 1 à 3 atomes de carbone dans la partie alcoyle, Y représente un groupe méthylène éventuellement substitué par un ou deux groupes alcoyle avec chacun 1 à 3 atomes de carbone et Z représente un groupe carboxy, on fait réagir un composé de formule générale XV

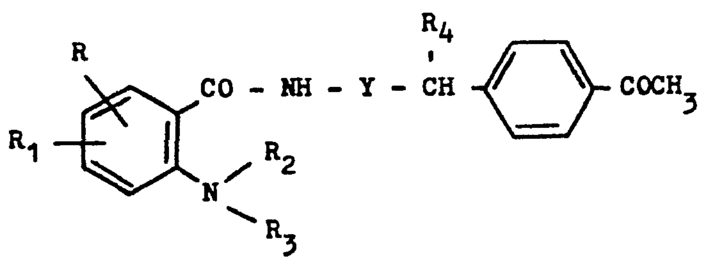

(XV)

dans laquelle

R et $R_2$ à $R_4$ sont définis comme au début,

$R_1$ et Y possèdent les significations mentionnées plus haut, avec un hypohalogénite éventuellement préparé dans le mélange réactionnel

et si on le désire, on transforme ensuite un composé ainsi obtenu de formule générale I dans laquelle Z représente un groupe carboxy, au moyen d'une estérification ou respectivement d'une amidation en un composé correspondant de formule générale I dans laquelle Z représente un groupe carboxy estéré ou un groupe aminocarbonyle éventuellement mono- ou disubstitué par un groupe alcoyle avec 1 à 7 atomes de carbone, un groupe cycloalcoyle avec 3 à 7 atomes de carbone et/ou alcényle avec 3 à 7 atomes de carbone, ou représente un groupe pipéridinocarbonyle, morpholinocarbonyle, thiomorpholinocarbonyle ou N-alcoylpipérazinocarbonyle et/ou

on transforme un composé obtenu de formule générale I dans laquelle $R_1$ et/ou Z représente un groupe nitro, au moyen d'une réduction en un composé correspondant de formule générale I dans laquelle $R_1$ et/ou Z représente un groupe amino et/ou

on transforme un composé obtenu de formule générale I dans laquelle $R_1$ et/ou Z représente un groupe amino, par l'intermédiaire d'un sel de diazonium correspondant en un composé correspondant de formule générale I, dans laquelle $R_1$ représente le groupe hydroxy ou cyano, un atome de fluor, de chlore ou de brome et/ou Z représente un atome de chlore ou de brome ou le groupe cyano, un composé éventuellement ainsi obtenu de formule générale I dans laquelle $R_1$ représente le groupe hydroxy pouvant être transformé au moyen d'une alcoylation en un composé correspondant de formule générale I dans laquelle $R_1$ représente un groupe alcoxy avec 1 à 6 atomes de carbone ou un groupe alcoxy avec 1 à 3 atomes de carbone substitué par un groupe phényle et/ou on transforme un composé obtenu de

formule générale I dans laquelle $R_1$ représente un groupe amino, au moyen d'une acylation en un composé correspondant de formule générale I dans laquelle $R_1$ représente un groupe alcanoylamino avec 1 à 3 atomes de carbone dans la partie alcanoyle, et/ou

on transforme un composé obtenu de formule générale I dans laquelle $R_1$ représente un groupe alcoxycarbonyle et/ou $R_2$ et $R_3$ représentent ensemble avec l'atome d'azote intermédiaire un groupe alcoylèneimino avec 4 à 6 atomes de carbone substitué par un groupe alcoxycarbonyle, la partie alcoxy pouvant dans chaque cas contenir 1 à 3 atomes de carbone, au moyen d'une hydrolyse en un composé correspondant de formule générale I dans laquelle $R_1$ représente le groupe carboxy et/ou $R_2$ et $R_3$ représentent ensemble avec l'atome d'azote intermédiaire un groupe alcoylèneimino avec 4 à 6 atomes de carbone dans le cycle imino, substitué par un groupe carboxy et/ou

on transforme un composé obtenu de formule générale I dans laquelle $R_2$ et $R_3$ représentent ensemble avec l'atome d'azote intermédiaire un groupe N-benzylpipérazino, au moyen d'une débenzylation en un composé correspondant de formule générale I dans laquelle $R_2$ et $R_3$ représentent ensemble avec l'atome d'azote intermédiaire un groupe pipérazino, et/ou

on transforme un composé obtenu de formule générale I dans laquelle $R_1$ représente un atome de chlore ou de brome au moyen d'une déshalogénation en un composé correspondant de formule générale I, dans laquelle $R_1$ représente un atome d'hydrogène et/ou

on transforme un carboxamide de formule générale I dans laquelle Z représente un groupe carboxy éventuellement estérifié, au moyen d'une réduction par un hydrure métallique en un composé correspondant de formule générale I dans laquelle Z représente le groupe hydroxyméthyle et/ou on transforme un composé obtenu de formule générale I dans laquelle Z représente le groupe hydroxyméthyle, au moyen d'une oxydation en un composé correspondant de formule générale I dans laquelle Z représente le groupe formyle et/ou

on transforme un composé obtenu de formule générale I dans laquelle Z représente le groupe hydroxyméthyle, au moyen d'une halogénation et par réaction subséquente avec un ester de l'acide malonique en un composé correspondant de formule générale I dans laquelle Z représente un groupe éthyle substitué par deux groupes alcoxycarbonyle et/ou on transforme un composé obtenu de formule générale I dans laquelle Z représente le groupe formyle au moyen d'une acétalisation en un composé correspondant de formule générale I dans laquelle Z représente un groupe dialcoxyméthyle, et/ou

on transforme un composé obtenu de formule générale I dans laquelle Z représente le groupe formyle, au moyen d'une condensation et éventuellement par hydrolyse subséquente en un composé correspondant de formule générale I dans laquelle Z représente un groupe éthylène substitué par un groupe hydroxycarbonyle ou alcoxycarbonyle et/ou

on transforme un composé obtenu de formule générale I dans laquelle Z représente un atome d'hydrogène, au moyen d'une acylation de Friedel-Crafts en un composé correspondant de formule générale I dans laquelle Z représente un groupe acétyle éventuellement substitué par un groupe alcoxycarbonyle et/ou

on transforme un composé obtenu de formule générale I dans laquelle Z représente un groupe nitrile, au moyen d'une alcoolyse, par l'intermédiaire d'un imino-ester correspondant, en un composé correspondant de formule générale I dans laquelle Z représente un groupe trialcoxyméthyle et/ou

on transforme un composé obtenu de formule générale I dans laquelle Z représente un groupe trialcoxyméthyle, au moyen d'une hydrolyse en un composé correspondant de formule générale I dans laquelle Z représente un groupe alcoxycarbonyle, et/ou

on transforme un composé obtenu de formule générale I dans laquelle Z représente le groupe acétyle, par chauffage avec une amine et du soufre et ensuite en présence d'une base minérale en un composé correspondant de formule générale I dans laquelle Z représente le groupe hydroxycarbonylméthyle, et/ou

on transforme un composé obtenu de formule générale I dans laquelle Z représente le groupe carboxy, par transformation en un hydrazide d'acide sulfonique et dismutation subséquente en un composé correspondant de formule générale I dans laquelle Z représente le groupe formyle et/ou

on transforme un composé obtenu de formule générale I dans laquelle $R_1$ représente un atome d'hydrogène, de fluor, de chlore ou de brome, un groupe alcoyle ou alcoxy avec à chaque fois 1 à 6 atomes de carbone ou un groupe alcoxy avec 1 à 3 atomes de carbone substitué par un groupe phényle, Y représente un groupe méthylène éventuellement substitué par un ou deux groupes alcoyle avec chacun 1 à 3 atomes de carbone et Z représente un atome de chlore ou de brome, par l'intermédiaire de son composé métallo-organique correspondant, au moyen de dioxyde de carbone en un composé correspondant de formule générale I dans laquelle Z représente le groupe carboxy, et/ou on transforme un composé obtenu de formule générale I en ses sels physiologiquement supportables avec des acides minéraux ou organiques et également des bases, lorsque Z contient un groupe carboxy.

**Revendications** (pour l'état contractant AT)

1. Procédé de préparation de nouveaux carboxamides de formule générale I

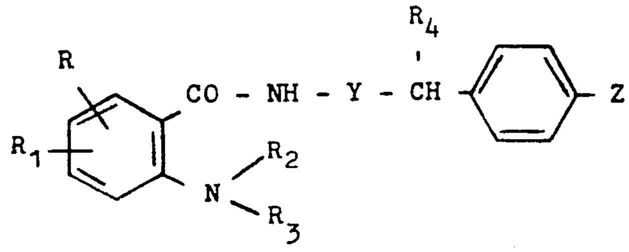

(I)

dans laquelle

R représente un atome d'hydrogène, de chlore ou de brome, ou un groupe alcoylèneimino cyclique avec 4 à 7 atomes de carbone dans le cycle imino,

$R_1$ représente un atome d'hydrogène, de fluor, de chlore ou de brome, un groupe alcoyle ou alcoxy avec à chaque fois 1 à 6 atomes de carbone, un groupe alcoxy avec 1 à 3 atomes de carbone substitué par un groupe phényle, ou représente un groupe hydroxy, nitro, amino, cyano ou carboxy, un groupe alcanoylamino, alcoxycarbonyle ou dialcoylamidosulfonyle, la partie alcoyle pouvant à chaque fois contenir 1 à 3 atomes de carbone,

$R_2$ et $R_3$, qui peuvent être identiques ou différents, représentent des groupes alcoyle avec 1 à 7 atomes de carbone, des groupes alcényle avec 3 à 7 atomes de carbone, des groupes cycloalcoyle avec 3 à 7 atomes de carbone, des groupes alcoyle avec 1 à 3 atomes de carbone substitués par un groupe phényle, des groupes phényle ou adamantyle ou

$R_2$ et $R_3$ représentent ensemble avec l'atome d'azote intermédiaire un groupe alcoylèneimino non ramifié avec 3 à 12 atomes de carbone dans le cycle imino, un groupe alcoylèneimino non ramifié avec 4 à 6 atomes de carbone dans le cycle imino qui est substitué par un ou deux groupes alcoyle avec à chaque fois 1 à 4 atomes de carbone, un groupe alcoxy avec 1 à 3 atomes de carbone, un groupe hydroxy, phényle, carboxy ou alcoxycarbonyle avec en tout 2 à 4 atomes de carbone et/ou dans lequel un groupe méthylène est remplacé par un groupe imino, lequel peut être substitué par un groupe alcoyle avec 1 à 3 atomes de carbone, un groupe alcoxycarbonyle avec en tout 2 à 4 atomes de carbone, un groupe phényle, halogénophényle, benzyle, pyridyle ou furoyle, ou est remplacé par un atome d'oxygène ou de soufre, par un groupe carbonyle, sulfoxyde ou sulfonyle, ou représentent ensemble un groupe azabicycloalcoyle saturé ou partiellement non saturé avec 7 à 10 atomes de carbone, un groupe pipéridino substitué en positions 3 et 5 par en tout 3 ou 4 groupes alcoyle avec chacun 1 à 3 atomes de carbone, ou représentent un groupe 1,4-dioxa-8-aza-spiro-alcoyle avec 6 à 9 atomes de carbone, un groupe pyrrolyle ou tétrahydropyridino,

$R_4$ représente un atome d'hydrogène ou un groupe alcoyle avec 1 à 3 atomes de carbone,

Y représente un atome d'oxygène, un groupe imino ou un groupe méthylène éventuellement substitué par un ou deux groupes alcoyle avec chacun 1 à 3 atomes de carbone, et

Z représente un atome d'hydrogène ou d'halogène, un groupe carboxy, cyano, formyle, hydroxyméthyle, hydroxycarbonyléthylène, nitro, amino, allyloxycarbonyle, phénoxycarbonyle, benzyloxycarbonyle ou phényléthoxycarbonyle, un groupe alcoxycarbonyle avec en tout 2 à 8 atomes de carbone, un groupe cycloalcoxycarbonyle avec en tout 4 à 8 atomes de carbone, un groupe méthyle éventuellement substitué par deux ou trois groupes alcoxy, par un groupe carboxy, alcoxycarbonyle ou éthylènedioxy, le groupe alcoxy pouvant dans chaque cas contenir 1 à 3 atomes de carbone, un groupe acétyle éventuellement substitué par un groupe carboxy ou alcoxycarbonyle avec en tout 2 à 4 atomes de carbone, un groupe éthyle ou éthylène substitué par un ou deux groupes alcoxycarbonyle avec en tout à chaque fois 2 à 4 atomes de carbone, ou par deux groupes carboxy, un groupe aminocarbonyle éventuellement mono- ou di-substitué par un groupe alcoyle avec 1 à 7 atomes de carbone, un groupe cycloalcoyle avec 3 à 7 atomes de carbone et/ou alcényle avec 3 à 7 atomes de carbone, ou représente un groupe pipéridinocarbonyle, morpholinocarbonyle, thiomorpholinocarbonyle ou N-alcoyl-pipérazinocarbonyle, le groupe alcoyle pouvant contenir 1 à 3 atomes de carbone, ou également un groupe éthyle substitué par un groupe carboxy, lorsque les radicaux $R_2$ et $R_3$ représentent ensemble avec l'atome d'azote intermédiaire l'un des radicaux imino cycliques mentionnés au début, et de leurs sels physiologiquement supportables avec des acides minéraux ou organiques et également des bases, lorsque Z représente ou contient un groupe carboxy, caractérisé en ce que

a) on fait réagir un acide aminobenzoïque de formule générale II

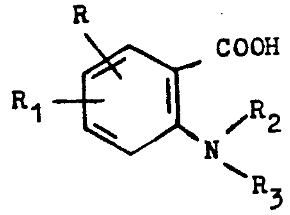

(II)

dans laquelle R, $R_1$, $R_2$ et $R_3$ sont définis comme au début, ou son dérivé réactif éventuellement préparé dans le mélange réactionnel, avec une amine de formule générale III

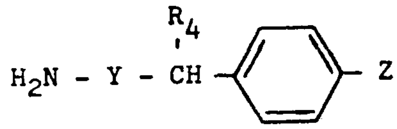

(III)

dans laquelle $R_4$, Y et Z sont définis comme au début, ou avec une amine N-activée de formule générale III éventuellement formée dans le mélange réactionnel, lorsqu'on utilise un acide aminobenzoïque de formule générale II et lorsque Z dans une amine N-activée de formule générale III ne contient pas de groupe carboxy ou amino, ou

b) pour la préparation de composés de formule générale I dans laquelle $R_1$ est défini comme au début à l'exception du groupe hydroxy et du groupe amino et Y représente un groupe méthylène éventuellement substitué par un ou deux groupes alcoyle avec chacun 1 à 3 atomes de carbone, on fait réagir un composé de formule générale IV

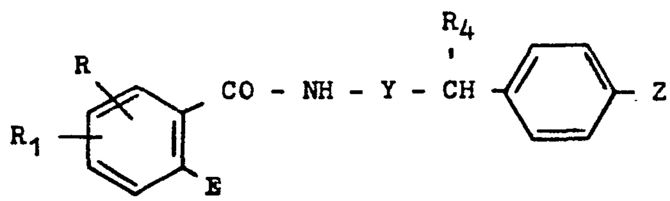

(IV)

dans laquelle

R, $R_4$ et Z sont définis comme au début,

$R_1$ et Y possèdent les significations indiquées plus haut et

E représente un atome d'halogène, avec une amine de formule générale V

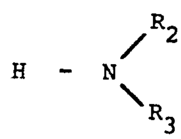

(V)

dans laquelle $R_2$ et $R_3$ sont définis comme au début, ou

c) pour la préparation de composés de formule générale I dans laquelle Z représente un groupe carboxy et Y ne représente pas un groupe NH, on oxyde un composé de formule générale VI

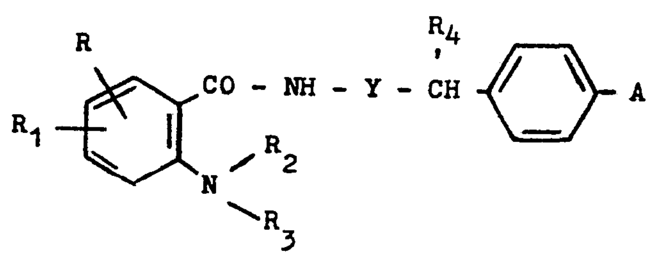

(VI)

dans laquelle

R et $R_1$ à $R_4$ sont définis comme au début,

Y est défini comme au début à l'exception du groupe NH et

A représente un groupe transformable par oxydation en un groupe carboxy, ou

d) pour la préparation de composés de formule générale I dans laquelle Z représente un groupe carboxy, on hydrolyse un composé de formule générale VII

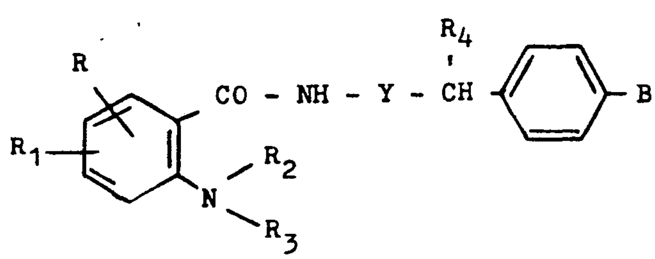

(VII)

dans laquelle R, $R_1$ à $R_4$ et Y sont définis comme au début et B représente un groupe transformable en un groupe carboxy par hydrolyse, ou

e) pour la préparation de composés de formule générale I dans laquelle $R_2$ représente un groupe alcoyle avec 1 à 7 atomes de carbone, un groupe cycloalcoyle avec 3 à 7 atomes de carbone, un groupe alcoyle avec 1 à 3 atomes de carbone substitué par un groupe phényle ou un groupe alcényle avec 3 à 7 atomes de carbone, $R_3$ représente un groupe alcoyle avec 1 à 7 atomes de carbone, un groupe cycloalcoyle avec 3 à 7 atomes de carbone, un groupe alcényle avec 3 à 7 atomes de carbone, un groupe alcoyle avec 1 à 3 atomes de carbone substitué par un groupe phényle ou un groupe adamantyle ou $R_2$ et $R_3$ représentent avec l'atome d'azote intermédiaire un cycle alcoylèneimino à 5 à 7 membres, un groupe pipéridino substitué par un groupe alcoyle avec 1 à 4 atomes de carbone ou un groupe pipéridino substitué en positions 3 et 5 à chaque fois par un groupe alcoyle avec 1 à 3 atomes de carbone et Y représente un groupe méthylène éventuellement substitué par un ou deux groupes alcoyle avec chacun 1 à 3 atomes de carbone, on fait réagir un composé éventuellement formé dans le mélange réactionnel, de formule générale VIII

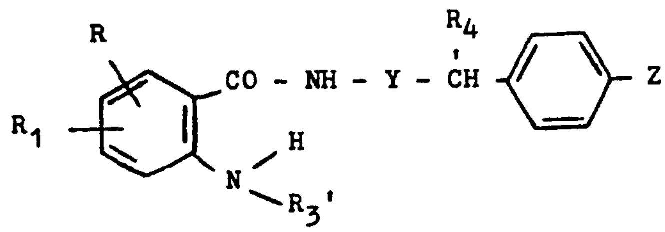

(VIII)

dans laquelle

R, $R_1$, $R_4$ et Z sont définis comme au début,

$R_3'$ représente un atome d'hydrogène ou possède les significations mentionnées plus haut pour $R_3$ et Y possède les significations mentionnées plus haut, avec un composé de formule générale IX

$$R_2'—G \quad (IX)$$

dans laquelle

$R_2'$ possède les significations mentionnées au début pour $R_2$ ou représente ensemble avec le radical $R_3'$ de la formule VIII un groupe alcoylène rectiligne avec 4 à 6 atomes de carbone, un groupe n-pentylène substitué par un groupe alcoyle avec 1 à 4 atomes de carbone, ou un groupe n-pentylène substitué en positions 2 et 4 à chaque fois par un groupe alcoyle avec 1 à 3 atomes de carbone et

G représente un groupe nucléophile partant tel qu'un atome d'halogène ou un groupe sulfonyloxy, ou

f) pour la préparation de composés de formule générale I dans laquelle Y représente le groupe NH ou un atome d'oxygène, on fait réagir un composé de formule générale X

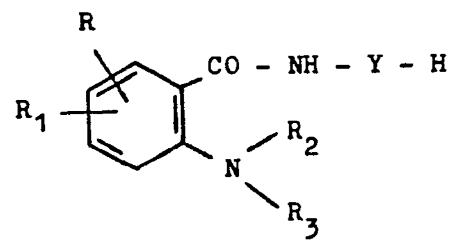

(X)

dans laquelle

R et $R_1$ et $R_3$ sont définis comme au début et

Y possède les significations mentionnées plus haut, ou son sel alcalin avec un dérivé phénylé de formule générale XI

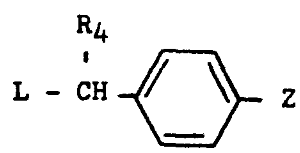

(XI)

dans laquelle

$R_4$ et Z sont définis comme au début et

L représente un groupe partant nucléophile tel qu'un atome d'halogène ou un groupe sulfonyloxy, ou

g) pour la préparation de composés de formule générale I dans laquelle $R_1$ est défini comme au début à l'exception du groupe hydroxy, carboxy, amino ou alcanoylamino et Y représente un groupe méthylène éventuellement substitué par un ou deux groupes alcoyle avec chacun 1 à 3 atomes de carbone, on fait réagir un amide de formule générale XII

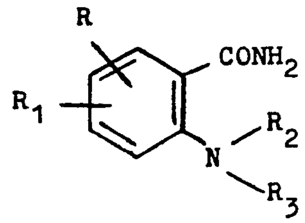

(XII)

dans laquelle

R, $R_2$ et $R_3$ sont définis comme au début et

$R_1$ possède les significations mentionnées plus haut, ou son sel alcalin avec un composé de formule générale XIII

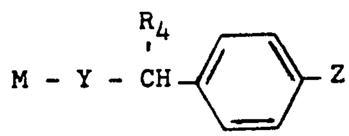

(XIII)

dans laquelle

$R_4$ et Z sont définis comme au début,

Y possède les significations mentionnées plus haut et

M représente un groupe partant nucléophile tel qu'un atome d'halogène ou un groupe sulfonyloxy, ou

h) pour la préparation de composés de formule générale I dans laquelle $R_1$ représente un atome d'hydrogène, de fluor, de chlore ou de brome, un groupe alcoyle ou alcoxy avec chacun 1 à 6 atomes de carbone, un groupe alcoxy avec 1 à 3 atomes de carbone substitué par un groupe phényle, un groupe hydroxy, nitro, carboxy ou alcanoylamino, Y représente un groupe méthylène éventuellement substitué par un ou deux groupes alcoyle avec chacun 1 à 3 atomes de carbone et Z représente un groupe carboxy, on acyle un composé de formule générale XIV

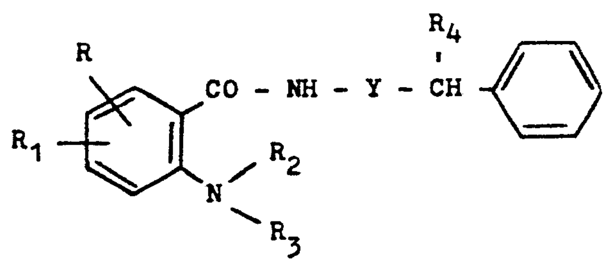

(XIV)

dans laquelle

R et $R_2$ à $R_4$ sont définis comme au début,

$R_1$ et Y possèdent les significations mentionnées plus haut, au moyen d'un halogénure d'oxalyle ou de phosgène en présence d'un acide de Lewis ou

i) pour la préparation de composés de formule générale I, dans laquelle $R_1$ représente un atome d'hydrogène, de fluor, de chlore ou de brome, un groupe alcoyle ou alcoxy avec chacun 1 à 6 atomes de carbone, un groupe alcoxy avec 1 à 3 atomes de carbone substitué par un groupe phényle, un groupe nitro, carboxy, alcanoylamino ou alcoxycarbonyle avec chacun 1 à 3 atomes de carbone dans la partie alcoyle, Y représente un groupe méthylène éventuellement substitué par un ou deux groupes alcoyle avec chacun 1 à 3 atomes de carbone et Z représente un groupe carboxy, on fait réagir un composé de formule générale XV

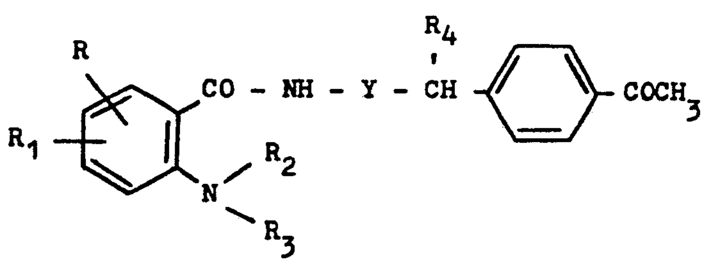

(XV)

dans laquelle

R et $R_2$ à $R_4$ sont définis comme au début,

$R_1$ et Y possèdent les significations mentionnées plus haut, avec un hypohalogénite éventuellement préparé dans le mélange réactionnel et si on le désire, on transforme ensuite un composé ainsi obtenu de formule générale I dans laquelle Z représente un groupe carboxy, au moyen d'une estérification ou respectivement d'une amidation en un composé correspondant de formule générale I dans laquelle Z représente un groupe carboxy estéré ou un groupe aminocarbonyle éventuellement mono- ou disubstitué par un groupe alcoyle avec 1 à 7 atomes de carbone, un groupe cycloalcoyle avec 3 à 7 atomes de carbone et/ou alcényle avec 3 à 7 atomes de carbone, ou représente un groupe pipéridinocarbonyle, morpholinocarbonyle, thiomorpholinocarbonyle ou N-alcoylpipérazinocarbonyle et/ou

on transforme un composé obtenu de formule générale I dans laquelle $R_1$ et/ou Z représente un groupe nitro, au moyen d'une réduction en un composé correspondant de formule générale I dans laquelle $R_1$ et/ou Z représente un groupe amino et/ou

on transforme un composé obtenu de formule générale I dans laquelle $R_1$ et/ou Z représente un groupe amino, par l'intermédiaire d'un sel de diazonium correspondant en un composé correspondant de formule générale I, dans laquelle $R_1$ représente le groupe hydroxy ou cyano, un atome de fluor, de chlore ou de brome et/ou Z représente un atome de chlore ou de brome ou le groupe cyano, un composé éventuellement ainsi obtenu de formule générale I dans laquelle $R_1$ représente le groupe hydroxy pouvant être transformé au moyen d'une alcoylation en un composé correspondant de formule générale I dans laquelle $R_1$ représente un groupe alcoxy avec 1 à 6 atomes de carbone ou un groupe alcoxy avec 1 à 3 atomes de carbone substitué par un groupe phényle et/ou

on transforme un composé obtenu de formule générale I dans laquelle $R_1$ représente un groupe amino, au moyen d'une acylation en un composé correspondant de formule générale I dans laquelle $R_1$ représente un groupe alcanoylamino avec 1 à 3 atomes de carbone dans la partie alcanoyle, et/ou

on transforme un composé obtenu de formule générale I dans laquelle $R_1$ représente un groupe alcoxycarbonyle et/ou $R_2$ et $R_3$ représentent ensemble avec l'atome d'azote intermédiaire un groupe alcoylèneimino avec 4 à 6 atomes de carbone substitué par un groupe alcoxycarbonyle, la partie alcoxy pouvant dans chaque cas contenir 1 à 3 atomes de carbone, au moyen d'une hydrolyse en un composé correspondant de formule générale I dans laquelle $R_1$ représente le groupe carboxy et/ou $R_2$ et $R_3$ représentent ensemble avec l'atome d'azote intermédiaire un groupe alcoylèneimino avec 4 à 6 atomes de carbone dans le cycle imino, substitué par un groupe carboxy et/ou

on transforme un composé obtenu de formule générale I dans laquelle $R_2$ et $R_3$ représentent ensemble avec l'atome d'azote intermédiaire un groupe N-benzylpipérazino, au moyen d'une débenzylation en un composé correspondant de formule générale I dans laquelle $R_2$ et $R_3$ représentent ensemble avec l'atome d'azote intermédiaire un groupe pipérazino, et/ou

on transforme un composé obtenu de formule générale I dans laquelle $R_1$ représente un atome de chlore ou de brome, au moyen d'une déshalogénation en un composé correspondant de formule générale I, dans laquelle $R_1$ représente un atome d'hydrogène et/ou

on transforme un carboxamide de formule générale I dans laquelle Z représente un groupe carboxy éventuellement estérifié, au moyen d'une réduction par un hydrure métallique en un composé correspondant de formule générale I dans laquelle Z représente le groupe hydroxyméthyle et/ou on transforme un composé obtenu de formule générale I dans laquelle Z représente le groupe hydroxyméthyle, au moyen d'une oxydation en un composé correspondant de formule générale I dans laquelle Z représente le groupe formyle et/ou

on transforme un composé obtenu de formule générale I dans laquelle Z représente le groupe hydroxyméthyle, au moyen d'une halogénation et par réaction subséquente avec un ester de l'acide malonique en un composé correspondant de formule générale I dans laquelle Z représente un groupe éthyle substitué par deux groupes alcoxycarbonyle et/ou on transforme un composé obtenu de formule générale I dans laquelle Z représente le groupe formyle, au moyen d'une acétalisation en un composé correspondant de formule générale I dans laquelle Z représente un groupe dialcoxyméthyle, et/ou

on transforme un composé obtenu de formule générale I dans laquelle Z représente le groupe formyle, au moyen d'une condensation et éventuellement par hydrolyse subséquente en un composé correspondant de formule générale I dans laquelle Z représente un groupe éthylène substitué par un groupe hydroxycarbonyle ou alcoxycarbonyle et/ou

on transforme un composé obtenu de formule générale I dans laquelle Z représente un atome d'hydrogène, au moyen d'une acylation de Friedel-Crafts en un composé correspondant de formule générale I dans laquelle Z représente un groupe acétyle éventuellement substitué par un groupe alcoxycarbonyle et/ou

on transforme un composé obtenu de formule générale I dans laquelle Z représente un groupe nitrile, au moyen d'une alcoolyse par l'intermédiaire d'un imino-ester correspondant en un composé correspondant de formule générale I dans laquelle Z représente un groupe trialcoxyméthyle et/ou

on transforme un composé obtenu de formule générale I dans laquelle Z représente un groupe trialcoxyméthyle, au moyen d'une hydrolyse en un composé correspondant de formule générale I dans laquelle Z représente un groupe alcoxycarbonyle, et/ou

on transforme un composé obtenu de formule générale I dans laquelle Z représente le groupe acétyle, par chauffage avec une amine et du soufre et ensuite en présence d'une base minérale en un composé correspondant de formule générale I dans laquelle Z représente le groupe hydroxycarbonylméthyle, et/ou

on transforme un composé obtenu de formule générale I dans laquelle Z représente le groupe carboxy, par transformation en un hydrazide d'acide sulfonique et dismutation subséquente en un composé correspondant de formule générale I dans laquelle Z représente le groupe formyle et/ou on transforme un composé obtenu de formule générale I dans laquelle $R_1$ représente un atome d'hydrogène, de fluor, de chlore ou de brome, un groupe alcoyle ou alcoxy avec à chaque fois 1 à 6 atomes de carbone ou un groupe alcoxy avec 1 à 3 atomes de carbone substitué par un groupe phényle, Y représente un groupe méthylène éventuellement substitué par un ou deux groupes alcoyle avec chacun 1 à 3 atomes de carbone et Z représente un atome de chlore ou de brome, par l'intermédiaire de son composé métallo-organique correspondant au moyen de dioxyde de carbone en un composé correspondant de formule générale I dans laquelle Z représente le groupe carboxy, et/ou on transforme un composé obtenu de formule générale I en ses sels physiologiquement supportables avec des acides minéraux ou organiques et également des bases, lorsque Z contient un groupe carboxy.

2. Procédé selon la revendication 1 pour la préparation de nouveaux carboxamides de formule générale I'

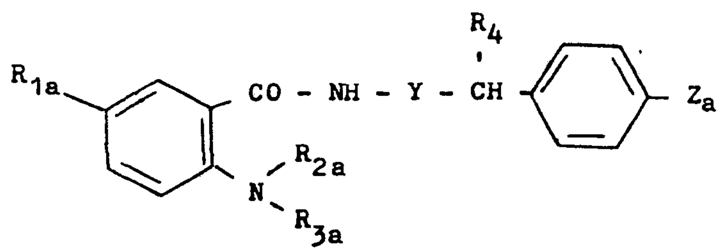

(I')

dans laquelle

$R_4$ est défini comme dans la revendication 1,

$R_{1a}$ représente un atome d'hydrogène, de fluor, de chlore ou de brome, un groupe nitro, amino, alcanoylamino, alcoxy, cyano, carboxy, alcoxycarbonyle ou dialcoylamidosulfonyle, la partie alcoyle pouvant dans chaque cas contenir 1 à 3 atomes de carbone,

$R_{2a}$ et $R_{3a}$ qui peuvent être identiques ou différents, représentent des groupes alcoyle avec 1 à 7 atomes de carbone ou cycloalcoyle avec 3 à 7 atomes de carbone ou

$R_{2a}$ et $R_{3a}$ représentent ensemble avec l'atome d'azote intermédiaire un groupe imino avec 4 à 6 atomes de carbone dans le cycle imino qui peut être substitué par un ou deux groupes alcoyle avec chacun 1 à 3 atomes de carbone, un groupe alcoxy avec 1 à 3 atomes de carbone, un groupe hydroxy, phényle, carboxy ou alcoxycarbonyle avec en tout 2 à 4 atomes de carbone, ou dans lequel un groupe méthylène du cycle imino peut être remplacé par un groupe cétal cyclique avec en tout 3 ou 4 atomes de carbone, par un atome d'oxygène ou de soufre, par un groupe sulfoxyde ou par un groupe imino qui est substitué par un groupe phényle ou pyridyle, ou représentent ensemble un groupe triméthylèneimino, pyrrolyle, tétrahydro-isoquinoléyle ou tétrahydro-benzazépinyle et

$Z_a$ représente un groupe carboxy ou alcoxycarbonyle avec en tout 2 à 5 atomes de carbone, et de leurs sels physiologiquement supportables avec des acides minéraux ou organiques ou également des bases, lorsque $Z_a$ représente un groupe carboxy, caractérisé en ce que :

a) on fait réagir un acide aminobenzoïque de formule générale IIa

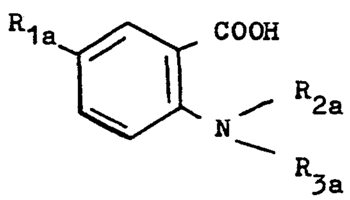

(IIa)

dans laquelle $R_{1a}$, $R_{2a}$ et $R_{3a}$ sont définis comme au début, ou son dérivé réactif éventuellement préparé dans le mélange réactionnel avec une phénylalcoylamine de formule générale IIIa

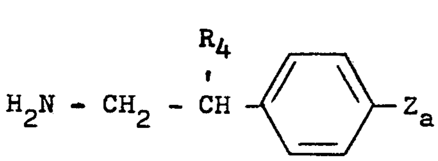

(IIIa)

dans laquelle $R_4$ et $Z_a$ sont définis comme au début, ou avec son dérivé réactif éventuellement formé dans le mélange réactionnel, lorsque l'on utilise un acide aminobenzoïque de formule générale IIa et $Z_a$ représente un groupe alcoxycarbonyle avec en tout 2 à 5 atomes de carbone, ou

b) pour la préparation d'un composé de formule générale I' dans laquelle $R_{1a}$ représente le groupe nitro, on fait réagir un nitrobenzamide de formule générale IVa

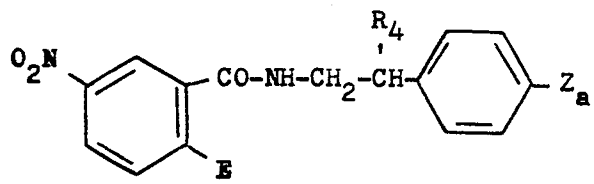

(IVa)

dans laquelle

$R_4$ et $Z_a$ sont définis comme au début et

E représente un atome d'halogène, avec une amine de formule générale Va

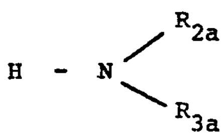

(Va)

dans laquelle $R_{2a}$ et $R_{3a}$ sont définis comme au début ou

c) pour la préparation d'un composé de formule générale I' dans laquelle $Z_a$ représente un groupe carboxy, on oxyde un composé de formule générale VIa

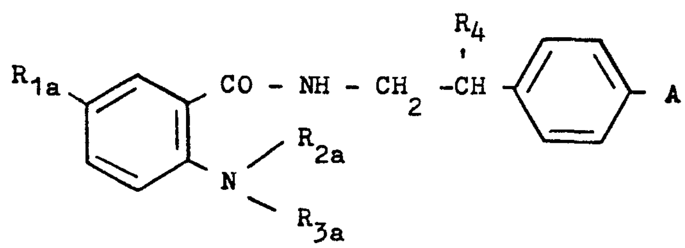

(VIa)

dans laquelle $R_4$ et A sont définis comme dans la revendication 1 et $R_{1a}$ à $R_{3a}$ sont définis comme au début ou

d) pour la préparation d'un composé de formule générale I' dans laquelle $Z_a$ représente un groupe carboxy, on hydrolyse un composé de formule générale VIIa

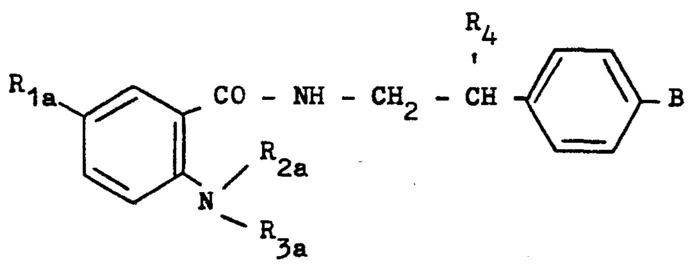

(VIIa)

dans laquelle $R_4$ et B sont définis comme dans la revendication 1 et $R_{1a}$ à $R_{3a}$ sont définis comme au début, ou

e) pour la préparation d'un composé de formule générale I' dans laquelle $R_{2a}$ et $R_{3a}$ qui peuvent être identiques ou différents, représentent des groupes alcoyle avec 1 à 7 atomes de carbone ou des groupes cycloalcoyle avec 3 à 7 atomes de carbone ou $R_{2a}$ et $R_{3a}$ représentent ensemble avec l'atome d'azote intermédiaire un cycle alcoylèneimino à 5 à 7 membres, un groupe pipéridino substitué par un groupe alcoyle avec 1 à 3 atomes de carbone ou un groupe pipéridino substitué en positions 3 et 5 à chaque fois par un groupe alcoyle avec dans chaque cas 1 à 3 atomes de carbone, on fait réagir un composé de formule générale VIIIa

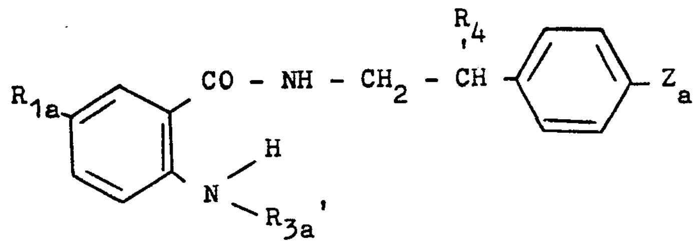

(VIIIa)

,

dans laquelle

$R_4$ est défini comme dans la revendication 1, $R_{1a}$ et $Z_a$ sont définis comme au début, et

$R_{3a}'$ représente un atome d'hydrogène ou possède les significations mentionnées plus haut pour $R_{3a}'$ avec un composé de formule générale IXa

$$R_{2a}'—G \quad \text{(IXa)}$$

dans laquelle

$R_{2a}'$ possède les significations mentionnées au début pour $R_{2a}$ ou représente ensemble avec le radical $R_{3a}'$ de la formule VIIIa un groupe alcoylène rectiligne avec 4 à 6 atomes de carbone, un groupe n-pentylène substitué par un groupe alcoyle avec 1 à 3 atomes de carbone, ou un groupe n-pentylène substitué en positions 2 et 4 à chaque fois par un groupe alcoyle avec 1 à 3 atomes de carbone, et

G représente un groupe partant nucléophile,

et si on le désire on transforme ensuite un composé obtenu de formule générale I' dans laquelle $Z_a$ représente un groupe carboxy, au moyen d'une estérification en un composé correspondant de formule générale I' dans laquelle $Z_a$ représente un groupe alcoxycarbonyle avec en tout 2 à 5 atomes de carbone, et/ou

on transforme un composé obtenu de formule générale I' dans laquelle $R_{1a}$ représente le groupe nitro, au moyen d'une réduction en un composé correspondant de formule générale Ia dans laquelle $R_{1a}$ représente le groupe amino, et/ou

on transforme un composé obtenu de formule générale I' dans laquelle $R_{1a}$ représente le groupe amino, par l'intermédiaire d'un sel de diazonium correspondant en un composé correspondant de formule générale I' dans laquelle $R_{1a}$ représente le groupe cyano, un atome de fluor, de chlore ou de brome, et/ou

on transforme un composé obtenu de formule générale I' dans laquelle $R_{1a}$ représente le groupe amino, au moyen d'une acylation en un composé correspondant de formule générale I' dans laquelle $R_{1a}$ représente un groupe alcanoylamino avec 1 à 3 atomes de carbone dans la partie alcanoyle, et/ou

on transforme un composé obtenu de formule générale I' dans laquelle $R_{1a}$ représente le groupe cyano, au moyen d'une alcoolyse en un composé correspondant de formule générale I' dans laquelle $R_{1a}$ représente un groupe alcoxycarbonyle avec 1 à 3 atomes de carbone dans la partie alcoxy, et/ou

on transforme un composé obtenu de formule générale I' dans laquelle $R_{1a}$ représente un groupe alcoxycarbonyle et/ou $R_{2a}$ et $R_{3a}$ représentent ensemble avec l'atome d'azote intermédiaire un groupe imino avec 4 à 6 atomes de carbone dans le cycle imino, substitué par un groupe alcoxycarbonyle, la partie alcoxy pouvant contenir dans chaque cas 1 à 3 atomes de carbone, au moyen d'une hydrolyse en un composé correspondant de formule générale I' dans laquelle $R_{1a}$ représente le groupe carboxy et/ou $R_{2a}$ et $R_{3a}$ représentent ensemble avec l'atome d'azote intermédiaire un groupe imino avec 4 à 6 atomes de carbone dans le cycle imino, substitué par un groupe carboxy, et/ou

on transforme un composé obtenu de formule générale I' en ses sels physiologiquement supportables avec des acides minéraux ou organiques ou également avec des bases, lorsque $Z_a$ représente un groupe carboxy.

3. Procédé selon la revendication 1 pour la préparation de nouveaux carboxamides de formule générale I''

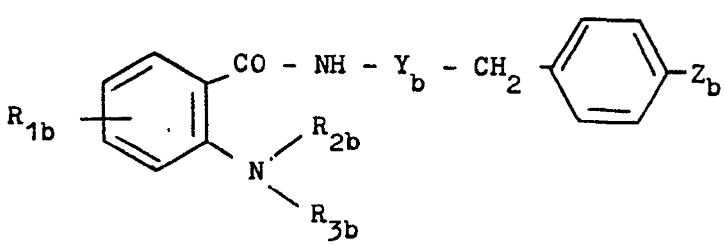

(I'')

dans laquelle

$R_{1b}$ représente un atome d'hydrogène, de chlore ou de brome, un groupe alcoxy avec 1 à 6 atomes

de carbone, un groupe benzyloxy, nitro ou amino,

$R_{2b}$ et $R_{3b}$ qui peuvent être identiques ou différents, représentent des groupes alcoyle avec 1 à 4 atomes de carbone, des groupes cycloalcoyle avec 3 à 7 atomes de carbone ou des groupes phényle, ou

$R_{2b}$ et $R_{3b}$ représentent ensemble avec l'atome d'azote intermédiaire un groupe alcoylèneimino non ramifié avec 5 à 12 atomes de carbone dans le cycle imino, un groupe pipéridino dans lequel un groupe méthylène est remplacé par un groupe méthylméthylène, méthoxyméthylène, carbonyle, imino ou benzylimino ou représentent un groupe pipéridino substitué en positions 3 et 5 à chaque fois par un groupe alcoyle avec 1 à 3 atomes de carbone, le groupe méthylène en position 4 pouvant en même temps être remplacé par un atome d'oxygène ou de soufre, un groupe sulfoxyde ou sulfone, ou représentent un groupe pipéridino substitué en positions 3 et 5 par en tout 3 ou 4 groupes alcoyle avec chacun 1 à 3 atomes de carbone, ou représentent un groupe dioxydothiomorpholino ou azabicycloalcoyle avec 7 à 9 atomes de carbone,

$Y_b$ représente un groupe méthylène éventuellement substitué par un groupe alcoyle avec 1 à 3 atomes de carbone et

$Z_b$ représente un groupe carboxy ou un groupe alcoxycarbonyle avec en tout 2 à 5 atomes de carbone, ainsi que de leurs sels physiologiquement supportables avec des acides minéraux ou organiques et également des bases, lorsque $Z_b$ représente un groupe carboxy, caractérisé en ce que

a) on fait réagir un acide aminobenzoïque de formule générale IIb

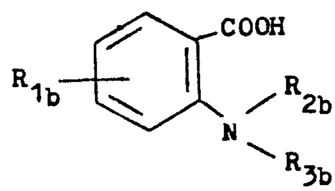

(IIb)

dans laquelle $R_{1b}$, $R_{2b}$ et $R_{3b}$ sont définis comme au début, ou son dérivé réactif éventuellement préparé dans le mélange réactionnel avec une phénylalcoylamine de formule générale IIIb

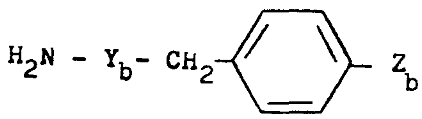

(IIIb)

dans laquelle $Z_b$ et $Y_b$ sont définis comme au début, ou avec une phénylalcoylamine N-activée de formule générale Vb éventuellement formée dans le mélange réactionnel, lorsque l'on utilise un acide aminobenzoïque de formule générale IVb et lorsque dans une phénylalcoylamine de formule générale Vb N-activée, $Z_b$ représente un groupe alcoxycarbonyle avec en tout 2 à 5 atomes de carbone, ou

b) pour la préparation d'un composé de formule générale I'' dans laquelle $R_{1b}$ représente le groupe nitro en position 3 ou 5, on fait réagir un nitrobenzamide de formule générale IVb

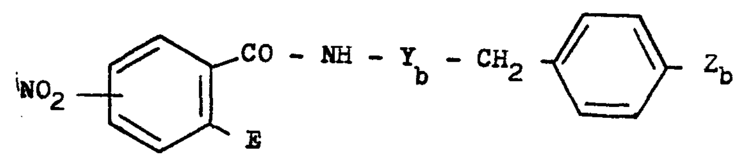

(IVb)

dans laquelle

$Z_b$ et $Y_b$ sont définis comme au début, et

E représente un atome d'halogène, avec une amine de formule générale Vb

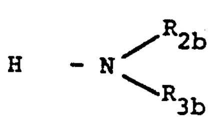

(Vb)

dans laquelle $R_{2b}$ et $R_{3b}$ sont définis comme au début, ou

c) pour la préparation d'un composé de formule générale I'' dans laquelle $Z_b$ représente un groupe carboxy, on oxyde un composé de formule générale VIb

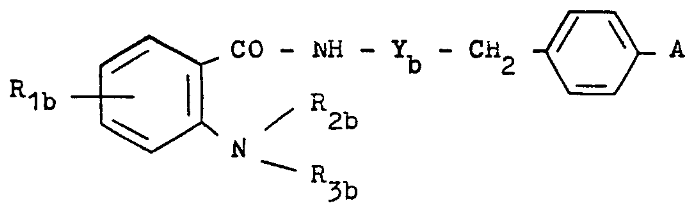

(VIb)

dans laquelle A est défini comme dans la revendication 1, $R_{1b}$ à $R_{3b}$ et $Y_b$ sont définis comme au début, ou

d) pour la préparation d'un composé de formule générale I″ dans laquelle $Z_b$ représente un groupe carboxy, on hydrolyse un composé de formule générale VIIb

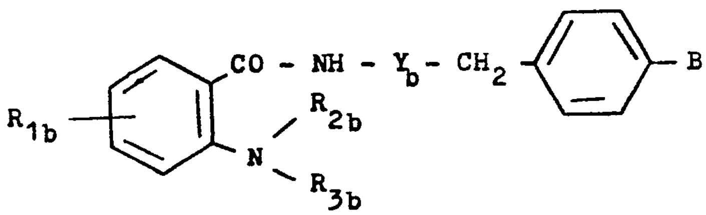

(VIIb)

dans laquelle B est défini comme dans la revendication 1, $R_{1b}$ à $R_{3b}$ et $Y_b$ sont définis comme au début, ou ou

e) pour la préparation d'un composé de formule générale I″ dans laquelle $R_{2b}$ et $R_{3b}$ qui peuvent être identiques ou différents, représentent des groupes alcoyle avec 1 à 4 atomes de carbone, des groupes cycloalcoyle avec 3 à 7 atomes de carbone ou des groupes phényle, ou $R_{2b}$ et $R_{3b}$ représentent ensemble avec l'atome d'azote intermédiaire un cycle imino à 6 ou 7 chaînons, un groupe méthylpipéridino ou un groupe pipéridino substitué en positions 3 et 5 par deux à quatre groupes alcoyle avec chacun 1 à 3 atomes de carbone, on fait réagir un composé de formule générale VIIIb

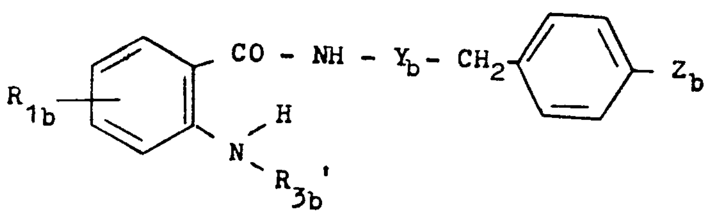

(VIIIb)

dans laquelle

$R_{1b}$, $Y_b$ et $Z_b$ sont définis comme au début, et

$R_{3b}'$ représente un atome d'hydrogène ou possède les significations mentionnées plus haut pour $R_{3b}'$ avec un composé de formule générale IXb

$$R_{2b}'—G \qquad \text{(IXb)}$$

dans laquelle

$R_{2b}'$ possède les significations mentionnées au début pour $R_{2b}$ ou représente ensemble avec le radical $R_{3b}'$ de la formule VIIIb un groupe alcoylène rectiligne avec 5 à 6 atomes de carbone, un groupe n-pentylène substitué par un groupe méthyle ou un groupe n-pentylène substitué en positions 2 et 4 par deux à quatre groupes alcoyle avec chacun 1 à 3 atomes de carbone, et

G représente un groupe partant nucléophile

et si on le désire, on transforme ensuite un composé obtenu de formule générale I″ dans laquelle $Z_b$ représente un groupe carboxy, au moyen d'une estérification en un composé correspondant de formule générale I″ dans laquelle $Z_5$ représente un groupe alcoxycarbonyle avec en tout 2 à 5 atomes de carbone, et/ou

on transforme un composé obtenu de formule générale I″ dans laquelle $R_{1b}$ représente le groupe nitro, au moyen d'une réduction en un composé correspondant de formule générale I″ dans laquelle $R_{1b}$ représente le groupe amino, et/ou

on transforme un composé obtenu de formule générale I″ dans laquelle $R_{1b}$ représente le groupe amino, par l'intermédiaire d'un sel de diazonium correspondant en un composé correspondant de formule générale I″ dans laquelle $R_{1b}$ représente un atome de chlore ou de brome ou le groupe hydroxy, et on transforme un composé ainsi éventuellement obtenu de formule générale I″ dans laquelle $R_{1b}$ représente le groupe hydroxy, au moyen d'une alcoylation ou d'une benzylation en un composé

correspondant de formule générale I" dans laquelle $R_{1b}$ représente un groupe benzyloxy ou alcoxy avec 1 à 4 atomes de carbone, et/ou

on transforme un composé obtenu de formule générale I" dans laquelle $R_{2b}$ et $R_{3b}$ représentent ensemble avec l'atome d'azote intermédiaire un groupe N-benzylpipérazino, au moyen d'une débenzylation en un composé correspondant de formule générale I" dans laquelle $R_{2b}$ et $R_{3b}$ représentent ensemble avec l'atome d'azote intermédiaire un groupe pipérazino, et/ou

on transforme un composé obtenu de formule générale I" dans laquelle $R_{1b}$ représente un atome de chlore ou de brome, au moyen d'une déshalogénation en un composé correspondant de formule générale I" dans laquelle $R_{1b}$ représente un atome d'hydrogène, et/ou

on transforme un composé obtenu de formule générale I" en ses sels physiologiquement supportables avec des acides minéraux ou organiques et également des bases, lorsque $Z_b$ représente un groupe carboxy.

4. Procédé selon la revendication 1 pour la préparation de nouveaux carboxamides de formule générale I'''

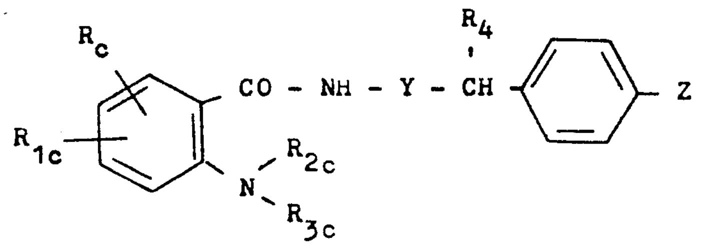

(I''')

dans laquelle

$R_4$, Y et Z sont définis comme dans la revendication 1,

$R_c$ représente un atome d'hydrogène ou de chlore ou un groupe pipéridino,

$R_{1c}$ représente un atome d'hydrogène ou d'halogène, un groupe alcoyle ou alcoxy avec chacun 1 à 6 atomes de carbone, un groupe hydroxy, amino, nitro ou cyano,

$R_{2c}$ représente un groupe alcoyle avec 1 à 3 atomes de carbone éventuellement substitué par un groupe phényle ou un groupe alcényle avec 3 à 7 atomes de carbone,

$R_{3c}$ représente un groupe alcoyle avec 1 à 7 atomes de carbone, un groupe alcényle avec 3 à 7 atomes de carbone ou un groupe adamantyle, ou

$R_{2c}$ et $R_{3c}$ représentent ensemble avec l'atome d'azote intermédiaire un groupe alcoylèneimino non ramifié avec 4 à 12 atomes de carbone dans le cycle imino, un groupe pipéridino substitué par un groupe alcoyle avec 1 à 4 atomes de carbone, ou représentent un groupe pipéridino substitué en positions 3 et 5 à chaque fois par un groupe alcoyle avec 1 à 3 atomes de carbone, ou représentent un groupe pipérazino substitué en position 4 par groupe alcoyle avec 1 à 3 atomes de carbone, un groupe halogénophényle, un groupe carbalcoxy avec en tout 2 à 4 atomes de carbone ou un groupe furoyle, ou représentent un groupe tétrahydropyridino, octahydroisoquinoléino, décahydro-isoquinoléino, décahydro-benzazépino, octahydro-isoindolo ou 1,4-dioxa-8-aza-spiroalcoyle avec 7 à 10 atomes de carbone, ainsi que de leurs sels d'addition d'acides physiologiquement supportables avec des acides minéraux ou organiques et également des bases, lorsque Z contient le groupe carboxy, caractérisé en ce que

a) On fait réagir un acide aminobenzoïque de formule générale IIc

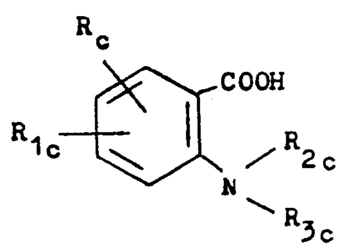

(IIc)

dans laquelle $R_c$, $R_{1c}$ à $R_{3c}$ sont définis comme au début, ou son dérivé réactif éventuellement préparé dans le mélange réactionnel, avec une amine de formule générale IIIc

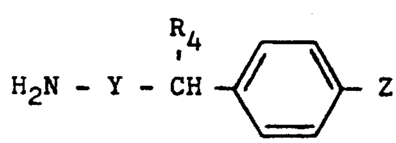

(IIIc)

dans laquelle $R_4$, Y et Z sont définis comme au début, ou avec une amine N-activée de formule

générale IIIc éventuellement formée dans le mélange réactionnel, lorsque l'on utilise un acide aminobenzoïque de formule générale IIc et lorsque Z ne contient pas de groupe carboxyle ou amino dans une amine N-activée de formule générale IIIc, ou

c) pour la préparation de composés de formule générale I''' dans laquelle Z représente un groupe carboxy et Y ne représente pas le groupe NH, on oxyde un composé de formule générale VIc

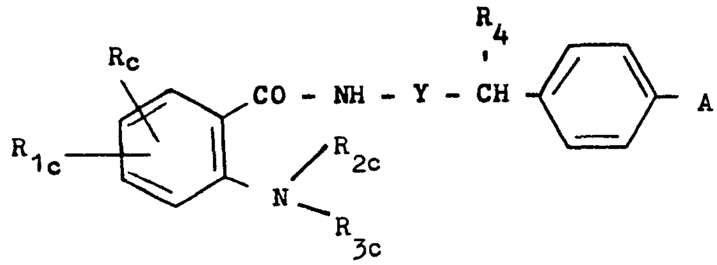

(VIc)

dans laquelle $R_4$ et A sont définis comme dans la revendication 1, $R_c$ et $R_{1c}$ à $R_{3c}$ et Y sont définis comme au début, ou

d) pour la préparation de composés de formule générale I''' dans laquelle Z représente un groupe carboxy, on hydrolyse un composé de formule générale VIIc

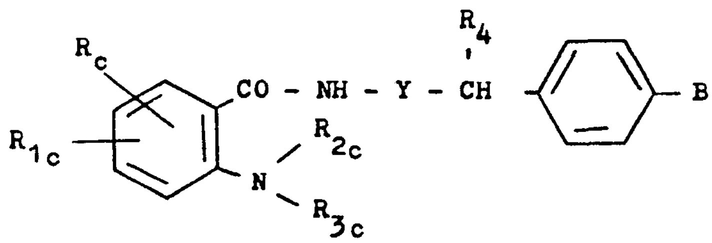

(VIIc)

dans laquelle $R_4$, B et Y sont définis comme dans la revendication 1, $R_c$ et $R_{1c}$ à $R_{3c}$ sont définis comme au début, ou

e) pour la préparation de composés de formule générale I''' dans laquelle $R_{2c}$ représente un groupe alcoyle avec 1 à 3 atomes de carbone, éventuellement substitué par un groupe phényle ou un groupe alcényle avec 3 à 7 atomes de carbone, $R_{3c}$ représente un groupe alcoyle avec 1 à 7 atomes de carbone, un groupe alcényle avec 3 à 7 atomes de carbone, un groupe adamantyle ou $R_{2c}$ et $R_{3c}$ représentent avec l'atome d'azote intermédiaire un cycle alcoylèneimino à 5 à 7 chaînons, un groupe pipéridino substitué par un groupe alcoyle avec 1 à 4 atomes de carbone ou un groupe pipéridino substitué en positions 3 et 5 à chaque fois par un groupe alcoyle avec 1 à 3 atomes de carbone et Y représente un groupe éventuellement substitué par un ou deux groupes alcoyle avec chacun 1 à 3 atomes de carbone, on fait réagir un composé, éventuellement formé dans le mélange réactionnel, de formule générale VIIIc

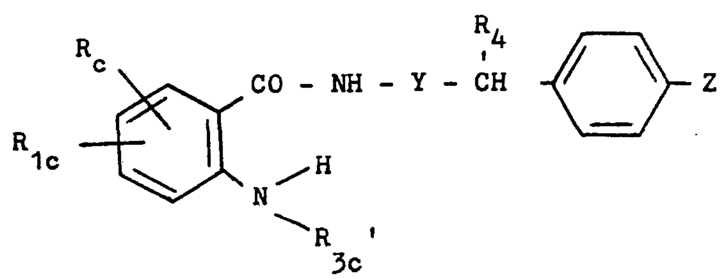

(VIIIc)

dans laquelle

$R_4$, Y et Z sont définis comme dans la revendication 1,

$R_c$ et $R_{1c}$ sont définis comme au début, et

$R_{3c}'$ représente un atome d'hydrogène ou possède les significations mentionnées plus haut pour $R_{3c}'$ avec un composé de formule générale IXc

$$R_{2c}'\text{—}G \qquad \text{(IXc)}$$

dans laquelle

G est défini comme dans la revendication 1, et

$R_{2c}'$ possède les significations mentionnées au début pour $R_{2c}$ ou représente ensemble avec le

radical $R_{3c}'$ de la formule VIIIc un groupe alcoylène rectiligne avec 4 à 6 atomes de carbone, un groupe n-pentylène substitué par un groupe alcoyle avec 1 à 4 atomes de carbone ou un groupe n-pentylène substitué en positions 2 et 4 à chaque fois par un groupe alcoyle avec 1 à 3 atomes de carbone, ou

f) pour la préparation de composés de formule générale I''' dans laquelle Y représente le groupe NH ou un atome d'oxygène, on fait réagir un composé de formule générale Xc

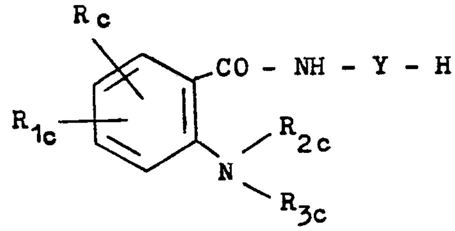

(Xc)

dans laquelle $R_c$, $R_{1c}$ à $R_{3c}$ et Y sont définis comme au début, ou son sel alcalin avec un dérivé phénylé de formule générale XIc

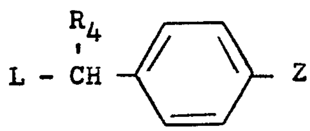

(XIc)

dans laquelle $R_4$, L et Z sont définis comme dans la revendication 1 et si on le désire on transforme ensuite un composé ainsi obtenu de formule générale I''' dans laquelle Z représente un groupe carboxy, au moyen d'une estérification ou respectivement d'une amidation en un composé correspondant de formule générale I''' dans laquelle Z représente un groupe carboxy estéré ou un groupe aminocarbonyle éventuellement mono- ou disubstitué par un groupe alcoyle avec 1 à 7 atomes de carbone, un groupe cycloalcoyle avec 3 à 7 atomes de carbone et/ou un groupe alcoylène avec 3 à 7 atomes de carbone, ou représente un groupe pipéridinocarbonyle, morpholinocarbonyle, thiomorpholinocarbonyle ou N-alcoyl-pipérazinocarbonyle, et/ou

on transforme un composé obtenu de formule générale I''' dans laquelle $R_{1c}$ et/ou Z représente un groupe nitro, au moyen d'une réduction en un composé correspondant de formule générale I''' dans laquelle $R_{1c}$ et/ou Z représente un groupe amino, et/ou

on transforme un composé obtenu de formule générale I''' dans laquelle $R_{1c}$ et/ou Z représente un groupe amino, par l'intermédiaire d'un sel de diazonium correspondant en un composé correspondant de formule générale I''' dans laquelle $R_{1c}$ représente le groupe hydroxy ou cyano, un atome de chlore ou de brome et/ou Z représente un atome de chlore ou le groupe cyano, un composé ainsi éventuellement obtenu de formule générale I''' dans laquelle $R_{1c}$ représente le groupe hydroxy, pouvant être transformé au moyen d'une alcoylation en un composé correspondant de formule générale I''' dans laquelle $R_{1c}$ représente un groupe alcoxy avec 1 à 4 atomes de carbone, et/ou

on transforme un composé obtenu de formule générale I''' dans laquelle Z représente le groupe hydroxyméthyle au moyen d'une oxydation en un composé correspondant de formule générale I''' dans laquelle Z représente le groupe formyle, et/ou

on transforme un composé obtenu de formule générale I''' dans laquelle Z représente le groupe hydroxyméthyle au moyen d'une halogénation et par réaction subséquent avec un ester de l'acide malonique en un composé correspondant de formule générale I''' dans laquelle Z représente un groupe éthyle substitué par deux groupes alcoxycarbonyle, et/ou

on transforme un composé obtenu de formule générale I''' dans laquelle Z représente le groupe formyle, au moyen d'une acétalisation en un composé correspondant de formule générale I''' dans laquelle Z représente un groupe dialcoxyméthyle, et/ou

on transforme un composé obtenu de formule générale I''' dans laquelle Z représente le groupe formyle, au moyen d'une condensation et éventuellement d'une hydrolyse subséquente en un composé correspondant de formule générale I''' dans laquelle Z représente un groupe éthylène substitué par un groupe hydroxycarbonyle ou alcoxycarbonyle, et/ou

on transforme un composé obtenu de formule générale I''' dans laquelle Z représente un atome d'hydrogène, au moyen d'une acylation selon Friedel-Crafts, en un composé correspondant de formule générale I''' dans laquelle Z représente un groupe acétyle éventuellement substitué par un groupe alcoxycarbonyle, et/ou

on transforme un composé obtenu de formule générale I''' dans laquelle Z représente un groupe nitrile, au moyen d'une alcoolyse par l'intermédiaire d'un imino-ester correspondant en un composé correspondant de formule générale I''' dans laquelle Z représente un groupe trialcoxyméthyle, et/ou

on transforme un composé obtenu de formule générale I''' dans laquelle Z représente un groupe trialcoxyméthyle, au moyen d'une hydrolyse en un composé correspondant de formule générale I''' dans

laquelle Z représente un groupe alcoxycarbonyle, et/ou

on transforme un composé obtenu de formule générale I''' dans laquelle Z représente le groupe acétyle, par chauffage avec une amine et du soufre et ensuite en présence d'une base organique en un composé correspondant de formule générale I''' dans laquelle Z représente le groupe hydroxycarbonylméthyle, et/ou

on transforme un composé obtenu de formule générale I''' dans laquelle Z représente le groupe carboxy, au moyen d'une transformation en un hydrazide d'acide sulfonique et par dismutation subséquente en un composé correspondant de formule générale I''' dans laquelle Z représente le groupe formyle, et/ou

on transforme un composé obtenu de formule générale I''' en ses sels physiologiquement supportables avec des acides minéraux ou organiques et également des bases, lorsque Z contient un groupe carboxyle.

5. Procédé selon les revendications 1 à 4, caractérisé en ce que la réaction est effectuée dans un solvant.

6. Procédé selon les revendications 1a, 2a, 3a, 4a et 5, caractérisé en ce que la réaction est effectuée en présence d'un agent activant les acides, d'un agent d'élimination de l'eau ou d'un agent activant l'amine, dans chaque cas éventuellement en présence d'une base minérale ou organique tertiaire et à des températures entre − 25 et 250 °C, de préférence cependant à des températures entre − 10 °C et la température d'ébullition du solvant utilisé.

7. Procédé selon les revendications 1b, 2b, 3b et 5, caractérisé en ce que la réaction est effectuée en présence d'une base minérale ou tertiaire organique, en présence d'un accélérateur de réaction tel que le cuivre et/ou dans un récipient sous pression et à des températures entre 20 et 150 °C, de préférence cependant à la température d'ébullition du mélange réactionnel.

8. Procédé selon les revendications 1c, 2c, 3c, 4c et 5, caractérisé en ce que la réaction est effectuée à des températures entre 0 et 100 °C, de préférence cependant à des températures entre 20 et 50 °C.

9. Procédé selon les revendications 1d, 2d, 3d, 4d et 5, caractérisé en ce que l'hydrolyse est effectuée en présence d'un acide ou d'une base et à la température d'ébullition du mélange réactionnel.

10. Procédé selon les revendications 1e, 2e, 3e, 4e et 5, caractérisé en ce que la méthylation au moyen d'aldéhyde formique est effectuée en présence d'un agent réducteur.